# EUROPEAN PATENT APPLICATION

(11) **EP 4 831 201 A1**
(43) Date of publication of application: **28.10.2026**
(21) Application number: 24906544.2
(22) Date of filing: 20.12.2024
(51) Int. Cl.: C07K 16/28, A61K 39/395, A61P 35/00, A61P 37/00, A61P 29/00

(54) **CD3-TARGETING ANTIBODY AND USE THEREOF**

(30) Priority: 22.12.2023 CN 202311782525
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: PU, Pu, Shanghai 200245 (CN); YANG, Hao, Shanghai 200245 (CN); JIN, Xinsheng, Shanghai 200245 (CN); LI, Xun, Shanghai 200245 (CN)
(74) Representative: Dragotti & Associati S.P.A.
(86) International application number: PCT/CN2024/141016
(87) International publication number: WO 2025/131071

(57) **Abstract**

The present invention provides an antibody specifically binding to CD3, a pharmaceutical composition containing the antibody, a preparation method for the antibody, and a use of the antibody.

## Description

The present application claims priority to Chinese Patent Application No. CN202311782525.0 filed on Dec. 22, 2023.

### TECHNICAL FIELD

The present disclosure pertains to the field of biotechnology. Specifically, the present disclosure relates to a CD3-targeting antibody and use thereof.

### BACKGROUND

The statements herein merely provide background information related to the present disclosure and may not necessarily constitute the prior art.

The TCR-CD3 complex consisting of the TCR-αβ heterodimer and the non-covalently associated signaling dimers, CD3εγ, CD3εδ, and CD3 ζ, plays an important role in the response and function of T cells, and is an important mechanism for initiating the activation and development of T cells. The TCR-αβ specifically recognizes and binds to an antigen peptide complex presented by an MHC molecule, and further transmits signals to the cytoplasm through an immunoreceptor tyrosine-based activation motif (ITAM) in the cytoplasmic segment of the CD3 subunit as a first activation signal to induce the activation of T cells. The variable regions of the TCR heterodimer are variable, but the CD3 subunit is expressed on the surface of all T cells (including αβ and γδ T cells) and serves as the primary signal transmitter for T cell activation. Therefore, CD3 becomes an ideal target for the regulation of T cell activity.

Currently, monoclonal antibodies, bispecific antibodies, or multispecific antibodies developed against the CD3 target have been widely used clinically. Among them, the monoclonal antibody, muromonab (OKT3), is the first monoclonal antibody approved for marketing for the treatment of acute rejection after organ transplantation. However, this antibody was withdrawn from the market in 2010 due to its relatively strong immunogenicity and cytokine storm side effects. The antibody teplizumab, which was humanized and Fc-engineered based on muromonab, was approved by the FDA for the treatment of type I diabetes in November 2022. However, in the TN-10 clinical trial, 57% of the treated patients still developed anti-drug antibodies (ADAs), of which 46% produced neutralizing antibodies, which could affect the distribution and clinical effectiveness of the drug. Other CD3-targeting monoclonal antibodies, otelixizumab, visilizumab, and the like, are also being developed for use in autoimmune diseases such as type I diabetes and ulcerative colitis. In addition, bispecific antibodies or multispecific antibodies targeting CD3 and tumor-associated antigens/tumor-specific antigens have attracted attention in tumor immunotherapy. A plurality of CD3 bispecific antibodies have been approved for marketing, and more CD3 bispecific antibodies are also under active clinical trials. However, CD3 bispecific antibodies have also elicited varying degrees of ADAs in clinical practice. Secondly, among the currently marketed antibodies, the CD3 antibody UCHT1 used in muromonab (OKT3), teplizumab, and tebentafusp shows no significant binding effect to cynomolgus monkey-derived CD3, which brings certain challenges to preclinical safety evaluation.

### SUMMARY

The present disclosure provides an anti-CD3 antibody comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. the HCDR1, the HCDR2, and the HCDR3 of the heavy chain variable region comprise the amino acid sequences of a HCDR1, a HCDR2, and a HCDR3 in SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, respectively, and the LCDR1, the LCDR2, and the LCDR3 of the light chain variable region comprise the amino acid sequences of a LCDR1, a LCDR2, and a LCDR3 in SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273, respectively; or
   the HCDR1, the HCDR2, and the HCDR3 of the heavy chain variable region comprise the amino acid sequences of a HCDR1, a HCDR2, and a HCDR3 in SEQ ID NO: 75, respectively, and the LCDR1, the LCDR2, and the LCDR3 of the light chain variable region comprise the amino acid sequences of a LCDR1, a LCDR2, and a LCDR3 in SEQ ID NO: 74, respectively; or
b. the HCDR1, the HCDR2, and the HCDR3 of the heavy chain variable region comprise the amino acid sequences of a HCDR1, a HCDR2, and a HCDR3 in SEQ ID NO: 108, 109, 110, 111, 112, 113, 114, 115, 116, 117, 118, or 119, respectively, and the LCDR1, the LCDR2, and the LCDR3 of the light chain variable region comprise the amino acid sequences of a LCDR1, a LCDR2, and a LCDR3 in SEQ ID NO: 101, 102, 103, 104, 105, 106, or 107, respectively; or
   the HCDR1, the HCDR2, and the HCDR3 of the heavy chain variable region comprise the amino acid sequences of a HCDR1, a HCDR2, and a HCDR3 in SEQ ID NO: 60, respectively, and the LCDR1, the LCDR2, and the LCDR3 of the light chain variable region comprise the amino acid sequences of a LCDR1, a LCDR2, and a LCDR3 in SEQ ID NO: 59, respectively; or
c. the HCDR1, the HCDR2, and the HCDR3 of the heavy chain variable region comprise the amino acid sequences of a HCDR1, a HCDR2, and a HCDR3 in SEQ ID NO: 58, respectively, and the LCDR1, the LCDR2, and the LCDR3 of the light chain variable region comprise the amino acid sequences of a LCDR1, a LCDR2, and a LCDR3 in SEQ ID NO: 57, respectively; or
d. the HCDR1, the HCDR2, and the HCDR3 of the heavy chain variable region comprise the amino acid sequences of a HCDR1, a HCDR2, and a HCDR3 in SEQ ID NO: 61, respectively, and the LCDR1, the LCDR2, and the LCDR3 of the light chain variable region comprise the amino acid sequences of a LCDR1, a LCDR2, and a LCDR3 in SEQ ID NO: 57, respectively; or
e. the HCDR1, the HCDR2, and the HCDR3 of the heavy chain variable region comprise the amino acid sequences of a HCDR1, a HCDR2, and a HCDR3 in SEQ ID NO: 63, respectively, and the LCDR1, the LCDR2, and the LCDR3 of the light chain variable region comprise the amino acid sequences of a LCDR1, a LCDR2, and a LCDR3 in SEQ ID NO: 62, respectively; or
f. the HCDR1, the HCDR2, and the HCDR3 of the heavy chain variable region comprise the amino acid sequences of a HCDR1, a HCDR2, and a HCDR3 in SEQ ID NO: 65, respectively, and the LCDR1, the LCDR2, and the LCDR3 of the light chain variable region comprise the amino acid sequences of a LCDR1, a LCDR2, and a LCDR3 in SEQ ID NO: 64, respectively; or
g. the HCDR1, the HCDR2, and the HCDR3 of the heavy chain variable region comprise the amino acid sequences of a HCDR1, a HCDR2, and a HCDR3 in SEQ ID NO: 67, respectively, and the LCDR1, the LCDR2, and the LCDR3 of the light chain variable region comprise the amino acid sequences of a LCDR1, a LCDR2, and a LCDR3 in SEQ ID NO: 66, respectively; or
h. the HCDR1, the HCDR2, and the HCDR3 of the heavy chain variable region comprise the amino acid sequences of a HCDR1, a HCDR2, and a HCDR3 in SEQ ID NO: 69, respectively, and the LCDR1, the LCDR2, and the LCDR3 of the light chain variable region comprise the amino acid sequences of a LCDR1, a LCDR2, and a LCDR3 in SEQ ID NO: 68, respectively; or
i. the HCDR1, the HCDR2, and the HCDR3 of the heavy chain variable region comprise the amino acid sequences of a HCDR1, a HCDR2, and a HCDR3 in SEQ ID NO: 71, respectively, and the LCDR1, the LCDR2, and the LCDR3 of the light chain variable region comprise the amino acid sequences of a LCDR1, a LCDR2, and a LCDR3 in SEQ ID NO: 70, respectively; or
j. the HCDR1, the HCDR2, and the HCDR3 of the heavy chain variable region comprise the amino acid sequences of a HCDR1, a HCDR2, and a HCDR3 in SEQ ID NO: 73, respectively, and the LCDR1, the LCDR2, and the LCDR3 of the light chain variable region comprise the amino acid sequences of a LCDR1, a LCDR2, and a LCDR3 in SEQ ID NO: 72, respectively; or
k. the HCDR1, the HCDR2, and the HCDR3 of the heavy chain variable region comprise the amino acid sequences of a HCDR1, a HCDR2, and a HCDR3 in SEQ ID NO: 76, respectively, and the LCDR1, the LCDR2, and the LCDR3 of the light chain variable region comprise the amino acid sequences of a LCDR1, a LCDR2, and a LCDR3 in SEQ ID NO: 74, respectively; or
l. the HCDR1, the HCDR2, and the HCDR3 of the heavy chain variable region comprise the amino acid sequences of a HCDR1, a HCDR2, and a HCDR3 in SEQ ID NO: 77, respectively, and the LCDR1, the LCDR2, and the LCDR3 of the light chain variable region comprise the amino acid sequences of a LCDR1, a LCDR2, and a LCDR3 in SEQ ID NO: 74, respectively; or
m. the HCDR1, the HCDR2, and the HCDR3 of the heavy chain variable region comprise the amino acid sequences of a HCDR1, a HCDR2, and a HCDR3 in SEQ ID NO: 79, respectively, and the LCDR1, the LCDR2, and the LCDR3 of the light chain variable region comprise the amino acid sequences of a LCDR1, a LCDR2, and a LCDR3 in SEQ ID NO: 78, respectively; or
n. the HCDR1, the HCDR2, and the HCDR3 of the heavy chain variable region comprise the amino acid sequences of a HCDR1, a HCDR2, and a HCDR3 in SEQ ID NO: 80, respectively, and the LCDR1, the LCDR2, and the LCDR3 of the light chain variable region comprise the amino acid sequences of a LCDR1, a LCDR2, and a LCDR3 in SEQ ID NO: 74, respectively; or
o. the HCDR1, the HCDR2, and the HCDR3 of the heavy chain variable region comprise the amino acid sequences of a HCDR1, a HCDR2, and a HCDR3 in SEQ ID NO: 82, respectively, and the LCDR1, the LCDR2, and the LCDR3 of the light chain variable region comprise the amino acid sequences of a LCDR1, a LCDR2, and a LCDR3 in SEQ ID NO: 81, respectively; or
p. the HCDR1, the HCDR2, and the HCDR3 of the heavy chain variable region comprise the amino acid sequences of a HCDR1, a HCDR2, and a HCDR3 in SEQ ID NO: 84, respectively, and the LCDR1, the LCDR2, and the LCDR3 of the light chain variable region comprise the amino acid sequences of a LCDR1, a LCDR2, and a LCDR3 in SEQ ID NO: 83, respectively; or
q. the HCDR1, the HCDR2, and the HCDR3 of the heavy chain variable region comprise the amino acid sequences of a HCDR1, a HCDR2, and a HCDR3 in SEQ ID NO: 86, respectively, and the LCDR1, the LCDR2, and the LCDR3 of the light chain variable region comprise the amino acid sequences of a LCDR1, a LCDR2, and a LCDR3 in SEQ ID NO: 85, respectively; or
r. the HCDR1, the HCDR2, and the HCDR3 of the heavy chain variable region comprise the amino acid sequences of a HCDR1, a HCDR2, and a HCDR3 in SEQ ID NO: 88, respectively, and the LCDR1, the LCDR2, and the LCDR3 of the light chain variable region comprise the amino acid sequences of a LCDR1, a LCDR2, and a LCDR3 in SEQ ID NO: 87, respectively.

In some embodiments, provided is the anti-CD3 antibody according to the foregoing, wherein:
a. the HCDR1, the HCDR2, and the HCDR3 of the heavy chain variable region comprise the amino acid sequences of a HCDR1, a HCDR2, and a HCDR3 in SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, respectively, and the LCDR1, the LCDR2, and the LCDR3 of the light chain variable region comprise the amino acid sequences of a LCDR1, a LCDR2, and a LCDR3 in SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273, respectively; or
b. the HCDR1, the HCDR2, and the HCDR3 of the heavy chain variable region comprise the amino acid sequences of a HCDR1, a HCDR2, and a HCDR3 in SEQ ID NO: 108, 109, 110, 111, 112, 113, 114, 115, 116, 117, 118, or 119, respectively, and the LCDR1, the LCDR2, and the LCDR3 of the light chain variable region comprise the amino acid sequences of a LCDR1, a LCDR2, and a LCDR3 in SEQ ID NO: 101, 102, 103, 104, 105, 106, or 107, respectively.

In some embodiments, provided is the anti-CD3 antibody according to any one of the foregoing, wherein:
(1) the HCDR1, the HCDR2, and the HCDR3 of the heavy chain variable region comprise the amino acid sequences of a HCDR1, a HCDR2, and a HCDR3 in SEQ ID NO: 255, respectively, and the LCDR1, the LCDR2, and the LCDR3 of the light chain variable region comprise the amino acid sequences of a LCDR1, a LCDR2, and a LCDR3 in SEQ ID NO: 236, respectively; or
(2) the HCDR1, the HCDR2, and the HCDR3 of the heavy chain variable region comprise the amino acid sequences of a HCDR1, a HCDR2, and a HCDR3 in SEQ ID NO: 255, respectively, and the LCDR1, the LCDR2, and the LCDR3 of the light chain variable region comprise the amino acid sequences of a LCDR1, a LCDR2, and a LCDR3 in SEQ ID NO: 237, respectively; or
(3) the HCDR1, the HCDR2, and the HCDR3 of the heavy chain variable region comprise the amino acid sequences of a HCDR1, a HCDR2, and a HCDR3 in SEQ ID NO: 255, respectively, and the LCDR1, the LCDR2, and the LCDR3 of the light chain variable region comprise the amino acid sequences of a LCDR1, a LCDR2, and a LCDR3 in SEQ ID NO: 238, respectively; or
(4) the HCDR1, the HCDR2, and the HCDR3 of the heavy chain variable region comprise the amino acid sequences of a HCDR1, a HCDR2, and a HCDR3 in SEQ ID NO: 278, respectively, and the LCDR1, the LCDR2, and the LCDR3 of the light chain variable region comprise the amino acid sequences of a LCDR1, a LCDR2, and a LCDR3 in SEQ ID NO: 268, respectively; or
(5) the HCDR1, the HCDR2, and the HCDR3 of the heavy chain variable region comprise the amino acid sequences of a HCDR1, a HCDR2, and a HCDR3 in SEQ ID NO: 275, respectively, and the LCDR1, the LCDR2, and the LCDR3 of the light chain variable region comprise the amino acid sequences of a LCDR1, a LCDR2, and a LCDR3 in SEQ ID NO: 269, respectively; or
(6) the HCDR1, the HCDR2, and the HCDR3 of the heavy chain variable region comprise the amino acid sequences of a HCDR1, a HCDR2, and a HCDR3 in SEQ ID NO: 278, respectively, and the LCDR1, the LCDR2, and the LCDR3 of the light chain variable region comprise the amino acid sequences of a LCDR1, a LCDR2, and a LCDR3 in SEQ ID NO: 269, respectively; or
(7) the HCDR1, the HCDR2, and the HCDR3 of the heavy chain variable region comprise the amino acid sequences of a HCDR1, a HCDR2, and a HCDR3 in SEQ ID NO: 279, respectively, and the LCDR1, the LCDR2, and the LCDR3 of the light chain variable region comprise the amino acid sequences of a LCDR1, a LCDR2, and a LCDR3 in SEQ ID NO: 269, respectively; or
(8) the HCDR1, the HCDR2, and the HCDR3 of the heavy chain variable region comprise the amino acid sequences of a HCDR1, a HCDR2, and a HCDR3 in SEQ ID NO: 157, respectively, and the LCDR1, the LCDR2, and the LCDR3 of the light chain variable region comprise the amino acid sequences of a LCDR1, a LCDR2, and a LCDR3 in SEQ ID NO: 236, respectively; or
(9) the HCDR1, the HCDR2, and the HCDR3 of the heavy chain variable region comprise the amino acid sequences of a HCDR1, a HCDR2, and a HCDR3 in SEQ ID NO: 275, respectively, and the LCDR1, the LCDR2, and the LCDR3 of the light chain variable region comprise the amino acid sequences of a LCDR1, a LCDR2, and a LCDR3 in SEQ ID NO: 268, respectively; or
(10) the HCDR1, the HCDR2, and the HCDR3 of the heavy chain variable region comprise the amino acid sequences of a HCDR1, a HCDR2, and a HCDR3 in SEQ ID NO: 279, respectively, and the LCDR1, the LCDR2, and the LCDR3 of the light chain variable region comprise the amino acid sequences of a LCDR1, a LCDR2, and a LCDR3 in SEQ ID NO: 268, respectively; or
(11) the HCDR1, the HCDR2, and the HCDR3 of the heavy chain variable region comprise the amino acid sequences of a HCDR1, a HCDR2, and a HCDR3 in SEQ ID NO: 157, respectively, and the LCDR1, the LCDR2, and the LCDR3 of the light chain variable region comprise the amino acid sequences of a LCDR1, a LCDR2, and a LCDR3 in SEQ ID NO: 271, respectively; or
(12) the HCDR1, the HCDR2, and the HCDR3 of the heavy chain variable region comprise the amino acid sequences of a HCDR1, a HCDR2, and a HCDR3 in SEQ ID NO: 157, respectively, and the LCDR1, the LCDR2, and the LCDR3 of the light chain variable region comprise the amino acid sequences of a LCDR1, a LCDR2, and a LCDR3 in SEQ ID NO: 272, respectively; or
(13) the HCDR1, the HCDR2, and the HCDR3 of the heavy chain variable region comprise the amino acid sequences of a HCDR1, a HCDR2, and a HCDR3 in SEQ ID NO: 281, respectively, and the LCDR1, the LCDR2, and the LCDR3 of the light chain variable region comprise the amino acid sequences of a LCDR1, a LCDR2, and a LCDR3 in SEQ ID NO: 236, respectively; or
(14) the HCDR1, the HCDR2, and the HCDR3 of the heavy chain variable region comprise the amino acid sequences of a HCDR1, a HCDR2, and a HCDR3 in SEQ ID NO: 282, respectively, and the LCDR1, the LCDR2, and the LCDR3 of the light chain variable region comprise the amino acid sequences of a LCDR1, a LCDR2, and a LCDR3 in SEQ ID NO: 236, respectively; or
(15) the HCDR1, the HCDR2, and the HCDR3 of the heavy chain variable region comprise the amino acid sequences of a HCDR1, a HCDR2, and a HCDR3 in SEQ ID NO: 283, respectively, and the LCDR1, the LCDR2, and the LCDR3 of the light chain variable region comprise the amino acid sequences of a LCDR1, a LCDR2, and a LCDR3 in SEQ ID NO: 236, respectively; or
(16) the HCDR1, the HCDR2, and the HCDR3 of the heavy chain variable region comprise the amino acid sequences of a HCDR1, a HCDR2, and a HCDR3 in SEQ ID NO: 282, respectively, and the LCDR1, the LCDR2, and the LCDR3 of the light chain variable region comprise the amino acid sequences of a LCDR1, a LCDR2, and a LCDR3 in SEQ ID NO: 267, respectively; or
(17) the HCDR1, the HCDR2, and the HCDR3 of the heavy chain variable region comprise the amino acid sequences of a HCDR1, a HCDR2, and a HCDR3 in SEQ ID NO: 282, respectively, and the LCDR1, the LCDR2, and the LCDR3 of the light chain variable region comprise the amino acid sequences of a LCDR1, a LCDR2, and a LCDR3 in SEQ ID NO: 268, respectively; or
(18) the HCDR1, the HCDR2, and the HCDR3 of the heavy chain variable region comprise the amino acid sequences of a HCDR1, a HCDR2, and a HCDR3 in SEQ ID NO: 282, respectively, and the LCDR1, the LCDR2, and the LCDR3 of the light chain variable region comprise the amino acid sequences of a LCDR1, a LCDR2, and a LCDR3 in SEQ ID NO: 269, respectively.

In some embodiments, provided is the anti-CD3 antibody according to any one of the foregoing, wherein:
(1) the HCDR1, the HCDR2, and the HCDR3 of the heavy chain variable region comprise the amino acid sequences of a HCDR1, a HCDR2, and a HCDR3 in SEQ ID NO: 255, respectively, and the LCDR1, the LCDR2, and the LCDR3 of the light chain variable region comprise the amino acid sequences of a LCDR1, a LCDR2, and a LCDR3 in SEQ ID NO: 236, respectively; or
(2) the HCDR1, the HCDR2, and the HCDR3 of the heavy chain variable region comprise the amino acid sequences of a HCDR1, a HCDR2, and a HCDR3 in SEQ ID NO: 255, respectively, and the LCDR1, the LCDR2, and the LCDR3 of the light chain variable region comprise the amino acid sequences of a LCDR1, a LCDR2, and a LCDR3 in SEQ ID NO: 237, respectively; or
(3) the HCDR1, the HCDR2, and the HCDR3 of the heavy chain variable region comprise the amino acid sequences of a HCDR1, a HCDR2, and a HCDR3 in SEQ ID NO: 255, respectively, and the LCDR1, the LCDR2, and the LCDR3 of the light chain variable region comprise the amino acid sequences of a LCDR1, a LCDR2, and a LCDR3 in SEQ ID NO: 238, respectively; or
(4) the HCDR1, the HCDR2, and the HCDR3 of the heavy chain variable region comprise the amino acid sequences of a HCDR1, a HCDR2, and a HCDR3 in SEQ ID NO: 278, respectively, and the LCDR1, the LCDR2, and the LCDR3 of the light chain variable region comprise the amino acid sequences of a LCDR1, a LCDR2, and a LCDR3 in SEQ ID NO: 268, respectively; or
(5) the HCDR1, the HCDR2, and the HCDR3 of the heavy chain variable region comprise the amino acid sequences of a HCDR1, a HCDR2, and a HCDR3 in SEQ ID NO: 275, respectively, and the LCDR1, the LCDR2, and the LCDR3 of the light chain variable region comprise the amino acid sequences of a LCDR1, a LCDR2, and a LCDR3 in SEQ ID NO: 269, respectively; or
(6) the HCDR1, the HCDR2, and the HCDR3 of the heavy chain variable region comprise the amino acid sequences of a HCDR1, a HCDR2, and a HCDR3 in SEQ ID NO: 278, respectively, and the LCDR1, the LCDR2, and the LCDR3 of the light chain variable region comprise the amino acid sequences of a LCDR1, a LCDR2, and a LCDR3 in SEQ ID NO: 269, respectively; or
(7) the HCDR1, the HCDR2, and the HCDR3 of the heavy chain variable region comprise the amino acid sequences of a HCDR1, a HCDR2, and a HCDR3 in SEQ ID NO: 279, respectively, and the LCDR1, the LCDR2, and the LCDR3 of the light chain variable region comprise the amino acid sequences of a LCDR1, a LCDR2, and a LCDR3 in SEQ ID NO: 269, respectively.

In some embodiments, provided is the anti-CD3 antibody according to any one of the foregoing, wherein the HCDR1, the HCDR2, and the HCDR3 of the heavy chain variable region and the LCDR1, the LCDR2, and the LCDR3 of the light chain variable region are defined according to a numbering scheme selected from the group consisting of Kabat, IMGT, Chothia, AbM, and Contact. In some embodiments, the HCDR1, the HCDR2, and the HCDR3 of the heavy chain variable region and the LCDR1, the LCDR2, and the LCDR3 of the light chain variable region are defined according to the Kabat numbering scheme. In some embodiments, the HCDR1, the HCDR2, and the HCDR3 of the heavy chain variable region and the LCDR1, the LCDR2, and the LCDR3 of the light chain variable region are defined according to the IMGT numbering scheme. In some embodiments, the HCDR1, the HCDR2, and the HCDR3 of the heavy chain variable region and the LCDR1, the LCDR2, and the LCDR3 of the light chain variable region are defined according to the Chothia numbering scheme. In some embodiments, the HCDR1, the HCDR2, and the HCDR3 of the heavy chain variable region and the LCDR1, the LCDR2, and the LCDR3 of the light chain variable region are defined according to the AbM numbering scheme. In some embodiments, the HCDR1, the HCDR2, and the HCDR3 of the heavy chain variable region and the LCDR1, the LCDR2, and the LCDR3 of the light chain variable region are defined according to the Contact numbering scheme. In another aspect, the present disclosure provides an anti-CD3 antibody, wherein:
a. in the heavy chain variable region, the HCDR1 comprises the amino acid sequence of SEQ ID NO: 343, the HCDR2 comprises the amino acid sequence of SEQ ID NO: 344, and the HCDR3 comprises the amino acid sequence of SEQ ID NO: 345, and in the light chain variable region, the LCDR1 comprises the amino acid sequence of SEQ ID NO: 346, the LCDR2 comprises the amino acid sequence of SEQ ID NO: 347, and the LCDR3 comprises the amino acid sequence of SEQ ID NO: 348, wherein
   the HCDR1 is selected from the group consisting of SX₁YX₂H SEQ ID NO: 343
   the HCDR2 is selected from the group consisting of WIX₃PGX₄X₅X₆X₇X₈YNEKFKG SEQ ID NO: 344
   the HCDR3 is selected from the group consisting of DGYSYYYFDX₉ SEQ ID NO: 345
   the LCDR1 is selected from the group consisting of X₁₀SSQSX₁₁LX₁₂X₁₃RX₁₄X₁₅X₁₆NYLA SEQ ID NO: 346
   the LCDR2 is selected from the group consisting of X₁₇ASTRES SEQ ID NO: 347
   the LCDR3 is selected from the group consisting of KQX₁₈X₁₉IX₂₀RT SEQ ID NO: 348 wherein X₁ is selected from the group consisting of Y, E, K, and Q, preferably Y; X₂ is selected from the group consisting of M, I, E, N, and Q, preferably M; X₃ is selected from the group consisting of N, Q, and S, preferably N or Q; X₄ is selected from the group consisting of D and E, preferably E; X₅ is selected from the group consisting of G and D, preferably G; X₆ is selected from the group consisting of S and A, preferably S; X₇ is selected from the group consisting of I and T; X₈ is selected from the group consisting of K and S; X₉ is selected from the group consisting of Y and F, preferably F; X₁₀ is selected from the group consisting of K and T, preferably K; X₁₁ is selected from the group consisting of L and V; X₁₂ is selected from the group consisting of N and Q, preferably N; X₁₃ is selected from the group consisting of S, D, E, K, P, Q, R, and W, preferably E or P; X₁₄ is selected from the group consisting of T and S; X₁₅ is selected from the group consisting of R and Q; X₁₆ is selected from the group consisting of K and Q, preferably K; X₁₇ is selected from the group consisting of Y and W, preferably W; X₁₈ is selected from the group consisting of S and N, preferably S; X₁₉ is selected from the group consisting of F and Y; X₂₀ is selected from the group consisting of L, I, and V, preferably L or V; or
b. in the heavy chain variable region, the HCDR1 comprises the amino acid sequence of SEQ ID NO: 8, the HCDR2 comprises the amino acid sequence of SEQ ID NO: 400, and the HCDR3 comprises the amino acid sequence of SEQ ID NO: 401, and in the light chain variable region, the LCDR1 comprises the amino acid sequence of SEQ ID NO: 402, the LCDR2 comprises the amino acid sequence of SEQ ID NO: 2, and the LCDR3 comprises the amino acid sequence of SEQ ID NO: 7, wherein:
   the HCDR2 is selected from the group consisting of NIX₂₁LSX₂₂GGTNYNEKFKT SEQ ID NO: 400
   the HCDR3 is selected from the group consisting of X₂₃SYSVYYFDY SEQ ID NO: 401
   the LCDR1 is selected from the group consisting of KSSQSLLX₂₄SRTRKNYLA SEQ ID NO: 402
   wherein X₂₁ is selected from the group consisting of N and Q; X₂₂ is selected from the group consisting of N and Q; X₂₃ is selected from the group consisting of D and E; X₂₄ is selected from the group consisting of N and Q; or
c. in the heavy chain variable region, the HCDR1 comprises the amino acid sequence of SEQ ID NO: 4, the HCDR2 comprises the amino acid sequence of SEQ ID NO: 5, and the HCDR3 comprises the amino acid sequence of SEQ ID NO: 6, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 3; or
d. in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 7; or
e. in the heavy chain variable region, the HCDR1 comprises the amino acid sequence of SEQ ID NO: 4, the HCDR2 comprises the amino acid sequence of SEQ ID NO: 15, and the HCDR3 comprises the amino acid sequence of SEQ ID NO: 13, and in the light chain variable region, the LCDR1 comprises the amino acid sequence of SEQ ID NO: 14, the LCDR2 comprises the amino acid sequence of SEQ ID NO: 2, and the LCDR3 comprises the amino acid sequence of SEQ ID NO: 7; or
f. in the heavy chain variable region, the HCDR1 comprises the amino acid sequence of SEQ ID NO: 13, the HCDR2 comprises the amino acid sequence of SEQ ID NO: 18, and the HCDR3 comprises the amino acid sequence of SEQ ID NO: 19, and in the light chain variable region, the LCDR1 comprises the amino acid sequence of SEQ ID NO: 14, the LCDR2 comprises the amino acid sequence of SEQ ID NO: 16, and the LCDR3 comprises the amino acid sequence of SEQ ID NO: 17; or
g. in the heavy chain variable region, the HCDR1 comprises the amino acid sequence of SEQ ID NO: 13, the HCDR2 comprises the amino acid sequence of SEQ ID NO: 21, and the HCDR3 comprises the amino acid sequence of SEQ ID NO: 19, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 20; or
h. in the heavy chain variable region, the HCDR1 comprises the amino acid sequence of SEQ ID NO: 25, the HCDR2 comprises the amino acid sequence of SEQ ID NO: 26, and the HCDR3 comprises the amino acid sequence of SEQ ID NO: 27, and in the light chain variable region, the LCDR1 comprises the amino acid sequence of SEQ ID NO: 22, the LCDR2 comprises the amino acid sequence of SEQ ID NO: 23, and the LCDR3 comprises the amino acid sequence of SEQ ID NO: 24; or
i. in the heavy chain variable region, the HCDR1 comprises the amino acid sequence of SEQ ID NO: 31, the HCDR2 comprises the amino acid sequence of SEQ ID NO: 32, and the HCDR3 comprises the amino acid sequence of SEQ ID NO: 33, and in the light chain variable region, the LCDR1 comprises the amino acid sequence of SEQ ID NO: 28, the LCDR2 comprises the amino acid sequence of SEQ ID NO: 29, and the LCDR3 comprises the amino acid sequence of SEQ ID NO: 30; or
j. in the heavy chain variable region, the HCDR1 comprises the amino acid sequence of SEQ ID NO: 37, the HCDR2 comprises the amino acid sequence of SEQ ID NO: 38, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17; or
k. in the heavy chain variable region, the HCDR1 comprises the amino acid sequence of SEQ ID NO: 34, the HCDR2 comprises the amino acid sequence of SEQ ID NO: 39, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17; or
l. in the heavy chain variable region, the HCDR1 comprises the amino acid sequence of SEQ ID NO: 41, the HCDR2 comprises the amino acid sequence of SEQ ID NO: 42, and the HCDR3 comprises the amino acid sequence of SEQ ID NO: 43, and in the light chain variable region, the LCDR1 comprises the amino acid sequence of SEQ ID NO: 40, the LCDR2 comprises the amino acid sequence of SEQ ID NO: 2, and the LCDR3 comprises the amino acid sequence of SEQ ID NO: 20; or
m. in the heavy chain variable region, the HCDR1 comprises the amino acid sequence of SEQ ID NO: 34, the HCDR2 comprises the amino acid sequence of SEQ ID NO: 35, and the HCDR3 comprises the amino acid sequence of SEQ ID NO: 44, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17; or
n. in the heavy chain variable region, the HCDR1 comprises the amino acid sequence of SEQ ID NO: 34, the HCDR2 comprises the amino acid sequence of SEQ ID NO: 38, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17; or
o. in the heavy chain variable region, the HCDR1 comprises the amino acid sequence of SEQ ID NO: 47, the HCDR2 comprises the amino acid sequence of SEQ ID NO: 48, and the HCDR3 comprises the amino acid sequence of SEQ ID NO: 49, and in the light chain variable region, the LCDR1 comprises the amino acid sequence of SEQ ID NO: 45, the LCDR2 comprises the amino acid sequence of SEQ ID NO: 2, and the LCDR3 comprises the amino acid sequence of SEQ ID NO: 46; or
p. in the heavy chain variable region, the HCDR1 comprises the amino acid sequence of SEQ ID NO: 11, the HCDR2 comprises the amino acid sequence of SEQ ID NO: 52, and the HCDR3 comprises the amino acid sequence of SEQ ID NO: 53, and in the light chain variable region, the LCDR1 comprises the amino acid sequence of SEQ ID NO: 50, the LCDR2 comprises the amino acid sequence of SEQ ID NO: 2, and the LCDR3 comprises the amino acid sequence of SEQ ID NO: 51; or
q. in the heavy chain variable region, the HCDR1 comprises the amino acid sequence of SEQ ID NO: 37, the HCDR2 comprises the amino acid sequence of SEQ ID NO: 55, and the HCDR3 comprises the amino acid sequence of SEQ ID NO: 56, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 54.

In some embodiments, the anti-CD3 antibody according to any one of the foregoing comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. in the heavy chain variable region, the HCDR1 comprises the amino acid sequence of SEQ ID NO: 343, the HCDR2 comprises the amino acid sequence of SEQ ID NO: 344, and the HCDR3 comprises the amino acid sequence of SEQ ID NO: 345, and in the light chain variable region, the LCDR1 comprises the amino acid sequence of SEQ ID NO: 346, the LCDR2 comprises the amino acid sequence of SEQ ID NO: 347, and the LCDR3 comprises the amino acid sequence of SEQ ID NO: 348; or
b. in the heavy chain variable region, the HCDR1 comprises the amino acid sequence of SEQ ID NO: 8, the HCDR2 comprises the amino acid sequence of SEQ ID NO: 400, and the HCDR3 comprises the amino acid sequence of SEQ ID NO: 401, and in the light chain variable region, the LCDR1 comprises the amino acid sequence of SEQ ID NO: 402, the LCDR2 comprises the amino acid sequence of SEQ ID NO: 2, and the LCDR3 comprises the amino acid sequence of SEQ ID NO: 7.

In some embodiments, provided is the anti-CD3 antibody according to any one of the foregoing, wherein:
a. in the heavy chain variable region, the HCDR1 comprises the amino acid sequence of SEQ ID NO: 137, 34, 193, 194, 195, 196, 197, or 199, the HCDR2 comprises the amino acid sequence of SEQ ID NO: 138, 217, 214, 218, 219, 220, 221, 35, 38, 215, or 216, and the HCDR3 comprises the amino acid sequence of SEQ ID NO: 36 or 43, and in the light chain variable region, the LCDR1 comprises the amino acid sequence of SEQ ID NO: 189, 187, 207, 208, 210, 211, 206, 1, 96, 186, 188, 190, 191, 192, 205, 209, or 212, the LCDR2 comprises the amino acid sequence of SEQ ID NO: 2 or 136, and the LCDR3 comprises the amino acid sequence of SEQ ID NO: 185, 183, 17, 182, or 184; or b. in the heavy chain variable region, the HCDR1 comprises the amino acid sequence of SEQ ID NO: 8, the HCDR2 comprises the amino acid sequence of SEQ ID NO: 9, 97, 98, or 100, and the HCDR3 comprises the amino acid sequence of SEQ ID NO: 10 or 99, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7. In some embodiments, provided is the anti-CD3 antibody according to any one of the foregoing, wherein in the heavy chain variable region, the HCDR1 comprises the amino acid sequence of SEQ ID NO: 137, the HCDR2 comprises the amino acid sequence of SEQ ID NO: 403, and the HCDR3 comprises the amino acid sequence of SEQ ID NO: 36, and in the light chain variable region, the LCDR1 comprises the amino acid sequence of SEQ ID NO: 404, the LCDR2 comprises the amino acid sequence of SEQ ID NO: 2, and the LCDR3 comprises the amino acid sequence of SEQ ID NO: 405,
   wherein:
   the HCDR2 is selected from the group consisting of WIX₂₅PGEGSX₂₆X₂₇YNEKFKG SEQ ID NO: 403
   the LCDR1 is selected from the group consisting of KSSQSX₂₈LNX₂₉RX₃₀X₃₁KNYLA SEQ ID NO: 404
   the LCDR3 is selected from the group consisting of KQSX₃₂IX₃₃RT SEQ ID NO: 405
   wherein X₂₅ is selected from the group consisting of N and Q; X₂₆ is selected from the group consisting of I and T; X₂₇ is selected from the group consisting of K and S; X₂₈ is selected from the group consisting of L and V; X₂₉ is selected from the group consisting of P and E; X₃₀ is selected from the group consisting of T and S; X₃₁ is selected from the group consisting of R and Q; X₃₂ is selected from the group consisting of F and Y; X₃₃ is selected from the group consisting of V and L.

In some embodiments, provided is the anti-CD3 antibody according to any one of the foregoing, wherein:
in the heavy chain variable region, the HCDR1 comprises the amino acid sequence of SEQ ID NO: 137, the HCDR2 comprises the amino acid sequence of SEQ ID NO: 138, 214, 217, 218, 219, 220, or 221, and the HCDR3 comprises the amino acid sequence of SEQ ID NO: 36, and in the light chain variable region, the LCDR1 comprises the amino acid sequence of SEQ ID NO: 189, 187, 206, 207, 208, 210, or 211, the LCDR2 comprises the amino acid sequence of SEQ ID NO: 2, and the LCDR3 comprises the amino acid sequence of SEQ ID NO: 185 or 183.

In some embodiments, provided is the anti-CD3 antibody according to any one of the foregoing, wherein:
(1) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(2) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 183; or
(3) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 187, the LCDR2 comprises the amino acid sequence of SEQ ID NO: 2, and the LCDR3 comprises the amino acid sequence of SEQ ID NO: 183; or
(4) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(5) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(6) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(7) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(8) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(9) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(10) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 210, the LCDR2 comprises the amino acid sequence of SEQ ID NO: 2, and the LCDR3 comprises the amino acid sequence of SEQ ID NO: 185; or
(11) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 211, the LCDR2 comprises the amino acid sequence of SEQ ID NO: 2, and the LCDR3 comprises the amino acid sequence of SEQ ID NO: 185; or
(12) in the heavy chain variable region, the HCDR1 comprises the amino acid sequence of SEQ ID NO: 137, the HCDR2 comprises the amino acid sequence of SEQ ID NO: 219, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(13) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(14) in the heavy chain variable region, the HCDR1 comprises the amino acid sequence of SEQ ID NO: 137, the HCDR2 comprises the amino acid sequence of SEQ ID NO: 221, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(15) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 206, the LCDR2 comprises the amino acid sequence of SEQ ID NO: 2, and the LCDR3 comprises the amino acid sequence of SEQ ID NO: 185; or
(16) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(17) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(18) in the heavy chain variable region, the HCDR1 comprises the amino acid sequence of SEQ ID NO: 34, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 1, 96, 187, or 189, the LCDR2 comprises the amino acid sequence of SEQ ID NO: 2, and the LCDR3 comprises the amino acid sequence of SEQ ID NO: 17; or
(19) in the heavy chain variable region, the HCDR1 comprises the amino acid sequence of SEQ ID NO: 34, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 1 or 96, the LCDR2 comprises the amino acid sequence of SEQ ID NO: 136, and the LCDR3 comprises the amino acid sequence of SEQ ID NO: 17; or
(20) in the heavy chain variable region, the HCDR1 comprises the amino acid sequence of SEQ ID NO: 34, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 1, 96, 186, 187, 188, 189, 190, 191, or 192, the LCDR2 comprises the amino acid sequence of SEQ ID NO: 2, and the LCDR3 comprises the amino acid sequence of SEQ ID NO: 17; or
(21) in the heavy chain variable region, the HCDR1 comprises the amino acid sequence of SEQ ID NO: 34, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 1 or 96, the LCDR2 comprises the amino acid sequence of SEQ ID NO: 136, and the LCDR3 comprises the amino acid sequence of SEQ ID NO: 17; or
(22) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 1, 186, 187, 188, 189, 190, 191, or 192, the LCDR2 comprises the amino acid sequence of SEQ ID NO: 2, and the LCDR3 comprises the amino acid sequence of SEQ ID NO: 17; or
(23) in the heavy chain variable region, the HCDR1 comprises the amino acid sequence of SEQ ID NO: 34, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 182, 183, 184, or 185; or
(24) in the heavy chain variable region, the HCDR1 comprises the amino acid sequence of SEQ ID NO: 193, 194, 195, 196, 197, or 199, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17; or
(25) in the heavy chain variable region, the HCDR1 comprises the amino acid sequence of SEQ ID NO: 196, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 188, 190, 191, or 192, the LCDR2 comprises the amino acid sequence of SEQ ID NO: 2, and the LCDR3 comprises the amino acid sequence of SEQ ID NO: 17; or
(26) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 184; or
(27) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 136, and the LCDR3 comprises the amino acid sequence of SEQ ID NO: 17; or
(28) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 187, 205, 206, 207, 208, 209, or 212, the LCDR2 comprises the amino acid sequence of SEQ ID NO: 2, and the LCDR3 comprises the amino acid sequence of SEQ ID NO: 185; or
(29) in the heavy chain variable region, the HCDR1 comprises the amino acid sequence of SEQ ID NO: 137, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 187, the LCDR2 comprises the amino acid sequence of SEQ ID NO: 2, and the LCDR3 comprises the amino acid sequence of SEQ ID NO: 183 or 185; or
(30) in the heavy chain variable region, the HCDR1 comprises the amino acid sequence of SEQ ID NO: 137, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 136, and the LCDR3 comprises the amino acid sequence of SEQ ID NO: 17; or
(31) in the heavy chain variable region, the HCDR1 comprises the amino acid sequence of SEQ ID NO: 197 or 34, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 136, and the LCDR3 comprises the amino acid sequence of SEQ ID NO: 184; or
(32) in the heavy chain variable region, the HCDR1 comprises the amino acid sequence of SEQ ID NO: 197, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 183, 184, or 185; or
(33) in the heavy chain variable region, the HCDR1 comprises the amino acid sequence of SEQ ID NO: 34, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 187, the LCDR2 comprises the amino acid sequence of SEQ ID NO: 2, and the LCDR3 comprises the amino acid sequence of SEQ ID NO: 183 or 185; or
(34) in the heavy chain variable region, the HCDR1 comprises the amino acid sequence of SEQ ID NO: 194, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 136, and the LCDR3 comprises the amino acid sequence of SEQ ID NO: 183 or 185; or
(35) in the heavy chain variable region, the HCDR1 comprises the amino acid sequence of SEQ ID NO: 34 or 197, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 136, and the LCDR3 comprises the amino acid sequence of SEQ ID NO: 183; or
(36) in the heavy chain variable region, the HCDR1 comprises the amino acid sequence of SEQ ID NO: 34 or 197, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 136, and the LCDR3 comprises the amino acid sequence of SEQ ID NO: 185; or
(37) in the heavy chain variable region, the HCDR1 comprises the amino acid sequence of SEQ ID NO: 137, the HCDR2 comprises the amino acid sequence of SEQ ID NO: 214, 215, 216, 217, or 218, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(38) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 43, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(39) in the heavy chain variable region, the HCDR1 comprises the amino acid sequence of SEQ ID NO: 137, the HCDR2 comprises the amino acid sequence of SEQ ID NO: 214, 217, or 218, and the HCDR3 comprises the amino acid sequence of SEQ ID NO: 36, and in the light chain variable region, the LCDR1 comprises the amino acid sequence of SEQ ID NO: 206, the LCDR2 comprises the amino acid sequence of SEQ ID NO: 2, and the LCDR3 comprises the amino acid sequence of SEQ ID NO: 185; or
(40) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 210, the LCDR2 comprises the amino acid sequence of SEQ ID NO: 2, and the LCDR3 comprises the amino acid sequence of SEQ ID NO: 185; or
(41) in the heavy chain variable region, the HCDR1 comprises the amino acid sequence of SEQ ID NO: 34, the HCDR2 comprises the amino acid sequence of SEQ ID NO: 38, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17.

In some embodiments, provided is the anti-CD3 antibody according to any one of the foregoing, wherein:
(1) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(2) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 183; or
(3) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 187, the LCDR2 comprises the amino acid sequence of SEQ ID NO: 2, and the LCDR3 comprises the amino acid sequence of SEQ ID NO: 183; or
(4) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(5) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(6) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(7) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(8) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(9) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(10) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 210, the LCDR2 comprises the amino acid sequence of SEQ ID NO: 2, and the LCDR3 comprises the amino acid sequence of SEQ ID NO: 185; or
(11) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 211, the LCDR2 comprises the amino acid sequence of SEQ ID NO: 2, and the LCDR3 comprises the amino acid sequence of SEQ ID NO: 185; or
(12) in the heavy chain variable region, the HCDR1 comprises the amino acid sequence of SEQ ID NO: 137, the HCDR2 comprises the amino acid sequence of SEQ ID NO: 219, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(13) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(14) in the heavy chain variable region, the HCDR1 comprises the amino acid sequence of SEQ ID NO: 137, the HCDR2 comprises the amino acid sequence of SEQ ID NO: 221, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(15) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 206, the LCDR2 comprises the amino acid sequence of SEQ ID NO: 2, and the LCDR3 comprises the amino acid sequence of SEQ ID NO: 185; or
(16) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(17) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185.

In some embodiments, provided is the anti-CD3 antibody according to any one of the foregoing, wherein:
(1) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
(2) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 183; or
(3) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 187, the LCDR2 comprises the amino acid sequence of SEQ ID NO: 2, and the LCDR3 comprises the amino acid sequence of SEQ ID NO: 183; or
(4) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
(5) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(6) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
(7) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185.

In some embodiments, provided is the anti-CD3 antibody according to any one of the foregoing, wherein:
(1) in the heavy chain variable region, the HCDR1 comprises the amino acid sequence of SEQ ID NO: 34, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 17; or
(2) in the heavy chain variable region, the HCDR1 comprises the amino acid sequence of SEQ ID NO: 34, the HCDR2 comprises the amino acid sequence of SEQ ID NO: 35, and the HCDR3 comprises the amino acid sequence of SEQ ID NO: 36, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 17; or
(3) in the heavy chain variable region, the HCDR1 comprises the amino acid sequence of SEQ ID NO: 34, the HCDR2 comprises the amino acid sequence of SEQ ID NO: 35 or 138, and the HCDR3 comprises the amino acid sequence of SEQ ID NO: 36, and in the light chain variable region, the LCDR1 comprises the amino acid sequence of SEQ ID NO: 1, the LCDR2 comprises the amino acid sequence of SEQ ID NO: 136, and the LCDR3 comprises the amino acid sequence of SEQ ID NO: 17.

In some embodiments, provided is the anti-CD3 antibody according to any one of the foregoing, wherein:
(1) in the heavy chain variable region, the HCDR1 comprises the amino acid sequence of SEQ ID NO: 8, the HCDR2 comprises the amino acid sequence of SEQ ID NO: 9, and the HCDR3 comprises the amino acid sequence of SEQ ID NO: 10, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7; or
(2) in the heavy chain variable region, the HCDR1 comprises the amino acid sequence of SEQ ID NO: 8, the HCDR2 comprises the amino acid sequence of SEQ ID NO: 97, and the HCDR3 comprises the amino acid sequence of SEQ ID NO: 10, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7; or
(3) in the heavy chain variable region, the HCDR1 comprises the amino acid sequence of SEQ ID NO: 8, the HCDR2 comprises the amino acid sequence of SEQ ID NO: 98, and the HCDR3 comprises the amino acid sequence of SEQ ID NO: 10, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7; or
(4) in the heavy chain variable region, the HCDR1 comprises the amino acid sequence of SEQ ID NO: 8, the HCDR2 comprises the amino acid sequence of SEQ ID NO: 9, and the HCDR3 comprises the amino acid sequence of SEQ ID NO: 99, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7.

In some embodiments, provided is the anti-CD3 antibody according to any one of the foregoing, wherein the HCDR1, the HCDR2, and the HCDR3 of the heavy chain variable region and the LCDR1, the LCDR2, and the LCDR3 of the light chain variable region are defined according to the Kabat numbering scheme.

In some embodiments, provided is the anti-CD3 antibody according to any one of the foregoing, wherein the anti-CD3 antibody is a murine-derived antibody, a chimeric antibody, a humanized antibody, or a fully human-derived antibody. In some embodiments, the anti-CD3 antibody is a chimeric antibody. In some embodiments, the anti-CD3 antibody is a humanized antibody. In some embodiments, provided is the anti-CD3 antibody according to any one of the foregoing, wherein the anti-CD3 antibody is modified by humanization, back mutation, affinity modulation (e.g., increasing or decreasing affinity), T cell epitope removal, reduction of antibody deamidation, and/or reduction of antibody isomerization.

In some embodiments, the anti-CD3 antibody according to any one of the foregoing comprises human antibody framework regions (FRs).

In some embodiments, in the anti-CD3 antibody according to any one of the foregoing, heavy chain framework regions of a human germline template used for humanization engineering are derived from IGHV1-18*01, IGHV1-46*01, IGHJ6*01, and/or IGHJ2*01; light chain framework regions of a human germline template used for humanization engineering are derived from IGKV4-1*01, IGKV2-28*01, IGKV1-27*01, and/or IGKJ4*01; or
heavy chain framework regions of a human germline template used for humanization engineering are derived from IGHV1-46*01, IGHJ6*01, and/or IGHJ4*01; light chain framework regions of a human germline template used for humanization engineering are derived from IGKV4-1*01, IGKV2-28*01, IGKV1-27*01, and/or IGKJ4*01.

In some embodiments, provided is the anti-CD3 antibody according to any one of the foregoing, wherein the heavy chain variable region comprises a FR1, a FR2, and a FR3 that are derived from IGHV1-18*01 or IGHV1-46*01 and a FR4 derived from IGHJ6*01 or IGHJ2*01, and the FRs are unsubstituted or have one or more amino acid substitutions selected from the group consisting of 1E, 28S, 30A, 37M, 40G, 69L, 71A, 73K, 78A, 82a R, 83T, 89I, 91F, and/or 107S; and/or the light chain variable region comprises a FR1, a FR2, and a FR3 that are derived from IGKV4-1*01, IGKV2-28*01, or IGKV1-27*01 and a FR4 derived from IGKJ4*01 or IGKJ1*01, and the FRs are unsubstituted or have one or more amino acid substitutions selected from the group consisting of 37Q, 43S, 45K, and/or 50Y. In some embodiments, provided is the anti-CD3 antibody, wherein in the heavy chain variable region, the HCDR1 comprises the amino acid sequence of SEQ ID NO: 137, 34, 193, 194, 195, 196, 197, or 199, the HCDR2 comprises the amino acid sequence of SEQ ID NO: 138, 217, 214, 218, 219, 220, 221, 35, 38, 215, or 216, and the HCDR3 comprises the amino acid sequence of SEQ ID NO: 36 or 43, and the FRs of the heavy chain variable region comprise one or more amino acid substitutions selected from the group consisting of 1E, 28S, 30A, 37M, 40G, 69L, 71A, 73K, 78A, 82a R, 83T, 89I, 91F, and/or 107S; and in the light chain variable region, the LCDR1 comprises the amino acid sequence of SEQ ID NO: 189, 187, 207, 208, 210, 211, 206, 1, 96, 186, 188, 190, 191, 192, 205, 209, or 212, the LCDR2 comprises the amino acid sequence of SEQ ID NO: 2 or 136, and the LCDR3 comprises the amino acid sequence of SEQ ID NO: 185, 183, 17, 182, or 184, and the FRs of the light chain variable region comprise one or more amino acid substitutions selected from the group consisting of 37Q, 43S, 45K, and/or 50Y. In some embodiments, provided is the anti-CD3 antibody, wherein in the heavy chain variable region, the HCDR1 comprises the amino acid sequence of SEQ ID NO: 34, the HCDR2 comprises the amino acid sequence of SEQ ID NO: 35, and the HCDR3 comprises the amino acid sequence of SEQ ID NO: 36, and the FRs of the heavy chain variable region comprise one or more amino acid substitutions selected from the group consisting of 1E, 28S, 30A, 37M, 40G, 69L, 71A, 73K, 78A, 82a R, 83T, 89I, 91F, and/or 107S; and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 17, and the FRs of the light chain variable region comprise one or more amino acid substitutions selected from the group consisting of 37Q, 43S, 45K, and/or 50Y. In some embodiments, the variable regions and CDRs described above are defined according to the Kabat numbering scheme.

In some embodiments, provided is the anti-CD3 antibody according to any one of the foregoing, wherein the heavy chain variable region comprises a FR1, a FR2, and a FR3 that are derived from IGHV1-46*01 and a FR4 derived from IGHJ6*01 or IGHJ4*01, and the FRs are unsubstituted or have one or more amino acid substitutions selected from the group consisting of 1E, 28N, 69L, 71A, 73K, 78A, 83T, and/or 91F; and/or the light chain variable region comprises a FR1, a FR2, and a FR3 that are derived from IGKV4-1*01, IGKV2-28*01, or IGKV1-27*01 and a FR4 derived from IGKJ4*01 or IGKJ1*01, and the FRs are unsubstituted or have one or more amino acid substitutions selected from the group consisting of 37Q, 43S, and/or 45K. In some embodiments, provided is the anti-CD3 antibody, wherein in the heavy chain variable region, the HCDR1 comprises the amino acid sequence of SEQ ID NO: 8, the HCDR2 comprises the amino acid sequence of SEQ ID NO: 9, 97, 98, or 100, and the HCDR3 comprises the amino acid sequence of SEQ ID NO: 10 or 99, and the FRs of the heavy chain variable region comprise one or more amino acid substitutions selected from the group consisting of 1E, 28N, 69L, 71A, 73K, 78A, 83T, and/or 91F; and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7, and the FRs of the light chain variable region comprise one or more amino acid substitutions selected from the group consisting of 37Q, 43S, and/or 45K. In some embodiments, provided is the anti-CD3 antibody, wherein in the heavy chain variable region, the HCDR1 comprises the amino acid sequence of SEQ ID NO: 8, the HCDR2 comprises the amino acid sequence of SEQ ID NO: 9, and the HCDR3 comprises the amino acid sequence of SEQ ID NO: 10, and the FRs of the heavy chain variable region comprise one or more amino acid substitutions selected from the group consisting of 1E, 28N, 69L, 71A, 73K, 78A, 83T, and/or 91F; and in the light chain variable region, the LCDR1 comprises the amino acid sequence of SEQ ID NO: 1, the LCDR2 comprises the amino acid sequence of SEQ ID NO: 2, and the LCDR3 comprises the amino acid sequence of SEQ ID NO: 7, and the FRs of the light chain variable region comprise one or more amino acid substitutions selected from the group consisting of 37Q, 43S, and/or 45K. In some embodiments, the variable regions and CDRs described above are defined according to the Kabat numbering scheme.

In some embodiments, provided is the anti-CD3 antibody according to any one of the foregoing, wherein:
a. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273; or
   the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 75, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 74; or
b. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 108, 109, 110, 111, 112, 113, 114, 115, 116, 117, 118, or 119, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 101, 102, 103, 104, 105, 106, or 107; or
   the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 60, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 59; or
c. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 58, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 57; or
d. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 61, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 57; or
e. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 63, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 62; or
f. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 65, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 64; or
g. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 67, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 66; or
h. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 69, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 68; or
i. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 71, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 70; or
j. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 73, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 72; or
k. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 76, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 74; or
l. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 77, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 74; or
m. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 79, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 78; or
n. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 80, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 74; or
o. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 82, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 81; or
p. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 84, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 83; or
q. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 86, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 85; or
r. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 88, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 87.

In some embodiments, provided is the anti-CD3 antibody according to any one of the foregoing, wherein:
a. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273; or
   the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 75, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 74; or
b. the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 108, 109, 110, 111, 112, 113, 114, 115, 116, 117, 118, or 119, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 101, 102, 103, 104, 105, 106, or 107; or
   the heavy chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 60, and the light chain variable region comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 59.

In some embodiments, provided is the anti-CD3 antibody according to any one of the foregoing, wherein:
a. the heavy chain variable region comprises the amino acid sequence of SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, and the light chain variable region comprises the amino acid sequence of SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273; or
   the heavy chain variable region comprises the amino acid sequence of SEQ ID NO: 75, and the light chain variable region comprises the amino acid sequence of SEQ ID NO: 74; or
b. the heavy chain variable region comprises the amino acid sequence of SEQ ID NO: 108, 109, 110, 111, 112, 113, 114, 115, 116, 117, 118, or 119, and the light chain variable region comprises the amino acid sequence of SEQ ID NO: 101, 102, 103, 104, 105, 106, or 107; or
   the heavy chain variable region comprises the amino acid sequence of SEQ ID NO: 60, and the light chain variable region comprises the amino acid sequence of SEQ ID NO: 59.

In some embodiments, provided is the anti-CD3 antibody according to any one of the foregoing, wherein the heavy chain variable region comprises the amino acid sequence of SEQ ID NO: 255, 157, 275, 278, 279, 281, 282, or 283, and the light chain variable region comprises the amino acid sequence of SEQ ID NO: 236, 237, 238, 267, 268, 269, 271, or 272.

In some embodiments, provided is the anti-CD3 antibody according to any one of the foregoing, wherein:
(1) the heavy chain variable region comprises the amino acid sequence of SEQ ID NO: 255, and the light chain variable region comprises the amino acid sequence of SEQ ID NO: 236; or
(2) the heavy chain variable region comprises the amino acid sequence of SEQ ID NO: 255, and the light chain variable region comprises the amino acid sequence of SEQ ID NO: 237; or
(3) the heavy chain variable region comprises the amino acid sequence of SEQ ID NO: 255, and the light chain variable region comprises the amino acid sequence of SEQ ID NO: 238; or
(4) the heavy chain variable region comprises the amino acid sequence of SEQ ID NO: 278, and the light chain variable region comprises the amino acid sequence of SEQ ID NO: 268; or
(5) the heavy chain variable region comprises the amino acid sequence of SEQ ID NO: 275, and the light chain variable region comprises the amino acid sequence of SEQ ID NO: 269; or
(6) the heavy chain variable region comprises the amino acid sequence of SEQ ID NO: 278, and the light chain variable region comprises the amino acid sequence of SEQ ID NO: 269; or
(7) the heavy chain variable region comprises the amino acid sequence of SEQ ID NO: 279, and the light chain variable region comprises the amino acid sequence of SEQ ID NO: 269; or
(8) the heavy chain variable region comprises the amino acid sequence of SEQ ID NO: 157, and the light chain variable region comprises the amino acid sequence of SEQ ID NO: 236; or
(9) the heavy chain variable region comprises the amino acid sequence of SEQ ID NO: 275, and the light chain variable region comprises the amino acid sequence of SEQ ID NO: 268; or
(10) the heavy chain variable region comprises the amino acid sequence of SEQ ID NO: 279, and the light chain variable region comprises the amino acid sequence of SEQ ID NO: 268; or
(11) the heavy chain variable region comprises the amino acid sequence of SEQ ID NO: 157, and the light chain variable region comprises the amino acid sequence of SEQ ID NO: 271; or
(12) the heavy chain variable region comprises the amino acid sequence of SEQ ID NO: 157, and the light chain variable region comprises the amino acid sequence of SEQ ID NO: 272; or
(13) the heavy chain variable region comprises the amino acid sequence of SEQ ID NO: 281, and the light chain variable region comprises the amino acid sequence of SEQ ID NO: 236; or
(14) the heavy chain variable region comprises the amino acid sequence of SEQ ID NO: 282, and the light chain variable region comprises the amino acid sequence of SEQ ID NO: 236; or
(15) the heavy chain variable region comprises the amino acid sequence of SEQ ID NO: 283, and the light chain variable region comprises the amino acid sequence of SEQ ID NO: 236; or
(16) the heavy chain variable region comprises the amino acid sequence of SEQ ID NO: 282, and the light chain variable region comprises the amino acid sequence of SEQ ID NO: 267; or
(17) the heavy chain variable region comprises the amino acid sequence of SEQ ID NO: 282, and the light chain variable region comprises the amino acid sequence of SEQ ID NO: 268; or
(18) the heavy chain variable region comprises the amino acid sequence of SEQ ID NO: 282, and the light chain variable region comprises the amino acid sequence of SEQ ID NO: 269; or
(19) the heavy chain variable region comprises the amino acid sequence of SEQ ID NO: 150, and the light chain variable region comprises the amino acid sequence of SEQ ID NO: 139, 140, 141, 142, 143, 144, or 145; or
(20) the heavy chain variable region comprises the amino acid sequence of SEQ ID NO: 151, and the light chain variable region comprises the amino acid sequence of SEQ ID NO: 139, 140, 141, 142, 143, 144, 145, or 146; or
(21) the heavy chain variable region comprises the amino acid sequence of SEQ ID NO: 152, and the light chain variable region comprises the amino acid sequence of SEQ ID NO: 144; or
(22) the heavy chain variable region comprises the amino acid sequence of SEQ ID NO: 153, and the light chain variable region comprises the amino acid sequence of SEQ ID NO: 139, 140, 141, 142, 143, 144, or 145; or
(23) the heavy chain variable region comprises the amino acid sequence of SEQ ID NO: 154, and the light chain variable region comprises the amino acid sequence of SEQ ID NO: 146, 147, 148, or 149; or
(24) the heavy chain variable region comprises the amino acid sequence of SEQ ID NO: 155, and the light chain variable region comprises the amino acid sequence of SEQ ID NO: 146, 147, 148, 149, 222, 223, 225, 226, 228, 229, 230, 231, 233, 234, or 235; or
(25) the heavy chain variable region comprises the amino acid sequence of SEQ ID NO: 156, 157, 245, 246, 248, 249, 250, 253, 255, 256, 264, 265, or 154, and the light chain variable region comprises the amino acid sequence of SEQ ID NO: 146; or
(26) the heavy chain variable region comprises the amino acid sequence of SEQ ID NO: 255, and the light chain variable region comprises the amino acid sequence of SEQ ID NO: 228, 229, 230, 231, 233, 234, 235, 239, 240, or 241; or
(27) the heavy chain variable region comprises the amino acid sequence of SEQ ID NO: 249, and the light chain variable region comprises the amino acid sequence of SEQ ID NO: 230, 233, 234, or 235; or
(28) the heavy chain variable region comprises the amino acid sequence of SEQ ID NO: 254, and the light chain variable region comprises the amino acid sequence of SEQ ID NO: 238, 240, or 241; or
(29) the heavy chain variable region comprises the amino acid sequence of SEQ ID NO: 157 or 156, and the light chain variable region comprises the amino acid sequence of SEQ ID NO: 229; or
(30) the heavy chain variable region comprises the amino acid sequence of SEQ ID NO: 157, and the light chain variable region comprises the amino acid sequence of SEQ ID NO: 231 or 273; or
(31) the heavy chain variable region comprises the amino acid sequence of SEQ ID NO: 152, and the light chain variable region comprises the amino acid sequence of SEQ ID NO: 229 or 231; or
(32) the heavy chain variable region comprises the amino acid sequence of SEQ ID NO: 151, and the light chain variable region comprises the amino acid sequence of SEQ ID NO: 229 or 231; or
(33) the heavy chain variable region comprises the amino acid sequence of SEQ ID NO: 258 or 259, and the light chain variable region comprises the amino acid sequence of SEQ ID NO: 242; or
(34) the heavy chain variable region comprises the amino acid sequence of SEQ ID NO: 258, and the light chain variable region comprises the amino acid sequence of SEQ ID NO: 236, 237, or 239; or
(35) the heavy chain variable region comprises the amino acid sequence of SEQ ID NO: 259, and the light chain variable region comprises the amino acid sequence of SEQ ID NO: 238 or 241; or
(36) the heavy chain variable region comprises the amino acid sequence of SEQ ID NO: 251, 259, or 260, and the light chain variable region comprises the amino acid sequence of SEQ ID NO: 243; or
(37) the heavy chain variable region comprises the amino acid sequence of SEQ ID NO: 251, 259, or 260, and the light chain variable region comprises the amino acid sequence of SEQ ID NO: 244; or
(38) the heavy chain variable region comprises the amino acid sequence of SEQ ID NO: 258, and the light chain variable region comprises the amino acid sequence of SEQ ID NO: 243 or 244; or
(39) the heavy chain variable region comprises the amino acid sequence of SEQ ID NO: 157, and the light chain variable region comprises the amino acid sequence of SEQ ID NO: 266, 267, 268, 269, or 270; or
(40) the heavy chain variable region comprises the amino acid sequence of SEQ ID NO: 274, 275, 276, 277, 278, 279, or 280, and the light chain variable region comprises the amino acid sequence of SEQ ID NO: 236; or
(41) the heavy chain variable region comprises the amino acid sequence of SEQ ID NO: 275, 278, or 279, and the light chain variable region comprises the amino acid sequence of SEQ ID NO: 267; or
(42) the heavy chain variable region comprises the amino acid sequence of SEQ ID NO: 282, and the light chain variable region comprises the amino acid sequence of SEQ ID NO: 271; or
(43) the heavy chain variable region comprises the amino acid sequence of SEQ ID NO: 356, and the light chain variable region comprises the amino acid sequence of SEQ ID NO: 236; or
(44) the heavy chain variable region comprises the amino acid sequence of SEQ ID NO: 356, and the light chain variable region comprises the amino acid sequence of SEQ ID NO: 237; or
(45) the heavy chain variable region comprises the amino acid sequence of SEQ ID NO: 356, and the light chain variable region comprises the amino acid sequence of SEQ ID NO: 238; or
(46) the heavy chain variable region comprises the amino acid sequence of SEQ ID NO: 357, and the light chain variable region comprises the amino acid sequence of SEQ ID NO: 268; or
(47) the heavy chain variable region comprises the amino acid sequence of SEQ ID NO: 358, and the light chain variable region comprises the amino acid sequence of SEQ ID NO: 269; or
(48) the heavy chain variable region comprises the amino acid sequence of SEQ ID NO: 357, and the light chain variable region comprises the amino acid sequence of SEQ ID NO: 269; or
(49) the heavy chain variable region comprises the amino acid sequence of SEQ ID NO: 359, and the light chain variable region comprises the amino acid sequence of SEQ ID NO: 269; or
(50) the heavy chain variable region comprises the amino acid sequence of SEQ ID NO: 161, and the light chain variable region comprises the amino acid sequence of SEQ ID NO: 236; or
(51) the heavy chain variable region comprises the amino acid sequence of SEQ ID NO: 358, and the light chain variable region comprises the amino acid sequence of SEQ ID NO: 268; or
(52) the heavy chain variable region comprises the amino acid sequence of SEQ ID NO: 359, and the light chain variable region comprises the amino acid sequence of SEQ ID NO: 268; or
(53) the heavy chain variable region comprises the amino acid sequence of SEQ ID NO: 161, and the light chain variable region comprises the amino acid sequence of SEQ ID NO: 271; or
(54) the heavy chain variable region comprises the amino acid sequence of SEQ ID NO: 161, and the light chain variable region comprises the amino acid sequence of SEQ ID NO: 272; or
(55) the heavy chain variable region comprises the amino acid sequence of SEQ ID NO: 360, and the light chain variable region comprises the amino acid sequence of SEQ ID NO: 236; or
(56) the heavy chain variable region comprises the amino acid sequence of SEQ ID NO: 361, and the light chain variable region comprises the amino acid sequence of SEQ ID NO: 236; or
(57) the heavy chain variable region comprises the amino acid sequence of SEQ ID NO: 362, and the light chain variable region comprises the amino acid sequence of SEQ ID NO: 236; or
(58) the heavy chain variable region comprises the amino acid sequence of SEQ ID NO: 361, and the light chain variable region comprises the amino acid sequence of SEQ ID NO: 267; or
(59) the heavy chain variable region comprises the amino acid sequence of SEQ ID NO: 361, and the light chain variable region comprises the amino acid sequence of SEQ ID NO: 268; or
(60) the heavy chain variable region comprises the amino acid sequence of SEQ ID NO: 361, and the light chain variable region comprises the amino acid sequence of SEQ ID NO: 269.

In some embodiments, provided is the anti-CD3 antibody according to any one of the foregoing, wherein:
1) the heavy chain variable region comprises the amino acid sequence of SEQ ID NO: 255, 157, 156, 158, 161, 162, 349, 356, or 274, and the light chain variable region comprises the amino acid sequence of SEQ ID NO: 236; or
2) the heavy chain variable region comprises the amino acid sequence of SEQ ID NO: 255, 157, 156, 158, 161, 162, 349, 356, or 274, and the light chain variable region comprises the amino acid sequence of SEQ ID NO: 237; or
3) the heavy chain variable region comprises the amino acid sequence of SEQ ID NO: 255, 157, 156, 158, 161, 162, 349, 356, or 274, and the light chain variable region comprises the amino acid sequence of SEQ ID NO: 238; or
4) the heavy chain variable region comprises the amino acid sequence of SEQ ID NO: 278 or 357, and the light chain variable region comprises the amino acid sequence of SEQ ID NO: 268; or
5) the heavy chain variable region comprises the amino acid sequence of SEQ ID NO: 275 or 358, and the light chain variable region comprises the amino acid sequence of SEQ ID NO: 269; or
6) the heavy chain variable region comprises the amino acid sequence of SEQ ID NO: 278 or 357, and the light chain variable region comprises the amino acid sequence of SEQ ID NO: 269; or
7) the heavy chain variable region comprises the amino acid sequence of SEQ ID NO: 279 or 359, and the light chain variable region comprises the amino acid sequence of SEQ ID NO: 269; or
8) the heavy chain variable region comprises the amino acid sequence of SEQ ID NO: 275 or 358, and the light chain variable region comprises the amino acid sequence of SEQ ID NO: 268; or
9) the heavy chain variable region comprises the amino acid sequence of SEQ ID NO: 279 or 359, and the light chain variable region comprises the amino acid sequence of SEQ ID NO: 268; or
10) the heavy chain variable region comprises the amino acid sequence of SEQ ID NO: 157, 255, 156, 158, 161, 162, 349, 356, or 274, and the light chain variable region comprises the amino acid sequence of SEQ ID NO: 271; or
11) the heavy chain variable region comprises the amino acid sequence of SEQ ID NO: 157, 255, 156, 158, 161, 162, 349, 356, or 274, and the light chain variable region comprises the amino acid sequence of SEQ ID NO: 272; or
12) the heavy chain variable region comprises the amino acid sequence of SEQ ID NO: 281 or 360, and the light chain variable region comprises the amino acid sequence of SEQ ID NO: 236; or
13) the heavy chain variable region comprises the amino acid sequence of SEQ ID NO: 282 or 361, and the light chain variable region comprises the amino acid sequence of SEQ ID NO: 236; or
14) the heavy chain variable region comprises the amino acid sequence of SEQ ID NO: 283, and the light chain variable region comprises the amino acid sequence of SEQ ID NO: 236; or
15) the heavy chain variable region comprises the amino acid sequence of SEQ ID NO: 282 or 361, and the light chain variable region comprises the amino acid sequence of SEQ ID NO: 267; or
16) the heavy chain variable region comprises the amino acid sequence of SEQ ID NO: 282 or 361, and the light chain variable region comprises the amino acid sequence of SEQ ID NO: 268; or
17) the heavy chain variable region comprises the amino acid sequence of SEQ ID NO: 282 or 361, and the light chain variable region comprises the amino acid sequence of SEQ ID NO: 269.

In some embodiments, provided is the anti-CD3 antibody according to any one of the foregoing, wherein:
(1) the heavy chain variable region comprises the amino acid sequence of SEQ ID NO: 255, and the light chain variable region comprises the amino acid sequence of SEQ ID NO: 236; or
   the heavy chain variable region comprises the amino acid sequence of SEQ ID NO: 157, and the light chain variable region comprises the amino acid sequence of SEQ ID NO: 236; or
(2) the heavy chain variable region comprises the amino acid sequence of SEQ ID NO: 255, and the light chain variable region comprises the amino acid sequence of SEQ ID NO: 237; or
(3) the heavy chain variable region comprises the amino acid sequence of SEQ ID NO: 255, and the light chain variable region comprises the amino acid sequence of SEQ ID NO: 238; or
(4) the heavy chain variable region comprises the amino acid sequence of SEQ ID NO: 278, and the light chain variable region comprises the amino acid sequence of SEQ ID NO: 268; or
(5) the heavy chain variable region comprises the amino acid sequence of SEQ ID NO: 275, and the light chain variable region comprises the amino acid sequence of SEQ ID NO: 269; or
(6) the heavy chain variable region comprises the amino acid sequence of SEQ ID NO: 278, and the light chain variable region comprises the amino acid sequence of SEQ ID NO: 269; or
(7) the heavy chain variable region comprises the amino acid sequence of SEQ ID NO: 279, and the light chain variable region comprises the amino acid sequence of SEQ ID NO: 269; or
(8) the heavy chain variable region comprises the amino acid sequence of SEQ ID NO: 275, and the light chain variable region comprises the amino acid sequence of SEQ ID NO: 268; or
(9) the heavy chain variable region comprises the amino acid sequence of SEQ ID NO: 279, and the light chain variable region comprises the amino acid sequence of SEQ ID NO: 268; or
(10) the heavy chain variable region comprises the amino acid sequence of SEQ ID NO: 157, and the light chain variable region comprises the amino acid sequence of SEQ ID NO: 271; or
(11) the heavy chain variable region comprises the amino acid sequence of SEQ ID NO: 157, and the light chain variable region comprises the amino acid sequence of SEQ ID NO: 272; or
(12) the heavy chain variable region comprises the amino acid sequence of SEQ ID NO: 281, and the light chain variable region comprises the amino acid sequence of SEQ ID NO: 236; or
(13) the heavy chain variable region comprises the amino acid sequence of SEQ ID NO: 282, and the light chain variable region comprises the amino acid sequence of SEQ ID NO: 236; or
(14) the heavy chain variable region comprises the amino acid sequence of SEQ ID NO: 283, and the light chain variable region comprises the amino acid sequence of SEQ ID NO: 236; or
(15) the heavy chain variable region comprises the amino acid sequence of SEQ ID NO: 282, and the light chain variable region comprises the amino acid sequence of SEQ ID NO: 267; or
(16) the heavy chain variable region comprises the amino acid sequence of SEQ ID NO: 282, and the light chain variable region comprises the amino acid sequence of SEQ ID NO: 268; or
(17) the heavy chain variable region comprises the amino acid sequence of SEQ ID NO: 282, and the light chain variable region comprises the amino acid sequence of SEQ ID NO: 269.

In some embodiments, provided is the anti-CD3 antibody according to any one of the foregoing, wherein:
(1) the heavy chain variable region comprises the amino acid sequence of SEQ ID NO: 255, and the light chain variable region comprises the amino acid sequence of SEQ ID NO: 236; or
(2) the heavy chain variable region comprises the amino acid sequence of SEQ ID NO: 255, and the light chain variable region comprises the amino acid sequence of SEQ ID NO: 237; or
(3) the heavy chain variable region comprises the amino acid sequence of SEQ ID NO: 255, and the light chain variable region comprises the amino acid sequence of SEQ ID NO: 238; or
(4) the heavy chain variable region comprises the amino acid sequence of SEQ ID NO: 278, and the light chain variable region comprises the amino acid sequence of SEQ ID NO: 268; or
(5) the heavy chain variable region comprises the amino acid sequence of SEQ ID NO: 275, and the light chain variable region comprises the amino acid sequence of SEQ ID NO: 269; or
(6) the heavy chain variable region comprises the amino acid sequence of SEQ ID NO: 278, and the light chain variable region comprises the amino acid sequence of SEQ ID NO: 269; or
(7) the heavy chain variable region comprises the amino acid sequence of SEQ ID NO: 279, and the light chain variable region comprises the amino acid sequence of SEQ ID NO: 269.

In some embodiments, provided is the anti-CD3 antibody according to any one of the foregoing, wherein:
(1) the heavy chain variable region comprises the amino acid sequence of SEQ ID NO: 356, and the light chain variable region comprises the amino acid sequence of SEQ ID NO: 236; or
   the heavy chain variable region comprises the amino acid sequence of SEQ ID NO: 161, and the light chain variable region comprises the amino acid sequence of SEQ ID NO: 236; or
(2) the heavy chain variable region comprises the amino acid sequence of SEQ ID NO: 356, and the light chain variable region comprises the amino acid sequence of SEQ ID NO: 237; or
(3) the heavy chain variable region comprises the amino acid sequence of SEQ ID NO: 356, and the light chain variable region comprises the amino acid sequence of SEQ ID NO: 238; or
(4) the heavy chain variable region comprises the amino acid sequence of SEQ ID NO: 357, and the light chain variable region comprises the amino acid sequence of SEQ ID NO: 268; or
(5) the heavy chain variable region comprises the amino acid sequence of SEQ ID NO: 358, and the light chain variable region comprises the amino acid sequence of SEQ ID NO: 269; or
(6) the heavy chain variable region comprises the amino acid sequence of SEQ ID NO: 357, and the light chain variable region comprises the amino acid sequence of SEQ ID NO: 269; or
(7) the heavy chain variable region comprises the amino acid sequence of SEQ ID NO: 359, and the light chain variable region comprises the amino acid sequence of SEQ ID NO: 269; or
(8) the heavy chain variable region comprises the amino acid sequence of SEQ ID NO: 358, and the light chain variable region comprises the amino acid sequence of SEQ ID NO: 268; or
(9) the heavy chain variable region comprises the amino acid sequence of SEQ ID NO: 359, and the light chain variable region comprises the amino acid sequence of SEQ ID NO: 268; or
(10) the heavy chain variable region comprises the amino acid sequence of SEQ ID NO: 161, and the light chain variable region comprises the amino acid sequence of SEQ ID NO: 271; or
(11) the heavy chain variable region comprises the amino acid sequence of SEQ ID NO: 161, and the light chain variable region comprises the amino acid sequence of SEQ ID NO: 272; or
(12) the heavy chain variable region comprises the amino acid sequence of SEQ ID NO: 360, and the light chain variable region comprises the amino acid sequence of SEQ ID NO: 236; or
(13) the heavy chain variable region comprises the amino acid sequence of SEQ ID NO: 361, and the light chain variable region comprises the amino acid sequence of SEQ ID NO: 236; or
(14) the heavy chain variable region comprises the amino acid sequence of SEQ ID NO: 362, and the light chain variable region comprises the amino acid sequence of SEQ ID NO: 236; or
(15) the heavy chain variable region comprises the amino acid sequence of SEQ ID NO: 361, and the light chain variable region comprises the amino acid sequence of SEQ ID NO: 267; or
(16) the heavy chain variable region comprises the amino acid sequence of SEQ ID NO: 361, and the light chain variable region comprises the amino acid sequence of SEQ ID NO: 268; or
(17) the heavy chain variable region comprises the amino acid sequence of SEQ ID NO: 361, and the light chain variable region comprises the amino acid sequence of SEQ ID NO: 269.

In some embodiments, provided is the anti-CD3 antibody according to any one of the foregoing, wherein:
(1) the heavy chain variable region comprises the amino acid sequence of SEQ ID NO: 153, and the light chain variable region comprises the amino acid sequence of SEQ ID NO: 140, 141, 142, 143, or 144; or
(2) the heavy chain variable region comprises the amino acid sequence of SEQ ID NO: 150, and the light chain variable region comprises the amino acid sequence of SEQ ID NO: 143; or
(3) the heavy chain variable region comprises the amino acid sequence of SEQ ID NO: 151, and the light chain variable region comprises the amino acid sequence of SEQ ID NO: 143 or 144; or
(4) the heavy chain variable region comprises the amino acid sequence of SEQ ID NO: 154, and the light chain variable region comprises the amino acid sequence of SEQ ID NO: 146, 147, or 148; or
(5) the heavy chain variable region comprises the amino acid sequence of SEQ ID NO: 155, and the light chain variable region comprises the amino acid sequence of SEQ ID NO: 146 or 148.

In some embodiments, provided is the anti-CD3 antibody according to any one of the foregoing, wherein:
(1) the heavy chain variable region comprises the amino acid sequence of SEQ ID NO: 109, and the light chain variable region comprises the amino acid sequence of SEQ ID NO: 102, 103, 104, or 105; or
(2) the heavy chain variable region comprises the amino acid sequence of SEQ ID NO: 110, and the light chain variable region comprises the amino acid sequence of SEQ ID NO: 101, 102, 103, 104, or 105; or
(3) the heavy chain variable region comprises the amino acid sequence of SEQ ID NO: 111, and the light chain variable region comprises the amino acid sequence of SEQ ID NO: 102, 103, 104, or 105; or
(4) the heavy chain variable region comprises the amino acid sequence of SEQ ID NO: 112, and the light chain variable region comprises the amino acid sequence of SEQ ID NO: 102, 103, 104, or 105; or
(5) the heavy chain variable region comprises the amino acid sequence of SEQ ID NO: 113, and the light chain variable region comprises the amino acid sequence of SEQ ID NO: 101, 102, 103, 104, or 105; or
(6) the heavy chain variable region comprises the amino acid sequence of SEQ ID NO: 115, and the light chain variable region comprises the amino acid sequence of SEQ ID NO: 106 or 107; or
(7) the heavy chain variable region comprises the amino acid sequence of SEQ ID NO: 116, and the light chain variable region comprises the amino acid sequence of SEQ ID NO: 106 or 107; or
(8) the heavy chain variable region comprises the amino acid sequence of SEQ ID NO: 118, and the light chain variable region comprises the amino acid sequence of SEQ ID NO: 106 or 107.

In some embodiments, provided is the anti-CD3 antibody according to the foregoing, wherein the anti-CD3 antibody is an antigen-binding fragment (antibody fragment); in some embodiments, the antigen-binding fragment (antibody fragment) is selected from the group consisting of a Fab, Fab', F(ab')2, Fd, Fv, scFv, dsFv, and dAb.

In some embodiments, the anti-CD3 antibody according to the foregoing comprises a heavy chain constant region and a light chain constant region. In some embodiments, the heavy chain constant region is a heavy chain constant region of human IgG1, IgG2, IgG3, or IgG4, or a variant thereof, and the light chain constant region is a light chain constant region of human kappa or lambda or a variant thereof. In some embodiments, the heavy chain constant region is a human IgG1 heavy chain constant region or a variant thereof. In some embodiments, the light chain constant region is a human kappa light chain constant region or a variant thereof. In some embodiments, the heavy chain constant region comprises the amino acid sequence of SEQ ID NO: 120, 90, 339, or 399, and the light chain constant region comprises the amino acid sequence of SEQ ID NO: 91 or 384. In some embodiments, the heavy chain constant region comprises the amino acid sequence of SEQ ID NO: 120, and the light chain constant region comprises the amino acid sequence of SEQ ID NO: 91.

In some embodiments, the anti-CD3 antibody according to any one of the foregoing comprises a heavy chain and a light chain, wherein:
a. the heavy chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 301, 326, 323, 327, 181, 331, 332, 333, 174, 175, 176, 177, 178, 179, 180, 295, 296, 297, 298, 299, 300, 302, 309, 311, 312, 314, 315, 322, 324, 325, 328, 335, 336, 350, 351, 352, 353, 354, 355, 363, 364, 365, 366, 367, 368, or 369, and the light chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 303, 304, 305, 319, 320, 329, 330, 318, 163, 164, 165, 166, 167, 168, 169, 170, 171, 172, 173, 198, 284, 285, 286, 287, 288, 289, 290, 291, 292, 293, 294, 306, 307, 308, 310, 313, 316, 317, 321, 334, or 342; or
   the heavy chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 95, and the light chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 94; or
b. the heavy chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 128, 129, 130, 131, 132, 133, 134, or 135, and the light chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 121, 122, 123, 124, 125, 126, or 127; or
   the heavy chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 93, and the light chain comprises an amino acid sequence having at least 80% (e.g., at least 81%, 82%, 83%, 84%, 85%, 86%, 87%, 88%, 89%, 90%, 91%, 92%, 93%, 94%, 95%, 96%, 97%, 98%, or 99%) sequence identity to SEQ ID NO: 92.

In some embodiments, provided is the anti-CD3 antibody according to any one of the foregoing, wherein:
a. the heavy chain comprises the amino acid sequence of SEQ ID NO: 301, 326, 323, 327, 181, 331, 332, 333, 174, 175, 176, 177, 178, 179, 180, 295, 296, 297, 298, 299, 300, 302, 309, 311, 312, 314, 315, 322, 324, 325, 328, 335, 336, 350, 351, 352, 353, 354, 355, 363, 364, 365, 366, 367, 368, or 369, and the light chain comprises the amino acid sequence of SEQ ID NO: 303, 304, 305, 319, 320, 329, 330, 318, 163, 164, 165, 166, 167, 168, 169, 170, 171, 172, 173, 198, 284, 285, 286, 287, 288, 289, 290, 291, 292, 293, 294, 306, 307, 308, 310, 313, 316, 317, 321, 334, or 342; or
   the heavy chain comprises the amino acid sequence of SEQ ID NO: 95, and the light chain comprises the amino acid sequence of SEQ ID NO: 94; or
b. the heavy chain comprises the amino acid sequence of SEQ ID NO: 128, 129, 130, 131, 132, 133, 134, or 135, and the light chain comprises the amino acid sequence of SEQ ID NO: 121, 122, 123, 124, 125, 126, or 127; or
   the heavy chain comprises the amino acid sequence of SEQ ID NO: 93, and the light chain comprises the amino acid sequence of SEQ ID NO: 92.

In some embodiments, provided is the anti-CD3 antibody according to any one of the foregoing, wherein:
(1) the heavy chain comprises the amino acid sequence of SEQ ID NO: 301, and the light chain comprises the amino acid sequence of SEQ ID NO: 303; or
(2) the heavy chain comprises the amino acid sequence of SEQ ID NO: 301, and the light chain comprises the amino acid sequence of SEQ ID NO: 304; or
(3) the heavy chain comprises the amino acid sequence of SEQ ID NO: 301, and the light chain comprises the amino acid sequence of SEQ ID NO: 305; or
(4) the heavy chain comprises the amino acid sequence of SEQ ID NO: 326, and the light chain comprises the amino acid sequence of SEQ ID NO: 319; or
(5) the heavy chain comprises the amino acid sequence of SEQ ID NO: 323, and the light chain comprises the amino acid sequence of SEQ ID NO: 320; or
(6) the heavy chain comprises the amino acid sequence of SEQ ID NO: 326, and the light chain comprises the amino acid sequence of SEQ ID NO: 320; or
(7) the heavy chain comprises the amino acid sequence of SEQ ID NO: 327, and the light chain comprises the amino acid sequence of SEQ ID NO: 320; or
(8) the heavy chain comprises the amino acid sequence of SEQ ID NO: 181, and the light chain comprises the amino acid sequence of SEQ ID NO: 303; or
(9) the heavy chain comprises the amino acid sequence of SEQ ID NO: 323, and the light chain comprises the amino acid sequence of SEQ ID NO: 319; or
(10) the heavy chain comprises the amino acid sequence of SEQ ID NO: 327, and the light chain comprises the amino acid sequence of SEQ ID NO: 319; or
(11) the heavy chain comprises the amino acid sequence of SEQ ID NO: 181, and the light chain comprises the amino acid sequence of SEQ ID NO: 329; or
(12) the heavy chain comprises the amino acid sequence of SEQ ID NO: 181, and the light chain comprises the amino acid sequence of SEQ ID NO: 330; or
(13) the heavy chain comprises the amino acid sequence of SEQ ID NO: 331, and the light chain comprises the amino acid sequence of SEQ ID NO: 303; or
(14) the heavy chain comprises the amino acid sequence of SEQ ID NO: 332, and the light chain comprises the amino acid sequence of SEQ ID NO: 303; or
(15) the heavy chain comprises the amino acid sequence of SEQ ID NO: 333, and the light chain comprises the amino acid sequence of SEQ ID NO: 303; or
(16) the heavy chain comprises the amino acid sequence of SEQ ID NO: 332, and the light chain comprises the amino acid sequence of SEQ ID NO: 318; or
(17) the heavy chain comprises the amino acid sequence of SEQ ID NO: 332, and the light chain comprises the amino acid sequence of SEQ ID NO: 319; or
(18) the heavy chain comprises the amino acid sequence of SEQ ID NO: 332, and the light chain comprises the amino acid sequence of SEQ ID NO: 320; or
(19) the heavy chain comprises the amino acid sequence of SEQ ID NO: 174, and the light chain comprises the amino acid sequence of SEQ ID NO: 163, 164, 165, 166, 167, 168, or 169; or
(20) the heavy chain comprises the amino acid sequence of SEQ ID NO: 175, and the light chain comprises the amino acid sequence of SEQ ID NO: 163, 164, 165, 166, 167, 168, 169, 170, 289, or 291; or
(21) the heavy chain comprises the amino acid sequence of SEQ ID NO: 176, and the light chain comprises the amino acid sequence of SEQ ID NO: 168, 289, or 291; or
(22) the heavy chain comprises the amino acid sequence of SEQ ID NO: 177, and the light chain comprises the amino acid sequence of SEQ ID NO: 163, 164, 165, 166, 167, 168, or 169; or
(23) the heavy chain comprises the amino acid sequence of SEQ ID NO: 178, and the light chain comprises the amino acid sequence of SEQ ID NO: 170, 171, 172, or 173; or
(24) the heavy chain comprises the amino acid sequence of SEQ ID NO: 179, and the light chain comprises the amino acid sequence of SEQ ID NO: 170, 171, 172, 173, 284, 285, 286, 287, 288, 289, 290, 291, 292, 293, or 294; or
(25) the heavy chain comprises the amino acid sequence of SEQ ID NO: 180, 181, 295, 296, 297, 298, 299, 300, 301, 302, 335, 336, or 178, and the light chain comprises the amino acid sequence of SEQ ID NO: 170; or
(26) the heavy chain comprises the amino acid sequence of SEQ ID NO: 301, and the light chain comprises the amino acid sequence of SEQ ID NO: 288, 289, 290, 291, 292, 293, 294, 306, 307, or 308; or
(27) the heavy chain comprises the amino acid sequence of SEQ ID NO: 298, and the light chain comprises the amino acid sequence of SEQ ID NO: 290, 292, 293, or 294; or
(28) the heavy chain comprises the amino acid sequence of SEQ ID NO: 309, and the light chain comprises the amino acid sequence of SEQ ID NO: 305, 307, or 308; or
(29) the heavy chain comprises the amino acid sequence of SEQ ID NO: 181, and the light chain comprises the amino acid sequence of SEQ ID NO: 289, 291, 317, 318, 319, 320, 321, or 334; or
(30) the heavy chain comprises the amino acid sequence of SEQ ID NO: 180, and the light chain comprises the amino acid sequence of SEQ ID NO: 289; or
(31) the heavy chain comprises the amino acid sequence of SEQ ID NO: 311, and the light chain comprises the amino acid sequence of SEQ ID NO: 303, 304, 306, 310, 313, or 316; or
(32) the heavy chain comprises the amino acid sequence of SEQ ID NO: 312, and the light chain comprises the amino acid sequence of SEQ ID NO: 305, 308, 313, or 316; or
(33) the heavy chain comprises the amino acid sequence of SEQ ID NO: 314, and the light chain comprises the amino acid sequence of SEQ ID NO: 313 or 316; or
(34) the heavy chain comprises the amino acid sequence of SEQ ID NO: 315, and the light chain comprises the amino acid sequence of SEQ ID NO: 313 or 316; or
(35) the heavy chain comprises the amino acid sequence of SEQ ID NO: 322, 323, 324, 325, 326, 327, or 328, and the light chain comprises the amino acid sequence of SEQ ID NO: 303; or
(36) the heavy chain comprises the amino acid sequence of SEQ ID NO: 323, 326, or 327, and the light chain comprises the amino acid sequence of SEQ ID NO: 318; or
(37) the heavy chain comprises the amino acid sequence of SEQ ID NO: 332, and the light chain comprises the amino acid sequence of SEQ ID NO: 329; or
(38) the heavy chain comprises the amino acid sequence of SEQ ID NO: 363, and the light chain comprises the amino acid sequence of SEQ ID NO: 303; or
(39) the heavy chain comprises the amino acid sequence of SEQ ID NO: 363, and the light chain comprises the amino acid sequence of SEQ ID NO: 304; or
(40) the heavy chain comprises the amino acid sequence of SEQ ID NO: 363, and the light chain comprises the amino acid sequence of SEQ ID NO: 305; or
(41) the heavy chain comprises the amino acid sequence of SEQ ID NO: 365, and the light chain comprises the amino acid sequence of SEQ ID NO: 319; or
(42) the heavy chain comprises the amino acid sequence of SEQ ID NO: 364, and the light chain comprises the amino acid sequence of SEQ ID NO: 320; or
(43) the heavy chain comprises the amino acid sequence of SEQ ID NO: 365, and the light chain comprises the amino acid sequence of SEQ ID NO: 320; or
(44) the heavy chain comprises the amino acid sequence of SEQ ID NO: 366, and the light chain comprises the amino acid sequence of SEQ ID NO: 320; or
(45) the heavy chain comprises the amino acid sequence of SEQ ID NO: 353, and the light chain comprises the amino acid sequence of SEQ ID NO: 303; or
(46) the heavy chain comprises the amino acid sequence of SEQ ID NO: 364, and the light chain comprises the amino acid sequence of SEQ ID NO: 319; or
(47) the heavy chain comprises the amino acid sequence of SEQ ID NO: 366, and the light chain comprises the amino acid sequence of SEQ ID NO: 319; or
(48) the heavy chain comprises the amino acid sequence of SEQ ID NO: 353, and the light chain comprises the amino acid sequence of SEQ ID NO: 329; or
(49) the heavy chain comprises the amino acid sequence of SEQ ID NO: 353, and the light chain comprises the amino acid sequence of SEQ ID NO: 330; or
(50) the heavy chain comprises the amino acid sequence of SEQ ID NO: 367, and the light chain comprises the amino acid sequence of SEQ ID NO: 303; or
(51) the heavy chain comprises the amino acid sequence of SEQ ID NO: 368, and the light chain comprises the amino acid sequence of SEQ ID NO: 303; or
(52) the heavy chain comprises the amino acid sequence of SEQ ID NO: 369, and the light chain comprises the amino acid sequence of SEQ ID NO: 303; or
(53) the heavy chain comprises the amino acid sequence of SEQ ID NO: 368, and the light chain comprises the amino acid sequence of SEQ ID NO: 318; or
(54) the heavy chain comprises the amino acid sequence of SEQ ID NO: 368, and the light chain comprises the amino acid sequence of SEQ ID NO: 319; or
(55) the heavy chain comprises the amino acid sequence of SEQ ID NO: 368, and the light chain comprises the amino acid sequence of SEQ ID NO: 320.

In some embodiments, provided is the anti-CD3 antibody according to any one of the foregoing, wherein:
1) the heavy chain comprises the amino acid sequence of SEQ ID NO: 301, 181, 180, 350, 353, 354, 355, 363, or 322, and the light chain comprises the amino acid sequence of SEQ ID NO: 303; or
2) the heavy chain comprises the amino acid sequence of SEQ ID NO: 301, 181, 180, 350, 353, 354, 355, 363, or 322, and the light chain comprises the amino acid sequence of SEQ ID NO: 304; or
3) the heavy chain comprises the amino acid sequence of SEQ ID NO: 301, 181, 180, 350, 353, 354, 355, 363, or 322, and the light chain comprises the amino acid sequence of SEQ ID NO: 305; or
4) the heavy chain comprises the amino acid sequence of SEQ ID NO: 326 or 365, and the light chain comprises the amino acid sequence of SEQ ID NO: 319; or
5) the heavy chain comprises the amino acid sequence of SEQ ID NO: 323 or 364, and the light chain comprises the amino acid sequence of SEQ ID NO: 320; or
6) the heavy chain comprises the amino acid sequence of SEQ ID NO: 326 or 365, and the light chain comprises the amino acid sequence of SEQ ID NO: 320; or
7) the heavy chain comprises the amino acid sequence of SEQ ID NO: 327 or 366, and the light chain comprises the amino acid sequence of SEQ ID NO: 320; or
8) the heavy chain comprises the amino acid sequence of SEQ ID NO: 323 or 364, and the light chain comprises the amino acid sequence of SEQ ID NO: 319; or
9) the heavy chain comprises the amino acid sequence of SEQ ID NO: 327 or 366, and the light chain comprises the amino acid sequence of SEQ ID NO: 319; or
10) the heavy chain comprises the amino acid sequence of SEQ ID NO: 181, 301, 180, 350, 353, 354, 355, 363, or 322, and the light chain comprises the amino acid sequence of SEQ ID NO: 329; or
11) the heavy chain comprises the amino acid sequence of SEQ ID NO: 181, 301, 180, 350, 353, 354, 355, 363, or 322, and the light chain comprises the amino acid sequence of SEQ ID NO: 330; or
12) the heavy chain comprises the amino acid sequence of SEQ ID NO: 331 or 367, and the light chain comprises the amino acid sequence of SEQ ID NO: 303; or
13) the heavy chain comprises the amino acid sequence of SEQ ID NO: 332 or 368, and the light chain comprises the amino acid sequence of SEQ ID NO: 303; or
14) the heavy chain comprises the amino acid sequence of SEQ ID NO: 333 or 369, and the light chain comprises the amino acid sequence of SEQ ID NO: 303; or
15) the heavy chain comprises the amino acid sequence of SEQ ID NO: 332 or 368, and the light chain comprises the amino acid sequence of SEQ ID NO: 318; or
16) the heavy chain comprises the amino acid sequence of SEQ ID NO: 332 or 368, and the light chain comprises the amino acid sequence of SEQ ID NO: 319; or
17) the heavy chain comprises the amino acid sequence of SEQ ID NO: 332 or 368, and the light chain comprises the amino acid sequence of SEQ ID NO: 320.

In some embodiments, provided is the anti-CD3 antibody according to any one of the foregoing, wherein:
(1) the heavy chain comprises the amino acid sequence of SEQ ID NO: 301, and the light chain comprises the amino acid sequence of SEQ ID NO: 303; or
   the heavy chain comprises the amino acid sequence of SEQ ID NO: 181, and the light chain comprises the amino acid sequence of SEQ ID NO: 303; or
(2) the heavy chain comprises the amino acid sequence of SEQ ID NO: 301, and the light chain comprises the amino acid sequence of SEQ ID NO: 304; or
(3) the heavy chain comprises the amino acid sequence of SEQ ID NO: 301, and the light chain comprises the amino acid sequence of SEQ ID NO: 305; or
(4) the heavy chain comprises the amino acid sequence of SEQ ID NO: 326, and the light chain comprises the amino acid sequence of SEQ ID NO: 319; or
(5) the heavy chain comprises the amino acid sequence of SEQ ID NO: 323, and the light chain comprises the amino acid sequence of SEQ ID NO: 320; or
(6) the heavy chain comprises the amino acid sequence of SEQ ID NO: 326, and the light chain comprises the amino acid sequence of SEQ ID NO: 320; or
(7) the heavy chain comprises the amino acid sequence of SEQ ID NO: 327, and the light chain comprises the amino acid sequence of SEQ ID NO: 320; or
(8) the heavy chain comprises the amino acid sequence of SEQ ID NO: 323, and the light chain comprises the amino acid sequence of SEQ ID NO: 319; or
(9) the heavy chain comprises the amino acid sequence of SEQ ID NO: 327, and the light chain comprises the amino acid sequence of SEQ ID NO: 319; or
(10) the heavy chain comprises the amino acid sequence of SEQ ID NO: 181, and the light chain comprises the amino acid sequence of SEQ ID NO: 329; or
(11) the heavy chain comprises the amino acid sequence of SEQ ID NO: 181, and the light chain comprises the amino acid sequence of SEQ ID NO: 330; or
(12) the heavy chain comprises the amino acid sequence of SEQ ID NO: 331, and the light chain comprises the amino acid sequence of SEQ ID NO: 303; or
(13) the heavy chain comprises the amino acid sequence of SEQ ID NO: 332, and the light chain comprises the amino acid sequence of SEQ ID NO: 303; or
(14) the heavy chain comprises the amino acid sequence of SEQ ID NO: 333, and the light chain comprises the amino acid sequence of SEQ ID NO: 303; or
(15) the heavy chain comprises the amino acid sequence of SEQ ID NO: 332, and the light chain comprises the amino acid sequence of SEQ ID NO: 318; or
(16) the heavy chain comprises the amino acid sequence of SEQ ID NO: 332, and the light chain comprises the amino acid sequence of SEQ ID NO: 319; or
(17) the heavy chain comprises the amino acid sequence of SEQ ID NO: 332, and the light chain comprises the amino acid sequence of SEQ ID NO: 320.

In some embodiments, provided is the anti-CD3 antibody according to any one of the foregoing, wherein:
(1) the heavy chain comprises the amino acid sequence of SEQ ID NO: 301, and the light chain comprises the amino acid sequence of SEQ ID NO: 303; or
(2) the heavy chain comprises the amino acid sequence of SEQ ID NO: 301, and the light chain comprises the amino acid sequence of SEQ ID NO: 304; or
(3) the heavy chain comprises the amino acid sequence of SEQ ID NO: 301, and the light chain comprises the amino acid sequence of SEQ ID NO: 305; or
(4) the heavy chain comprises the amino acid sequence of SEQ ID NO: 326, and the light chain comprises the amino acid sequence of SEQ ID NO: 319; or
(5) the heavy chain comprises the amino acid sequence of SEQ ID NO: 323, and the light chain comprises the amino acid sequence of SEQ ID NO: 320; or
(6) the heavy chain comprises the amino acid sequence of SEQ ID NO: 326, and the light chain comprises the amino acid sequence of SEQ ID NO: 320; or
(7) the heavy chain comprises the amino acid sequence of SEQ ID NO: 327, and the light chain comprises the amino acid sequence of SEQ ID NO: 320.

In some embodiments, provided is the anti-CD3 antibody according to any one of the foregoing, wherein:
(1) the heavy chain comprises the amino acid sequence of SEQ ID NO: 363, and the light chain comprises the amino acid sequence of SEQ ID NO: 303; or
   the heavy chain comprises the amino acid sequence of SEQ ID NO: 353, and the light chain comprises the amino acid sequence of SEQ ID NO: 303; or
(2) the heavy chain comprises the amino acid sequence of SEQ ID NO: 363, and the light chain comprises the amino acid sequence of SEQ ID NO: 304; or
(3) the heavy chain comprises the amino acid sequence of SEQ ID NO: 363, and the light chain comprises the amino acid sequence of SEQ ID NO: 305; or
(4) the heavy chain comprises the amino acid sequence of SEQ ID NO: 365, and the light chain comprises the amino acid sequence of SEQ ID NO: 319; or
(5) the heavy chain comprises the amino acid sequence of SEQ ID NO: 364, and the light chain comprises the amino acid sequence of SEQ ID NO: 320; or
(6) the heavy chain comprises the amino acid sequence of SEQ ID NO: 365, and the light chain comprises the amino acid sequence of SEQ ID NO: 320; or
(7) the heavy chain comprises the amino acid sequence of SEQ ID NO: 366, and the light chain comprises the amino acid sequence of SEQ ID NO: 320; or
(8) the heavy chain comprises the amino acid sequence of SEQ ID NO: 364, and the light chain comprises the amino acid sequence of SEQ ID NO: 319; or
(9) the heavy chain comprises the amino acid sequence of SEQ ID NO: 366, and the light chain comprises the amino acid sequence of SEQ ID NO: 319; or
(10) the heavy chain comprises the amino acid sequence of SEQ ID NO: 353, and the light chain comprises the amino acid sequence of SEQ ID NO: 329; or
(11) the heavy chain comprises the amino acid sequence of SEQ ID NO: 353, and the light chain comprises the amino acid sequence of SEQ ID NO: 330; or
(12) the heavy chain comprises the amino acid sequence of SEQ ID NO: 367, and the light chain comprises the amino acid sequence of SEQ ID NO: 303; or
(13) the heavy chain comprises the amino acid sequence of SEQ ID NO: 368, and the light chain comprises the amino acid sequence of SEQ ID NO: 303; or
(14) the heavy chain comprises the amino acid sequence of SEQ ID NO: 369, and the light chain comprises the amino acid sequence of SEQ ID NO: 303; or
(15) the heavy chain comprises the amino acid sequence of SEQ ID NO: 368, and the light chain comprises the amino acid sequence of SEQ ID NO: 318; or
(16) the heavy chain comprises the amino acid sequence of SEQ ID NO: 368, and the light chain comprises the amino acid sequence of SEQ ID NO: 319; or
(17) the heavy chain comprises the amino acid sequence of SEQ ID NO: 368, and the light chain comprises the amino acid sequence of SEQ ID NO: 320.

In some embodiments, provided is the anti-CD3 antibody according to any one of the foregoing, wherein:
(1) the heavy chain comprises the amino acid sequence of SEQ ID NO: 177, and the light chain comprises the amino acid sequence of SEQ ID NO: 164; or
(2) the heavy chain comprises the amino acid sequence of SEQ ID NO: 177, and the light chain comprises the amino acid sequence of SEQ ID NO: 165; or
(3) the heavy chain comprises the amino acid sequence of SEQ ID NO: 177, and the light chain comprises the amino acid sequence of SEQ ID NO: 166; or
(4) the heavy chain comprises the amino acid sequence of SEQ ID NO: 174, and the light chain comprises the amino acid sequence of SEQ ID NO: 167; or
(5) the heavy chain comprises the amino acid sequence of SEQ ID NO: 175, and the light chain comprises the amino acid sequence of SEQ ID NO: 167; or
(6) the heavy chain comprises the amino acid sequence of SEQ ID NO: 177, and the light chain comprises the amino acid sequence of SEQ ID NO: 167; or
(7) the heavy chain comprises the amino acid sequence of SEQ ID NO: 175, and the light chain comprises the amino acid sequence of SEQ ID NO: 168; or
(8) the heavy chain comprises the amino acid sequence of SEQ ID NO: 177, and the light chain comprises the amino acid sequence of SEQ ID NO: 168; or
(9) the heavy chain comprises the amino acid sequence of SEQ ID NO: 178, and the light chain comprises the amino acid sequence of SEQ ID NO: 170; or
(10) the heavy chain comprises the amino acid sequence of SEQ ID NO: 179, and the light chain comprises the amino acid sequence of SEQ ID NO: 170; or
(11) the heavy chain comprises the amino acid sequence of SEQ ID NO: 178, and the light chain comprises the amino acid sequence of SEQ ID NO: 171; or
(12) the heavy chain comprises the amino acid sequence of SEQ ID NO: 178, and the light chain comprises the amino acid sequence of SEQ ID NO: 172; or
(13) the heavy chain comprises the amino acid sequence of SEQ ID NO: 179, and the light chain comprises the amino acid sequence of SEQ ID NO: 172.

In some embodiments, provided is the anti-CD3 antibody according to any one of the foregoing, wherein:
(1) the heavy chain comprises the amino acid sequence of SEQ ID NO: 128, and the light chain comprises the amino acid sequence of SEQ ID NO: 122, 123, 124, or 125; or
(2) the heavy chain comprises the amino acid sequence of SEQ ID NO: 129, and the light chain comprises the amino acid sequence of SEQ ID NO: 121, 122, 123, 124, or 125; or
(3) the heavy chain comprises the amino acid sequence of SEQ ID NO: 130, and the light chain comprises the amino acid sequence of SEQ ID NO: 122, 123, 124, or 125; or
(4) the heavy chain comprises the amino acid sequence of SEQ ID NO: 131, and the light chain comprises the amino acid sequence of SEQ ID NO: 122, 123, 124, or 125; or
(5) the heavy chain comprises the amino acid sequence of SEQ ID NO: 132, and the light chain comprises the amino acid sequence of SEQ ID NO: 121, 122, 123, 124, or 125; or
(6) the heavy chain comprises the amino acid sequence of SEQ ID NO: 133, and the light chain comprises the amino acid sequence of SEQ ID NO: 126 or 127; or
(7) the heavy chain comprises the amino acid sequence of SEQ ID NO: 134, and the light chain comprises the amino acid sequence of SEQ ID NO: 126 or 127; or
(8) the heavy chain comprises the amino acid sequence of SEQ ID NO: 135, and the light chain comprises the amino acid sequence of SEQ ID NO: 126 or 127.

In some embodiments, the present disclosure further provides an isolated anti-CD3 antibody that competes for binding to human CD3 or an epitope thereof with the anti-CD3 antibody according to any one of the foregoing.

In some embodiments, the anti-CD3 antibody provided by the present disclosure binds to human CD3 with a KD value of greater than 0.1 nM (e.g., greater than 0.5 nM, greater than 1 nM, greater than 2 nM, greater than 3 nM, greater than 4 nM, greater than 5 nM, greater than 6 nM, greater than 7 nM, greater than 8 nM, greater than 9 nM, greater than 10 nM, greater than 20 nM, greater than 30 nM, greater than 40 nM, greater than 50 nM, greater than 60 nM, greater than 70 nM, greater than 80 nM, greater than 90 nM, greater than 100 nM, greater than 150 nM, greater than 200 nM, greater than 250 nM, greater than 300 nM, greater than 350 nM, greater than 400 nM, greater than 450 nM, greater than 500 nM, greater than 550 nM, greater than 600 nM, greater than 650 nM, greater than 700 nM, greater than 750 nM, greater than 800 nM, greater than 850 nM, greater than 900 nM, greater than 950 nM, greater than 1000 nM, greater than 1100 nM, greater than 1200 nM, greater than 1300 nM, greater than 1400 nM, greater than 1450 nM, greater than 1500 nM, greater than 1800 nM, or greater than 2000 nM), as measured by Biacore. In some embodiments, the anti-CD3 antibody provided by the present disclosure binds to human CD3 with a KD value of less than 2000 nM (e.g., less than 1800 nM, less than 1500 nM, less than 1450 nM, less than 1400 nM, less than 1300 nM, less than 1200 nM, less than 1100 nM, less than 1000 nM, less than 950 nM, less than 900 nM, less than 850 nM, less than 800 nM, less than 750 nM, less than 700 nM, less than 650 nM, less than 600 nM, less than 550 nM, less than 500 nM, less than 450 nM, less than 400 nM, less than 350 nM, less than 300 nM, less than 250 nM, less than 200 nM, less than 150 nM, less than 100 nM, less than 90 nM, less than 80 nM, less than 70 nM, less than 60 nM, less than 50 nM, less than 40 nM, less than 30 nM, less than 20 nM, less than 10 nM, less than 9 nM, less than 8 nM, less than 7 nM, less than 6 nM, less than 5 nM, less than 4 nM, less than 3 nM, less than 2 nM, less than 1 nM, less than 0.5 nM, or less than 0.2 nM), as measured by Biacore.

In some embodiments, the anti-CD3 antibody provided by the present disclosure binds to human CD3 with an EC₅₀ value of greater than 0.008 nM (e.g., greater than 0.009 nM, greater than 0.01 nM, greater than 0.1 nM, greater than 0.2 nM, greater than 0.3 nM, greater than 0.4 nM, greater than 0.5 nM, greater than 0.6 nM, greater than 0.7 nM, greater than 0.8 nM, greater than 0.9 nM, greater than 1 nM, greater than 2 nM, greater than 3 nM, greater than 4 nM, greater than 5 nM, greater than 6 nM, greater than 7 nM, greater than 8 nM, greater than 9 nM, greater than 10 nM, greater than 15 nM, greater than 20 nM, greater than 30 nM, greater than 40 nM, greater than 50 nM, greater than 60 nM, greater than 70 nM, greater than 80 nM, greater than 90 nM, greater than 100 nM, greater than 150 nM, greater than 200 nM, greater than 250 nM, greater than 300 nM, greater than 350 nM, greater than 400 nM, greater than 450 nM, or greater than 500 nM), as measured by FACS.

In some embodiments, the anti-CD3 antibody provided by the present disclosure binds to human CD3 with an EC₅₀ value of less than 1500 nM (e.g., less than 1000 nM, less than 950 nM, less than 900 nM, less than 850 nM, less than 800 nM, less than 750 nM, less than 700 nM, less than 650 nM, less than 600 nM, less than 550 nM, less than 500 nM, less than 450 nM, less than 400 nM, less than 350 nM, less than 300 nM, less than 250 nM, less than 200 nM, less than 150 nM, less than 100 nM, less than 90 nM, less than 80 nM, less than 70 nM, less than 60 nM, less than 50 nM, less than 40 nM, less than 30 nM, less than 20 nM, less than 10 nM, less than 9 nM, less than 8 nM, less than 7 nM, less than 6 nM, less than 5 nM, less than 4 nM, less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, less than 0.2 nM, less than 0.1 nM, or less than 0.01 nM), as measured by FACS.

In some embodiments, the anti-CD3 antibody provided by the present disclosure is capable of cross-binding to human CD3 and cynomolgus monkey CD3.

In some embodiments, the anti-CD3 antibody according to any one of the foregoing is a multispecific antibody. In some embodiments, provided is the anti-CD3 antibody according to any one of the foregoing, wherein the multispecific antibody comprises a first antigen-binding domain that binds to human CD3, and one to four additional antigen-binding domains that bind to one or more selected from the group consisting of (a) a tumor antigen and (b) a cell surface antigen, receptor, or receptor ligand. In some embodiments, provided is the anti-CD3 antibody according to any one of the foregoing, wherein the tumor antigen is an antigen on a tumor cell selected from the group consisting of cells of breast cancer, prostate cancer, gastric cancer, lung cancer, colorectal cancer (including colon cancer and rectal cancer), pancreatic cancer, liver cancer, ovarian cancer, oral cancer, nasopharyngeal carcinoma, esophageal cancer, laryngeal cancer, bone cancer, skin cancer, melanoma, uterine cancer, testicular cancer, bladder cancer, renal cancer, brain cancer, glioblastoma, thyroid cancer, lymphoma, myeloma, and leukemia.

In another aspect, the present disclosure provides a multispecific antibody comprising the anti-CD3 antibody according to any one of the foregoing. In some embodiments, the multispecific antibody specifically binds to human CD3 and further binds to a tumor-associated antigen (TAA) or a cell surface antigen, receptor, or receptor ligand. In some embodiments, the multispecific antibody binds to human CD3 and further binds to one or more antigens selected from the group consisting of CD20, BCMA, and PSMA.

In some embodiments, provided is the multispecific antibody according to the foregoing, wherein the tumor antigen is an antigen on a tumor cell selected from the group consisting of cells of breast cancer, prostate cancer, gastric cancer, lung cancer, colorectal cancer (including colon cancer and rectal cancer), pancreatic cancer, liver cancer, ovarian cancer, oral cancer, nasopharyngeal carcinoma, esophageal cancer, laryngeal cancer, bone cancer, skin cancer, melanoma, uterine cancer, testicular cancer, bladder cancer, renal cancer, brain cancer, glioblastoma, thyroid cancer, lymphoma, myeloma, and leukemia.

In some embodiments, the multispecific antibody according to any one of the foregoing is a bispecific antibody. In some embodiments, the bispecific antibody is an anti-CD20/CD3 bispecific antibody.

In some embodiments, the anti-CD20/CD3 bispecific antibody according to the foregoing comprises one first antigen-binding domain that specifically binds to CD3 and one second antigen-binding domain that specifically binds to CD20, wherein the first antigen-binding domain that specifically binds to CD3 comprises a heavy chain variable region CD3-VH and a light chain variable region CD3-VL, and the second antigen-binding domain that specifically binds to CD20 comprises a heavy chain variable region CD20-VH and a light chain variable region CD20-VL, wherein:
(1) the CD3-VH comprises a CD3-HCDR1, a CD3-HCDR2, and a CD3-HCDR3, wherein the CD3-HCDR1 comprises the amino acid sequence of SEQ ID NO: 34, the CD3-HCDR2 comprises the amino acid sequence of SEQ ID NO: 138 or 35, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 1, the CD3-LCDR2 comprises the amino acid sequence of SEQ ID NO: 2, and the CD3-LCDR3 comprises the amino acid sequence of SEQ ID NO: 17; or
(2) the CD3-VH comprises a CD3-HCDR1, a CD3-HCDR2, and a CD3-HCDR3, wherein the CD3-HCDR1 comprises the amino acid sequence of SEQ ID NO: 34, the CD3-HCDR2 comprises the amino acid sequence of SEQ ID NO: 138 or 35, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 96, the CD3-LCDR2 comprises the amino acid sequence of SEQ ID NO: 2, and the CD3-LCDR3 comprises the amino acid sequence of SEQ ID NO: 17; or
(3) the CD3-VH comprises a CD3-HCDR1, a CD3-HCDR2, and a CD3-HCDR3, wherein the CD3-HCDR1 comprises the amino acid sequence of SEQ ID NO: 34, the CD3-HCDR2 comprises the amino acid sequence of SEQ ID NO: 138 or 35, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 1, the CD3-LCDR2 comprises the amino acid sequence of SEQ ID NO: 136, and the CD3-LCDR3 comprises the amino acid sequence of SEQ ID NO: 17.

In some embodiments, provided is the anti-CD20/CD3 bispecific antibody according to any one of the foregoing, wherein:
the CD20-VH comprises a CD20-HCDR1, a CD20-HCDR2, and a CD20-HCDR3, wherein the CD20-HCDR1 comprises the amino acid sequence of SEQ ID NO: 373, the CD20-HCDR2 comprises the amino acid sequence of SEQ ID NO: 374, and the CD20-HCDR3 comprises the amino acid sequence of SEQ ID NO: 375; and the CD20-VL comprises a CD20-LCDR1, a CD20-LCDR2, and a CD20-LCDR3, wherein the CD20-LCDR1 comprises the amino acid sequence of SEQ ID NO: 370, the CD20-LCDR2 comprises the amino acid sequence of SEQ ID NO: 371, and the CD20-LCDR3 comprises the amino acid sequence of SEQ ID NO: 372.

In some embodiments, provided is the anti-CD20/CD3 bispecific antibody according to any one of the foregoing, wherein:
(1) the CD3-HCDR1 comprises a CD3-HCDR1 comprising the amino acid sequence of SEQ ID NO: 34, the CD3-HCDR2 comprises the amino acid sequence of SEQ ID NO: 138 or 35, and the CD3-HCDR3 comprises the amino acid sequence of SEQ ID NO: 36; and the CD3-LCDR1 comprises the amino acid sequence of SEQ ID NO: 1, the CD3-LCDR2 comprises the amino acid sequence of SEQ ID NO: 2, and the CD3-LCDR3 comprises the amino acid sequence of SEQ ID NO: 17;
   furthermore, the CD20-VH comprises: a CD20-HCDR1 comprising the amino acid sequence of SEQ ID NO: 373, a CD20-HCDR2 comprising the amino acid sequence of SEQ ID NO: 374, and a CD20-HCDR3 comprising the amino acid sequence of SEQ ID NO: 375; and the CD20-VL comprises: a CD20-LCDR1 comprising the amino acid sequence of SEQ ID NO: 370, a CD20-LCDR2 comprising the amino acid sequence of SEQ ID NO: 371, and a CD20-LCDR3 comprising the amino acid sequence of SEQ ID NO: 372; or
(2) the CD3-VH comprises: a CD3-HCDR1 comprising the amino acid sequence of SEQ ID NO: 34, a CD3-HCDR2 comprising the amino acid sequence of SEQ ID NO: 138 or 35, and a CD3-HCDR3 comprising the amino acid sequence of SEQ ID NO: 36; and the CD3-VL comprises: a CD3-LCDR1 comprising the amino acid sequence of SEQ ID NO: 96, a CD3-LCDR2 comprising the amino acid sequence of SEQ ID NO: 2, and a CD3-LCDR3 comprising the amino acid sequence of SEQ ID NO: 17;
   furthermore, the CD20-HCDR1 comprises the amino acid sequence of SEQ ID NO: 373, the CD20-HCDR2 comprises the amino acid sequence of SEQ ID NO: 374, and the CD20-HCDR3 comprises the amino acid sequence of SEQ ID NO: 375; and the CD20-LCDR1 comprises the amino acid sequence of SEQ ID NO: 370, the CD20-LCDR2 comprises the amino acid sequence of SEQ ID NO: 371, and the CD20-LCDR3 comprises the amino acid sequence of SEQ ID NO: 372; or
(3) the CD3-HCDR1 comprises a CD3-HCDR1 comprising the amino acid sequence of SEQ ID NO: 34, the CD3-HCDR2 comprises the amino acid sequence of SEQ ID NO: 138 or 35, and the CD3-HCDR3 comprises the amino acid sequence of SEQ ID NO: 36; and the CD3-LCDR1 comprises the amino acid sequence of SEQ ID NO: 1, the CD3-LCDR2 comprises the amino acid sequence of SEQ ID NO: 136, and the CD3-LCDR3 comprises the amino acid sequence of SEQ ID NO: 17;
   furthermore, the CD20-HCDR1 comprises the amino acid sequence of SEQ ID NO: 373, the CD20-HCDR2 comprises the amino acid sequence of SEQ ID NO: 374, and the CD20-HCDR3 comprises the amino acid sequence of SEQ ID NO: 375; and the CD20-LCDR1 comprises the amino acid sequence of SEQ ID NO: 370, the CD20-LCDR2 comprises the amino acid sequence of SEQ ID NO: 371, and the CD20-LCDR3 comprises the amino acid sequence of SEQ ID NO: 372.

In some embodiments, provided is the anti-CD20/CD3 bispecific antibody according to any one of the foregoing, wherein:
(1) the CD3-VH comprises the amino acid sequence of SEQ ID NO: 153, and the CD3-VL comprises the amino acid sequence of SEQ ID NO: 140, 141, 142, 143, or 144; or
(2) the CD3-VH comprises the amino acid sequence of SEQ ID NO: 150, and the CD3-VL comprises the amino acid sequence of SEQ ID NO: 143; or
(3) the CD3-VH comprises the amino acid sequence of SEQ ID NO: 151, and the CD3-VL comprises the amino acid sequence of SEQ ID NO: 143 or 144; or
(4) the CD3-VH comprises the amino acid sequence of SEQ ID NO: 154, and the CD3-VL comprises the amino acid sequence of SEQ ID NO: 146, 147, or 148; or
(5) the CD3-VH comprises the amino acid sequence of SEQ ID NO: 155, and the CD3-VL comprises the amino acid sequence of SEQ ID NO: 146 or 148.

In some embodiments, provided is the anti-CD20/CD3 bispecific antibody according to any one of the foregoing, wherein
the CD20-VH comprises the amino acid sequence of SEQ ID NO: 338, and the CD20-VL comprises the amino acid sequence of SEQ ID NO: 337.

In some embodiments, provided is the anti-CD20/CD3 bispecific antibody according to any one of the foregoing, wherein:
(1) the CD3-VH comprises the amino acid sequence of SEQ ID NO: 153, and the CD3-VL comprises the amino acid sequence of SEQ ID NO: 140, 141, 142, 143, or 144; and the CD20-VH comprises the amino acid sequence of SEQ ID NO: 338, and the CD20-VL comprises the amino acid sequence of SEQ ID NO: 337; or
(2) the CD3-VH comprises the amino acid sequence of SEQ ID NO: 150, and the CD3-VL comprises the amino acid sequence of SEQ ID NO: 143; and the CD20-VH comprises the amino acid sequence of SEQ ID NO: 338, and the CD20-VL comprises the amino acid sequence of SEQ ID NO: 337; or
(3) the CD3-VH comprises the amino acid sequence of SEQ ID NO: 151, and the CD3-VL comprises the amino acid sequence of SEQ ID NO: 143 or 144; and the CD20-VH comprises the amino acid sequence of SEQ ID NO: 338, and the CD20-VL comprises the amino acid sequence of SEQ ID NO: 337; or
(4) the CD3-VH comprises the amino acid sequence of SEQ ID NO: 154, and the CD3-VL comprises the amino acid sequence of SEQ ID NO: 146, 147, or 148; and the CD20-VH comprises the amino acid sequence of SEQ ID NO: 338, and the CD20-VL comprises the amino acid sequence of SEQ ID NO: 337; or
(5) the CD3-VH comprises the amino acid sequence of SEQ ID NO: 155, and the CD3-VL comprises the amino acid sequence of SEQ ID NO: 146 or 148; and the CD20-VH comprises the amino acid sequence of SEQ ID NO: 338, and the CD20-VL comprises the amino acid sequence of SEQ ID NO: 337.

In some embodiments, provided is the anti-CD20/CD3 bispecific antibody according to any one of the foregoing, wherein:
the bispecific antibody further comprises a heavy chain constant region and a light chain constant region. In some embodiments, the heavy chain constant region is a human IgG1 heavy chain constant region or a variant thereof. In some embodiments, the light chain constant region is a human kappa light chain constant region or a variant thereof. In some embodiments, the heavy chain constant region comprises the amino acid sequence of SEQ ID NO: 120, 90, or 339, and the light chain constant region comprises the amino acid sequence of SEQ ID NO: 91. In some embodiments, the heavy chain constant region comprises the amino acid sequence of SEQ ID NO: 120, and the light chain constant region comprises the amino acid sequence of SEQ ID NO: 91. In some embodiments, the heavy chain constant region comprises the amino acid sequence of SEQ ID NO: 339, and the light chain constant region comprises the amino acid sequence of SEQ ID NO: 91.

In some embodiments, provided is the anti-CD20/CD3 bispecific antibody according to any one of the foregoing, wherein the bispecific antibody comprises:
(1) one first chain comprising the amino acid sequence of SEQ ID NO: 341, one second chain comprising the amino acid sequence of SEQ ID NO: 340, one third chain comprising the amino acid sequence of SEQ ID NO: 164, and one fourth chain comprising the amino acid sequence of SEQ ID NO: 177; or
(2) one first chain comprising the amino acid sequence of SEQ ID NO: 341, one second chain comprising the amino acid sequence of SEQ ID NO: 340, one third chain comprising the amino acid sequence of SEQ ID NO: 165, and one fourth chain comprising the amino acid sequence of SEQ ID NO: 177; or
(3) one first chain comprising the amino acid sequence of SEQ ID NO: 341, one second chain comprising the amino acid sequence of SEQ ID NO: 340, one third chain comprising the amino acid sequence of SEQ ID NO: 166, and one fourth chain comprising the amino acid sequence of SEQ ID NO: 177; or
(4) one first chain comprising the amino acid sequence of SEQ ID NO: 341, one second chain comprising the amino acid sequence of SEQ ID NO: 340, one third chain comprising the amino acid sequence of SEQ ID NO: 167, and one fourth chain comprising the amino acid sequence of SEQ ID NO: 174; or
(5) one first chain comprising the amino acid sequence of SEQ ID NO: 341, one second chain comprising the amino acid sequence of SEQ ID NO: 340, one third chain comprising the amino acid sequence of SEQ ID NO: 167, and one fourth chain comprising the amino acid sequence of SEQ ID NO: 175; or
(6) one first chain comprising the amino acid sequence of SEQ ID NO: 341, one second chain comprising the amino acid sequence of SEQ ID NO: 340, one third chain comprising the amino acid sequence of SEQ ID NO: 167, and one fourth chain comprising the amino acid sequence of SEQ ID NO: 177; or
(7) one first chain comprising the amino acid sequence of SEQ ID NO: 341, one second chain comprising the amino acid sequence of SEQ ID NO: 340, one third chain comprising the amino acid sequence of SEQ ID NO: 168, and one fourth chain comprising the amino acid sequence of SEQ ID NO: 175; or
(8) one first chain comprising the amino acid sequence of SEQ ID NO: 341, one second chain comprising the amino acid sequence of SEQ ID NO: 340, one third chain comprising the amino acid sequence of SEQ ID NO: 168, and one fourth chain comprising the amino acid sequence of SEQ ID NO: 177; or
(9) one first chain comprising the amino acid sequence of SEQ ID NO: 341, one second chain comprising the amino acid sequence of SEQ ID NO: 340, one third chain comprising the amino acid sequence of SEQ ID NO: 170, and one fourth chain comprising the amino acid sequence of SEQ ID NO: 178; or
(10) one first chain comprising the amino acid sequence of SEQ ID NO: 341, one second chain comprising the amino acid sequence of SEQ ID NO: 340, one third chain comprising the amino acid sequence of SEQ ID NO: 170, and one fourth chain comprising the amino acid sequence of SEQ ID NO: 179; or
(11) one first chain comprising the amino acid sequence of SEQ ID NO: 341, one second chain comprising the amino acid sequence of SEQ ID NO: 340, one third chain comprising the amino acid sequence of SEQ ID NO: 171, and one fourth chain comprising the amino acid sequence of SEQ ID NO: 178; or
(12) one first chain comprising the amino acid sequence of SEQ ID NO: 341, one second chain comprising the amino acid sequence of SEQ ID NO: 340, one third chain comprising the amino acid sequence of SEQ ID NO: 172, and one fourth chain comprising the amino acid sequence of SEQ ID NO: 178; or
(13) one first chain comprising the amino acid sequence of SEQ ID NO: 341, one second chain comprising the amino acid sequence of SEQ ID NO: 340, one third chain comprising the amino acid sequence of SEQ ID NO: 172, and one fourth chain comprising the amino acid sequence of SEQ ID NO: 179.

In some embodiments, the bispecific antibody is an anti-BCMA/CD3 bispecific antibody. In some embodiments, the anti-BCMA/CD3 bispecific antibody according to the foregoing comprises a first antigen-binding domain that specifically binds to CD3 and a second antigen-binding domain that specifically binds to BCMA, wherein the first antigen-binding domain that specifically binds to CD3 comprises a heavy chain variable region CD3-VH and a light chain variable region CD3-VL, and the second antigen-binding domain that specifically binds to BCMA comprises a heavy chain variable region BCMA-VH and a light chain variable region BCMA-VL, wherein:
(1) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(2) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 183; or
(3) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 187, the CD3-LCDR2 comprises the amino acid sequence of SEQ ID NO: 2, and the CD3-LCDR3 comprises the amino acid sequence of SEQ ID NO: 183; or
(4) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(5) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(6) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(7) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 218, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(8) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(9) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 210, the CD3-LCDR2 comprises the amino acid sequence of SEQ ID NO: 2, and the CD3-LCDR3 comprises the amino acid sequence of SEQ ID NO: 185; or
(10) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 211, the CD3-LCDR2 comprises the amino acid sequence of SEQ ID NO: 2, and the CD3-LCDR3 comprises the amino acid sequence of SEQ ID NO: 185; or
(11) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 219, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(12) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(13) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 221, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(14) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185.

In some embodiments, provided is the anti-BCMA/CD3 bispecific antibody according to any one of the foregoing, wherein the BCMA-VH comprises a BCMA-HCDR1, a BCMA-HCDR2, and a BCMA-HCDR3, wherein the BCMA-HCDR1 comprises the amino acid sequence of SEQ ID NO: 379, the BCMA-HCDR2 comprises the amino acid sequence of SEQ ID NO: 380, and the BCMA-HCDR3 comprises the amino acid sequence of SEQ ID NO: 381; and the BCMA-VL comprises a BCMA-LCDR1, a BCMA-LCDR2, and a BCMA-LCDR3, wherein the BCMA-LCDR1 comprises the amino acid sequence of SEQ ID NO: 376, the BCMA-LCDR2 comprises the amino acid sequence of SEQ ID NO: 377, and the BCMA-LCDR3 comprises the amino acid sequence of SEQ ID NO: 378.

In some embodiments, provided is the anti-BCMA/CD3 bispecific antibody according to any one of the foregoing, wherein:
(1) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(2) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 183; or
(3) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 187, the CD3-LCDR2 comprises the amino acid sequence of SEQ ID NO: 2, and the CD3-LCDR3 comprises the amino acid sequence of SEQ ID NO: 183; or
(4) the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(5) the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(6) the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(7) the CD3-HCDR1 comprises the amino acid sequence of SEQ ID NO: 137, the CD3-HCDR2 comprises the amino acid sequence of SEQ ID NO: 218, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(8) the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(9) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 210, the CD3-LCDR2 comprises the amino acid sequence of SEQ ID NO: 2, and the CD3-LCDR3 comprises the amino acid sequence of SEQ ID NO: 185; or
(10) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 211, the CD3-LCDR2 comprises the amino acid sequence of SEQ ID NO: 2, and the CD3-LCDR3 comprises the amino acid sequence of SEQ ID NO: 185; or
(11) the CD3-HCDR1 comprises the amino acid sequence of SEQ ID NO: 137, the CD3-HCDR2 comprises the amino acid sequence of SEQ ID NO: 219, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(12) the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(13) the CD3-HCDR1 comprises the amino acid sequence of SEQ ID NO: 137, the CD3-HCDR2 comprises the amino acid sequence of SEQ ID NO: 221, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(14) the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185;
furthermore, the BCMA-HCDR1 comprises the amino acid sequence of SEQ ID NO: 379, the BCMA-HCDR2 comprises the amino acid sequence of SEQ ID NO: 380, and the BCMA-HCDR3 comprises the amino acid sequence of SEQ ID NO: 381; and the BCMA-LCDR1 comprises the amino acid sequence of SEQ ID NO: 376, the BCMA-LCDR2 comprises the amino acid sequence of SEQ ID NO: 377, and the BCMA-LCDR3 comprises the amino acid sequence of SEQ ID NO: 378.

In some embodiments, provided is the anti-BCMA/CD3 bispecific antibody according to any one of the foregoing, wherein:
(1) the CD3-VH comprises the amino acid sequence of SEQ ID NO: 255, and the CD3-VL comprises the amino acid sequence of SEQ ID NO: 236; or
(2) the CD3-VH comprises the amino acid sequence of SEQ ID NO: 255, and the CD3-VL comprises the amino acid sequence of SEQ ID NO: 237; or
(3) the CD3-VH comprises the amino acid sequence of SEQ ID NO: 255, and the CD3-VL comprises the amino acid sequence of SEQ ID NO: 238; or
(4) the CD3-VH comprises the amino acid sequence of SEQ ID NO: 278, and the CD3-VL comprises the amino acid sequence of SEQ ID NO: 268; or
(5) the CD3-VH comprises the amino acid sequence of SEQ ID NO: 275, and the CD3-VL comprises the amino acid sequence of SEQ ID NO: 269; or
(6) the CD3-VH comprises the amino acid sequence of SEQ ID NO: 278, and the CD3-VL comprises the amino acid sequence of SEQ ID NO: 269; or
(7) the CD3-VH comprises the amino acid sequence of SEQ ID NO: 279, and the CD3-VL comprises the amino acid sequence of SEQ ID NO: 269; or
(8) the CD3-VH comprises the amino acid sequence of SEQ ID NO: 157, and the CD3-VL comprises the amino acid sequence of SEQ ID NO: 236; or
(9) the CD3-VH comprises the amino acid sequence of SEQ ID NO: 275, and the CD3-VL comprises the amino acid sequence of SEQ ID NO: 268; or
(10) the CD3-VH comprises the amino acid sequence of SEQ ID NO: 157, and the CD3-VL comprises the amino acid sequence of SEQ ID NO: 271; or
(11) the CD3-VH comprises the amino acid sequence of SEQ ID NO: 157, and the CD3-VL comprises the amino acid sequence of SEQ ID NO: 272; or
(12) the CD3-VH comprises the amino acid sequence of SEQ ID NO: 281, and the CD3-VL comprises the amino acid sequence of SEQ ID NO: 236; or
(13) the CD3-VH comprises the amino acid sequence of SEQ ID NO: 282, and the CD3-VL comprises the amino acid sequence of SEQ ID NO: 236; or
(14) the CD3-VH comprises the amino acid sequence of SEQ ID NO: 283, and the CD3-VL comprises the amino acid sequence of SEQ ID NO: 236; or
(15) the CD3-VH comprises the amino acid sequence of SEQ ID NO: 282, and the CD3-VL comprises the amino acid sequence of SEQ ID NO: 269.

In some embodiments, provided is the anti-BCMA/CD3 bispecific antibody according to any one of the foregoing, wherein the BCMA-VH comprises the amino acid sequence of SEQ ID NO: 383, and the BCMA-VL comprises the amino acid sequence of SEQ ID NO: 382.

In some embodiments, provided is the anti-BCMA/CD3 bispecific antibody according to any one of the foregoing, wherein:
(1) the CD3-VH comprises the amino acid sequence of SEQ ID NO: 255, and the CD3-VL comprises the amino acid sequence of SEQ ID NO: 236; and the BCMA-VH comprises the amino acid sequence of SEQ ID NO: 383, and the BCMA-VL comprises the amino acid sequence of SEQ ID NO: 382; or
(2) the CD3-VH comprises the amino acid sequence of SEQ ID NO: 255, and the CD3-VL comprises the amino acid sequence of SEQ ID NO: 237; and the BCMA-VH comprises the amino acid sequence of SEQ ID NO: 383, and the BCMA-VL comprises the amino acid sequence of SEQ ID NO: 382; or
(3) the CD3-VH comprises the amino acid sequence of SEQ ID NO: 255, and the CD3-VL comprises the amino acid sequence of SEQ ID NO: 238; and the BCMA-VH comprises the amino acid sequence of SEQ ID NO: 383, and the BCMA-VL comprises the amino acid sequence of SEQ ID NO: 382; or
(4) the CD3-VH comprises the amino acid sequence of SEQ ID NO: 278, and the CD3-VL comprises the amino acid sequence of SEQ ID NO: 268; and the BCMA-VH comprises the amino acid sequence of SEQ ID NO: 383, and the BCMA-VL comprises the amino acid sequence of SEQ ID NO: 382; or
(5) the CD3-VH comprises the amino acid sequence of SEQ ID NO: 275, and the CD3-VL comprises the amino acid sequence of SEQ ID NO: 269; and the BCMA-VH comprises the amino acid sequence of SEQ ID NO: 383, and the BCMA-VL comprises the amino acid sequence of SEQ ID NO: 382; or
(6) the CD3-VH comprises the amino acid sequence of SEQ ID NO: 278, and the CD3-VL comprises the amino acid sequence of SEQ ID NO: 269; and the BCMA-VH comprises the amino acid sequence of SEQ ID NO: 383, and the BCMA-VL comprises the amino acid sequence of SEQ ID NO: 382; or
(7) the CD3-VH comprises the amino acid sequence of SEQ ID NO: 279, and the CD3-VL comprises the amino acid sequence of SEQ ID NO: 269; and the BCMA-VH comprises the amino acid sequence of SEQ ID NO: 383, and the BCMA-VL comprises the amino acid sequence of SEQ ID NO: 382; or
(8) the CD3-VH comprises the amino acid sequence of SEQ ID NO: 157, and the CD3-VL comprises the amino acid sequence of SEQ ID NO: 236; and the BCMA-VH comprises the amino acid sequence of SEQ ID NO: 383, and the BCMA-VL comprises the amino acid sequence of SEQ ID NO: 382; or
(9) the CD3-VH comprises the amino acid sequence of SEQ ID NO: 275, and the CD3-VL comprises the amino acid sequence of SEQ ID NO: 268; and the BCMA-VH comprises the amino acid sequence of SEQ ID NO: 383, and the BCMA-VL comprises the amino acid sequence of SEQ ID NO: 382; or
(10) the CD3-VH comprises the amino acid sequence of SEQ ID NO: 157, and the CD3-VL comprises the amino acid sequence of SEQ ID NO: 271; and the BCMA-VH comprises the amino acid sequence of SEQ ID NO: 383, and the BCMA-VL comprises the amino acid sequence of SEQ ID NO: 382; or
(11) the CD3-VH comprises the amino acid sequence of SEQ ID NO: 157, and the CD3-VL comprises the amino acid sequence of SEQ ID NO: 272; and the BCMA-VH comprises the amino acid sequence of SEQ ID NO: 383, and the BCMA-VL comprises the amino acid sequence of SEQ ID NO: 382; or
(12) the CD3-VH comprises the amino acid sequence of SEQ ID NO: 281, and the CD3-VL comprises the amino acid sequence of SEQ ID NO: 236; and the BCMA-VH comprises the amino acid sequence of SEQ ID NO: 383, and the BCMA-VL comprises the amino acid sequence of SEQ ID NO: 382; or
(13) the CD3-VH comprises the amino acid sequence of SEQ ID NO: 282, and the CD3-VL comprises the amino acid sequence of SEQ ID NO: 236; and the BCMA-VH comprises the amino acid sequence of SEQ ID NO: 383, and the BCMA-VL comprises the amino acid sequence of SEQ ID NO: 382; or
(14) the CD3-VH comprises the amino acid sequence of SEQ ID NO: 283, and the CD3-VL comprises the amino acid sequence of SEQ ID NO: 236; and the BCMA-VH comprises the amino acid sequence of SEQ ID NO: 383, and the BCMA-VL comprises the amino acid sequence of SEQ ID NO: 382; or
(15) the CD3-VH comprises the amino acid sequence of SEQ ID NO: 282, and the CD3-VL comprises the amino acid sequence of SEQ ID NO: 269; and the BCMA-VH comprises the amino acid sequence of SEQ ID NO: 383, and the BCMA-VL comprises the amino acid sequence of SEQ ID NO: 382.

In some embodiments, provided is the anti-BCMA/CD3 bispecific antibody according to any one of the foregoing, wherein the anti-BCMA/CD3 bispecific antibody comprises a heavy chain constant region and a light chain constant region. In some embodiments, provided is the anti-BCMA/CD3 bispecific antibody according to any one of the foregoing, wherein the heavy chain constant region is a heavy chain constant region of human IgG1, IgG2, IgG3, or IgG4, or a variant thereof. In some embodiments, the heavy chain constant region of the anti-BCMA/CD3 bispecific antibody according to any one of the foregoing comprises an amino acid substitution of F405L or K409R. In some embodiments, provided is the anti-BCMA/CD3 bispecific antibody according to any one of the foregoing, wherein the light chain constant region is a light chain constant region of human lambda or kappa or a variant thereof. In some embodiments, provided is the anti-BCMA/CD3 bispecific antibody according to any one of the foregoing, wherein the second antigen-binding domain that specifically binds to BCMA comprises a heavy chain constant region and a light chain constant region, wherein the heavy chain constant region of the second antigen-binding domain that specifically binds to BCMA comprises the amino acid sequence of SEQ ID NO: 339, and the light chain constant region of the second antigen-binding domain that specifically binds to BCMA comprises the amino acid sequence of SEQ ID NO: 384. In some embodiments, provided is the anti-BCMA/CD3 bispecific antibody according to any one of the foregoing, wherein the first antigen-binding domain that specifically binds to CD3 comprises a heavy chain constant region and a light chain constant region, wherein the heavy chain constant region of the first antigen-binding domain that specifically binds to CD3 comprises the amino acid sequence of SEQ ID NO: 120, and the light chain constant region of the first antigen-binding domain that specifically binds to CD3 comprises the amino acid sequence of SEQ ID NO: 91.

In some embodiments, provided is the anti-BCMA/CD3 bispecific antibody according to any one of the foregoing, wherein the bispecific antibody comprises:
(1) one first chain comprising the amino acid sequence of SEQ ID NO: 386, one second chain comprising the amino acid sequence of SEQ ID NO: 385, one third chain comprising the amino acid sequence of SEQ ID NO: 303, and one fourth chain comprising the amino acid sequence of SEQ ID NO: 301; or
(2) one first chain comprising the amino acid sequence of SEQ ID NO: 386, one second chain comprising the amino acid sequence of SEQ ID NO: 385, one third chain comprising the amino acid sequence of SEQ ID NO: 304, and one fourth chain comprising the amino acid sequence of SEQ ID NO: 301; or
(3) one first chain comprising the amino acid sequence of SEQ ID NO: 386, one second chain comprising the amino acid sequence of SEQ ID NO: 385, one third chain comprising the amino acid sequence of SEQ ID NO: 305, and one fourth chain comprising the amino acid sequence of SEQ ID NO: 301; or
(4) one first chain comprising the amino acid sequence of SEQ ID NO: 386, one second chain comprising the amino acid sequence of SEQ ID NO: 385, one third chain comprising the amino acid sequence of SEQ ID NO: 319, and one fourth chain comprising the amino acid sequence of SEQ ID NO: 326; or
(5) one first chain comprising the amino acid sequence of SEQ ID NO: 386, one second chain comprising the amino acid sequence of SEQ ID NO: 385, one third chain comprising the amino acid sequence of SEQ ID NO: 320, and one fourth chain comprising the amino acid sequence of SEQ ID NO: 323; or
(6) one first chain comprising the amino acid sequence of SEQ ID NO: 386, one second chain comprising the amino acid sequence of SEQ ID NO: 385, one third chain comprising the amino acid sequence of SEQ ID NO: 320, and one fourth chain comprising the amino acid sequence of SEQ ID NO: 326; or
(7) one first chain comprising the amino acid sequence of SEQ ID NO: 386, one second chain comprising the amino acid sequence of SEQ ID NO: 385, one third chain comprising the amino acid sequence of SEQ ID NO: 320, and one fourth chain comprising the amino acid sequence of SEQ ID NO: 327; or
(8) one first chain comprising the amino acid sequence of SEQ ID NO: 386, one second chain comprising the amino acid sequence of SEQ ID NO: 385, one third chain comprising the amino acid sequence of SEQ ID NO: 303, and one fourth chain comprising the amino acid sequence of SEQ ID NO: 181; or
(9) one first chain comprising the amino acid sequence of SEQ ID NO: 386, one second chain comprising the amino acid sequence of SEQ ID NO: 385, one third chain comprising the amino acid sequence of SEQ ID NO: 319, and one fourth chain comprising the amino acid sequence of SEQ ID NO: 323; or
(10) one first chain comprising the amino acid sequence of SEQ ID NO: 386, one second chain comprising the amino acid sequence of SEQ ID NO: 385, one third chain comprising the amino acid sequence of SEQ ID NO: 329, and one fourth chain comprising the amino acid sequence of SEQ ID NO: 181; or
(11) one first chain comprising the amino acid sequence of SEQ ID NO: 386, one second chain comprising the amino acid sequence of SEQ ID NO: 385, one third chain comprising the amino acid sequence of SEQ ID NO: 330, and one fourth chain comprising the amino acid sequence of SEQ ID NO: 181; or
(12) one first chain comprising the amino acid sequence of SEQ ID NO: 386, one second chain comprising the amino acid sequence of SEQ ID NO: 385, one third chain comprising the amino acid sequence of SEQ ID NO: 303, and one fourth chain comprising the amino acid sequence of SEQ ID NO: 331; or
(13) one first chain comprising the amino acid sequence of SEQ ID NO: 386, one second chain comprising the amino acid sequence of SEQ ID NO: 385, one third chain comprising the amino acid sequence of SEQ ID NO: 303, and one fourth chain comprising the amino acid sequence of SEQ ID NO: 332; or
(14) one first chain comprising the amino acid sequence of SEQ ID NO: 386, one second chain comprising the amino acid sequence of SEQ ID NO: 385, one third chain comprising the amino acid sequence of SEQ ID NO: 303, and one fourth chain comprising the amino acid sequence of SEQ ID NO: 333; or
(15) one first chain comprising the amino acid sequence of SEQ ID NO: 386, one second chain comprising the amino acid sequence of SEQ ID NO: 385, one third chain comprising the amino acid sequence of SEQ ID NO: 320, and one fourth chain comprising the amino acid sequence of SEQ ID NO: 332.

In some embodiments, the bispecific antibody is an anti-PSMA/CD3 bispecific antibody. In some embodiments, the anti-PSMA/CD3 bispecific antibody according to the foregoing comprises a first antigen-binding domain that specifically binds to CD3 and a second antigen-binding domain that specifically binds to PSMA, wherein the first antigen-binding domain that specifically binds to CD3 comprises a heavy chain variable region CD3-VH and a light chain variable region CD3-VL, and the second antigen-binding domain that specifically binds to PSMA comprises a heavy chain variable region PSMA-VH and a light chain variable region PSMA-VL, wherein:
(1) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(2) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 183; or
(3) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 187, the CD3-LCDR2 comprises the amino acid sequence of SEQ ID NO: 2, and the CD3-LCDR3 comprises the amino acid sequence of SEQ ID NO: 183; or
(4) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(5) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(6) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(7) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 218, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(8) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(9) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 218, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(10) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 210, the CD3-LCDR2 comprises the amino acid sequence of SEQ ID NO: 2, and the CD3-LCDR3 comprises the amino acid sequence of SEQ ID NO: 185; or
(11) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 211, the CD3-LCDR2 comprises the amino acid sequence of SEQ ID NO: 2, and the CD3-LCDR3 comprises the amino acid sequence of SEQ ID NO: 185; or
(12) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 219, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(13) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(14) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 221, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(15) the CD3-VH comprises a CD3-HCDR1, a CD3-HCDR2, and a CD3-HCDR3, wherein the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-VL comprises a CD3-LCDR1, a CD3-LCDR2, and a CD3-LCDR3, wherein the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185.

In some embodiments, provided is the anti-PSMA/CD3 bispecific antibody according to any one of the foregoing, wherein the PSMA-VH comprises a PSMA-HCDR1, a PSMA-HCDR2, and a PSMA-HCDR3, wherein the PSMA-HCDR1 comprises the amino acid sequence of SEQ ID NO: 390, the PSMA-HCDR2 comprises the amino acid sequence of SEQ ID NO: 391, and the PSMA-HCDR3 comprises the amino acid sequence of SEQ ID NO: 392; and the PSMA-VL comprises a PSMA-LCDR1, a PSMA-LCDR2, and a PSMA-LCDR3, wherein the PSMA-LCDR1 comprises the amino acid sequence of SEQ ID NO: 387, the PSMA-LCDR2 comprises the amino acid sequence of SEQ ID NO: 388, and the PSMA-LCDR3 comprises the amino acid sequence of SEQ ID NO: 389.

In some embodiments, provided is the anti-PSMA/CD3 bispecific antibody according to any one of the foregoing, wherein:
(1) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(2) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 183; or
(3) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 187, the CD3-LCDR2 comprises the amino acid sequence of SEQ ID NO: 2, and the CD3-LCDR3 comprises the amino acid sequence of SEQ ID NO: 183; or
(4) the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(5) the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(6) the CD3-HCDR1 comprises the amino acid sequence of SEQ ID NO: 137, the CD3-HCDR2 comprises the amino acid sequence of SEQ ID NO: 217, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(7) the CD3-HCDR1 comprises the amino acid sequence of SEQ ID NO: 137, the CD3-HCDR2 comprises the amino acid sequence of SEQ ID NO: 218, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185; or
(8) the CD3-HCDR1 comprises the amino acid sequence of SEQ ID NO: 137, the CD3-HCDR2 comprises the amino acid sequence of SEQ ID NO: 214, and the CD3-HCDR3 comprises the amino acid sequence of SEQ ID NO: 36; and the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(9) the CD3-HCDR1 comprises the amino acid sequence of SEQ ID NO: 137, the CD3-HCDR2 comprises the amino acid sequence of SEQ ID NO: 218, and the CD3-HCDR3 comprises the amino acid sequence of SEQ ID NO: 36; and the CD3-LCDR1 comprises the amino acid sequence of SEQ ID NO: 207, the CD3-LCDR2 comprises the amino acid sequence of SEQ ID NO: 2, and the CD3-LCDR3 comprises the amino acid sequence of SEQ ID NO: 185; or
(10) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 210, the CD3-LCDR2 comprises the amino acid sequence of SEQ ID NO: 2, and the CD3-LCDR3 comprises the amino acid sequence of SEQ ID NO: 185; or
(11) the CD3-HCDR1 comprises the amino acid sequence of SEQ ID NO: 137, the CD3-HCDR2 comprises the amino acid sequence of SEQ ID NO: 138, and the CD3-HCDR3 comprises the amino acid sequence of SEQ ID NO: 36; and the CD3-LCDR1 comprises the amino acid sequence of SEQ ID NO: 211, the CD3-LCDR2 comprises the amino acid sequence of SEQ ID NO: 2, and the CD3-LCDR3 comprises the amino acid sequence of SEQ ID NO: 185; or
(12) the CD3-HCDR1 comprises the amino acid sequence of SEQ ID NO: 137, the CD3-HCDR2 comprises the amino acid sequence of SEQ ID NO: 219, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(13) the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(14) the CD3-HCDR1 comprises the amino acid sequence of SEQ ID NO: 137, the CD3-HCDR2 comprises the amino acid sequence of SEQ ID NO: 221, and the CD3-HCDR3 comprises the amino acid sequence of SEQ ID NO: 36; and the CD3-LCDR1 comprises the amino acid sequence of SEQ ID NO: 189, the CD3-LCDR2 comprises the amino acid sequence of SEQ ID NO: 2, and the CD3-LCDR3 comprises the amino acid sequence of SEQ ID NO: 185; or
(15) the CD3-HCDR1 comprises the amino acid sequence of SEQ ID NO: 137, the CD3-HCDR2 comprises the amino acid sequence of SEQ ID NO: 220, and the CD3-HCDR3 comprises the amino acid sequence of SEQ ID NO: 36; and the CD3-LCDR1 comprises the amino acid sequence of SEQ ID NO: 208, the CD3-LCDR2 comprises the amino acid sequence of SEQ ID NO: 2, and the CD3-LCDR3 comprises the amino acid sequence of SEQ ID NO: 185;
furthermore, the PSMA-HCDR1 comprises the amino acid sequence of SEQ ID NO: 390, the PSMA-HCDR2 comprises the amino acid sequence of SEQ ID NO: 391, and the PSMA-HCDR3 comprises the amino acid sequence of SEQ ID NO: 392; and the PSMA-LCDR1 comprises the amino acid sequence of SEQ ID NO: 387, the PSMA-LCDR2 comprises the amino acid sequence of SEQ ID NO: 388, and the PSMA-LCDR3 comprises the amino acid sequence of SEQ ID NO: 389.

In some embodiments, provided is the anti-PSMA/CD3 bispecific antibody according to any one of the foregoing, wherein:
(1) the CD3-VH comprises the amino acid sequence of SEQ ID NO: 255, and the CD3-VL comprises the amino acid sequence of SEQ ID NO: 236; or
(2) the CD3-VH comprises the amino acid sequence of SEQ ID NO: 255, and the CD3-VL comprises the amino acid sequence of SEQ ID NO: 237; or
(3) the CD3-VH comprises the amino acid sequence of SEQ ID NO: 255, and the CD3-VL comprises the amino acid sequence of SEQ ID NO: 238; or
(4) the CD3-VH comprises the amino acid sequence of SEQ ID NO: 278, and the CD3-VL comprises the amino acid sequence of SEQ ID NO: 268; or
(5) the CD3-VH comprises the amino acid sequence of SEQ ID NO: 275, and the CD3-VL comprises the amino acid sequence of SEQ ID NO: 269; or
(6) the CD3-VH comprises the amino acid sequence of SEQ ID NO: 278, and the CD3-VL comprises the amino acid sequence of SEQ ID NO: 269; or
(7) the CD3-VH comprises the amino acid sequence of SEQ ID NO: 279, and the CD3-VL comprises the amino acid sequence of SEQ ID NO: 269; or
(8) the CD3-VH comprises the amino acid sequence of SEQ ID NO: 157, and the CD3-VL comprises the amino acid sequence of SEQ ID NO: 236; or
(9) the CD3-VH comprises the amino acid sequence of SEQ ID NO: 275, and the CD3-VL comprises the amino acid sequence of SEQ ID NO: 268; or
(10) the CD3-VH comprises the amino acid sequence of SEQ ID NO: 279, and the CD3-VL comprises the amino acid sequence of SEQ ID NO: 268; or
(11) the CD3-VH comprises the amino acid sequence of SEQ ID NO: 157, and the CD3-VL comprises the amino acid sequence of SEQ ID NO: 271; or
(12) the CD3-VH comprises the amino acid sequence of SEQ ID NO: 157, and the CD3-VL comprises the amino acid sequence of SEQ ID NO: 272; or
(13) the CD3-VH comprises the amino acid sequence of SEQ ID NO: 281, and the CD3-VL comprises the amino acid sequence of SEQ ID NO: 236; or
(15) the CD3-VH comprises the amino acid sequence of SEQ ID NO: 282, and the CD3-VL comprises the amino acid sequence of SEQ ID NO: 236; or
(15) the CD3-VH comprises the amino acid sequence of SEQ ID NO: 283, and the CD3-VL comprises the amino acid sequence of SEQ ID NO: 236; or
(16) the CD3-VH comprises the amino acid sequence of SEQ ID NO: 282, and the CD3-VL comprises the amino acid sequence of SEQ ID NO: 269.

In some embodiments, provided is the anti-PSMA/CD3 bispecific antibody according to any one of the foregoing, wherein the PSMA-VH comprises the amino acid sequence of SEQ ID NO: 394, and the PSMA-VL comprises the amino acid sequence of SEQ ID NO: 393.

In some embodiments, provided is the anti-PSMA/CD3 bispecific antibody according to any one of the foregoing, wherein:
(1) the CD3-VH comprises the amino acid sequence of SEQ ID NO: 255, and the CD3-VL comprises the amino acid sequence of SEQ ID NO: 236; and the PSMA-VH comprises the amino acid sequence of SEQ ID NO: 394, and the PSMA-VL comprises the amino acid sequence of SEQ ID NO: 393; or
(2) the CD3-VH comprises the amino acid sequence of SEQ ID NO: 255, and the CD3-VL comprises the amino acid sequence of SEQ ID NO: 237; and the PSMA-VH comprises the amino acid sequence of SEQ ID NO: 394, and the PSMA-VL comprises the amino acid sequence of SEQ ID NO: 393; or
(3) the CD3-VH comprises the amino acid sequence of SEQ ID NO: 255, and the CD3-VL comprises the amino acid sequence of SEQ ID NO: 238; and the PSMA-VH comprises the amino acid sequence of SEQ ID NO: 394, and the PSMA-VL comprises the amino acid sequence of SEQ ID NO: 393; or
(4) the CD3-VH comprises the amino acid sequence of SEQ ID NO: 278, and the CD3-VL comprises the amino acid sequence of SEQ ID NO: 268; and the PSMA-VH comprises the amino acid sequence of SEQ ID NO: 394, and the PSMA-VL comprises the amino acid sequence of SEQ ID NO: 393; or
(5) the CD3-VH comprises the amino acid sequence of SEQ ID NO: 275, and the CD3-VL comprises the amino acid sequence of SEQ ID NO: 269; and the PSMA-VH comprises the amino acid sequence of SEQ ID NO: 394, and the PSMA-VL comprises the amino acid sequence of SEQ ID NO: 393; or
(6) the CD3-VH comprises the amino acid sequence of SEQ ID NO: 278, and the CD3-VL comprises the amino acid sequence of SEQ ID NO: 269; and the PSMA-VH comprises the amino acid sequence of SEQ ID NO: 394, and the PSMA-VL comprises the amino acid sequence of SEQ ID NO: 393; or
(7) the CD3-VH comprises the amino acid sequence of SEQ ID NO: 279, and the CD3-VL comprises the amino acid sequence of SEQ ID NO: 269; and the PSMA-VH comprises the amino acid sequence of SEQ ID NO: 394, and the PSMA-VL comprises the amino acid sequence of SEQ ID NO: 393; or
(8) the CD3-VH comprises the amino acid sequence of SEQ ID NO: 157, and the CD3-VL comprises the amino acid sequence of SEQ ID NO: 236; and the PSMA-VH comprises the amino acid sequence of SEQ ID NO: 394, and the PSMA-VL comprises the amino acid sequence of SEQ ID NO: 393; or
(9) the CD3-VH comprises the amino acid sequence of SEQ ID NO: 275, and the CD3-VL comprises the amino acid sequence of SEQ ID NO: 268; and the PSMA-VH comprises the amino acid sequence of SEQ ID NO: 394, and the PSMA-VL comprises the amino acid sequence of SEQ ID NO: 393; or
(10) the CD3-VH comprises the amino acid sequence of SEQ ID NO: 279, and the CD3-VL comprises the amino acid sequence of SEQ ID NO: 268; and the PSMA-VH comprises the amino acid sequence of SEQ ID NO: 394, and the PSMA-VL comprises the amino acid sequence of SEQ ID NO: 393; or
(11) the CD3-VH comprises the amino acid sequence of SEQ ID NO: 157, and the CD3-VL comprises the amino acid sequence of SEQ ID NO: 271; and the PSMA-VH comprises the amino acid sequence of SEQ ID NO: 394, and the PSMA-VL comprises the amino acid sequence of SEQ ID NO: 393; or
(12) the CD3-VH comprises the amino acid sequence of SEQ ID NO: 157, and the CD3-VL comprises the amino acid sequence of SEQ ID NO: 272; and the PSMA-VH comprises the amino acid sequence of SEQ ID NO: 394, and the PSMA-VL comprises the amino acid sequence of SEQ ID NO: 393; or
(13) the CD3-VH comprises the amino acid sequence of SEQ ID NO: 281, and the CD3-VL comprises the amino acid sequence of SEQ ID NO: 236; and the PSMA-VH comprises the amino acid sequence of SEQ ID NO: 394, and the PSMA-VL comprises the amino acid sequence of SEQ ID NO: 393; or
(15) the CD3-VH comprises the amino acid sequence of SEQ ID NO: 282, and the CD3-VL comprises the amino acid sequence of SEQ ID NO: 236; and the PSMA-VH comprises the amino acid sequence of SEQ ID NO: 394, and the PSMA-VL comprises the amino acid sequence of SEQ ID NO: 393; or
(15) the CD3-VH comprises the amino acid sequence of SEQ ID NO: 283, and the CD3-VL comprises the amino acid sequence of SEQ ID NO: 236; and the PSMA-VH comprises the amino acid sequence of SEQ ID NO: 394, and the PSMA-VL comprises the amino acid sequence of SEQ ID NO: 393; or
(16) the CD3-VH comprises the amino acid sequence of SEQ ID NO: 282, and the CD3-VL comprises the amino acid sequence of SEQ ID NO: 269; and the PSMA-VH comprises the amino acid sequence of SEQ ID NO: 394, and the PSMA-VL comprises the amino acid sequence of SEQ ID NO: 393.

In some embodiments, provided is the anti-PSMA/CD3 bispecific antibody according to any one of the foregoing, wherein the anti-PSMA/CD3 bispecific antibody comprises a heavy chain constant region and a light chain constant region. In some embodiments, provided is the anti-PSMA/CD3 bispecific antibody according to any one of the foregoing, wherein the heavy chain constant region is a heavy chain constant region of human IgG1, IgG2, IgG3, or IgG4, or a variant thereof. In some embodiments, the heavy chain constant region of the anti-PSMA/CD3 bispecific antibody according to any one of the foregoing comprises an amino acid substitution of F405L or K409R. In some embodiments, provided is the anti-PSMA/CD3 bispecific antibody according to any one of the foregoing, wherein the light chain constant region is a light chain constant region of human lambda or kappa or a variant thereof. In some embodiments, provided is the anti-PSMA/CD3 bispecific antibody according to any one of the foregoing, wherein the second antigen-binding domain that specifically binds to PSMA comprises a heavy chain constant region and a light chain constant region, wherein the heavy chain constant region of the second antigen-binding domain that specifically binds to PSMA comprises the amino acid sequence of SEQ ID NO: 399, and the light chain constant region of the second antigen-binding domain that specifically binds to PSMA comprises the amino acid sequence of SEQ ID NO: 91. In some embodiments, provided is the anti-PSMA/CD3 bispecific antibody according to any one of the foregoing, wherein the first antigen-binding domain that specifically binds to CD3 comprises a heavy chain constant region and a light chain constant region, wherein the heavy chain constant region of the first antigen-binding domain that specifically binds to CD3 comprises the amino acid sequence of SEQ ID NO: 120, and the light chain constant region of the first antigen-binding domain that specifically binds to CD3 comprises the amino acid sequence of SEQ ID NO: 91.

In some embodiments, provided is the anti-PSMA/CD3 bispecific antibody according to any one of the foregoing, wherein the bispecific antibody comprises:
(1) one first chain comprising the amino acid sequence of SEQ ID NO: 395, one second chain comprising the amino acid sequence of SEQ ID NO: 396, one third chain comprising the amino acid sequence of SEQ ID NO: 303, and one fourth chain comprising the amino acid sequence of SEQ ID NO: 301; or
(2) one first chain comprising the amino acid sequence of SEQ ID NO: 395, one second chain comprising the amino acid sequence of SEQ ID NO: 396, one third chain comprising the amino acid sequence of SEQ ID NO: 304, and one fourth chain comprising the amino acid sequence of SEQ ID NO: 301; or
(3) one first chain comprising the amino acid sequence of SEQ ID NO: 395, one second chain comprising the amino acid sequence of SEQ ID NO: 396, one third chain comprising the amino acid sequence of SEQ ID NO: 305, and one fourth chain comprising the amino acid sequence of SEQ ID NO: 301; or
(4) one first chain comprising the amino acid sequence of SEQ ID NO: 395, one second chain comprising the amino acid sequence of SEQ ID NO: 396, one third chain comprising the amino acid sequence of SEQ ID NO: 319, and one fourth chain comprising the amino acid sequence of SEQ ID NO: 326; or
(5) one first chain comprising the amino acid sequence of SEQ ID NO: 395, one second chain comprising the amino acid sequence of SEQ ID NO: 396, one third chain comprising the amino acid sequence of SEQ ID NO: 320, and one fourth chain comprising the amino acid sequence of SEQ ID NO: 323; or
(6) one first chain comprising the amino acid sequence of SEQ ID NO: 395, one second chain comprising the amino acid sequence of SEQ ID NO: 396, one third chain comprising the amino acid sequence of SEQ ID NO: 320, and one fourth chain comprising the amino acid sequence of SEQ ID NO: 326; or
(7) one first chain comprising the amino acid sequence of SEQ ID NO: 395, one second chain comprising the amino acid sequence of SEQ ID NO: 396, one third chain comprising the amino acid sequence of SEQ ID NO: 320, and one fourth chain comprising the amino acid sequence of SEQ ID NO: 327; or
(8) one first chain comprising the amino acid sequence of SEQ ID NO: 395, one second chain comprising the amino acid sequence of SEQ ID NO: 396, one third chain comprising the amino acid sequence of SEQ ID NO: 303, and one fourth chain comprising the amino acid sequence of SEQ ID NO: 181; or
(9) one first chain comprising the amino acid sequence of SEQ ID NO: 395, one second chain comprising the amino acid sequence of SEQ ID NO: 396, one third chain comprising the amino acid sequence of SEQ ID NO: 319, and one fourth chain comprising the amino acid sequence of SEQ ID NO: 323; or
(10) one first chain comprising the amino acid sequence of SEQ ID NO: 395, one second chain comprising the amino acid sequence of SEQ ID NO: 396, one third chain comprising the amino acid sequence of SEQ ID NO: 319, and one fourth chain comprising the amino acid sequence of SEQ ID NO: 327; or
(11) one first chain comprising the amino acid sequence of SEQ ID NO: 395, one second chain comprising the amino acid sequence of SEQ ID NO: 396, one third chain comprising the amino acid sequence of SEQ ID NO: 329, and one fourth chain comprising the amino acid sequence of SEQ ID NO: 181; or
(12) one first chain comprising the amino acid sequence of SEQ ID NO: 395, one second chain comprising the amino acid sequence of SEQ ID NO: 396, one third chain comprising the amino acid sequence of SEQ ID NO: 330, and one fourth chain comprising the amino acid sequence of SEQ ID NO: 181; or
(13) one first chain comprising the amino acid sequence of SEQ ID NO: 395, one second chain comprising the amino acid sequence of SEQ ID NO: 396, one third chain comprising the amino acid sequence of SEQ ID NO: 303, and one fourth chain comprising the amino acid sequence of SEQ ID NO: 331; or
(14) one first chain comprising the amino acid sequence of SEQ ID NO: 395, one second chain comprising the amino acid sequence of SEQ ID NO: 396, one third chain comprising the amino acid sequence of SEQ ID NO: 303, and one fourth chain comprising the amino acid sequence of SEQ ID NO: 332; or
(15) one first chain comprising the amino acid sequence of SEQ ID NO: 395, one second chain comprising the amino acid sequence of SEQ ID NO: 396, one third chain comprising the amino acid sequence of SEQ ID NO: 303, and one fourth chain comprising the amino acid sequence of SEQ ID NO: 333; or
(16) one first chain comprising the amino acid sequence of SEQ ID NO: 395, one second chain comprising the amino acid sequence of SEQ ID NO: 396, one third chain comprising the amino acid sequence of SEQ ID NO: 320, and one fourth chain comprising the amino acid sequence of SEQ ID NO: 332.

In another aspect, the present disclosure provides a pharmaceutical composition comprising a therapeutically effective amount of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, and one or more pharmaceutically acceptable carriers, diluents, or excipients. In some embodiments, the pharmaceutical composition according to any one of the foregoing is used for activating T cells. In some embodiments, the pharmaceutical composition according to any one of the foregoing is used for treating a tumor, an autoimmune disease, or an inflammatory disease.

In some embodiments, based on the total weight of the composition, the pharmaceutical composition comprises 0.01%-99.99% of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing. In some embodiments, the pharmaceutical composition comprises 0.1%-99.9% of the anti-CD3 antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing. In some embodiments, the pharmaceutical composition comprises 0.5%-99.5% of the anti-CD3 antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing. In some embodiments, the pharmaceutical composition comprises 1%-99% of the anti-CD3 antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing. In some embodiments, the pharmaceutical composition comprises 2%-98% of the anti-CD3 antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing.

In some embodiments, based on the total weight of the composition, the pharmaceutical composition comprises 0.01%-99.99% of the pharmaceutically acceptable excipient. In some embodiments, the pharmaceutical composition comprises 0.1%-99.9% of the pharmaceutically acceptable excipient. In some embodiments, the pharmaceutical composition comprises 0.5%-99.5% of the pharmaceutically acceptable excipient. In some embodiments, the pharmaceutical composition comprises 1%-99% of the pharmaceutically acceptable excipient. In some embodiments, the pharmaceutical composition comprises 2%-98% of the pharmaceutically acceptable excipient.

In another aspect, the present disclosure provides an isolated nucleic acid encoding the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing.

In another aspect, the present disclosure provides a vector comprising the nucleic acid molecule according to any one of the foregoing.

In another aspect, the present disclosure provides a host cell comprising the isolated nucleic acid according to any one of the foregoing or the vector according to any one of the foregoing.

In another aspect, the present disclosure provides a method for producing the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, comprising a step of culturing the host cell according to any one of the foregoing in a culture medium to form and accumulate the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, and recovering the antibody, or the multispecific antibody, or the anti-CD20/CD3 bispecific antibody, or the anti-BCMA/CD3 bispecific antibody, or the anti-PSMA/CD3 bispecific antibody from the culture.

In another aspect, the present disclosure provides the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing, for use as a medicament. In some embodiments, the medicament is a medicament for activating T cells. In some embodiments, the medicament is a medicament for treating, preventing, or ameliorating a disease or disorder.

In another aspect, the present disclosure provides use of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing, in the manufacture of a medicament for activating T cells.

In another aspect, the present disclosure provides use of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing, in the manufacture of a medicament for treating, preventing, or ameliorating a disease or disorder.

In another aspect, the present disclosure provides a method for activating T cells, comprising administering to a subject a therapeutically effective amount of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing. In some embodiments, the method comprises administering to a subject a unit dose of a composition comprising 0.1-3000 mg (more preferably 1-1000 mg) of the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing.

In another aspect, the present disclosure provides a method for treating, preventing, or ameliorating a disease or disorder, comprising administering to a subject a therapeutically, prophylactically, or amelioratively effective amount of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing.

In some embodiments, the method comprises administering to a subject a unit dose of a composition comprising 0.1-3000 mg (more preferably 1-1000 mg) of the anti-CD3 antibody according to any one of the foregoing, or the multispecific antibody according to any one of the foregoing, or the anti-CD20/CD3 bispecific antibody according to any one of the foregoing, or the anti-BCMA/CD3 bispecific antibody according to any one of the foregoing, or the anti-PSMA/CD3 bispecific antibody according to any one of the foregoing, or the pharmaceutical composition according to any one of the foregoing, or the isolated nucleic acid molecule according to any one of the foregoing, or the vector according to any one of the foregoing, or the host cell according to any one of the foregoing.

In some embodiments, the disease or disorder is a disease or disorder associated with CD3. In some embodiments, the disease or disorder is a tumor, an autoimmune disease, or an inflammatory disease.

In some embodiments, the tumor according to any one of the foregoing is selected from the group consisting of a solid tumor (including advanced solid tumors) and a hematological tumor. In some embodiments, the tumor according to any one of the foregoing is selected from the group consisting of lymphoma, blastoma, sarcoma, and leukemia. In some embodiments, the tumor according to any one of the foregoing is selected from the group consisting of Hodgkin lymphoma and non-Hodgkin lymphoma. More specific examples of the tumor include squamous cell carcinoma, myeloma, small-cell lung cancer, non-small cell lung cancer (NSCLC), head and neck squamous cell carcinoma (HNSCC), neuroglioma, Hodgkin lymphoma, non-Hodgkin lymphoma, diffuse large B-cell lymphoma (DLBCL), follicular lymphoma, cutaneous T-cell lymphoma, non-Hodgkin lymphoma,, acute lymphoblastic leukemia (ALL), acute myeloid leukemia (AML), acute myelogenous leukemia, chronic lymphocytic leukemia (CLL) (e.g., B-cell chronic lymphocytic leukemia), chronic myeloid leukemia (CML), primary mediastinal large B-cell lymphoma, diffuse large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal (tract) cancer (gastric cancer), renal cancer, ovarian cancer, liver cancer, lymphoblastic leukemia, lymphocytic leukemia, colorectal cancer (including colon cancer and rectal cancer), endometrial cancer, renal cancer, prostate cancer, thyroid cancer, melanoma, chondrosarcoma, neuroblastoma, pancreatic cancer, glioblastoma multiforme, gastric cancer, bone cancer, Ewing sarcoma, cervical cancer, brain cancer, bladder cancer, hepatoma, breast cancer, colon cancer, hepatocellular carcinoma (HCC), clear cell renal cell carcinoma (RCC), head and neck cancer, throat cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative neoplasms, neuroendocrine neoplasms, Merkel cell carcinoma, testicular cancer, and skin cancer. In some embodiments, the tumor is breast cancer, ovarian cancer, prostate cancer, pancreatic cancer, renal cancer, lung cancer, liver cancer, gastric cancer, colon cancer, bladder cancer, esophageal cancer, cervical cancer, gallbladder cancer, glioblastoma, and melanoma.

In some embodiments, the autoimmune disease or inflammatory disease according to any one of the foregoing is selected from the group consisting of: rheumatoid arthritis, psoriasis, psoriasis, psoriatic arthritis, Crohn's disease, ankylosing spondylitis, multiple sclerosis, type I diabetes (type I insulin-dependent diabetes mellitus), hepatitis (autoimmune hepatitis), myocarditis, Sjogren's syndrome, autoimmune hemolytic anemia after transplant rejection, bullous pemphigoid, Graves' disease, Hashimoto's thyroiditis, systemic lupus erythematosus (SLE), myasthenia gravis, pemphigus, pernicious anemia, inflammatory bowel disease, myasthenia gravis, celiac disease, asthma, allergic rhinitis (seasonal allergic rhinitis), effects from organ transplantation (organ transplant rejection), graft-versus-host disease (GVHD), chronic urticaria, and sepsis.

The present disclosure provides a novel anti-CD3 antibody that is capable of simultaneously binding to a human or cynomolgus monkey CD3 antigen and further reducing T cell recognition epitopes of the anti-CD3 antibody by engineering, thereby providing a new therapeutic potential for the fields of diseases such as autoimmune diseases and tumors. The anti-CD3 antibody of the present disclosure is capable of binding to a human or cynomolgus monkey CD3 antigen, particularly to human or cynomolgus monkey CD3ε and a heterodimer thereof. Therefore, a primate, cynomolgus monkey, can be selected for preclinical safety and toxicology assessment, facilitating preclinical pharmacology and toxicology studies. The anti-CD3 antibody of the present disclosure has excellent biological activity, and is capable of effectively activating downstream signaling pathways in T cells to exert the T cell activity regulation function. In addition, the anti-CD3 antibody of the present disclosure has fewer T cell recognition epitopes, further reducing the risk of immunogenicity.

### BRIEF DESCRIPTION OF THE DRAWINGS

FIG. 1A shows the T cell activation effects of CD20/CD3 bispecific antibodies, CD20-82L2H3, CD20-82L3H3, CD20-82L4H3, CD20-82L8H4, and CD20-82L8H5, in the presence of Jeko-1 cells.
FIG. 1B shows the T cell activation effects of CD20/CD3 bispecific antibodies, CD20-82L5H1, CD20-82L5H2, CD20-82L5H3, and CD20-82L6H2, in the presence of Jeko-1 cells.
FIG. 1C shows the T cell activation effect of a CD20/CD3 bispecific antibody, CD20-82L6H3, CD20-82L9H4, CD20-82L10H4, or CD20L10H5, in the presence of Jeko-1 cells.
FIG. 2A shows the killing effects of CD20/CD3 bispecific antibodies, CD20-82L2H3, CD20-82L3H3, CD20-82L4H3, CD20-82L8H4, and CD20-82L8H5, on Daudi-luc tumor cells.
FIG. 2B shows the killing effects of CD20/CD3 bispecific antibodies, CD20-82L5H1, CD20-82L5H2, CD20-82L5H3, and CD20-82L6H2, on Daudi-luc tumor cells.
FIG. 2C shows the killing effects of CD20/CD3 bispecific antibodies, CD20-82L6H3, CD20-82L9H4, CD20-82L10H4, and CD20-82L10H5, on Daudi-luc tumor cells.
FIG. 3A shows the killing effects of BCMA/CD3 bispecific antibodies, BCMA-82L8_S27e P,L94V/H7, BCMA-82L8_S27e P,L94V,T28S/H7_I57T, and BCMA-82L8_S27e P,L94V,R29Q/H7_I57T, on NCI-H929 tumor cells.
FIG. 3B shows the killing effects of BCMA/CD3 bispecific antibodies, BCMA-82L8_S27e P,L94V,R29Q/H7_N52Q, BCMA-82L8_S27e P,L94V,L27bV,R29Q/H7, and BCMA-82L8_S27e P,L94V/H7_I57T,K58S, on NCI-H929 tumor cells.
FIG. 3C shows the killing effects of BCMA/CD3 bispecific antibodies, BCMA-82L8_S27e P,L94V,T28S/H7_N52Q and BCMA-82L8_S27e P,L94V,R29Q/H7_K58S, on NCI-H929 tumor cells.
FIG. 3D shows the killing effects of BCMA/CD3 bispecific antibodies, BCMA-82L8_S27e P,L94V,L27b V,T28S/H7, BCMA-82L8_S27e P,L94V/H7_N52Q,I57T, and BCMA-82L8_S27e P,L94V/H7_N52Q,K58S, on NCI-H929 tumor cells.
FIG. 3E shows the killing effects of BCMA/CD3 bispecific antibodies, BCMA-82L8_S27e P,L94V/H5_I34M, BCMA-82L8_S27e P,F92Y/H5_I34M, and BCMA-82L8_S27e E,F92Y/H5_I34M, on NCI-H929 tumor cells.
FIG. 3F shows the killing effect of a BCMA/CD3 bispecific antibody, BCMA-82L8_S27e P,L94V,R29Q/H7_N52Q,K58S, on NCI-H929 tumor cells.
FIG. 4A shows the killing effects of PSMA/CD3 bispecific antibodies, PSMA-82L8_S27e P,L94V/H7, PSMA-82L8_S27e P,L94V,T28S/H7_I57T, and PSMA-82L8_S27e P,L94V,R29Q/H7_I57T, on LnCAP tumor cells.
FIG. 4B shows the killing effects of PSMA/CD3 bispecific antibodies, PSMA-82L8_S27e P,L94V,R29Q/H7_N52Q and PSMA-82L8_S27e P,L94V/H7_I57T,K58S, on LnCAP tumor cells.
FIG. 4C shows the killing effects of PSMA/CD3 bispecific antibodies, PSMA-82L8_S27e P,L94V,R29Q/H7_K58S, PSMA-82L8_S27e P,F92Y/H5_I34M, and PSMA-82L8_S27e E,F92Y/H5_I34M, on LnCAP tumor cells.
FIG. 4D shows the killing effects of PSMA/CD3 bispecific antibodies, PSMA-82L8_S27e P,L94V/H5_I34M and PSMA-82L8_S27e P,L94V/H7_N52Q,K58S, on LnCAP tumor cells.
FIG. 4E shows the killing effects of PSMA/CD3 bispecific antibodies, PSMA-82L8_S27e P,L94V,T28S/H7_N52Q and PSMA-82L8_S27e P,L94V,T28S/H7_K58S, on LnCAP tumor cells.
FIG. 4F shows the killing effects of PSMA/CD3 bispecific antibodies, PSMA-82L8_S27e P,L94V,L27b V,R29Q/H7 and PSMA-82L8_S27e P,L94V/H7_N52Q,I57T, on LnCAP tumor cells.
FIG. 4G shows the killing effect of a PSMA/CD3 bispecific antibody, PSMA-82L8_S27e P,L94V,R29Q/H7_N52Q,K58S, on LnCAP tumor cells.
FIG. 4H shows the killing effect of a PSMA/CD3 bispecific antibody, PSMA-82L8_S27e P,L94V,L27b V,T28S/H7, on LnCAP tumor cells.

### DETAILED DESCRIPTION

### Terminology

To facilitate the understanding of the present disclosure, certain technical and scientific terms are described below. Unless otherwise specifically defined herein, all technical and scientific terms used herein have the same meaning as commonly understood by those of ordinary skill in the art.

The singular forms "a", "an", and "the" used in the description and claims include plural reference unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

The term "and/or" is meant to include the two meanings "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used in the present disclosure are as described in J. Biol. Chem., 243, p3558 (1968).

The term "CD3" refers to an antigen expressed on a T cell as part of a multi-molecular T cell receptor (TCR), and it consists of a homodimer or heterodimer formed from two of the following four receptor chains: CD3-ε (CD3E or CD3 epsilon), CD3-δ (CD3D), CD3-ζ, and CD3-γ. Human CD3-ε (hCD3ε) comprises the amino acid sequence described in UniProtKB/Swiss-Prot: P07766.2. Human CD3-δ (hCD3δ) comprises the amino acid sequence described in UniProtKB/Swiss-Prot: P04234.1. Thus, the term "CD3" refers to human CD3 unless it is explicitly indicated to be from a non-human species, such as "mouse CD3" or "monkey CD3".

The term "amino acid" refers to naturally occurring and synthetic amino acids, as well as amino acid analogs and amino acid mimics that function similarly to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have an identical basic chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain an identical basic chemical structure to naturally occurring amino acids. Amino acid mimics refer to chemical compounds that have a structure different from the general chemical structure of amino acids, but function similarly to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the amino acid residue at a particular position may be denoted by position + amino acid residue; for example, 107S means that the amino acid residue at position 107 is S. T107S means that the amino acid residue at position 107 is mutated from T to S. When the residue at a characteristic position is defined with "position + amino acid residue" in a claim, the original residue at that position does not limit the scope of protection.

The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments, and antigen-binding fragments (or antigen-binding moieties), as long as they exhibit the desired antigen-binding activity.

The term "natural antibody" refers to a naturally occurring immunoglobulin molecule. For example, a natural IgG antibody is a heterotetrameric glycoprotein of about 150,000 Daltons composed of two identical light chains and two identical heavy chains linked by a disulfide bond. From the N-terminus to the C-terminus, each heavy chain comprises one variable region (VH), also known as variable heavy domain or heavy chain variable region, followed by heavy chain constant regions. Generally, a natural IgG heavy chain constant region comprises three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL).

The terms "full-length antibody", "intact antibody", and "whole antibody" are used herein interchangeably and refer to an antibody having a structure substantially similar to a natural antibody structure or comprising a heavy chain with an Fc region as defined herein. In a native intact antibody, the light chain comprises light chain variable region VL and constant region CL, the VL being positioned at the amino terminus of the light chain, and the light chain constant region comprises a κ chain and a λ chain; the heavy chain comprises a variable region VH and constant regions (CH1, CH2, and CH3), the VH being positioned at the amino terminus of the heavy chain, and the constant regions being positioned at the carboxyl terminus, wherein CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The boundaries of the amino acid sequences of CDRs can be determined by a variety of well-known schemes, for example, the "Kabat" numbering scheme (see Kabat et al., (1991), Sequences of Proteins of Immunological Interest, 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), etc. The corresponding relationships between the various numbering schemes are well known to those skilled in the art and, illustratively, are shown in Table 1 below.

**Table 1. The relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure. Although the Kabat numbering scheme is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that binds to an antigen to which the intact antibody binds. Examples of antibody fragments include, but are not limited to, Fv, dsFv, Fab, Fab', Fab'-SH, Fd, F(ab')2, single-domain antibodies (sdAbs, e.g., VH, VL, VHH, or V_{HH}), single-chain Fab (scFab), diabodies, linear antibodies, single-chain antibodies (e.g., scFv and sc(Fv)2), and multispecific antibodies formed from antibody fragments.

The term "antigen-binding fragment" encompasses a full-length antibody, a Fab, a modified Fab, a Fab', a Fab'-SH, a modified Fab', a F(ab')2, an Fv, a dsFv, a Fab-Fv, a Fab-dsFv, an Fd, a single-domain antibody (sdAb, e.g., VH or VL or VHH), a single-chain Fab (scFab), a single-chain antibody (e.g., scFv and sc(Fv)2), a diabody, a linear antibody, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9): 1126-1136; Adair and Lawson, 2005, Drug Design Reviews-Online 2(3), 209-217). Methods for producing and preparing such antigen-binding fragments are well known in the art (see, e.g., Verma et al., 1998, Journal of Immunological Methods, 216, 165-181).

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of an Fc region may also vary; for example, the C-terminal lysine of the Fc region (residue 447 according to the EU numbering system) is deleted, or the C-terminal glycine and lysine of the Fc region (residues 446 and 447 according to the EU numbering system) are deleted. Unless otherwise stated, the numbering scheme for the Fc region is the EU numbering system, also known as the EU index.

The term "chimeric" antibody refers to an antibody in which a portion of the heavy and/or light chain is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The terms "human antibody", "humanized antibody", "fully human antibody", and "complete human antibody" are used interchangeably and refer to antibodies in which the variable regions and constant regions are human sequences. The term encompasses antibodies that are derived from human genes but have, for example, sequences that have been altered to, e.g., reduce possible immunogenicity, increase affinity, and eliminate cysteines or glycosylation sites that may cause undesired folding. The term encompasses antibodies recombinantly produced in non-human cells (that may confer glycosylation not characteristic of human cells). The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "CD3-VH" refers to a heavy chain variable region of an antigen-binding domain that specifically binds to CD3. The term "CD3-VL" refers to a light chain variable region of an antigen-binding domain that specifically binds to CD3. The term "CD3-HCDR1" refers to a HCDR1 of an antigen-binding domain that specifically binds to CD3. The term "CD3-HCDR2" refers to a HCDR2 of an antigen-binding domain that specifically binds to CD3. The term "CD3-HCDR3" refers to a HCDR3 of an antigen-binding domain that specifically binds to CD3. The term "CD3-LCDR1" refers to a LCDR1 of an antigen-binding domain that specifically binds to CD3. The term "CD3-LCDR2" refers to a LCDR2 of an antigen-binding domain that specifically binds to CD3. The term "CD3-LCDR3" refers to a LCDR3 of an antigen-binding domain that specifically binds to CD3. The term "CD20-VH" refers to a heavy chain variable region of an antigen-binding domain that specifically binds to CD20. The term "CD20-VL" refers to a light chain variable region of an antigen-binding domain that specifically binds to CD20. The term "CD20-HCDR1" refers to a HCDR1 of an antigen-binding domain that specifically binds to CD20. The term "CD20-HCDR2" refers to a HCDR2 of an antigen-binding domain that specifically binds to CD20. The term "CD20-HCDR3" refers to a HCDR3 of an antigen-binding domain that specifically binds to CD20. The term "CD20-LCDR1" refers to a LCDR1 of an antigen-binding domain that specifically binds to CD20. The term "CD20-LCDR2" refers to a LCDR2 of an antigen-binding domain that specifically binds to CD20. The term "CD20-LCDR3" refers to a LCDR3 of an antigen-binding domain that specifically binds to CD20.

The term "BCMA-VH" refers to a heavy chain variable region of an antigen-binding domain that specifically binds to BCMA. The term "BCMA-VL" refers to a light chain variable region of an antigen-binding domain that specifically binds to BCMA. The term "BCMA-HCDR1" refers to a HCDR1 of an antigen-binding domain that specifically binds to BCMA. The term "BCMA-HCDR2" refers to a HCDR2 of an antigen-binding domain that specifically binds to BCMA. The term "BCMA-HCDR3" refers to a HCDR3 of an antigen-binding domain that specifically binds to BCMA. The term "BCMA-LCDR1" refers to a LCDR1 of an antigen-binding domain that specifically binds to BCMA. The term "BCMA-LCDR2" refers to a LCDR2 of an antigen-binding domain that specifically binds to BCMA. The term "BCMA-LCDR3" refers to a LCDR3 of an antigen-binding domain that specifically binds to BCMA.

The term "PSMA-VH" refers to a heavy chain variable region of an antigen-binding domain that specifically binds to PSMA. The term "PSMA-VL" refers to a light chain variable region of an antigen-binding domain that specifically binds to PSMA. The term "PSMA-HCDR1" refers to a HCDR1 of an antigen-binding domain that specifically binds to PSMA. The term "PSMA-HCDR2" refers to a HCDR2 of an antigen-binding domain that specifically binds to PSMA. The term "PSMA-HCDR3" refers to a HCDR3 of an antigen-binding domain that specifically binds to PSMA. The term "PSMA-LCDR1" refers to a LCDR1 of an antigen-binding domain that specifically binds to PSMA. The term "PSMA-LCDR2" refers to a LCDR2 of an antigen-binding domain that specifically binds to PSMA. The term "PSMA-LCDR3" refers to a LCDR3 of an antigen-binding domain that specifically binds to PSMA.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1:1 interaction between members of a binding pair (e.g., an antibody and an antigen). The affinity of molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is calculated as the ratio of kd to ka (i.e., kd/ka) and expressed as a molar concentration (M). The KD value of an antibody can be determined using methods well known in the art. For example, surface plasmon resonance is measured using a biosensing system such as a system (e.g., Biacore), or the affinity in a solution is measured by solution equilibrium titration (SET).

The term "surface plasmon resonance" refers to an optical phenomenon that allows for the analysis of real-time interactions by detecting changes in protein concentrations within a biosensor matrix, for example, using the BIAcoreTM system (Biacore LifeSciences division of GE Healthcare, Piscataway, NJ).

The term "effector function" refers to biological activities that can be attributed to the Fc region of an antibody (a natural sequence Fc region or an amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, a polyclonal antibody formulation generally comprises several different antibodies comprising different amino acid sequences in their variable domains, which are generally specific for different epitopes. "Monoclonal" refers to the characteristics of an antibody obtained from a substantially homogeneous antibody population and should not be construed as requiring the production of the antibody by any particular method. In some embodiments, the antibody provided by the present disclosure is a monoclonal antibody.

The term "antigen" refers to a molecule or portion of a molecule that is capable of being bound by a selective binding agent, such as an antigen-binding protein (including, for example, an antibody), and that is also capable of being used in an animal to produce an antibody that is capable of binding to the antigen. An antigen may have one or more epitopes capable of interacting with different antigen-binding proteins (e.g., antibodies). The term "epitope" refers to an area or region on an antigen that is capable of specifically binding to an antibody or an antigen-binding fragment thereof. Epitopes can be formed from contiguous strings of amino acids (linear epitopes) or comprise non-contiguous amino acids (conformational epitopes), e.g., coming in spatial proximity due to the folding of the antigen (i.e., by the tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically bind", or "bind" means that an antibody is capable of binding to a certain antigen or an epitope of the antigen with higher affinity than to other antigens or epitopes. In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE^{®} surface plasmon resonance assay. However, an antibody that specifically binds to an antigen or an epitope in the antigen may have cross-reactivity with other related antigens, for example, with corresponding antigens from other species (homologous) (e.g., humans or monkeys, such as Macaca fascicularis (cynomolgus, cyno), Pan troglodytes (chimpanzee, chimp), or Callithrix jacchus (commonmarmoset, marmoset)).

The term "no binding" means that an antibody is not capable of binding to a certain antigen or an epitope thereof in the specific binding manner described above.

The terms "anti-CD3 antibody" and "antibody that binds to CD3" refer to an antibody that is capable of binding to CD3 with sufficient affinity.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcγ receptor (FcγR) expressed on the effector cells. For example, NK cells express FcγRIIIa, while monocytes express FcγRI, FcγRII, and FcγRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis (ADCP)" refers to a mechanism by which antibody-coated target cells are eliminated by internalization of phagocytic cells (such as macrophages or dendritic cells).

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. The activation of a complement may also result in the deposition of complement components on the surface of target cells, and these complement components promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized similarly to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule contained in a cell that generally comprises the nucleic acid molecule, but the nucleic acid molecule is present extrachromosomally or at a chromosomal position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron. The terms "host cell", "host cell line", and "host cell culture" are used interchangeably and refer to cells into which exogenous nucleic acids have been introduced, including the progeny of such cells. Host cells include "transformants" and "transformed cells", which include primary transformed cells and progeny derived therefrom, regardless of the number of passages. Progeny may not be completely identical to parent cells in terms of nucleic acid content and may contain mutations. Mutant progeny that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichia pastoris*, *Pichia finlandica*, *Pichia trehalophila*, *Pichia koclamae*, *Pichia membranaefaciens*, *Pichia minuta* (*Ogataea minuta*, *Pichia lindneri*), *Pichia opuntiae*, *Pichia thermotolerans*, *Pichia salictaria*, *Pichia guercuum*, *Pichia pijperi*, *Pichia stiptis*, *Pichia methanolica*, *Pichia*, *Saccharomyces cerevisiae*, *Saccharomyces*, *Hansenula polymorpha*, *Kluyveromyces*, *Kluyveromyces lactis*, *Candida albicans*, *Aspergillus*, *Aspergillus nidulans*, *Aspergillus niger*, *Aspergillus oryzae*, *Trichoderma reesei*, *Chrysosporium lucknowense*, *Fusarium sp.*, *Fusarium gramineum*, *Fusarium venenatum*, *Physcomitrella patens*, and *Neurospora crassa*.

As used in the present application, the expressions "cell", "cell line", and "cell culture" are used interchangeably, and all such designations include their progeny. Thus, the words "transformant" and "transformed cell" include the primary subject cell and cultures derived therefrom, regardless of the number of passages. It is also understood that not all progeny have precisely identical DNA contents due to deliberate or inadvertent mutations. Variant progeny that have the same function or biological activity as the original transformed cell are included.

"Optional" or "optionally" means that the event or circumstance subsequently described may, but does not necessarily, occur. This description includes the instance where the event or circumstance occurs or does not occur.

The term "pharmaceutical composition" refers to a mixture comprising one or more of the anti-CD3 antibodies described herein and other chemical components; the other components are, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless indicated, the terms "patient" and "subject" are used interchangeably herein. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells, or tissues isolated from a subject, as well as fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, palliating symptoms, palliating/reducing any direct or indirect pathological consequences of the disease, preventing metastasis, decreasing the rate of disease progression, ameliorating or palliating the disease state, and achieving regression or improved prognosis. In some embodiments, an antibody of the present disclosure is used to delay the development of or slow the progression of a disease.

The term "recurrence", "relapse", or "relapsed" refers to the return of a cancer or disease after clinical assessment of the disappearance of disease. A diagnosis of distant metastasis or local recurrence can be considered a relapse.

The term "refractory" or "resistant" refers to a cancer or disease that has not responded to treatment.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

### Anti-CD3 Antibodies of the Present Disclosure

The present disclosure provides anti-CD3 antibodies having a number of advantageous properties, such as *in vitro* T cell activation ability, killing ability, good therapeutic activity, safety, pharmacokinetic properties, and druggability (e.g., solubility, viscosity, purity, and stability).

### Exemplary Anti-CD3 Antibodies

Examples of the present disclosure disclose antibody series 82#, 12#, 8#, 21#, 29#, 31#, 57#, 61#, 63#, 80#, 89#, 92-2#, 93#, 96#, 103#, 104#, 105#, and 106#. Antibody 82# is described below as an example of the antibodies of the present disclosure.

Illustratively, provided is an anti-CD3 antibody of the present disclosure, wherein the anti-CD3 antibody comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein: the heavy chain variable region comprises a HCDR1 whose amino acid sequence is set forth in SEQ ID NO: 343, a HCDR2 whose amino acid sequence is set forth in SEQ ID NO: 344, and a HCDR3 whose amino acid sequence is set forth in SEQ ID NO: 345, and the light chain variable region comprises a LCDR whose amino acid sequence is set forth in SEQ ID NO: 346, a LCDR2 whose amino acid sequence is set forth in SEQ ID NO: 347, and a LCDR3 whose amino acid sequence is set forth in SEQ ID NO: 348, wherein:

| | |
|---|---|
| HCDR1: SX₁YX₂H | SEQ ID NO: 343 |
| HCDR2: WIX₃PGX₄X₅X₆X₇X₈YNEKFKG | SEQ ID NO: 344 |
| HCDR3: DGYSYYYFDX₉ | SEQ ID NO: 345 |
| LCDR1: X₁₀SSQSX₁₁LX₁₂X₁₃RX₁₄X₁₅X₁₆NYLA | SEQ ID NO: 346 |
| LCDR2: X₁₇ASTRES | SEQ ID NO: 347 |
| LCDR3: KQX₁₈X₁₉IX₂₀RT | SEQ ID NO: 348 |

wherein X₁ is selected from the group consisting of Y, E, K, and Q, preferably Y; X₂ is selected from the group consisting of M, I, E, N, and Q, preferably M; X₃ is selected from the group consisting of N, Q, and S, preferably N or Q; X₄ is selected from the group consisting of E and D, preferably E; X₅ is selected from the group consisting of G and D, preferably G; X₆ is selected from the group consisting of S and A, preferably S; X₇ is selected from the group consisting of I and T; X₈ is selected from the group consisting of K and S; X₉ is selected from the group consisting of F and Y, preferably F; X₁₀ is selected from the group consisting of K and T, preferably K; X₁₁ is selected from the group consisting of L and V; X₁₂ is selected from the group consisting of N and Q, preferably N; X₁₃ is selected from the group consisting of E, P, S, D, K, Q, R, and W, preferably E or P; X₁₄ is selected from the group consisting of T and S; X₁₅ is selected from the group consisting of R and Q; X₁₆ is selected from the group consisting of K and Q, preferably K; X₁₇ is selected from the group consisting of W and Y, preferably W; X₁₈ is selected from the group consisting of S and N, preferably S; X₁₉ is selected from the group consisting of F and Y; X₂₀ is selected from the group consisting of L, V, and I, preferably L or V.

Illustratively, provided is an anti-CD3 antibody of the present disclosure, wherein:
the heavy chain variable region comprises a HCDR1 whose amino acid sequence is set forth in SEQ ID NO: 137, a HCDR2 whose amino acid sequence is set forth in SEQ ID NO: 403, and a HCDR3 whose amino acid sequence is set forth in SEQ ID NO: 36, and the light chain variable region comprises a LCDR1 whose amino acid sequence is set forth in SEQ ID NO: 404, a LCDR2 whose amino acid sequence is set forth in SEQ ID NO: 2, and a LCDR3 whose amino acid sequence is set forth in SEQ ID NO: 405;
wherein:

| | |
|---|---|
| HCDR2: WIX₂₅PGEGSX₂₆X₂₇YNEKFKG | SEQ ID NO: 403 |
| LCDR1: KSSQSX₂₈LNX₂₉RX₃₀X₃₁KNYLA | SEQ ID NO: 404 |
| LCDR3: KQSX₃₂IX₃₃RT | SEQ ID NO: 405 |

wherein X₂₅ is selected from the group consisting of N and Q; X₂₆ is selected from the group consisting of I and T; X₂₇ is selected from the group consisting of K and S; X₂₈ is selected from the group consisting of L and V; X₂₉ is selected from the group consisting of P and E; X₃₀ is selected from the group consisting of T and S; X₃₁ is selected from the group consisting of R and Q; X₃₂ is selected from the group consisting of F and Y; X₃₃ is selected from the group consisting of V and L.

In some embodiments, provided is the anti-CD3 antibody according to the foregoing, wherein:
(1) if X₂₅ is N, X₂₆ is I, and X₂₇ is K, then X₂₈ is L, X₂₉ is P, X₃₀ is T, X₃₁ is R, X₃₂ is F, and X₃₃ is V; or
(2) if X₂₅ is N, X₂₆ is I, and X₂₇ is K, then X₂₈ is L, X₂₉ is P, X₃₀ is T, X₃₁ is R, X₃₂ is Y, and X₃₃ is L; or
(3) if X₂₅ is N, X₂₆ is I, and X₂₇ is K, then X₂₈ is L, X₂₉ is E, X₃₀ is T, X₃₁ is R, X₃₂ is Y, and X₃₃ is L; or
(4) if X₂₅ is N, X₂₆ is T, and X₂₇ is K, then X₂₈ is L, X₂₉ is P, X₃₀ is S, X₃₁ is R, X₃₂ is F, and X₃₃ is V; or
(5) if X₂₅ is Q, X₂₆ is I, and X₂₇ is K, then X₂₈ is L, X₂₉ is P, X₃₀ is T, X₃₁ is Q, X₃₂ is F, and X₃₃ is V; or
(6) if X₂₅ is N, X₂₆ is T, and X₂₇ is K, then X₂₈ is L, X₂₉ is P, X₃₀ is T, X₃₁ is Q, X₃₂ is F, and X₃₃ is V; or
(7) if X₂₅ is N, X₂₆ is I, and X₂₇ is S, then X₂₈ is L, X₂₉ is P, X₃₀ is T, X₃₁ is Q, X₃₂ is F, and X₃₃ is V; or
(8) if X₂₅ is Q, X₂₆ is I, and X₂₇ is K, then X₂₈ is L, X₂₉ is P, X₃₀ is S, X₃₁ is R, X₃₂ is F, and X₃₃ is V; or
(9) if X₂₅ is N, X₂₆ is I, and X₂₇ is S, then X₂₈ is L, X₂₉ is P, X₃₀ is S, X₃₁ is R, X₃₂ is F, and X₃₃ is V; or
(10) if X₂₅ is N, X₂₆ is I, and X₂₇ is K, then X₂₈ is V, X₂₉ is P, X₃₀ is S, X₃₁ is R, X₃₂ is F, and X₃₃ is V; or
(11) if X₂₅ is N, X₂₆ is I, and X₂₇ is K, then X₂₈ is V, X₂₉ is P, X₃₀ is T, X₃₁ is Q, X₃₂ is F, and X₃₃ is V; or
(12) if X₂₅ is Q, X₂₆ is T, and X₂₇ is K, then X₂₈ is L, X₂₉ is P, X₃₀ is T, X₃₁ is R, X₃₂ is F, and X₃₃ is V; or
(13) if X₂₅ is Q, X₂₆ is I, and X₂₇ is S, then X₂₈ is L, X₂₉ is P, X₃₀ is T, X₃₁ is R, X₃₂ is F, and X₃₃ is V; or
(14) if X₂₅ is N, X₂₆ is T, and X₂₇ is S, then X₂₈ is L, X₂₉ is P, X₃₀ is T, X₃₁ is R, X₃₂ is F, and X₃₃ is V; or
(15) if X₂₅ is Q, X₂₆ is I, and X₂₇ is S, then X₂₈ is V, X₂₉ is P, X₃₀ is T, X₃₁ is R, X₃₂ is F, and X₃₃ is V; or
(16) if X₂₅ is Q, X₂₆ is I, and X₂₇ is S, then X₂₈ is L, X₂₉ is P, X₃₀ is S, X₃₁ is R, X₃₂ is F, and X₃₃ is V; or
(17) if X₂₅ is Q, X₂₆ is I, and X₂₇ is S, then X₂₈ is L, X₂₉ is P, X₃₀ is T, X₃₁ is Q, X₃₂ is F, and X₃₃ is V.

Illustratively, provided is an anti-CD3 antibody of the present disclosure, wherein the anti-CD3 antibody comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
(1) the heavy chain variable region comprises a HCDR1 whose amino acid sequence is set forth in SEQ ID NO: 137, a HCDR2 whose amino acid sequence is set forth in SEQ ID NO: 138, and a HCDR3 whose amino acid sequence is set forth in SEQ ID NO: 36, and the light chain variable region comprises a LCDR1 whose amino acid sequence is set forth in SEQ ID NO: 189, a LCDR2 whose amino acid sequence is set forth in SEQ ID NO: 2, and a LCDR3 whose amino acid sequence is set forth in SEQ ID NO: 185; or
(2) the heavy chain variable region comprises a HCDR1 whose amino acid sequence is set forth in SEQ ID NO: 137, a HCDR2 whose amino acid sequence is set forth in SEQ ID NO: 138, and a HCDR3 whose amino acid sequence is set forth in SEQ ID NO: 36, and the light chain variable region comprises a LCDR1 whose amino acid sequence is set forth in SEQ ID NO: 189, a LCDR2 whose amino acid sequence is set forth in SEQ ID NO: 2, and a LCDR3 whose amino acid sequence is set forth in SEQ ID NO: 183; or
(3) the heavy chain variable region comprises a HCDR1 whose amino acid sequence is set forth in SEQ ID NO: 137, a HCDR2 whose amino acid sequence is set forth in SEQ ID NO: 138, and a HCDR3 whose amino acid sequence is set forth in SEQ ID NO: 36, and the light chain variable region comprises a LCDR1 whose amino acid sequence is set forth in SEQ ID NO: 187, a LCDR2 whose amino acid sequence is set forth in SEQ ID NO: 2, and a LCDR3 whose amino acid sequence is set forth in SEQ ID NO: 183; or
(4) the heavy chain variable region comprises a HCDR1 whose amino acid sequence is set forth in SEQ ID NO: 137, a HCDR2 whose amino acid sequence is set forth in SEQ ID NO: 217, and a HCDR3 whose amino acid sequence is set forth in SEQ ID NO: 36, and the light chain variable region comprises a LCDR1 whose amino acid sequence is set forth in SEQ ID NO: 207, a LCDR2 whose amino acid sequence is set forth in SEQ ID NO: 2, and a LCDR3 whose amino acid sequence is set forth in SEQ ID NO: 185; or
(5) the heavy chain variable region comprises a HCDR1 whose amino acid sequence is set forth in SEQ ID NO: 137, a HCDR2 whose amino acid sequence is set forth in SEQ ID NO: 214, and a HCDR3 whose amino acid sequence is set forth in SEQ ID NO: 36, and the light chain variable region comprises a LCDR1 whose amino acid sequence is set forth in SEQ ID NO: 208, a LCDR2 whose amino acid sequence is set forth in SEQ ID NO: 2, and a LCDR3 whose amino acid sequence is set forth in SEQ ID NO: 185; or
(6) the heavy chain variable region comprises a HCDR1 whose amino acid sequence is set forth in SEQ ID NO: 137, a HCDR2 whose amino acid sequence is set forth in SEQ ID NO: 217, and a HCDR3 whose amino acid sequence is set forth in SEQ ID NO: 36, and the light chain variable region comprises a LCDR1 whose amino acid sequence is set forth in SEQ ID NO: 208, a LCDR2 whose amino acid sequence is set forth in SEQ ID NO: 2, and a LCDR3 whose amino acid sequence is set forth in SEQ ID NO: 185; or
(7) the heavy chain variable region comprises a HCDR1 whose amino acid sequence is set forth in SEQ ID NO: 137, a HCDR2 whose amino acid sequence is set forth in SEQ ID NO: 218, and a HCDR3 whose amino acid sequence is set forth in SEQ ID NO: 36, and the light chain variable region comprises a LCDR1 whose amino acid sequence is set forth in SEQ ID NO: 208, a LCDR2 whose amino acid sequence is set forth in SEQ ID NO: 2, and a LCDR3 whose amino acid sequence is set forth in SEQ ID NO: 185; or
(8) the heavy chain variable region comprises a HCDR1 whose amino acid sequence is set forth in SEQ ID NO: 137, a HCDR2 whose amino acid sequence is set forth in SEQ ID NO: 214, and a HCDR3 whose amino acid sequence is set forth in SEQ ID NO: 36, and the light chain variable region comprises a LCDR1 whose amino acid sequence is set forth in SEQ ID NO: 207, a LCDR2 whose amino acid sequence is set forth in SEQ ID NO: 2, and a LCDR3 whose amino acid sequence is set forth in SEQ ID NO: 185; or
(9) the heavy chain variable region comprises a HCDR1 whose amino acid sequence is set forth in SEQ ID NO: 137, a HCDR2 whose amino acid sequence is set forth in SEQ ID NO: 218, and a HCDR3 whose amino acid sequence is set forth in SEQ ID NO: 36, and the light chain variable region comprises a LCDR1 whose amino acid sequence is set forth in SEQ ID NO: 207, a LCDR2 whose amino acid sequence is set forth in SEQ ID NO: 2, and a LCDR3 whose amino acid sequence is set forth in SEQ ID NO: 185; or
(10) the heavy chain variable region comprises a HCDR1 whose amino acid sequence is set forth in SEQ ID NO: 137, a HCDR2 whose amino acid sequence is set forth in SEQ ID NO: 138, and a HCDR3 whose amino acid sequence is set forth in SEQ ID NO: 36, and the light chain variable region comprises a LCDR1 whose amino acid sequence is set forth in SEQ ID NO: 210, a LCDR2 whose amino acid sequence is set forth in SEQ ID NO: 2, and a LCDR3 whose amino acid sequence is set forth in SEQ ID NO: 185; or
(11) the heavy chain variable region comprises a HCDR1 whose amino acid sequence is set forth in SEQ ID NO: 137, a HCDR2 whose amino acid sequence is set forth in SEQ ID NO: 138, and a HCDR3 whose amino acid sequence is set forth in SEQ ID NO: 36, and the light chain variable region comprises a LCDR1 whose amino acid sequence is set forth in SEQ ID NO: 211, a LCDR2 whose amino acid sequence is set forth in SEQ ID NO: 2, and a LCDR3 whose amino acid sequence is set forth in SEQ ID NO: 185; or
(12) the heavy chain variable region comprises a HCDR1 whose amino acid sequence is set forth in SEQ ID NO: 137, a HCDR2 whose amino acid sequence is set forth in SEQ ID NO: 219, and a HCDR3 whose amino acid sequence is set forth in SEQ ID NO: 36, and the light chain variable region comprises a LCDR1 whose amino acid sequence is set forth in SEQ ID NO: 189, a LCDR2 whose amino acid sequence is set forth in SEQ ID NO: 2, and a LCDR3 whose amino acid sequence is set forth in SEQ ID NO: 185; or
(13) the heavy chain variable region comprises a HCDR1 whose amino acid sequence is set forth in SEQ ID NO: 137, a HCDR2 whose amino acid sequence is set forth in SEQ ID NO: 220, and a HCDR3 whose amino acid sequence is set forth in SEQ ID NO: 36, and the light chain variable region comprises a LCDR1 whose amino acid sequence is set forth in SEQ ID NO: 189, a LCDR2 whose amino acid sequence is set forth in SEQ ID NO: 2, and a LCDR3 whose amino acid sequence is set forth in SEQ ID NO: 185; or
(14) the heavy chain variable region comprises a HCDR1 whose amino acid sequence is set forth in SEQ ID NO: 137, a HCDR2 whose amino acid sequence is set forth in SEQ ID NO: 221, and a HCDR3 whose amino acid sequence is set forth in SEQ ID NO: 36, and the light chain variable region comprises a LCDR1 whose amino acid sequence is set forth in SEQ ID NO: 189, a LCDR2 whose amino acid sequence is set forth in SEQ ID NO: 2, and a LCDR3 whose amino acid sequence is set forth in SEQ ID NO: 185; or
(15) the heavy chain variable region comprises a HCDR1 whose amino acid sequence is set forth in SEQ ID NO: 137, a HCDR2 whose amino acid sequence is set forth in SEQ ID NO: 220, and a HCDR3 whose amino acid sequence is set forth in SEQ ID NO: 36, and the light chain variable region comprises a LCDR1 whose amino acid sequence is set forth in SEQ ID NO: 206, a LCDR2 whose amino acid sequence is set forth in SEQ ID NO: 2, and a LCDR3 whose amino acid sequence is set forth in SEQ ID NO: 185; or
(16) the heavy chain variable region comprises a HCDR1 whose amino acid sequence is set forth in SEQ ID NO: 137, a HCDR2 whose amino acid sequence is set forth in SEQ ID NO: 220, and a HCDR3 whose amino acid sequence is set forth in SEQ ID NO: 36, and the light chain variable region comprises a LCDR1 whose amino acid sequence is set forth in SEQ ID NO: 207, a LCDR2 whose amino acid sequence is set forth in SEQ ID NO: 2, and a LCDR3 whose amino acid sequence is set forth in SEQ ID NO: 185; or
(17) the heavy chain variable region comprises a HCDR1 whose amino acid sequence is set forth in SEQ ID NO: 137, a HCDR2 whose amino acid sequence is set forth in SEQ ID NO: 220, and a HCDR3 whose amino acid sequence is set forth in SEQ ID NO: 36, and the light chain variable region comprises a LCDR1 whose amino acid sequence is set forth in SEQ ID NO: 208, a LCDR2 whose amino acid sequence is set forth in SEQ ID NO: 2, and a LCDR3 whose amino acid sequence is set forth in SEQ ID NO: 185.

Illustratively, provided is an anti-CD3 antibody of the present disclosure, wherein the HCDR1, the HCDR2, and the HCDR3 of the heavy chain variable region and the LCDR1, the LCDR2, and the LCDR3 of the light chain variable region are defined according to the Kabat numbering scheme.

Illustratively, provided is an anti-CD3 antibody of the present disclosure, wherein the anti-CD3 antibody is a murine-derived antibody, a chimeric antibody, a humanized antibody, or a fully human-derived antibody. In some embodiments, the anti-CD3 antibody is a chimeric antibody. In some embodiments, the anti-CD3 antibody is a humanized antibody.

Illustratively, provided is an anti-CD3 antibody of the present disclosure, wherein the anti-CD3 antibody comprises a heavy chain variable region and a light chain variable region, wherein:
(1) the amino acid sequence of the heavy chain variable region is set forth in SEQ ID NO: 255, and the amino acid sequence of the light chain variable region is set forth in SEQ ID NO: 236; or
   the amino acid sequence of the heavy chain variable region is set forth in SEQ ID NO: 157, and the amino acid sequence of the light chain variable region is set forth in SEQ ID NO: 236; or
(2) the amino acid sequence of the heavy chain variable region is set forth in SEQ ID NO: 255, and the amino acid sequence of the light chain variable region is set forth in SEQ ID NO: 237; or
(3) the amino acid sequence of the heavy chain variable region is set forth in SEQ ID NO: 255, and the amino acid sequence of the light chain variable region is set forth in SEQ ID NO: 238; or
(4) the amino acid sequence of the heavy chain variable region is set forth in SEQ ID NO: 278, and the amino acid sequence of the light chain variable region is set forth in SEQ ID NO: 268; or
(5) the amino acid sequence of the heavy chain variable region is set forth in SEQ ID NO: 275, and the amino acid sequence of the light chain variable region is set forth in SEQ ID NO: 269; or
(6) the amino acid sequence of the heavy chain variable region is set forth in SEQ ID NO: 278, and the amino acid sequence of the light chain variable region is set forth in SEQ ID NO: 269; or
(7) the amino acid sequence of the heavy chain variable region is set forth in SEQ ID NO: 279, and the amino acid sequence of the light chain variable region is set forth in SEQ ID NO: 269; or
(8) the amino acid sequence of the heavy chain variable region is set forth in SEQ ID NO: 275, and the amino acid sequence of the light chain variable region is set forth in SEQ ID NO: 268; or
(9) the amino acid sequence of the heavy chain variable region is set forth in SEQ ID NO: 279, and the amino acid sequence of the light chain variable region is set forth in SEQ ID NO: 268; or
(10) the amino acid sequence of the heavy chain variable region is set forth in SEQ ID NO: 157, and the amino acid sequence of the light chain variable region is set forth in SEQ ID NO: 271; or
(11) the amino acid sequence of the heavy chain variable region is set forth in SEQ ID NO: 157, and the amino acid sequence of the light chain variable region is set forth in SEQ ID NO: 272; or
(12) the amino acid sequence of the heavy chain variable region is set forth in SEQ ID NO: 281, and the amino acid sequence of the light chain variable region is set forth in SEQ ID NO: 236; or
(13) the amino acid sequence of the heavy chain variable region is set forth in SEQ ID NO: 282, and the amino acid sequence of the light chain variable region is set forth in SEQ ID NO: 236; or
(14) the amino acid sequence of the heavy chain variable region is set forth in SEQ ID NO: 283, and the amino acid sequence of the light chain variable region is set forth in SEQ ID NO: 236; or
(15) the amino acid sequence of the heavy chain variable region is set forth in SEQ ID NO: 282, and the amino acid sequence of the light chain variable region is set forth in SEQ ID NO: 267; or
(16) the amino acid sequence of the heavy chain variable region is set forth in SEQ ID NO: 282, and the amino acid sequence of the light chain variable region is set forth in SEQ ID NO: 268; or
(17) the amino acid sequence of the heavy chain variable region is set forth in SEQ ID NO: 282, and the amino acid sequence of the light chain variable region is set forth in SEQ ID NO: 269.

Illustratively, provided is an anti-CD3 antibody of the present disclosure, wherein the anti-CD3 antibody is an antigen-binding fragment; in some embodiments, the antigen-binding fragment is selected from the group consisting of a Fab, Fab', F(ab')2, Fd, Fv, scFv, dsFv, and dAb.

Illustratively, provided is an anti-CD3 antibody of the present disclosure, wherein the anti-CD3 antibody comprises a heavy chain constant region and a light chain constant region. In some embodiments, the heavy chain constant region is a human IgG1 heavy chain constant region or a variant thereof. In some embodiments, the light chain constant region is a human kappa light chain constant region or a variant thereof. In some embodiments, the heavy chain constant region comprises the amino acid sequence of SEQ ID NO: 120, 90, 339, or 399, and the light chain constant region comprises the amino acid sequence of SEQ ID NO: 91 or 384. In some embodiments, the heavy chain constant region comprises the amino acid sequence of SEQ ID NO: 120, and the light chain constant region comprises the amino acid sequence of SEQ ID NO: 91.

Illustratively, provided is an anti-CD3 antibody of the present disclosure, wherein the anti-CD3 antibody comprises a heavy chain and a light chain, wherein:
(1) the amino acid sequence of the heavy chain is set forth in SEQ ID NO: 301, and the amino acid sequence of the light chain is set forth in SEQ ID NO: 303; or the amino acid sequence of the heavy chain is set forth in SEQ ID NO: 181, and the amino acid sequence of the light chain is set forth in SEQ ID NO: 303; or
(2) the amino acid sequence of the heavy chain is set forth in SEQ ID NO: 301, and the amino acid sequence of the light chain is set forth in SEQ ID NO: 304; or
(3) the amino acid sequence of the heavy chain is set forth in SEQ ID NO: 301, and the amino acid sequence of the light chain is set forth in SEQ ID NO: 305; or
(4) the amino acid sequence of the heavy chain is set forth in SEQ ID NO: 326, and the amino acid sequence of the light chain is set forth in SEQ ID NO: 319; or
(5) the amino acid sequence of the heavy chain is set forth in SEQ ID NO: 323, and the amino acid sequence of the light chain is set forth in SEQ ID NO: 320; or
(6) the amino acid sequence of the heavy chain is set forth in SEQ ID NO: 326, and the amino acid sequence of the light chain is set forth in SEQ ID NO: 320; or
(7) the amino acid sequence of the heavy chain is set forth in SEQ ID NO: 327, and the amino acid sequence of the light chain is set forth in SEQ ID NO: 320; or
(8) the amino acid sequence of the heavy chain is set forth in SEQ ID NO: 323, and the amino acid sequence of the light chain is set forth in SEQ ID NO: 319; or
(9) the amino acid sequence of the heavy chain is set forth in SEQ ID NO: 327, and the amino acid sequence of the light chain is set forth in SEQ ID NO: 319; or
(10) the amino acid sequence of the heavy chain is set forth in SEQ ID NO: 181, and the amino acid sequence of the light chain is set forth in SEQ ID NO: 329; or
(11) the amino acid sequence of the heavy chain is set forth in SEQ ID NO: 181, and the amino acid sequence of the light chain is set forth in SEQ ID NO: 330; or
(12) the amino acid sequence of the heavy chain is set forth in SEQ ID NO: 331, and the amino acid sequence of the light chain is set forth in SEQ ID NO: 303; or
(13) the amino acid sequence of the heavy chain is set forth in SEQ ID NO: 332, and the amino acid sequence of the light chain is set forth in SEQ ID NO: 303; or
(14) the amino acid sequence of the heavy chain is set forth in SEQ ID NO: 333, and the amino acid sequence of the light chain is set forth in SEQ ID NO: 303; or
(15) the amino acid sequence of the heavy chain is set forth in SEQ ID NO: 332, and the amino acid sequence of the light chain is set forth in SEQ ID NO: 318; or
(16) the amino acid sequence of the heavy chain is set forth in SEQ ID NO: 332, and the amino acid sequence of the light chain is set forth in SEQ ID NO: 319; or
(17) the amino acid sequence of the heavy chain is set forth in SEQ ID NO: 332, and the amino acid sequence of the light chain is set forth in SEQ ID NO: 320.

Illustratively, provided is an anti-CD3 antibody of the present disclosure, wherein the anti-CD3 antibody comprises a heavy chain and a light chain, wherein:
(1) the amino acid sequence of the heavy chain consists of amino acids at positions 1-448 in SEQ ID NO: 301, and the amino acid sequence of the light chain consists of amino acids at positions 1-219 in SEQ ID NO: 303; or
   the amino acid sequence of the heavy chain consists of amino acids at positions 1-448 in SEQ ID NO: 181, and the amino acid sequence of the light chain consists of amino acids at positions 1-219 in SEQ ID NO: 303; or
(2) the amino acid sequence of the heavy chain consists of amino acids at positions 1-448 in SEQ ID NO: 301, and the amino acid sequence of the light chain consists of amino acids at positions 1-219 in SEQ ID NO: 304; or
(3) the amino acid sequence of the heavy chain consists of amino acids at positions 1-448 in SEQ ID NO: 301, and the amino acid sequence of the light chain consists of amino acids at positions 1-219 in SEQ ID NO: 305; or
(4) the amino acid sequence of the heavy chain consists of amino acids at positions 1-448 in SEQ ID NO: 326, and the amino acid sequence of the light chain consists of amino acids at positions 1-219 in SEQ ID NO: 319; or
(5) the amino acid sequence of the heavy chain consists of amino acids at positions 1-448 in SEQ ID NO: 323, and the amino acid sequence of the light chain consists of amino acids at positions 1-219 in SEQ ID NO: 320; or
(6) the amino acid sequence of the heavy chain consists of amino acids at positions 1-448 in SEQ ID NO: 326, and the amino acid sequence of the light chain consists of amino acids at positions 1-219 in SEQ ID NO: 320; or
(7) the amino acid sequence of the heavy chain consists of amino acids at positions 1-448 in SEQ ID NO: 327, and the amino acid sequence of the light chain consists of amino acids at positions 1-219 in SEQ ID NO: 320; or
(8) the amino acid sequence of the heavy chain consists of amino acids at positions 1-448 in SEQ ID NO: 323, and the amino acid sequence of the light chain consists of amino acids at positions 1-219 in SEQ ID NO: 319; or
(9) the amino acid sequence of the heavy chain consists of amino acids at positions 1-448 in SEQ ID NO: 327, and the amino acid sequence of the light chain consists of amino acids at positions 1-219 in SEQ ID NO: 319; or
(10) the amino acid sequence of the heavy chain consists of amino acids at positions 1-448 in SEQ ID NO: 181, and the amino acid sequence of the light chain consists of amino acids at positions 1-219 in SEQ ID NO: 329; or
(11) the amino acid sequence of the heavy chain consists of amino acids at positions 1-448 in SEQ ID NO: 181, and the amino acid sequence of the light chain consists of amino acids at positions 1-219 in SEQ ID NO: 330; or
(12) the amino acid sequence of the heavy chain consists of amino acids at positions 1-448 in SEQ ID NO: 331, and the amino acid sequence of the light chain consists of amino acids at positions 1-219 in SEQ ID NO: 303; or
(13) the amino acid sequence of the heavy chain consists of amino acids at positions 1-448 in SEQ ID NO: 332, and the amino acid sequence of the light chain consists of amino acids at positions 1-219 in SEQ ID NO: 303; or
(14) the amino acid sequence of the heavy chain consists of amino acids at positions 1-448 in SEQ ID NO: 333, and the amino acid sequence of the light chain consists of amino acids at positions 1-219 in SEQ ID NO: 303; or
(15) the amino acid sequence of the heavy chain consists of amino acids at positions 1-448 in SEQ ID NO: 332, and the amino acid sequence of the light chain consists of amino acids at positions 1-219 in SEQ ID NO: 318; or
(16) the amino acid sequence of the heavy chain consists of amino acids at positions 1-448 in SEQ ID NO: 332, and the amino acid sequence of the light chain consists of amino acids at positions 1-219 in SEQ ID NO: 319; or
(17) the amino acid sequence of the heavy chain consists of amino acids at positions 1-448 in SEQ ID NO: 332, and the amino acid sequence of the light chain consists of amino acids at positions 1-219 in SEQ ID NO: 320.

### Antibody Structure

In certain embodiments, the antibody provided herein is a full-length antibody.

In certain embodiments, the antibody provided herein is an antibody fragment.

In one embodiment, the antibody fragment is a Fab, Fab', Fab'-SH, or F(ab')2 fragment, particularly a Fab fragment. "Fab" is a monovalent fragment consisting of the VL, VH, CL, and CH1 domains. "Fab fragment" may be produced by papain cleavage of an antibody. "Fab'" comprises VL, CL, and VH and CH1, and further comprises the region between the CH1 and CH2 domains, so that an interchain disulfide bond can be formed between the two heavy chains of two Fab' fragments to form a F(ab')2 molecule. "Fab'-SH" is a Fab' fragment in which the cysteine residue in the constant region has a free sulfhydryl group. "F(ab')2" is a bivalent fragment comprising two Fab fragments linked by a disulfide bond at the hinge region.

In another embodiment, the antibody fragment is a diabody, a triabody, or a tetrabody. Diabodies are antibody fragments comprising two antigen-binding sites. The fragment comprises a VH and a VL linked in the same polypeptide chain (VH-VL). By using a linker that is too short to allow pairing between two domains on the same chain, the domains are forced to pair with the complementary domains of another chain, thereby resulting in two antigen-binding sites. The two antigens may be identical or different.

In another embodiment, the antibody fragment is a single-chain Fab fragment. "Single-chain Fab fragment" or "scFab" is a polypeptide consisting of VH, CH1, VL, CL, and a linker, wherein the antibody domains and the linker have one of the following orders in the N-terminus to C-terminus direction: a) VH-CH1-linker-VL-CL, b) VL-CL-linker-VH-CH1, c) VH-CL-linker-VL-CH1, or d) VL-CH1-linker-VH-CL. In one embodiment, the linker is a polypeptide having at least 30 amino acids. In another embodiment, the linker is a polypeptide having between 32 and 50 amino acids. The single-chain Fab fragment is stabilized via the natural disulfide bond between CL and CH1. In addition, these single-chain Fab molecules may be further stabilized by generating interchain disulfide bonds through the insertion of cysteine residues (e.g., position 44 in the heavy chain variable region and position 100 in the light chain variable region, according to the Kabat numbering).

In another embodiment, the antibody fragment is an Fv fragment consisting of the VH and VL domains of a single arm of the antibody.

In another embodiment, the antibody fragment is a single-chain variable fragment (scFv). "scFv" is a fusion protein comprising at least one antibody fragment comprising a light chain variable region and at least one antibody fragment comprising a heavy chain variable region, wherein the light and heavy chain variable regions are contiguously linked by a short flexible peptide linker and are capable of being expressed as a single-chain polypeptide, and wherein the scFv retains the specificity of the intact antibody from which it is derived. Unless specifically indicated, an scFv may comprise VL and VH variable regions in any order herein; for example, with respect to the N-terminus and C-terminus of the polypeptide, the scFv may comprise VL-linker-VH or may comprise VH-linker-VL.

In another embodiment, the antibody fragment is a dsFv obtained by linking polypeptides in which one amino acid residue of each of VH and VL is substituted with a cysteine residue by a disulfide bond between the cysteine residues. The amino acid residues substituted with cysteine residues can be selected according to known methods (Protein Engineering, 7:697 (1994)) based on the prediction of the three-dimensional structure of the antibody.

In another embodiment, the antibody fragment is a single-domain antibody (dAb). Single-domain antibodies are antibody fragments comprising all or a portion of the heavy chain variable domain or all or a portion of the light chain variable domain of an antibody.

In certain embodiments, the antibody provided herein is a chimeric antibody. In one example, a chimeric antibody comprises a non-human variable region (e.g., a variable region derived from a mouse, rat, hamster, domestic rabbit, or non-human primate, such as a monkey) and a human constant region. In another example, a chimeric antibody is a "class switched" antibody in which the class or subclass has been changed from the class or subclass of the parent antibody.

In certain embodiments, the antibody is a humanized antibody. Typically, non-human antibodies are humanized to reduce immunogenicity to humans while retaining the specificity and affinity of the parent non-human antibody. Generally, the humanized antibody comprises one or more variable regions in which the CDRs or portions thereof are derived from a non-human antibody and the FRs or portions thereof are derived from a human antibody. Optionally, the humanized antibody can further comprise a portion of a human constant region. In some embodiments, some FR residues in a humanized antibody may be replaced with corresponding residues from a non-human antibody (e.g., an antibody that provides CDR sequences).

Humanized antibodies and methods for their production are reviewed, e.g., in Almagro and Fransson, Front. Biosci. 13:1619-1633 (2008), and further described, e.g., in Riechmann et al., Nature 332:323-329 (1988); Queen et al., Proc. Nat'l Acad. Sci. USA 86:10029-10033 (1989); U.S. Patent Nos. 5,821,337, 7,527,791, 6,982,321, and 7,087,409; Kashmiri et al., Methods 36:25-34 (2005) (describing specificity-determining region (SDR) grafting); Padlan, Mol. Immunol. 28:489-498 (1991) (describing "resurfuacing"); Dall'Acqua et al., Methods 36:43-60 (2005) (describing "FR shuffling"); and Osbourn et al., Methods 36:61-68 (2005) and Klimka et al., Br. J. Cancer 83:252-260 (2000) (describing the "guided selection" method for FR shuffling).

Human framework regions that can be used for humanization include, but are not limited to: framework regions selected using the "best-fit" method (see, e.g., Sims et al., J. Immunol. 151:2296 (1993)); framework regions derived from the consensus sequence of human antibodies of a particular subgroup of light or heavy chain variable regions (see, e.g., Carter et al., Proc. Natl. Acad. Sci. USA, 89:4285 (1992); and Presta et al., J. Immunol., 151:2623 (1993)); human mature (somatically mutated) framework regions or human germline framework regions (see, e.g., Almagro and Fransson, Front. Biosci. 13:1619-1633 (2008)); and framework regions obtained by screening FR libraries (see, e.g., Baca et al., J. Biol. Chem. 272:10678-10684 (1997) and Rosok et al., J. Biol. Chem. 271:22611-22618 (1996)).

### Variants of Anti-CD3 Antibodies

In certain embodiments, amino acid sequence variants of the anti-CD3 antibodies provided herein are encompassed. For example, it may be desirable to improve the binding affinity and/or other biological properties of the antibodies. The amino acid sequence variants of the antibodies can be prepared by introducing appropriate modifications into the nucleotide sequences that encode the antibodies, or by peptide synthesis. Such modifications include, for example, deletions from, and/or insertions into, and/or substitutions of residues within the amino acid sequence of the anti-CD3 antibody. Any combination of deletions, insertions, and substitutions can be made to obtain the final construct, as long as the final construct possesses the desired characteristics, e.g., antigen binding properties.

### Substitution, Insertion, and Deletion Variants

In certain embodiments, antibody variants comprising one or more amino acid substitutions are provided. Positions of interest for substitutional mutagenesis include CDRs and FRs. Conservative substitutions are shown in Table 2 under the heading "Preferred substitution". More substantial changes are provided in Table 2 under the heading "Exemplary substitution", and as further described below with reference to amino acid side chain classes. Amino acid substitutions can be introduced into an antibody of interest, and the products are screened for a desired activity, e.g., retained/improved antigen binding, reduced immunogenicity, or improved ADCC or CDC.

**Table 2. Amino acid substitutions**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

One type of substitution variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated, and the variant antibodies are displayed on phages and screened for a particular biological activity (e.g., binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling. Particularly, HCDR3 and LCDR3 are often targeted.

In certain embodiments, substitutions, insertions, or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside the antigen-contacting residues in CDRs. In certain embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or a group of target residues (e.g., charged residues such as Arg, Asp, His, Lys, and Glu) is identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides containing 100 or more residues, and intrasequence insertions of single or multiple amino acid residues. Examples of terminal insertions include an antibody with an N-terminal methionyl residue. Other insertion variants of the antibody molecule include the fusion of the N- or C-terminus of the antibody to an enzyme or a polypeptide that increases the serum half-life of the antibody.

### Recombination Methods

Anti-CD3 antibodies can be produced using recombinant methods. For these methods, one or more isolated nucleic acids encoding the anti-CD3 antibodies are provided.

In one embodiment, the present disclosure provides isolated nucleic acids encoding the aforementioned anti-CD3 antibodies. Such nucleic acids may each independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In one embodiment, provided is a method for preparing a polypeptide or fusion protein, wherein the method comprises culturing a host cell comprising a nucleic acid encoding the polypeptide or fusion protein, as provided above, under conditions suitable for expression, and optionally recovering the anti-CD3 antibody from the host cell (or host cell culture medium).

For recombinant production of the anti-CD3 antibody, the nucleic acid encoding the protein is isolated and inserted into one or more vectors for further cloning and/or expression in the host cell. Such nucleic acids can be readily isolated and sequenced using conventional procedures, or produced by recombination methods, or obtained by chemical synthesis.

Suitable host cells for cloning or expressing a vector encoding the anti-CD3 antibody include the prokaryotic or eukaryotic cells described herein. For example, it can be produced in bacteria, particularly when glycosylation and Fc effector functions are not needed. After expression, it can be isolated from the bacterial cell paste in a soluble fraction and can be further purified.

In addition to prokaryotes, eukaryotic microorganisms such as filamentous fungi or yeast are suitable cloning or expression hosts for vectors encoding fusion proteins, including fungal and yeast strains. Suitable host cells for expression of the fusion protein may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978, and US6417429; vertebrate cells can also be used as hosts, e.g., mammalian cell lines adapted for growth in a suspension. Other examples of suitable mammalian host cell lines include an SV40-transformed monkey kidney CVl line (COS-7); a human embryonic kidney line (293 or 293T cells); baby hamster kidney cells (BHK); mouse Sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for antibody production, see, e.g., Yazaki, P. and Wu, A.M., Methods in Molecular Biology, Vol. 248, Lo, B.K.C. (eds.), Humana Press, Totowa, NJ (2004), pp. 255-268.

### Assays

The physical/chemical characteristics and/or biological activity of the anti-CD3 antibodies provided herein can be identified, screened, or characterized through a variety of assays known in the art. In one aspect, the anti-CD3 antibody of the present disclosure is tested for activity, for example, by known methods such as ELISA and western blot.

### Treatment Method and Route of Administration

Any anti-CD3 antibody provided by the present disclosure can be used in a treatment method. In yet another aspect, the present disclosure provides use of the anti-CD3 antibody in the manufacture or preparation of a medicament. In some embodiments, the disease is a disease or disorder associated with CD3. In some embodiments, the disease or disorder is selected from the group consisting of a tumor, an autoimmune disease, and an inflammatory disease.

In yet another aspect, provided is a pharmaceutical composition comprising the anti-CD3 antibody, e.g., for use in any one of the above pharmaceutical uses or treatment methods. In one embodiment, the pharmaceutical composition comprises any anti-CD3 antibody or antigen-binding fragment thereof provided herein and a pharmaceutically acceptable carrier. In another embodiment, the pharmaceutical composition further comprises at least one additional therapeutic agent.

The anti-CD3 antibody of the present disclosure may be used alone or in combination with other agents for treatment. For example, the antibody of the present disclosure can be co-administered with at least one additional therapeutic agent.

The anti-CD3 antibody of the present disclosure (and any additional therapeutic agent) may be administered by any suitable means, including parenteral, intrapulmonary, and intranasal administration, and, if local treatment is needed, intralesional administration. Parenteral infusion includes intramuscular, intravenous, intra-arterial, intraperitoneal, or subcutaneous administration. Administration may be performed via any suitable route, e.g., by injection, such as intravenous or subcutaneous injection, depending in part on whether the administration is short-term or long-term. Various administration schedules are contemplated herein, including but not limited to single administration or multiple administrations at multiple time points, bolus injection administration, and pulse infusion. The anti-CD3 antibody of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the administration method, the timing of administration, and other factors known to medical practitioners. The anti-CD3 antibody can be formulated with or without one or more agents currently used for preventing or treating the disorder. The effective amount of such additional agents depends on the amount present in the pharmaceutical composition, the type of the disorder or treatment, and other factors. These are generally used at the same dosage and via the same route of administration as described herein, or at about 1% to 99% of the dosage described herein, or at other dosages, and via any route empirically/clinically determined to be appropriate.

For the prevention or treatment of a disease, the appropriate dosage of the anti-CD3 antibody of the present disclosure (when used alone or in combination with one or more additional therapeutic agents) will depend on the type of the disease to be treated, the type of the therapeutic molecule, the severity and course of the disease, the purpose of administration (prophylactic or therapeutic), the previous treatment, the patient's clinical history and response to the therapeutic molecule, and the discretion of the attending physician. The therapeutic molecule is suitably administered to a patient in one treatment or a series of treatments.

### Product

In another aspect of the present disclosure, provided is a product comprising materials useful for the treatment, prevention, and/or diagnosis of the disorders described above. The product comprises a container and a label or package insert on or associated with the container. Suitable containers include, for example, bottles, vials, syringes, IV solution bags, and the like. The container may be formed of a variety of materials such as glass or plastic. The container contains a composition effective in the treatment, prevention, and/or diagnosis of a disease, either alone or in combination with another composition, and may have a sterile access port (e.g., the container may be an intravenous solution bag or a vial with a stopper pierceable by a hypodermic injection needle). At least one active agent in the composition is the anti-CD3 antibody of the present disclosure. The label or package insert indicates that the composition is used to treat the selected condition. In addition, the product may comprise: (a) a first container containing a composition, wherein the composition comprises the anti-CD3 antibody of the present disclosure; and (b) a second container containing a composition, wherein the composition comprises an additional cytotoxic agent or a therapeutic agent of other aspects. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further comprise other required materials, including other buffers, diluents, filters, needles, and syringes.

### Example

The present disclosure is further described below with reference to examples and test examples. However, these examples and test examples do not limit the scope of the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Preparation and Screening of CD3 Antibodies

Monoclonal antibodies targeting human CD3 were prepared by the hybridoma technology in the present disclosure. Mice were cross-immunized with human and monkey CD3E and heterodimer proteins of CD3D&CD3E as immunoreagents and with TiterMax^{®} Gold Adjuvant (Sigma Cat No. T2684) and Thermo Imject^{®} Alum (Thermo Cat No. 77161) as adjuvants. After primary immunization and boost immunization, mice 1-1, 1-4, 2-1, 2-5, 4-2, and 4-5#, with high antibody titers in serum, were selected for splenocyte fusion. The Human CD3 epsilon/CD3E (hFc&his) protein was purchased from Sinobiological; the human CD3D&CD3E heterodimer (hFc, his&flag) protein was purchased from Sinobiological; the Cynomolgus CD3E (Fc&his) protein was purchased from ACRO Biosystems; and the Cynomolgus CD3D&CD3E heterodimer (hFc, his&flag) protein was purchased from ACRO Biosystems.

After fusion, the hybridoma culture supernatant was assayed based on the growth density of hybridoma cells, and antibodies capable of specifically binding to human and cynomolgus monkey CD3 antigens, capable of specifically binding to Jurkat cells, and having a T cell activation function were screened. Monoclonal hybridoma cell strains with good activity were obtained by screening. Hybridoma cells at logarithmic growth phase were harvested, and the RNA was extracted using NucleoZol (MN) (following the procedures in the kit instructions) and reverse-transcribed (PrimeScript^{™} Reverse Transcriptase, Takara, cat # 2680A). The cDNA obtained by the reverse transcription was amplified by PCR using mouse Ig-Primer Set (Novagen, TB326 Rev.B 0503) and then sent for sequencing. The amino acid sequences of the CDRs and variable regions of the monoclonal hybridoma cell strains obtained by screening are as follows:

**Table 3. CDR sequences of CD3 antibodies obtained by hybridoma screening**

| No. | Light chain | | Heavy chain | |
|---|---|---|---|---|
| 8# | LCDR1 | KSSQSLLNSRTRKNYLA (SEQ ID NO: 1) | HCDR1 | NYWMH (SEQ ID NO: 4) |
| | LCDR2 | WASTRES (SEQ ID NO: 2) | HCDR2 | NINLSNGGTNYKEEFKT (SEQ ID NO: 5) |
| | LCDR3 | TQSFILRT (SEQ ID NO: 3) | HCDR3 | DAYSLYYFDY (SEQ ID NO: 6) |
| 12# | LCDR1 | KSSQSLLNSRTRKNYLA (SEQ ID NO: 1) | HCDR1 | SFWMH (SEQ ID NO: 8) |
| | LCDR2 | WASTRES (SEQ ID NO: 2) | HCDR2 | NINLSNGGTNYNEKFKT (SEQ ID NO: 9) |
| | LCDR3 | TQSYILRT (SEQ ID NO: 7) | HCDR3 | DSYSVYYFDY (SEQ ID NO: 10) |
| 21# | LCDR1 | KSSQSLLNSRTRKNYLA (SEQ ID NO: 1) | HCDR1 | NYWMH (SEQ ID NO: 4) |
| | LCDR2 | WASTRES (SEQ ID NO: 2) | HCDR2 | NINLSNGGTNYKEEFKT (SEQ ID NO: 5) |
| | LCDR3 | TQSFILRT (SEQ ID NO: 3) | HCDR3 | DAYSLYYFDY (SEQ ID NO: 6) |
| 29# | LCDR1 | KSSQSLLNSRTRKNYLA (SEQ ID NO: 1) | HCDR1 | SYWMH (SEQ ID NO: 11) |
| | LCDR2 | WASTRES (SEQ ID NO: 2) | HCDR2 | NINLSNGDTNYKENFKT (SEQ ID NO: 12) |
| | LCDR3 | TQSYILRT (SEQ ID NO: 7) | HCDR3 | DAYSVYYFDY (SEQ ID NO: 13) |
| 31# | LCDR1 | KSSQSLFNSRTRKNYLA (SEQ ID NO: 14) | HCDR1 | NYWMH (SEQ ID NO: 4) |
| | LCDR2 | WASTRES (SEQ ID NO: 2) | HCDR2 | NINLSNGDTNYKEKFKT (SEQ ID NO: 15) |
| | LCDR3 | TQSYILRT (SEQ ID NO: 7) | HCDR3 | DAYSVYYFDY (SEQ ID NO: 13) |
| 57# | LCDR1 | KSSQSLFNSRTRKNYLA (SEQ ID NO: 14) | HCDR1 | DAYSVYYFDY (SEQ ID NO: 13) |
| | LCDR2 | WSSTRES (SEQ ID NO: 16) | HCDR2 | WIYPGSDNTRYNEKFKD (SEQ ID NO: 18) |
| | LCDR3 | KQSFILRT (SEQ ID NO: 17) | HCDR3 | DGNYYFDY (SEQ ID NO: 19) |
| 61# | LCDR1 | KSSQSLLNSRTRKNYLA (SEQ ID NO: 1) | HCDR1 | DAYSVYYFDY (SEQ ID NO: 13) |
| | LCDR2 | WASTRES (SEQ ID NO: 2) | HCDR2 | WIYTGSDNTKYNEKFKG (SEQ ID NO: 21) |
| | LCDR3 | KQSYTLRT (SEQ ID NO: 20) | HCDR3 | DGNYYFDY (SEQ ID NO: 19) |
| 63# | LCDR1 | KSSQSLLHSDGKTYLN (SEQ ID NO: 22) | HCDR1 | DHYIN (SEQ ID NO: 25) |
| | LCDR2 | LVSKVDS (SEQ ID NO: 23) | HCDR2 | WIFPGSVSTYYNAKFKG (SEQ ID NO: 26) |
| | LCDR3 | WQGTHFPRT (SEQ ID NO: 24) | HCDR3 | SGNGSY (SEQ ID NO: 27) |
| 80# | LCDR1 | RASGNIHNYLA (SEQ ID NO: 28) | HCDR1 | EYTIH (SEQ ID NO: 31) |
| | LCDR2 | NAKILAD (SEQ ID NO: 29) | HCDR2 | GFNPNTGGTNYNQNFKG (SEQ ID NO: 32) |
| | LCDR3 | QHFWRTPFT (SEQ ID NO: 30) | HCDR3 | GYDNAPYWYFDV (SEQ ID NO: 33) |
| 82# | LCDR1 | KSSQSLLNSRTRKNYLA (SEQ ID NO: 1) | HCDR1 | SYYIH (SEQ ID NO: 34) |
| | LCDR2 | WASTRES (SEQ ID NO: 2) | HCDR2 | WINPGDGSIKYNEKFKG (SEQ ID NO: 35) |
| | LCDR3 | KQSFILRT (SEQ ID NO: 17) | HCDR3 | DGYSYYYFDF (SEQ ID NO: 36) |
| 89# | LCDR1 | KSSQSLLNSRTRKNYLA (SEQ ID NO: 1) | HCDR1 | TYYIH (SEQ ID NO: 37) |
| | LCDR2 | WASTRES (SEQ ID NO: 2) | HCDR2 | WINPGDDSIKYNEKFKG (SEQ ID NO: 38) |
| | LCDR3 | KQSFILRT (SEQ ID NO: 17) | HCDR3 | DGYSYYYFDF (SEQ ID NO: 36) |
| 92-2# | LCDR1 | KSSQSLLNSRTRKNYLA (SEQ ID NO: 1) | HCDR1 | SYYIH (SEQ ID NO: 34) |
| | LCDR2 | WASTRES (SEQ ID NO: 2) | HCDR2 | WINPGDGTIKYNEKFKG (SEQ ID NO: 39) |
| | LCDR3 | KQSFILRT (SEQ ID NO: 17) | HCDR3 | DGYSYYYFDF (SEQ ID NO: 36) |
| 93# | LCDR1 | KSSQSLLNIRTRKNYLA (SEQ ID NO: 40) | HCDR1 | NYYIH (SEQ ID NO: 41) |
| | LCDR2 | WASTRES (SEQ ID NO: 2) | HCDR2 | WLYPGDGSIKFNENFKG (SEQ ID NO: 42) |
| | LCDR3 | KQSYTLRT (SEQ ID NO: 20) | HCDR3 | DGYSYYYFDY (SEQ ID NO: 43) |
| 96# | LCDR1 | KSSQSLLNSRTRKNYLA (SEQ ID NO: 1) | HCDR1 | SYYIH (SEQ ID NO: 34) |
| | LCDR2 | WASTRES (SEQ ID NO: 2) | HCDR2 | WINPGDGSIKYNEKFKG (SEQ ID NO: 35) |
| | LCDR3 | KQSFILRT (SEQ ID NO: 17) | HCDR3 | DGYSYYFFDF (SEQ ID NO: 44) |
| 103# | LCDR1 | KSSQSLLNSRTRKNYLA (SEQ ID NO: 1) | HCDR1 | SYYIH (SEQ ID NO: 34) |
| | LCDR2 | WASTRES (SEQ ID NO: 2) | HCDR2 | WINPGDDSIKYNEKFKG (SEQ ID NO: 38) |
| | LCDR3 | KQSFILRT (SEQ ID NO: 17) | HCDR3 | DGYSYYYFDF (SEQ ID NO: 36) |
| 104# | LCDR1 | KSSQSLFNSRIRKNYLA (SEQ ID NO: 45) | HCDR1 | DTYMH (SEQ ID NO: 47) |
| | LCDR2 | WASTRES (SEQ ID NO: 2) | HCDR2 | RIDPANGNTRYDPKFQV (SEQ ID NO: 48) |
| | LCDR3 | KQFYTLWT (SEQ ID NO: 46) | HCDR3 | ISYNYDAWFTY (SEQ ID NO: 49) |
| 105# | LCDR1 | KSSQSLLNSGTRKNYLA (SEQ ID NO: 50) | HCDR1 | SYWMH (SEQ ID NO: 11) |
| | LCDR2 | WASTRES (SEQ ID NO: 2) | HCDR2 | NIDPVSGTINYDEKFKS (SEQ ID NO: 52) |
| | LCDR3 | IQSYILRT (SEQ ID NO: 51) | HCDR3 | DGYSFYYFDY (SEQ ID NO: 53) |
| 106# | LCDR1 | KSSQSLLNSRTRKNYLA (SEQ ID NO: 1) | HCDR1 | TYYIH (SEQ ID NO: 37) |
| | LCDR2 | WASTRES (SEQ ID NO: 2) | HCDR2 | WIYPGDDSSKFNEKFRG (SEQ ID NO: 55) |
| | LCDR3 | KQSYSLRT (SEQ ID NO: 54) | HCDR3 | DSYGSYYFDY (SEQ ID NO: 56) |

The variable region sequences are as follows:
> 8# murine-derived light chain variable region 
DIVMSQSPSSLAVSAGEKVTMSCKSSQSLLNSRTRKNYLAWYQQKPGQSPKLLI
YWASTRESGVPDRFTGSGSGTDFTLTISGVQAEDLAVYYCTQSFILRTFGGGTKL
EIK
SEQ ID NO: 57
> 8# murine-derived heavy chain variable region 
QVQLQQPGTELVKPGTSVKLSCKASGYTFTNYWMHWVKQRPGQGLEWIGNIN
LSNGGTNYKEEFKTKATLTVDKSSSTAYIQLSSLTSEDSAVYYCARDAYSLYYFD
YWGQGTTLTVSS
SEQ ID NO: 58
> 12# murine-derived light chain variable region 
DIVMSQSPSSLAVSAGEKVTMNCKSSQSLLNSRTRKNYLAWYQQKPGQSPKLLI
YWASTRESGVPDRFTGSGSGTDFTLTISGVQAEDLAVYYCTQSYILRTFGGGTKL
EIK
SEQ ID NO: 59
> 12# murine-derived heavy chain variable region 
QVQLQQPGTELVKPGASVKLSCKASGYNFTSFWMHWVKQRPGQGLEWIGNIN
LSNGGTNYNEKFKTKATLTADKSSSTAYMQLSSLTSEDSAVYFCARDSYSVYYF
DYWGQGTTLTVSS
SEQ ID NO: 60
> 21# murine-derived light chain variable region 
DIVMSQSPSSLAVSAGEKVTMSCKSSQSLLNSRTRKNYLAWYQQKPGQSPKLLI
YWASTRESGVPDRFTGSGSGTDFTLTISGVQAEDLAVYYCTQSFILRTFGGGTKL
EIK
SEQ ID NO: 57
> 21# murine-derived heavy chain variable region 
QVQLQQPGIELVKPGTSVKLSCKASGYTFTNYWMHWVKQRPGQGLEWIGNIN
LSNGGTNYKEEFKTKATLTVDKSSSTAYIQLSSLTSEDSAVYYCARDAYSLYYFD
YWGQGTTLTVSS
SEQ ID NO: 61
> 29# murine-derived light chain variable region 
DIVLSQSPSSLAVSAGEKVTMNCKSSQSLLNSRTRKNYLAWYQQKPGQSPKVLI
YWASTRESGVPDRFTGSGSGTDFTLTISGVQAEDLAVYYCTQSYILRTFGGGTKL
 
EIK
SEQ ID NO: 62
> 29# murine-derived heavy chain variable region 
QVQLQQPGTEQVKPGASVKLSCKASGYTFTSYWMHWVKQRPGQGLEWIGNIN
LSNGDTNYKENFKTKATLTVDKSSSTAYMQLSSLTSEDSAVYYCARDAYSVYYF
DYWGQGTTLTVSS
SEQ ID NO: 63
> 31# murine-derived light chain variable region 
DIVMSQSPSSLAVSAGEKVTMNCKSSQSLFNSRTRKNYLAWYQQKPGQSPKLLI
YWASTRESGVPDRFTGSGSGTDFTLTISGVQAEDLAVYYCTQSYILRTFGGGTKL
EIK
SEQ ID NO: 64
> 31# murine-derived heavy chain variable region 
QVQLQQPGTELVKPGASVKLSCKASGYTFTNYWMHWVKQRPGQGLEWIGNIN
LSNGDTNYKEKFKTKATLTVDKSSSTVYMQLSSLTSEDSAVYYCARDAYSVYYF
DYWGQGTTLTVSS
SEQ ID NO: 65
> 57# murine-derived light chain variable region 
DIVMSQSPSSLAVSAGEKVTMSCKSSQSLFNSRTRKNYLAWYQQKPGQSPKLLI
YWSSTRESGVPDRFTGSGSGTDFTLTISSVQAEDLAVYYCKQSFILRTFGGGTRL
EIK
SEQ ID NO: 66
> 57# murine-derived heavy chain variable region 
QVQLQQSGPELVNPGASVKMSCKASGYSFTDAYSVYYFDYWVKQRPGQGLEW
IGWIYPGSDNTRYNEKFKDKATLTADTSSSTAYMQLSSLTSEDSAVYYCTRDGN
YYFDYWGQGTTLTVSS
SEQ ID NO: 67
> 61# murine-derived light chain variable region 
DIVMSQSPSSLAVSAGEKVSMSCKSSQSLLNSRTRKNYLAWYQQKPGQSPKLLI
YWASTRESGVPDRFTGSGSGTDFTLTISSVQAEDLAVYYCKQSYTLRTFGGGTK
LEIK
SEQ ID NO: 68
> 61# murine-derived heavy chain variable region 
QVQLQQSGPELVKPGASVKMSCKASGYSFTDAYSVYYFDYWVKQRPGQGLEW
IGWIYTGSDNTKYNEKFKGKATLTADTSSRTAYMQLSSLTSEDSAVYYCTRDGN
YYFDYWGQGTTLTVSS
 SEQ ID NO: 69
> 63# murine-derived light chain variable region 
DVVLTQTPLTLSVTIGQPASISCKSSQSLLHSDGKTYLNWLLQRPGQSPKRLIYLV
SKVDSGVPDRFTGSGSGTDFTLKISRVEAEDLGVYYCWQGTHFPRTFGGGTKLE
IR
SEQ ID NO: 70
> 63# murine-derived heavy chain variable region 
QVQLQQSGPELVKPGASVKISCKTSGYTLTDHYINWVKQRPGQGLEWIGWIFPG
SVSTYYNAKFKGKATLTVDISSSTAYMLLSSLTSEDSAVYFCARSGNGSYWGQG
TTLTVSS
SEQ ID NO: 71
> 80# murine-derived light chain variable region 
DIQMTQSPASLSASVGETVTITCRASGNIHNYLAWYQQKQGKSPQLLVYNAKIL
ADGVPSRFSGSGSGTQYSLKINSLQPEDFGSYHCQHFWRTPFTFGSGTKLEIK
SEQ ID NO: 72
> 80# murine-derived heavy chain variable region 
EVQLQQSGPELVKPGASVKISCKTSGYTFTEYTIHWVKQSHGKSLEWIGGFNPN
TGGTNYNQNFKGKATLTVDKSSSTAYMELRSLTSEDSAVYYCVRGYDNAPYWY
FDVWGAGTTVTVSS
SEQ ID NO: 73
> 82# murine-derived light chain variable region 
DIVMSQSPSSLAVSAGEQVTMSCKSSQSLLNSRTRKNYLAWYQQKPGQSPKLLI
YWASTRESGVPDRFTGSGSGTDFTLTISNVQAEDLAVYYCKQSFILRTFGGGTKL
EIK
SEQ ID NO: 74
> 82# murine-derived heavy chain variable region 
QVQLQQSGPELVKPGASVKMSCKASGYTFTSYYIHWMQQRPGQGLEWIGWIN
PGDGSIKYNEKFKGRTTLTADKSSSTAYMLLRSLTSEDSAIYFCARDGYSYYYFD
FWGQGTTLTVSS
SEQ ID NO: 75
> 89# murine-derived light chain variable region 
DIVMSQSPSSLAVSAGEQVTMSCKSSQSLLNSRTRKNYLAWYQQKPGQSPKLLI
YWASTRESGVPDRFTGSGSGTDFTLTISNVQAEDLAVYYCKQSFILRTFGGGTKL
EIK
SEQ ID NO: 74
> 89# murine-derived heavy chain variable region 
QVQLQQSGPELVKPGASVKMSCKASGYTFATYYIHWMQQRPGQGLEWIGWIN
PGDDSIKYNEKFKGRTTLTADKSSSTAYMLLRSLTSEDSAIYFCARDGYSYYYFD
FWGQGTTLTVSS
SEQ ID NO: 76
> 92-2# murine-derived light chain variable region 
DIVMSQSPSSLAVSAGEQVTMSCKSSQSLLNSRTRKNYLAWYQQKPGQSPKLLI
YWASTRESGVPDRFTGSGSGTDFTLTISNVQAEDLAVYYCKQSFILRTFGGGTKL
EIK
SEQ ID NO: 74
> 92-2# murine-derived heavy chain variable region 
QVQLQQSGPELVKPGASVKMSCKASGYTFTSYYIHWMQQRPGQGLEWIGWIN
PGDGTIKYNEKFKGRTTLTADKSSSTAYMLLRSLTFEDSAIYFCARDGYSYYYFD
FWGQGTTLTVSS
SEQ ID NO: 77
> 93# murine-derived light chain variable region 
DIVMSQSPSSLAVSAGEKVIMSCKSSQSLLNIRTRKNYLAWYQQKPGQSPKLLIY
WASTRESGVPDRFTGSGSGTDFTLTISSVQAEDLAIYYCKQSYTLRTFGGGTKLE
IK
SEQ ID NO: 78
> 93# murine-derived heavy chain variable region 
QVQLQQSGPELVKPGASVKMSCKASGYNFTNYYIHWVKQRPGQGLEWIGWLY
PGDGSIKFNENFKGKTALTADKSSTTAYMWLSSLTSEDSAIYFCARDGYSYYYFD
YWGQGTTLTVSS
SEQ ID NO: 79
> 96# murine-derived light chain variable region 
DIVMSQSPSSLAVSAGEQVTMSCKSSQSLLNSRTRKNYLAWYQQKPGQSPKLLI
YWASTRESGVPDRFTGSGSGTDFTLTISNVQAEDLAVYYCKQSFILRTFGGGTKL
EIK
SEQ ID NO: 74
> 96# murine-derived heavy chain variable region 
QVQLQQSGPELVKPGASVKMSCKASGYTFTSYYIHWMQQRPGQGLEWIGWIN
PGDGSIKYNEKFKGRTTLTADKSSSTAYMLLRSLTSEDSAIYFCARDGYSYYFFD
FWGQGTTLTVSS
SEQ ID NO: 80
> 103# murine-derived light chain variable region DIVMSQSPSSLAVSAGEQVTMSCKSSQSLLNSRTRKNYLAWYQQKPGQSPKLLI 
YWASTRESGVPDRFTGSGSGTDFTLTINNVQAEDLAVYYCKQSFILRTFGGGTK
LEIK
SEQ ID NO: 81
> 103# murine-derived heavy chain variable region 
QVQLQQSGPELVKPGASVKMSCKASGYTFASYYIHWMQQRPGQGLEWIGWIN
PGDDSIKYNEKFKGRTTLTADKSSSTAYMLLRSLTSEDSAIYFCARDGYSYYYFD
FWGQGSTLTVSS
SEQ ID NO: 82
> 104# murine-derived light chain variable region 
DIVMSQSPSSLAVSAGEKVTMSCKSSQSLFNSRIRKNYLAWFQQKPGQSPKLLIY
WASTRESGVPDRFTGSGSGTDFTLTISSVQAEDLAVYYCKQFYTLWTFGGGTKL
EIK
SEQ ID NO: 83
> 104# murine-derived heavy chain variable region 
EIQLQQSGAELVKPGASVKLSCTASGFNIKDTYMHWVKQRPDQGLDWIGRIDPA
NGNTRYDPKFQVKATITADTSSNTAYLHLSSLTSEDTAVYYCSRISYNYDAWFTY
WGQGTLVTVSA
SEQ ID NO: 84
> 105# murine-derived light chain variable region 
DIVMSQSPSSLAVSAGERVTMTCKSSQSLLNSGTRKNYLAWYQQKPGQSPKLLI
YWASTRESGVPDRFTGSGSGTDFTLTINSVQAEDLAVYYCIQSYILRTFGGGTKL
EIK
SEQ ID NO: 85
> 105# murine-derived heavy chain variable region 
QVQLQQPGSELVRPGASVKLSCKASGYTFTSYWMHWVKQRPGQGLEWIGNID
PVSGTINYDEKFKSKATLTVDTSSSTAYMQLSSLTSEDSAVYYCTRDGYSFYYFD
YWGQGTTLTVSS
SEQ ID NO: 86
> 106# murine-derived light chain variable region 
DIVMSQSPSSLAVSAGEKVTMSCKSSQSLLNSRTRKNYLAWYQQKPGQSPKLLI
YWASTRESGVPDRFTGSGSGTDFTLTISNVQAEDLAVYYCKQSYSLRTFGGGTK
LEIK
SEQ ID NO: 87
> 106# murine-derived heavy chain variable region

QVQLQQSGPELVKPGASVKMSCKASAYTFTTYYIHWVRQRPGQGLEWIGWIYP
GDDSSKFNEKFRGKATLTADKSSSTAYMLLSSLTSEDSAIYFCARDSYGSYYFDY

WGQGITLTVSS
SEQ ID NO: 88

Note: In the above sequences, the regions are arranged in the following order: FR1-CDR1-FR2-CDR2-FR3-CDR3-FR4; the CDR sequences determined according to the Kabat numbering scheme are underlined, and the portions that are not underlined are the FR sequences.

The heavy chain variable region and the light chain variable region of the murine-derived anti-CD3 antibody were recombined with the heavy chain constant region (hIgG1:CH1-Fc) and the kappa light chain constant region (kappa CL) of human immunoglobulin IgG1, respectively, to obtain the full-length chimeric antibody. Illustratively, Chi12 and Chi82 represent chimeric antibodies comprising 12# and 82# murine-derived heavy and light chain variable regions and the constant regions set forth in SEQ ID NO: 90 and SEQ ID NO: 91. This similarly applies to Chi8, Chi21, Chi29, Chi31, Chi57, Chi61, Chi63, Chi80, Chi89, Chi92-2, Chi93, Chi96, Chi103, Chi104, Chi105, Chi106, and the like.
> CH1: 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC
SEQ ID NO: 89
> hIgG1:CH1-Fc: 







SEQ ID NO: 90
> kappa CL: 


SEQ ID NO: 91
> Chi12 light chain: 
DIVMSQSPSSLAVSAGEKVTMNCKSSQSLLNSRTRKNYLAWYQQKPGQSPKLLI
YWASTRESGVPDRFTGSGSGTDFTLTISGVQAEDLAVYYCTQSYILRTFGGGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
 SEQ ID NO: 92
> Chi12 heavy chain: 
QVQLQQPGTELVKPGASVKLSCKASGYNFTSFWMHWVKQRPGQGLEWIGNIN
LSNGGTNYNEKFKTKATLTADKSSSTAYMQLSSLTSEDSAVYFCARDSYSVYYF
DYWGQGTTLTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK
SEQ ID NO: 93
> Chi82 light chain: 
DIVMSQSPSSLAVSAGEQVTMSCKSSQSLLNSRTRKNYLAWYQQKPGQSPKLLI
YWASTRESGVPDRFTGSGSGTDFTLTISNVQAEDLAVYYCKQSFILRTFGGGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 94
> Chi82 heavy chain:

QVQLQQSGPELVKPGASVKMSCKASGYTFTSYYIHWMQQRPGQGLEWIGWIN
PGDGSIKYNEKFKGRTTLTADKSSSTAYMLLRSLTSEDSAIYFCARDGYSYYYFD
FWGQGTTLTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK
SEQ ID NO: 95

Note: The CDRs are single-underlined; the constant regions are italicized; the CDRs are numbered according to Kabat.

### Example 2. Humanization of Anti-CD3 Antibody 12#

By alignment with the IMGT human antibody heavy and light chain variable region germline gene database through the MOE software, heavy and light chain variable region germline genes highly homologous with 12# were selected as templates, and CDRs of this murine-derived antibody were grafted into corresponding human-derived templates to form variable region sequences in the following order: FR1-CDR1-FR2-CDR2-FR3-CDR3-FR4. Illustratively, the CDR amino acid residues in the specific examples below were determined and annotated using the Kabat numbering scheme.

For the humanized antibody of antibody 12#, the FR1, FR2, and FR3 of IGKV4-1*01, IGKV2-28*01, or IGKV1-27*01 and the FR4 of IGKJ4*01 or IGKJ1*01 were selected as light chain framework region templates; the FR1, FR2, and FR3 of IGHV1-46*01 and the FR4 of IGHJ6*01 or IGHJ4*01 were selected as heavy chain framework region templates. Optionally, the amino acid residue(s) at position(s) 27d, 37, 43, and/or 45 in the light chain variable region of the humanized antibody was/were substituted; and/or the amino acid residue(s) at position(s) 1, 28, 52, 54, 69, 71, 73, 78, 83, 91, and/or 95 in the heavy chain variable region of the humanized antibody was/were substituted.

**Table 4-1. 12# humanized antibodies**

| **VL** | | **VH** | |
|---|---|---|---|
| VL1 | Graft (IGKV4-1*01) + P43S | VH1 | Graft (IGHV1-46*01) + R71A,T73K + Q1E |
| VL2 | Graft (IGKV2-28*01) | VH2 | Graft (IGHV1-46*01) + M69L,R71A,T73K,V78A + Q1E |
| VL3 | Graft (IGKV2-28*01) + L37Q,Q45K | VH3 | Graft (IGHV1-46*01) + T28N,M69L,R71A,T73K,V78A + Q1E |
| VL4 | Graft (IGKV1-27*01) + V43S | VH4 | Graft (IGHV1-46*01) + M69L,R71A,T73K,V78A + Q1E,N52Q |
| VL5 | Graft (IGKV4-1*01) + P43S + N27d Q | VH5 | Graft (IGHV1-46*01) + M69L,R71A,T73K,V78A + Q1E,N54Q |
| | | VH6 | Graft (IGHV1-46*01) + M69L,R71A,T73K,V78A + Q1E,D95E |
| | | VH7 | Graft (IGHV1-46*01) + M69L,R71A,T73K,V78A + Q1E,N52Q,N54Q,D95E |
| FR4 | IGKJ4*01 | FR4 | IGHJ6*01 |

| | | | |
|---|---|---|---|
| Note: P43S indicates that P at position 43 is mutated to S according to the Kabat numbering scheme. This similarly applies to the others. | | | |

**Table 4-2. 12# humanized antibodies**

| **VL** | | **VH** | |
|---|---|---|---|
| VL6 | Graft (IGKV4-1*01) | VH8 | Graft (IGHV1-46*01) + M69L,R71A,T73K,V78A,R83T,Y91F + Q1E |
| VL7 | Graft (IGKV4-1*01) + N27d Q | VH9 | Graft (IGHV1-46*01) + M69L,R71A,T73K,V78A,R83T,Y91F+ Q1E,N52Q |
| | | VH10 | Graft (IGHV1-46*01) + M69L,R71A,T73K,V78A,R83T,Y91F+ Q1E,N54Q |
| | | VH11 | Graft (IGHV1-46*01) + M69L,R71A,T73K,V78A,R83T,Y91F+ Q1E,D95E |
| | | VH12 | Graft (IGHV1-46*01) + M69L,R71A,T73K,V78A,R83T,Y91F + Q1E,N52Q,N54Q,D95E |
| FR4 | IGKJ1*01 | FR4 | IGHJ4*01 |

The CDRs of the 12# humanized antibodies are as follows:

**Table 5. The CDRs of 12# humanized antibodies**

| Variable region of 12# | CDR | Sequence | SEQ ID NO |
|---|---|---|---|
| VL5/VL7 | LCDR1 | KSSQSLL**Q**SRTRKNYLA | SEQ ID NO: 96 |
| VH4/VH9 | HCDR2 | NI**Q**LSNGGTNYNEKFKT | SEQ ID NO: 97 |
| VH5/VH10 | HCDR2 | NINLS**Q**GGTNYNEKFKT | SEQ ID NO: 98 |
| VH6/VH7/VH11/VH12 | HCDR3 | **E**SYSVYYFDY | SEQ ID NO: 99 |
| VH7/VH12 | HCDR2 | NI**Q**LS**Q**GGTNYNEKFKT | SEQ ID NO: 100 |

The specific variable region sequences of the humanized antibodies of 12# are as follows:
>12VL1 
DIVMTQSPDSLAVSLGERATINCKSSQSLLNSRTRKNYLAWYQQKPGQSPKLLIY
WASTRESGVPDRFSGSGSGTDFTLTISSLQAEDVAVYYCTQSYILRTFGGGTKVEI
K
SEQ ID NO: 101
>12VL2 
DIVMTQSPLSLPVTPGEPASISCKSSQSLLNSRTRKNYLAWYLQKPGQSPQLLIY
WASTRESGVPDRFSGSGSGTDFTLKISRVEAEDVGVYYCTQSYILRTFGGGTKV
EIK
SEQ ID NO: 102
>12VL3 
DIVMTQSPLSLPVTPGEPASISCKSSQSLLNSRTRKNYLAWYQQKPGQSPKLLIY
WASTRESGVPDRFSGSGSGTDFTLKISRVEAEDVGVYYCTQSYILRTFGGGTKV
EIK
SEQ ID NO: 103
>12VL4 DIQMTQSPSSLSASVGDRVTITCKSSQSLLNSRTRKNYLAWYQQKPGKSPKLLIY 
WASTRESGVPSRFSGSGSGTDFTLTISSLQPEDVATYYCTQSYILRTFGGGTKVEI
K
SEQ ID NO: 104
>12VL5 
DIVMTQSPDSLAVSLGERATINCKSSQSLLQSRTRKNYLAWYQQKPGQSPKLLIY
WASTRESGVPDRFSGSGSGTDFTLTISSLQAEDVAVYYCTQSYILRTFGGGTKVEI
K
SEQ ID NO: 105
>12VL6 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCTQSYILRTFGGGTKVEI
K
SEQ ID NO: 106
>12VL7 
DIVMTQSPDSLAVSLGERATINCKSSQSLLQSRTRKNYLAWYQQKPGQPPKLLIY
WASTRESGVPDRFSGSGSGTDFTLTISSLQAEDVAVYYCTQSYILRTFGGGTKVEI
K
SEQ ID NO: 107
>12VH1 
EVQLVQSGAEVKKPGASVKVSCKASGYTFTSFWMHWVRQAPGQGLEWMGNI
NLSNGGTNYNEKFKTRVTMTADKSTSTVYMELSSLRSEDTAVYYCARDSYSVY
YFDYWGQGTTVTVSS
SEQ ID NO: 108
>12VH2 
EVQLVQSGAEVKKPGASVKVSCKASGYTFTSFWMHWVRQAPGQGLEWMGNI
NLSNGGTNYNEKFKTRVTLTADKSTSTAYMELSSLRSEDTAVYYCARDSYSVY
YFDYWGQGTTVTVSS
SEQ ID NO: 109
>12VH3 
EVQLVQSGAEVKKPGASVKVSCKASGYNFTSFWMHWVRQAPGQGLEWMGNI
NLSNGGTNYNEKFKTRVTLTADKSTSTAYMELSSLRSEDTAVYYCARDSYSVY
YFDYWGQGTTVTVSS
SEQ ID NO: 110
>12VH4 
EVQLVQSGAEVKKPGASVKVSCKASGYTFTSFWMHWVRQAPGQGLEWMGNI
QLSNGGTNYNEKFKTRVTLTADKSTSTAYMELSSLRSEDTAVYYCARDSYSVY
 
YFDYWGQGTTVTVSS
SEQ ID NO: 111
>12VH5 
EVQLVQSGAEVKKPGASVKVSCKASGYTFTSFWMHWVRQAPGQGLEWMGNI
NLSQGGTNYNEKFKTRVTLTADKSTSTAYMELSSLRSEDTAVYYCARDSYSVY
YFDYWGQGTTVTVSS
SEQ ID NO: 112
>12VH6 
EVQLVQSGAEVKKPGASVKVSCKASGYTFTSFWMHWVRQAPGQGLEWMGNI
NLSNGGTNYNEKFKTRVTLTADKSTSTAYMELSSLRSEDTAVYYCARESYSVY
YFDYWGQGTTVTVSS
SEQ ID NO: 113
>12VH7 
EVQLVQSGAEVKKPGASVKVSCKASGYTFTSFWMHWVRQAPGQGLEWMGNI
QLSQGGTNYNEKFKTRVTLTADKSTSTAYMELSSLRSEDTAVYYCARESYSVY
YFDYWGQGTTVTVSS
SEQ ID NO: 114
>12VH8 
EVQLVQSGAEVKKPGASVKVSCKASGYTFTSFWMHWVRQAPGQGLEWMGNI
NLSNGGTNYNEKFKTRVTLTADKSTSTAYMELSSLTSEDTAVYFCARDSYSVY
YFDYWGQGTTVTVSS
SEQ ID NO: 115
>12VH9 
EVQLVQSGAEVKKPGASVKVSCKASGYTFTSFWMHWVRQAPGQGLEWMGNI
QLSNGGTNYNEKFKTRVTLTADKSTSTAYMELSSLTSEDTAVYFCARDSYSVY
YFDYWGQGTTVTVSS
SEQ ID NO: 116
>12VH10 
EVQLVQSGAEVKKPGASVKVSCKASGYTFTSFWMHWVRQAPGQGLEWMGNI
NLSQGGTNYNEKFKTRVTLTADKSTSTAYMELSSLTSEDTAVYFCARDSYSVY
YFDYWGQGTTVTVSS
SEQ ID NO: 117
>12VH11 
EVQLVQSGAEVKKPGASVKVSCKASGYTFTSFWMHWVRQAPGQGLEWMGNI
NLSNGGTNYNEKFKTRVTLTADKSTSTAYMELSSLTSEDTAVYFCARESYSVY
YFDYWGQGTTVTVSS
 SEQ ID NO: 118
>12VH12

EVQLVQSGAEVKKPGASVKVSCKASGYTFTSFWMHWVRQAPGQGLEWMGNI
QLSQGGTNYNEKFKTRVTLTADKSTSTAYMELSSLTSEDTAVYFCARESYSVY
YFDYWGQGTTVTVSS
SEQ ID NO: 119

Note: In the above sequences, the regions are arranged in the following order: FR1-CDR1-FR2-CDR2-FR3-CDR3-FR4; the CDR sequences determined according to the Kabat numbering scheme are underlined; the FR sequences are not underlined; the amino acid point mutations are double-underlined and shown in bold.

The heavy chain variable region and the light chain variable region of the 12# humanized anti-CD3 antibody were recombined with the heavy chain constant region hIgG1: CH1-Fc-2 (SEQ ID NO: 120) and the light chain constant region kappa CL (SEQ ID NO: 91), respectively, to obtain humanized antibodies as shown in Table 6 below.

**Table 6. 12# humanized antibodies**

| Variable region | VL1 | VL2 | VL3 | VL4 | VL5 | VL6 | VL7 |
|---|---|---|---|---|---|---|---|
| VH1 | | | | | | | |
| VH2 | | 12L2H2 | 12L3H2 | 12L4H2 | 12L5H2 | | |
| VH3 | 12L1H3 | 12L2H3 | 12L3H3 | 12L4H3 | 12L5H3 | | |
| VH4 | | 12L2H4 | 12L3H4 | 12L4H4 | 12L5H4 | | |
| VH5 | | 12L2H5 | 12L3H5 | 12L4H5 | 12L5H5 | | |
| VH6 | 12L1H6 | 12L2H6 | 12L3H6 | 12L4H6 | 12L5H6 | | |
| VH7 | | | | | | | |
| VH8 | | | | | | 12L6H8 | 12L7H8 |
| VH9 | | | | | | 12L6H9 | 12L7H9 |
| VH10 | | | | | | | |
| VH11 | | | | | | 12L6H11 | 12L7H11 |
| VH12 | | | | | | | |

Note: In the table, "12L1H3" represents a humanized antibody whose heavy chain variable region is VH3 (SEQ ID NO: 110), whose light chain variable region is VL1 (SEQ ID NO: 101), whose heavy chain constant region is set forth in SEQ ID NO: 120, and whose light chain constant region is set forth in SEQ ID NO: 91; this similarly applies to the other antibodies.
> hIgG1: CH1-Fc-2:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSRLTVDKSRWQQGNVFSCSVMHEALHNHYTQKSL
SLSPGK
SEQ ID NO: 120

Illustratively, the full-length sequences of the anti-CD3 humanized antibodies are as follows:
> Light chain for 12L1H3 and 12L1H6 (12VL1-CL) 
DIVMTQSPDSLAVSLGERATINCKSSQSLLNSRTRKNYLAWYQQKPGQSPKLLIY
WASTRESGVPDRFSGSGSGTDFTLTISSLQAEDVAVYYCTQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 121
> Light chain for 12L2H2, 12L2H3, 12L2H4, 12L2H5, and 12L2H6 (12VL2-CL) 
DIVMTQSPLSLPVTPGEPASISCKSSQSLLNSRTRKNYLAWYLQKPGQSPQLLIY
WASTRESGVPDRFSGSGSGTDFTLKISRVEAEDVGVYYCTQSYILRTFGGGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 122
> Light chain for 12L3H2, 12L3H3, 12L3H4, 12L3H5, and 12L3H6 (12VL3-CL) 
DIVMTQSPLSLPVTPGEPASISCKSSQSLLNSRTRKNYLAWYQQKPGQSPKLLIY
WASTRESGVPDRFSGSGSGTDFTLKISRVEAEDVGVYYCTQSYILRTFGGGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 123
> Light chain for 12L4H2, 12L4H3, 12L4H4, 12L4H5, and 12L4H6 (12VL4-CL) 
DIQMTQSPSSLSASVGDRVTITCKSSQSLLNSRTRKNYLAWYQQKPGKSPKLLIY
WASTRESGVPSRFSGSGSGTDFTLTISSLQPEDVATYYCTQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 124
> Light chain for 12L5H2, 12L5H3, 12L5H4, 12L5H5, and 12L5H6 (12VL5-CL) DIVMTQSPDSLAVSLGERATINCKSSQSLLQSRTRKNYLAWYQQKPGQSPKLLIY 
WASTRESGVPDRFSGSGSGTDFTLTISSLQAEDVAVYYCTQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 125
> Light chain for 12L6H8, 12L6H9, and 12L6H11 (12VL6-CL) 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCTQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 126
> Light chain for 12L7H8, 12L7H9, and 12L7H11 (12VL7-CL) 
DIVMTQSPDSLAVSLGERATINCKSSQSLLQSRTRKNYLAWYQQKPGQPPKLLIY
WASTRESGVPDRFSGSGSGTDFTLTISSLQAEDVAVYYCTQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 127
> Heavy chain for 12L2H2, 12L3H2, 12L4H2, and 12L5H2 (12VH2-CH1-Fc-2) 
EVQLVQSGAEVKKPGASVKVSCKASGYTFTSFWMHWVRQAPGQGLEWMGNI
NLSNGGTNYNEKFKTRVTLTADKSTSTAYMELSSLRSEDTAVYYCARDSYSVYY
FDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 128
> Heavy chain for 12L1H3, 12L2H3, 12L3H3, 12L4H3, and 12L5H3 (12VH3-CH1-Fc-2) 
EVQLVQSGAEVKKPGASVKVSCKASGYNFTSFWMHWVRQAPGQGLEWMGNI
NLSNGGTNYNEKFKTRVTLTADKSTSTAYMELSSLRSEDTAVYYCARDSYSVYY
FDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
 SEQ ID NO: 129
> Heavy chain for 12L2H4, 12L3H4, 12L4H4, and 12L5H4 (12VH4-CH1-Fc-2) 
EVQLVQSGAEVKKPGASVKVSCKASGYTFTSFWMHWVRQAPGQGLEWMGNI
QLSNGGTNYNEKFKTRVTLTADKSTSTAYMELSSLRSEDTAVYYCARDSYSVYY
FDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 130
> Heavy chain for 12L2H5, 12L3H5, 12L4H5, and 12L5H5 (12VH5-CH1-Fc-2) 
EVQLVQSGAEVKKPGASVKVSCKASGYTFTSFWMHWVRQAPGQGLEWMGNI
NLSQGGTNYNEKFKTRVTLTADKSTSTAYMELSSLRSEDTAVYYCARDSYSVYY
FDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 131
> Heavy chain for 12L1H6, 12L2H6, 12L3H6, 12L4H6, and 12L5H6 (12VH6-CH1-Fc-2) 
EVQLVQSGAEVKKPGASVKVSCKASGYTFTSFWMHWVRQAPGQGLEWMGNI
NLSNGGTNYNEKFKTRVTLTADKSTSTAYMELSSLRSEDTAVYYCARESYSVYY
FDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 132
> Heavy chain for 12L6H8 and 12L7H8 (12VH8-CH1-Fc-2) 
EVQLVQSGAEVKKPGASVKVSCKASGYTFTSFWMHWVRQAPGQGLEWMGNI
NLSNGGTNYNEKFKTRVTLTADKSTSTAYMELSSLTSEDTAVYFCARDSYSVYY
FDYWGQGTTVTVSSASTKGPSVFPLAPSSKSTSGGTAALGCLVKDYFPEPVTVSWNS
 
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 133
> Heavy chain for 12L6H9 and 12L7H9 (12VH9-CH1-Fc-2) 
EVQLVQSGAEVKKPGASVKVSCKASGYTFTSFWMHWVRQAPGQGLEWMGNI
QLSNGGTNYNEKFKTRVTLTADKSTSTAYMELSSLTSEDTAVYFCARDSYSVYY
FDYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 134
> Heavy chain for 12L6H11 and 12L7H11 (12VH11-CH1-Fc-2)

EVQLVQSGAEVKKPGASVKVSCKASGYTFTSFWMHWVRQAPGQGLEWMGNI
NLSNGGTNYNEKFKTRVTLTADKSTSTAYMELSSLTSEDTAVYFCARESYSVYYF
DYWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 135

Note: In the sequences described above, the CDR sequences determined according to the Kabat numbering scheme are underlined, and the antibody constant regions are italicized.

The negative control antibody used in the present disclosure is C25-hIgG1 (in which the VH/VL sequences are derived from the antibody C25 of the patent US6114143A).
>C25-HC 
QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDK
 
THTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK
SEQ ID NO: 397
>C25-LC

DIQMTQSPSSLSASVGDRVTMSCKSSQSLLNSGDQKNYLTWYQQKPGKAPKLLI
YWASTGESGVPSRFSGSGSGTDFTFTISSLQPEDIATYYCQNDYSYPWTFGQGTK
VEIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQ ID NO: 398

### Example 3. Humanization of CD3 Antibody 82#

By alignment with the IMGT human antibody heavy and light chain variable region germline gene database through the MOE software, heavy and light chain variable region germline genes highly homologous with 82# were selected as templates, and CDRs of this murine-derived antibody were grafted into corresponding human-derived templates to form variable region sequences in the following order: FR1-CDR1-FR2-CDR2-FR3-CDR3-FR4. Illustratively, the CDR amino acid residues in the specific examples below were determined and annotated using the Kabat numbering scheme.

For the humanized antibody of antibody 82#, the FR1, FR2, and FR3 of IGKV4-1*01, IGKV2-28*01, or IGKV1-27*01 and the FR4 of IGKJ4*01 or IGKJ1*01 were selected as light chain framework region templates; the FR1, FR2, and FR3 of IGHV1-18*01 or IGHV1-46*01 and the FR4 of IGHJ6*01 or IGHJ2*01 were selected as heavy chain framework region templates. Optionally, the amino acid residue(s) at position(s) 27d, 37, 43, 45, and/or 50 in the light chain variable region of the humanized antibody was/were substituted; and/or the amino acid residue(s) at position(s) 1, 34, 37, 54, 69, 71, 73, 78, 82a, 83, 89, and/or 91 in the heavy chain variable region of the humanized antibody was/were substituted.

**Table 7-1. 82# humanized antibodies**

| **VL** | | **VH** | |
|---|---|---|---|
| VL1 | Graft (IGKV4-1*01) + P43S | VH1 | Graft (IGHV1-18*01) + T71A,T73K+Q1E |
| VL2 | Graft (IGKV2-28*01) | VH2 | Graft (IGHV1-18*01) + V37M,M69L,T71A,T73 K+Q1E |
| VL3 | Graft (IGKV2-28*01) + L37Q,Q45K | VH3 | Graft (IGHV1-18*01) + V37M,M69L,T71A,T73 K + Q1E,D54E |
| VL4 | Graft (IGKV1-27*01) + V43S | VH2_M37V | Graft (IGHV1-18*01) + M69L,T71A,T73K+Q1E |
| VL5 | Graft (IGKV4-1*01) + P43S + N27d Q | | |
| VL6 | Graft (IGKV4-1*01) + P43S + W50Y | | |
| VL7 | Graft (IGKV4-1*01) + P43S + N27d Q,W50Y | | |
| FR4 | IGKJ4*01 | FR4 | IGHJ6*01 |

**Table 7-2. Humanized antibodies of 82**

| VL | | VH | |
|---|---|---|---|
| VL8 | Graft (IGKV4-1*01) | VH4 | Graft (IGHV1-46*01) + M69L,V78A,S82a R,R83T,V89I,Y91F |
| VL9 | Graft (IGKV4-1*01) + N27d Q | VH5 | Graft (IGHV1-46*01) + M69L,V78A,S82a R,R83T,V89I,Y91F + D54E |
| VL10 | Graft (IGKV4-1*01) + W50Y | VH6 | Graft (IGHV1-46*01)+ M69L,V78A,V89I,Y91F + D54E,I34M |
| VL11 | Graft (IGKV4-1*01) + N27d Q,W50Y | VH7 | Graft (IGHV1-46*01) + M69L,V78A,S82a R,R83T + D54E,I34M |
| | | VH8 | Graft (IGHV1-46*01) + M69L,V78A +D54E,I34M |
| | | VH9 | Graft (IGHV1-46*01) + M69L,V78A,S82a R,R83T,V89I,Y91F+Q1E |
| | | VH10 | Graft (IGHV1-46*01) + M69L,V78A,S82a R,R83T,V89I,Y91F + Q1E,D54E |
| | | VH11 | Graft (IGHV1-46*01) + M69L,V78A,S82a R,R83T + Q1E,D54E,I34M |
| | | VH12 | Graft (IGHV1-46*01) + M69L,V78A + Q1E,D54E,I34M |
| | | VH13 | Graft (IGHV1-46*01)+ M69L,V78A,V89I,Y91F + Q1E,D54E,I34M |
| FR4 | IGKJ1*01 | FR4 | IGHJ2*01 |

**Table 8. The CDRs of 82# humanized antibodies**

| Variable region of 82# | CDR | Sequence | SEQ ID NO |
|---|---|---|---|
| VL5/VL7/VL9/VL11 | LCDR1 | KSSQSLL**Q**SRTRKNYLA | SEQ ID NO: 96 |
| VL6/VL7/VL10/VL11 | LCDR2 | **Y**ASTRES | SEQ ID NO: 136 |
| VH6/VH7/VH8/VH11/VH12/ VH13 | HCDR1 | SYY**M**H | SEQ ID NO: 137 |
| VH3/VH5/VH6/VH7/VH8/VH 10/VH11/VH12/VH13 | HCDR2 | WINPG**E**GSIKYNEKFKG | SEQ ID NO: 138 |

>82VL1 
DIVMTQSPDSLAVSLGERATINCKSSQSLLNSRTRKNYLAWYQQKPGQSPKLLIY
WASTRESGVPDRFSGSGSGTDFTLTISSLQAEDVAVYYCKQSFILRTFGGGTKVEI
K
SEQ ID NO: 139
>82VL2 
DIVMTQSPLSLPVTPGEPASISCKSSQSLLNSRTRKNYLAWYLQKPGQSPQLLIY
WASTRESGVPDRFSGSGSGTDFTLKISRVEAEDVGVYYCKQSFILRTFGGGTKVE
IK
SEQ ID NO: 140
>82VL3 
DIVMTQSPLSLPVTPGEPASISCKSSQSLLNSRTRKNYLAWYQQKPGQSPKLLIY
WASTRESGVPDRFSGSGSGTDFTLKISRVEAEDVGVYYCKQSFILRTFGGGTKVE
IK
SEQ ID NO: 141
>82VL4 
DIQMTQSPSSLSASVGDRVTITCKSSQSLLNSRTRKNYLAWYQQKPGKSPKLLIY
WASTRESGVPSRFSGSGSGTDFTLTISSLQPEDVATYYCKQSFILRTFGGGTKVEI
K
SEQ ID NO: 142
>82VL5 
DIVMTQSPDSLAVSLGERATINCKSSQSLLQSRTRKNYLAWYQQKPGQSPKLLIY
WASTRESGVPDRFSGSGSGTDFTLTISSLQAEDVAVYYCKQSFILRTFGGGTKVEI
K
SEQ ID NO: 143
>82VL6 
DIVMTQSPDSLAVSLGERATINCKSSQSLLNSRTRKNYLAWYQQKPGQSPKLLIY
YASTRESGVPDRFSGSGSGTDFTLTISSLQAEDVAVYYCKQSFILRTFGGGTKVEI
K
SEQ ID NO: 144
>82VL7 
DIVMTQSPDSLAVSLGERATINCKSSQSLLQSRTRKNYLAWYQQKPGQSPKLLIY
YASTRESGVPDRFSGSGSGTDFTLTISSLQAEDVAVYYCKQSFILRTFGGGTKVEI
K
SEQ ID NO: 145
>82VL8 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSFILRTFGQGTKVEI
K
SEQ ID NO: 146
>82VL9 
DIVMTQSPDSLAVSLGERATINCKSSQSLLQSRTRKNYLAWYQQKPGQPPKLLIY
WASTRESGVPDRFSGSGSGTDFTLTISSLQAEDVAVYYCKQSFILRTFGQGTKVEI
K
SEQ ID NO: 147
>82VL10 
DIVMTQSPDSLAVSLGERATINCKSSQSLLNSRTRKNYLAWYQQKPGQPPKLLIY
YASTRESGVPDRFSGSGSGTDFTLTISSLQAEDVAVYYCKQSFILRTFGQGTKVEI
K
SEQ ID NO: 148
>82VL11 
DIVMTQSPDSLAVSLGERATINCKSSQSLLQSRTRKNYLAWYQQKPGQPPKLLIY
YASTRESGVPDRFSGSGSGTDFTLTISSLQAEDVAVYYCKQSFILRTFGQGTKVEI
K
SEQ ID NO: 149
>82VH1 
EVQLVQSGAEVKKPGASVKVSCKASGYTFTSYYIHWVRQAPGQGLEWMGWIN
PGDGSIKYNEKFKGRVTMTADKSTSTAYMELRSLRSDDTAVYYCARDGYSYYY
FDFWGQGTTVTVSS
SEQ ID NO: 150
>82VH2 EVQLVQSGAEVKKPGASVKVSCKASGYTFTSYYIHWMRQAPGQGLEWMGWI 
NPGDGSIKYNEKFKGRVTLTADKSTSTAYMELRSLRSDDTAVYYCARDGYSYY
YFDFWGQGTTVTVSS
SEQ ID NO: 151
>82VH2_M37V 
EVQLVQSGAEVKKPGASVKVSCKASGYTFTSYYIHWVRQAPGQGLEWMGWIN
PGDGSIKYNEKFKGRVTLTADKSTSTAYMELRSLRSDDTAVYYCARDGYSYYY
FDFWGQGTTVTVSS
SEQ ID NO: 152
>82VH3 
EVQLVQSGAEVKKPGASVKVSCKASGYTFTSYYIHWMRQAPGQGLEWMGWI
NPGEGSIKYNEKFKGRVTLTADKSTSTAYMELRSLRSDDTAVYYCARDGYSYY
YFDFWGQGTTVTVSS
SEQ ID NO: 153
>82VH4 
QVQLVQSGAEVKKPGASVKVSCKASGYTFTSYYIHWVRQAPGQGLEWMGWIN
PGDGSIKYNEKFKGRVTLTRDTSTSTAYMELRSLTSEDTAIYFCARDGYSYYYF
DFWGQGTLVTVSS
SEQ ID NO: 154
>82VH5 
QVQLVQSGAEVKKPGASVKVSCKASGYTFTSYYIHWVRQAPGQGLEWMGWIN
PGEGSIKYNEKFKGRVTLTRDTSTSTAYMELRSLTSEDTAIYFCARDGYSYYYF
DFWGQGTLVTVSS
SEQ ID NO: 155
>82VH6 
QVQLVQSGAEVKKPGASVKVSCKASGYTFTSYYMHWVRQAPGQGLEWMGWI
NPGEGSIKYNEKFKGRVTLTRDTSTSTAYMELSSLRSEDTAIYFCARDGYSYYY
FDFWGQGTLVTVSS
SEQ ID NO: 156
>82VH7 
QVQLVQSGAEVKKPGASVKVSCKASGYTFTSYYMHWVRQAPGQGLEWMGWI
NPGEGSIKYNEKFKGRVTLTRDTSTSTAYMELRSLTSEDTAVYYCARDGYSYY
YFDFWGQGTLVTVSS
SEQ ID NO: 157
>82VH8 
QVQLVQSGAEVKKPGASVKVSCKASGYTFTSYYMHWVRQAPGQGLEWMGWI
NPGEGSIKYNEKFKGRVTLTRDTSTSTAYMELSSLRSEDTAVYYCARDGYSYYY
 
FDFWGQGTLVTVSS
SEQ ID NO: 158
>82VH9 
EVQLVQSGAEVKKPGASVKVSCKASGYTFTSYYIHWVRQAPGQGLEWMGWIN
PGDGSIKYNEKFKGRVTLTRDTSTSTAYMELRSLTSEDTAIYFCARDGYSYYYF
DFWGQGTLVTVSS
SEQ ID NO: 159
>82VH10 
EVQLVQSGAEVKKPGASVKVSCKASGYTFTSYYIHWVRQAPGQGLEWMGWIN
PGEGSIKYNEKFKGRVTLTRDTSTSTAYMELRSLTSEDTAIYFCARDGYSYYYF
DFWGQGTLVTVSS
SEQ ID NO: 160
>82VH11 
EVQLVQSGAEVKKPGASVKVSCKASGYTFTSYYMHWVRQAPGQGLEWMGWI
NPGEGSIKYNEKFKGRVTLTRDTSTSTAYMELRSLTSEDTAVYYCARDGYSYY
YFDFWGQGTLVTVSS
SEQ ID NO: 161
>82VH12 
EVQLVQSGAEVKKPGASVKVSCKASGYTFTSYYMHWVRQAPGQGLEWMGWI
NPGEGSIKYNEKFKGRVTLTRDTSTSTAYMELSSLRSEDTAVYYCARDGYSYYY
FDFWGQGTLVTVSS
SEQ ID NO: 162
>82VH13

EVQLVQSGAEVKKPGASVKVSCKASGYTFTSYYMHWVRQAPGQGLEWMGWI
NPGEGSIKYNEKFKGRVTLTRDTSTSTAYMELSSLRSEDTAIYFCARDGYSYYY
FDFWGQGTLVTVSS
SEQ ID NO: 349

Note: In the above sequences, the regions are arranged in the following order: FR1-CDR1-FR2-CDR2-FR3-CDR3-FR4; the CDR sequences determined according to the Kabat numbering scheme are underlined; the FR sequences are not underlined; the amino acid point mutations are double-underlined and shown in bold.

The heavy chain variable region and the light chain variable region of the 82# humanized anti-CD3 antibody were recombined with the heavy chain constant region hIgG1: CH1-Fc-2 (SEQ ID NO: 120) and the light chain constant region kappa CL (SEQ ID NO: 91), respectively, to obtain humanized antibodies as shown in Table 9 below.

**Table 9. 82# humanized antibodies**

| Variable region | VL1 | VL2 | VL3 | VL4 | VL5 | VL6 | VL7 | VL8 | VL9 | VL10 | VL11 |
|---|---|---|---|---|---|---|---|---|---|---|---|
| VH1 | 82L1H1 | 82L2H1 | 82L3H1 | 82L4H1 | 82L5H1 | 82L6H1 | 82L7H1 | | | | |
| VH2 | 82L1H2 | 82L2H2 | 82L3H2 | 82L4H2 | 82L5H2 | 82L6H2 | 82L7H2 | 82L8H2 | | | |
| VH2_ M37V | | | | | | 82L6H 2_M37 V | | | | | |
| VH3 | 82L1H 3 | 82L2H 3 | 82L3H 3 | 82L4H 3 | 82L5H 3 | 82L6H 3 | 82L7H 3 | | | | |
| VH4 | | | | | | | | 82L8H 4 | 82L9H 4 | 82L10 H4 | 82L11 H4 |
| VH5 | | | | | | | | 82L8H 5 | 82L9H 5 | 82L10 H5 | 82L11 H5 |
| VH6 | | | | | | | | 82L8H 6 | | | |
| VH7 | | | | | | | | 82L8H 7 | | | |
| VH8 | | | | | | | | | | | |
| VH9 | | | | | | | | | | | |
| VH10 | | | | | | | | | | | |
| VH11 | | | | | | | | | | | |
| VH12 | | | | | | | | | | | |
| VH13 | | | | | | | | | | | |

Note: In the table, "82L1H1" represents a humanized antibody whose heavy chain variable region is VH1 (SEQ ID NO: 150), whose light chain variable region is VL1 (SEQ ID NO: 139), whose heavy chain constant region is set forth in SEQ ID NO: 120, and whose light chain constant region is set forth in SEQ ID NO: 91; this similarly applies to the other antibodies.

Illustratively, the full-length sequences of the anti-CD3 humanized antibodies are as follows:
> Light chain for 82L1H1, 82L1H2, and 82L1H3 (82VL1-CL) 
DIVMTQSPDSLAVSLGERATINCKSSQSLLNSRTRKNYLAWYQQKPGQSPKLLIY
WASTRESGVPDRFSGSGSGTDFTLTISSLQAEDVAVYYCKQSFILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 163
> Light chain for 82L2H1, 82L2H2, and 82L2H3 (82VL2-CL) 
DIVMTQSPLSLPVTPGEPASISCKSSQSLLNSRTRKNYLAWYLQKPGQSPQLLIY
WASTRESGVPDRFSGSGSGTDFTLKISRVEAEDVGVYYCKQSFILRTFGGGTKVE
IKRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 164
> Light chain for 82L3H1, 82L3H2, and 82L3H3 (82VL3-CL) 
DIVMTQSPLSLPVTPGEPASISCKSSQSLLNSRTRKNYLAWYQQKPGQSPKLLIY
WASTRESGVPDRFSGSGSGTDFTLKISRVEAEDVGVYYCKQSFILRTFGGGTKVE
IKRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 165
> Light chain for 82L4H1, 82L4H2, and 82L4H3 (82VL4-CL) 
DIQMTQSPSSLSASVGDRVTITCKSSQSLLNSRTRKNYLAWYQQKPGKSPKLLIY
WASTRESGVPSRFSGSGSGTDFTLTISSLQPEDVATYYCKQSFILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 166
> Light chain for 82L5H1, 82L5H2, and 82L5H3 (82VL5-CL) 
DIVMTQSPDSLAVSLGERATINCKSSQSLLQSRTRKNYLAWYQQKPGQSPKLLIY
WASTRESGVPDRFSGSGSGTDFTLTISSLQAEDVAVYYCKQSFILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 167
> Light chain for 82L6H1, 82L6H2, 82L6H2_M37V, and 82L6H3 (82VL6-CL) 
DIVMTQSPDSLAVSLGERATINCKSSQSLLNSRTRKNYLAWYQQKPGQSPKLLIY
YASTRESGVPDRFSGSGSGTDFTLTISSLQAEDVAVYYCKQSFILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 168
> Light chain for 82L7H1, 82L7H2, and 82L7H3 (82VL7-CL) 
DIVMTQSPDSLAVSLGERATINCKSSQSLLQSRTRKNYLAWYQQKPGQSPKLLIY
YASTRESGVPDRFSGSGSGTDFTLTISSLQAEDVAVYYCKQSFILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 169
> Light chain for 82L8H2, 82L8H4, 82L8H5, 82L8H6, and 82L8H7 (82VL8-CL) 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 170
> Light chain for 82L9H4 and 82L9H5 (82VL9-CL) 
DIVMTQSPDSLAVSLGERATINCKSSQSLLQSRTRKNYLAWYQQKPGQPPKLLIY
WASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 171
> Light chain for 82L10H4 and 82L10H5 (82VL10-CL) 
DIVMTQSPDSLAVSLGERATINCKSSQSLLNSRTRKNYLAWYQQKPGQPPKLLIY
YASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 172
> Light chain for 82L11H4 and 82L11H5 (82VL11-CL) 
DIVMTQSPDSLAVSLGERATINCKSSQSLLQSRTRKNYLAWYQQKPGQPPKLLIY
YASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 173
> Heavy chain for 82L1H1, 82L2H1, 82L3H1, 82L4H1, 82L5H1, 82L6H1, and 82L7H1 (82VH1-CH1-Fc-2) 
EVQLVQSGAEVKKPGASVKVSCKASGYTFTSYYIHWVRQAPGQGLEWMGWIN
PGDGSIKYNEKFKGRVTMTADKSTSTAYMELRSLRSDDTAVYYCARDGYSYYY
FDFWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 174
> Heavy chain for 82L1H2, 82L2H2, 82L3H2, 82L4H2, 82L5H2, 82L6H2, 82L7H2, and 82L8H2 (82VH2-CH1-Fc-2) 
EVQLVQSGAEVKKPGASVKVSCKASGYTFTSYYIHWMRQAPGQGLEWMGWI
NPGDGSIKYNEKFKGRVTLTADKSTSTAYMELRSLRSDDTAVYYCARDGYSYYY
FDFWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 175
> Heavy chain for 82L6H2_M37V (82VH2_M37V-CH1-Fc-2) 
EVQLVQSGAEVKKPGASVKVSCKASGYTFTSYYIHWVRQAPGQGLEWMGWIN
PGDGSIKYNEKFKGRVTLTADKSTSTAYMELRSLRSDDTAVYYCARDGYSYYYF
DFWGQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 176
> Heavy chain for 82L1H3, 82L2H3, 82L3H3, 82L4H3, 82L5H3, 82L6H3, and 82L7H3 (82VH3-CH1-Fc-2) 
EVQLVQSGAEVKKPGASVKVSCKASGYTFTSYYIHWMRQAPGQGLEWMGWI
NPGEGSIKYNEKFKGRVTLTADKSTSTAYMELRSLRSDDTAVYYCARDGYSYYY
FDFWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 177
> Heavy chain for 82L8H4, 82L9H4, 82L10H4, and 82L11H4 (82VH4-CH1-Fc-2) 
QVQLVQSGAEVKKPGASVKVSCKASGYTFTSYYIHWVRQAPGQGLEWMGWIN
PGDGSIKYNEKFKGRVTLTRDTSTSTAYMELRSLTSEDTAIYFCARDGYSYYYFD
FWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNWY
 
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSRLTVDKSRWQQGNVFSCSVMHEALHNHYTQKSLSLSPGK
SEQ ID NO: 178
> Heavy chain for 82L8H5, 82L9H5, 82L10H5, and 82L11H5 (82VH5-CH1-Fc-2) 
QVQLVQSGAEVKKPGASVKVSCKASGYTFTSYYIHWVRQAPGQGLEWMGWIN
PGEGSIKYNEKFKGRVTLTRDTSTSTAYMELRSLTSEDTAIYFCARDGYSYYYFD
FWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSRLTVDKSRWQQGNVFSCSVMHEALHNHYTQKSLSLSPGK
SEQ ID NO: 179
> Heavy chain for 82L8H6 (82VH6-CH1-Fc-2) 
QVQLVQSGAEVKKPGASVKVSCKASGYTFTSYYMHWVRQAPGQGLEWMGWI
NPGEGSIKYNEKFKGRVTLTRDTSTSTAYMELSSLRSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 180
> Heavy chain for 82L8H7 (82VH7-CH1-Fc-2) 
QVQLVQSGAEVKKPGASVKVSCKASGYTFTSYYMHWVRQAPGQGLEWMGWI
NPGEGSIKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 181
>82VH8-CH1-Fc-2 QVQLVQSGAEVKKPGASVKVSCKASGYTFTSYYMHWVRQAPGQGLEWMGWI 
NPGEGSIKYNEKFKGRVTLTRDTSTSTAYMELSSLRSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 350
>82VH9-CH1-Fc-2 
EVQLVQSGAEVKKPGASVKVSCKASGYTFTSYYIHWVRQAPGQGLEWMGWIN
PGDGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 351
>82VH10-CH1-Fc-2 
EVQLVQSGAEVKKPGASVKVSCKASGYTFTSYYIHWVRQAPGQGLEWMGWIN
PGEGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 352
>82VH11-CH1-Fc-2 
EVQLVQSGAEVKKPGASVKVSCKASGYTFTSYYMHWVRQAPGQGLEWMGWI
NPGEGSIKYNEKFKGRVTLTRDTSTSTAYMELRSLTSEDTAVYYCARDGYSYY
YFDFWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPEN
 
NYKTTPPVLDSDGSFFLYSRLTVDKSRWQQGNVFSCSVMHEALHNHYTQKSLSLSPG
K
SEQ ID NO: 353
>82VH12-CH1-Fc-2 
EVQLVQSGAEVKKPGASVKVSCKASGYTFTSYYMHWVRQAPGQGLEWMGWI
NPGEGSIKYNEKFKGRVTLTRDTSTSTAYMELSSLRSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 354
>82VH13-CH1-Fc-2

EVQLVQSGAEVKKPGASVKVSCKASGYTFTSYYMHWVRQAPGQGLEWMGWI
NPGEGSIKYNEKFKGRVTLTRDTSTSTAYMELSSLRSEDTAIYFCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 355

Note: In the sequences described above, the CDR sequences determined according to the Kabat numbering scheme are underlined, and the antibody constant regions are italicized.

### Example 4. CDR and FR Engineering of Anti-CD3 Antibody 82#

### 1. Engineering by removal of TCE and hotspot of CDRs and FRs of anti-CD3 antibody 82#

The CDRs or FRs of the humanized antibodies of antibody 82# were engineered by point mutation to remove TCE and hotspot. Optionally, single-point mutations were combined. Illustratively, the following mutation engineering, including single-point and multi-point combination mutations, was performed on 82L8 and 82L6 to obtain different variable regions and antibodies.

**Table 10-1. CDR and FR engineering of 82# humanized antibodies**

| Variable region of 82# | CDR | Sequence | SEQ ID NO |
|---|---|---|---|
| VL8_S91N | LCDR3 | KQ**N**FILRT | SEQ ID NO: 182 |
| VL8/VL6_F92Y | LCDR3 | KQS**Y**ILRT | SEQ ID NO: 183 |
| VL8_L94I | LCDR3 | KQSFI**I**RT | SEQ ID NO: 184 |
| VL8/VL6_L94V | LCDR3 | KQSFI**V**RT | SEQ ID NO: 185 |
| VL8_S27e D | LCDR1 | KSSQSLLN**D**RTRKNYLA | SEQ ID NO: 186 |
| VL8_S27e E | LCDR1 | KSSQSLLN**E**RTRKNYLA | SEQ ID NO: 187 |
| VL8_S27e K | LCDR1 | KSSQSLLN**K**RTRKNYLA | SEQ ID NO: 188 |
| VL8/VL6_S27e P | LCDR1 | KSSQSLLN**P**RTRKNYLA | SEQ ID NO: 189 |
| VL8_S27e Q | LCDR1 | KSSQSLLN**Q**RTRKNYLA | SEQ ID NO: 190 |
| VL8_S27e R | LCDR1 | KSSQSLLN**R**RTRKNYLA | SEQ ID NO: 191 |
| VL8_S27e W | LCDR1 | KSSQSLLN**W**RTRKNYLA | SEQ ID NO: 192 |
| VL8_W50Y | LCDR2 | **Y**ASTRES | SEQ ID NO: 136 |

Illustratively, the following mutation engineering, including single-point and multi-point combination mutations, was performed on 82H1, 82H2, 82H4, and 82H5 to obtain different variable regions and antibodies.

**Table 10-2. CDR and FR engineering of 82# humanized antibodies**

| Variable region of 82# | CDR/FR | Sequence | SEQ ID NO |
|---|---|---|---|
| VH5/VH10_Y32E | HCDR1 | S**E**YIH | SEQ ID NO: 193 |
| VH5/VH2/VH10_Y32K | HCDR1 | S**K**YIH | SEQ ID NO: 194 |
| VH5/VH10_Y32Q | HCDR1 | S**Q**YIH | SEQ ID NO: 195 |
| VH5/VH10_I34E | HCDR1 | SYY**E**H | SEQ ID NO: 196 |
| VH5/VH1/VH2/VH10_I34N | HCDR1 | SYY**N**H | SEQ ID NO: 197 |
| VH5/VH10_I34Q | HCDR1 | SYY**Q**H | SEQ ID NO: 199 |
| VH5/VH4/VH10_I34M | HCDR1 | SYY**M**H | SEQ ID NO: 137 |
| VH2_A40G | FR2 | WMRQ**G**PGQGLEWMG | SEQ ID NO: 200 |
| VH2_M37V | FR2 | W**V**RQAPGQGLEWMG | SEQ ID NO: 201 |
| VH5 _A40G /VH2_M37V,A40G/VH 10_A40G | FR2 | W**V**RQ**G**PGQGLEWMG | SEQ ID NO: 202 |
| VH2_G55D | HCDR2 | WINPGD**D**SIKYNEKFKG | SEQ ID NO: 38 |
| VH2_T30A | FR1 | EVQLVQSGAEVKKPGASVK VSCKASGYTFA | SEQ ID NO: 203 |
| VH2_T107S | FR4 | WGQG**S**TVTVSS | SEQ ID NO: 204 |

### 2. Engineering by reducing affinity of CDRs and FRs of CD3 antibody 82#

The CDRs or FRs of the humanized antibodies of antibody 82# were engineered by point mutation to reduce the affinity of antibody 82#. Optionally, point mutations were combined. Illustratively, the following mutation engineering, including single-point and multi-point combination mutations, was performed on 82VL8_S27e P,L94V to obtain different variable regions and antibodies.

**Table 11-1. CDR and FR engineering of 82# humanized antibodies**

| Variable region of 82# | CDR | Sequence | SEQ ID NO |
|---|---|---|---|
| VL8_S27e P,L94V,K24T | LCDR1 | **T**SSQSLLN**P**RTRKNYLA | SEQ ID NO: 205 |
| VL8_S27e P,L94V,L27b V | LCDR1 | KSSQSVLNPRTRKNYL A | SEQ ID NO: 206 |
| VL8_S27e P,L94V,T28S | LCDR1 | KSSQSLLN**P**R**S**RKNYLA | SEQ ID NO: 207 |
| VL8_S27e P,L94V,R29Q | LCDR1 | KSSQSLLNPRTQKNYL A | SEQ ID NO: 208 |
| VL8_S27e P,L94V,K30Q | LCDR1 | KSSQSLLNPRTRQNYL A | SEQ ID NO: 209 |
| VL8_S27e P,L94V,L27b V,T28S | LCDR1 | KSSQSVLNPRSRKNYL A | SEQ ID NO: 210 |
| VL8_S27e P,L94V,L27b V,R29Q | LCDR1 | KSSQSVLNPRTQKNYL A | SEQ ID NO: 211 |
| VL8_S27e P,L94V,T28S,R29Q | LCDR1 | KSSQSLLNPRSQKNYL A | SEQ ID NO: 212 |

Illustratively, the following mutation engineering, including single-point and multi-point combination mutations, was performed on 82H7 to obtain different variable regions and antibodies.

**Table 11-2. CDR and FR engineering of 82# humanized antibody**

| Variable region of 82# | CDR | Sequence | SEQ ID NO |
|---|---|---|---|
| VH7/VH11_T28S | FR1 | QVQLVQSGAEVKKPGASVK VSCKASGYSFT | SEQ ID NO: 213 |
| VH7/VH11_N52Q | HCDR2 | WI**Q**PG**E**GSIKYNEKFKG | SEQ ID NO: 214 |
| VH7/VH11_N52S | HCDR2 | WI**S**PG**E**GSIKYNEKFKG | SEQ ID NO: 215 |
| VH7/VH11_S56A | HCDR2 | WINPG**E**G**A**IKYNEKFKG | SEQ ID NO: 216 |
| VH7/VH11_I57T | HCDR2 | WINPG**E**GS**T**KYNEKFKG | SEQ ID NO: 217 |
| VH7/VH11_K58S | HCDR2 | WINPG**E**GSI**S**YNEKFKG | SEQ ID NO: 218 |
| VH7/VH11_F102Y | HCDR3 | DGYSYYYFD**Y** | SEQ ID NO: 43 |
| VH7/VH11_N52Q,I57T | HCDR2 | WI**Q**PG**E**GS**T**KYNEKFKG | SEQ ID NO: 219 |
| VH7/VH11_N52Q,K58S | HCDR2 | WI**Q**PG**E**GSI**S**YNEKFKG | SEQ ID NO: 220 |
| VH7/VH11_I57T,K58S | HCDR2 | WINPG**E**G**STS**YNEKFKG | SEQ ID NO: 221 |

Illustratively, the variable region sequences of various antibodies obtained are as follows:
>82VL8_S91N 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQNFILRTFGQGTKVEI
K
SEQ ID NO: 222
>82VL8_F92Y 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSYILRTFGQGTKVEI
K
SEQ ID NO: 223
>82VL6_F92Y 
DIVMTQSPDSLAVSLGERATINCKSSQSLLNSRTRKNYLAWYQQKPGQSPKLLIY
YASTRESGVPDRFSGSGSGTDFTLTISSLQAEDVAVYYCKQSYILRTFGGGTKVEI
K
SEQ ID NO: 224
>82VL8_L94I 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSFIIRTFGQGTKVEI
K
SEQ ID NO: 225
>82VL8_L94V 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSFIVRTFGQGTKVEI
K
SEQ ID NO: 226
>82VL6_L94V 
DIVMTQSPDSLAVSLGERATINCKSSQSLLNSRTRKNYLAWYQQKPGQSPKLLIY
YASTRESGVPDRFSGSGSGTDFTLTISSLQAEDVAVYYCKQSFIVRTFGGGTKVEI
K
SEQ ID NO: 227
>82VL8_S27e D 
DIVMTQSPDSLAVSLGERATINCKSSQSLLNDRTRKNYLAWYQQKPGQPPKLLI
YWASTRESGVPDRFSGSGSGTDFTLTISSLQAEDVAVYYCKQSFILRTFGQGTKV
EIK
SEQ ID NO: 228
>82VL8_S27e E 
DIVMTQSPDSLAVSLGERATINCKSSQSLLNERTRKNYLAWYQQKPGQPPKLLIY
WASTRESGVPDRFSGSGSGTDFTLTISSLQAEDVAVYYCKQSFILRTFGQGTKVEI
K
SEQ ID NO: 229
>82VL8_S27e K 
DIVMTQSPDSLAVSLGERATINCKSSQSLLNKRTRKNYLAWYQQKPGQPPKLLI
YWASTRESGVPDRFSGSGSGTDFTLTISSLQAEDVAVYYCKQSFILRTFGQGTKV
EIK
SEQ ID NO: 230
>82VL8_S27e P 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSFILRTFGQGTKVEI
K
SEQ ID NO: 231
>82VL6_S27e P 
DIVMTQSPDSLAVSLGERATINCKSSQSLLNPRTRKNYLAWYQQKPGQSPKLLIY
YASTRESGVPDRFSGSGSGTDFTLTISSLQAEDVAVYYCKQSFILRTFGGGTKVEI
K
SEQ ID NO: 232
>82VL8_S27e Q 
DIVMTQSPDSLAVSLGERATINCKSSQSLLNQRTRKNYLAWYQQKPGQPPKLLI
YWASTRESGVPDRFSGSGSGTDFTLTISSLQAEDVAVYYCKQSFILRTFGQGTKV
EIK
SEQ ID NO: 233
>82VL8_S27e R DIVMTQSPDSLAVSLGERATINCKSSQSLLNRRTRKNYLAWYQQKPGQPPKLLI 
YWASTRESGVPDRFSGSGSGTDFTLTISSLQAEDVAVYYCKQSFILRTFGQGTKV
EIK
SEQ ID NO: 234
>82VL8_S27e W 
DIVMTQSPDSLAVSLGERATINCKSSQSLLNWRTRKNYLAWYQQKPGQPPKLLI
YWASTRESGVPDRFSGSGSGTDFTLTISSLQAEDVAVYYCKQSFILRTFGQGTKV
EIK
SEQ ID NO: 235
>82VL8_S27e P,L94V 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSFIVRTFGQGTKVEI
K
SEQ ID NO: 236
>82VL8_S27e P,F92Y 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSYILRTFGQGTKVEI
K
SEQ ID NO: 237
>82VL8_S27e E,F92Y 
DIVMTQSPDSLAVSLGERATINCKSSQSLLNERTRKNYLAWYQQKPGQPPKLLIY
WASTRESGVPDRFSGSGSGTDFTLTISSLQAEDVAVYYCKQSYILRTFGQGTKVEI
K
SEQ ID NO: 238
>82VL8_S27e P,L94I 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSFIIRTFGQGTKVEI
K
SEQ ID NO: 239
>82VL8_S27e P,W50Y 
DIVMTQSPDSLAVSLGERATINCKSSQSLLNPRTRKNYLAWYQQKPGQPPKLLIY
YASTRESGVPDRFSGSGSGTDFTLTISSLQAEDVAVYYCKQSFILRTFGQGTKVEIK
SEQ ID NO: 240
>82VL8_S27e E,L94V 
DIVMTQSPDSLAVSLGERATINCKSSQSLLNERTRKNYLAWYQQKPGQPPKLLIY
WASTRESGVPDRFSGSGSGTDFTLTISSLQAEDVAVYYCKQSFIVRTFGQGTKVEI
K
 SEQ ID NO: 241
>82VL8_S27e P,L94I,W50Y 
DIVMTQSPDSLAVSLGERATINCKSSQSLLNPRTRKNYLAWYQQKPGQPPKLLIY
YASTRESGVPDRFSGSGSGTDFTLTISSLQAEDVAVYYCKQSFIIRTFGQGTKVEI
K
SEQ ID NO: 242
>82VL6_S27e P,F92Y 
DIVMTQSPDSLAVSLGERATINCKSSQSLLNPRTRKNYLAWYQQKPGQSPKLLIY
YASTRESGVPDRFSGSGSGTDFTLTISSLQAEDVAVYYCKQSYILRTFGGGTKVEI
K
SEQ ID NO: 243
>82VL6_S27e P,L94V 
DIVMTQSPDSLAVSLGERATINCKSSQSLLNPRTRKNYLAWYQQKPGQSPKLLIY
YASTRESGVPDRFSGSGSGTDFTLTISSLQAEDVAVYYCKQSFIVRTFGGGTKVEI
K
SEQ ID NO: 244
>82VH5_Y32E 
QVQLVQSGAEVKKPGASVKVSCKASGYTFTSEYIHWVRQAPGQGLEWMGWIN
PGEGSIKYNEKFKGRVTLTRDTSTSTAYMELRSLTSEDTAIYFCARDGYSYYYFD
FWGQGTLVTVSS
SEQ ID NO: 245
>82VH5_Y32K 
QVQLVQSGAEVKKPGASVKVSCKASGYTFTSKYIHWVRQAPGQGLEWMGWIN
PGEGSIKYNEKFKGRVTLTRDTSTSTAYMELRSLTSEDTAIYFCARDGYSYYYFD
FWGQGTLVTVSS
SEQ ID NO: 246
>82VH2_Y32K 
EVQLVQSGAEVKKPGASVKVSCKASGYTFTSKYIHWMRQAPGQGLEWMGWI
NPGDGSIKYNEKFKGRVTLTADKSTSTAYMELRSLRSDDTAVYYCARDGYSYYY
FDFWGQGTTVTVSS
SEQ ID NO: 247
>82VH5_Y32Q 
QVQLVQSGAEVKKPGASVKVSCKASGYTFTSQYIHWVRQAPGQGLEWMGWIN
PGEGSIKYNEKFKGRVTLTRDTSTSTAYMELRSLTSEDTAIYFCARDGYSYYYFD
FWGQGTLVTVSS
SEQ ID NO: 248
>82VH5_I34E 
QVQLVQSGAEVKKPGASVKVSCKASGYTFTSYYEHWVRQAPGQGLEWMGWI
NPGEGSIKYNEKFKGRVTLTRDTSTSTAYMELRSLTSEDTAIYFCARDGYSYYYF
DFWGQGTLVTVSS
SEQ ID NO: 249
>82VH5_I34N 
QVQLVQSGAEVKKPGASVKVSCKASGYTFTSYYNHWVRQAPGQGLEWMGWI
NPGEGSIKYNEKFKGRVTLTRDTSTSTAYMELRSLTSEDTAIYFCARDGYSYYYF
DFWGQGTLVTVSS
SEQ ID NO: 250
>82VH1_I34N 
EVQLVQSGAEVKKPGASVKVSCKASGYTFTSYYNHWVRQAPGQGLEWMGWI
NPGDGSIKYNEKFKGRVTMTADKSTSTAYMELRSLRSDDTAVYYCARDGYSYY
YFDFWGQGTTVTVSS
SEQ ID NO: 251
>82VH2_I34N 
EVQLVQSGAEVKKPGASVKVSCKASGYTFTSYYNHWMRQAPGQGLEWMGWI
NPGDGSIKYNEKFKGRVTLTADKSTSTAYMELRSLRSDDTAVYYCARDGYSYYY
FDFWGQGTTVTVSS
SEQ ID NO: 252
>82VH5_I34Q 
QVQLVQSGAEVKKPGASVKVSCKASGYTFTSYYQHWVRQAPGQGLEWMGWI
NPGEGSIKYNEKFKGRVTLTRDTSTSTAYMELRSLTSEDTAIYFCARDGYSYYYF
DFWGQGTLVTVSS
SEQ ID NO: 253
>82VH4_I34M 
QVQLVQSGAEVKKPGASVKVSCKASGYTFTSYYMHWVRQAPGQGLEWMGWI
NPGDGSIKYNEKFKGRVTLTRDTSTSTAYMELRSLTSEDTAIYFCARDGYSYYYF
DFWGQGTLVTVSS
SEQ ID NO: 254
>82VH5_I34M 
QVQLVQSGAEVKKPGASVKVSCKASGYTFTSYYMHWVRQAPGQGLEWMGWI
NPGEGSIKYNEKFKGRVTLTRDTSTSTAYMELRSLTSEDTAIYFCARDGYSYYYF
DFWGQGTLVTVSS
SEQ ID NO: 255
>82VH10_I34M 
EVQLVQSGAEVKKPGASVKVSCKASGYTFTSYYMHWVRQAPGQGLEWMGWI
NPGEGSIKYNEKFKGRVTLTRDTSTSTAYMELRSLTSEDTAIYFCARDGYSYYYF
DFWGQGTLVTVSS
SEQ ID NO: 356
>82VH5_A40G 
QVQLVQSGAEVKKPGASVKVSCKASGYTFTSYYIHWVRQGPGQGLEWMGWIN
PGEGSIKYNEKFKGRVTLTRDTSTSTAYMELRSLTSEDTAIYFCARDGYSYYYFD
FWGQGTLVTVSS
SEQ ID NO: 256
>82VH2_A40G 
EVQLVQSGAEVKKPGASVKVSCKASGYTFTSYYIHWMRQGPGQGLEWMGWI
NPGDGSIKYNEKFKGRVTLTADKSTSTAYMELRSLRSDDTAVYYCARDGYSYYY
FDFWGQGTTVTVSS
SEQ ID NO: 257
>82VH2_M37V,I34N 
EVQLVQSGAEVKKPGASVKVSCKASGYTFTSYYNHWVRQAPGQGLEWMGWI
NPGDGSIKYNEKFKGRVTLTADKSTSTAYMELRSLRSDDTAVYYCARDGYSYYY
FDFWGQGTTVTVSS
SEQ ID NO: 258
>82VH2_M37V,A40G 
EVQLVQSGAEVKKPGASVKVSCKASGYTFTSYYIHWVRQGPGQGLEWMGWIN
PGDGSIKYNEKFKGRVTLTADKSTSTAYMELRSLRSDDTAVYYCARDGYSYYYF
DFWGQGTTVTVSS
SEQ ID NO: 259
>82VH2_M37V,Y32K 
EVQLVQSGAEVKKPGASVKVSCKASGYTFTSKYIHWVRQAPGQGLEWMGWIN
PGDGSIKYNEKFKGRVTLTADKSTSTAYMELRSLRSDDTAVYYCARDGYSYYYF
DFWGQGTTVTVSS
SEQ ID NO: 260
>82VH2_G55D 
EVQLVQSGAEVKKPGASVKVSCKASGYTFTSYYIHWMRQAPGQGLEWMGWI
NPGDDSIKYNEKFKGRVTLTADKSTSTAYMELRSLRSDDTAVYYCARDGYSYYY
FDFWGQGTTVTVSS
SEQ ID NO: 261
>82VH2_T30A 
EVQLVQSGAEVKKPGASVKVSCKASGYTFASYYIHWMRQAPGQGLEWMGWI
NPGDGSIKYNEKFKGRVTLTADKSTSTAYMELRSLRSDDTAVYYCARDGYSYYY
FDFWGQGTTVTVSS
SEQ ID NO: 262
>82VH2_T107S 
EVQLVQSGAEVKKPGASVKVSCKASGYTFTSYYIHWMRQAPGQGLEWMGWI
NPGDGSIKYNEKFKGRVTLTADKSTSTAYMELRSLRSDDTAVYYCARDGYSYYY
FDFWGQGSTVTVSS
SEQ ID NO: 263
>82VH2_M37V,T30A,G55D 
EVQLVQSGAEVKKPGASVKVSCKASGYTFASYYIHWVRQAPGQGLEWMGWIN
PGDDSIKYNEKFKGRVTLTADKSTSTAYMELRSLRSDDTAVYYCARDGYSYYYF
DFWGQGTTVTVSS
SEQ ID NO: 264
>82VH2_M37V,T30A,G55D,T107S 
EVQLVQSGAEVKKPGASVKVSCKASGYTFASYYIHWVRQAPGQGLEWMGWIN
PGDDSIKYNEKFKGRVTLTADKSTSTAYMELRSLRSDDTAVYYCARDGYSYYYF
DFWGQGSTVTVSS
SEQ ID NO: 265
>82VL8_S27e P,L94V,K24T 
DIVMTQSPDSLAVSLGERATINCTSSQSLLNPRTRKNYLAWYQQKPGQPPKLLIY
WASTRESGVPDRFSGSGSGTDFTLTISSLQAEDVAVYYCKQSFIVRTFGQGTKVEI
K
SEQ ID NO: 266
>82VL8_S27e P,L94V,L27b V 
DIVMTQSPDSLAVSLGERATINCKSSQSVLNPRTRKNYLAWYQQKPGQPPKLLI
YWASTRESGVPDRFSGSGSGTDFTLTISSLQAEDVAVYYCKQSFIVRTFGQGTKV
EIK
SEQ ID NO: 267
>82VL8_S27e P,L94V,T28S 
DIVMTQSPDSLAVSLGERATINCKSSQSLLNPRSRKNYLAWYQQKPGQPPKLLIY
WASTRESGVPDRFSGSGSGTDFTLTISSLQAEDVAVYYCKQSFIVRTFGQGTKVEI
K
SEQ ID NO: 268
>82VL8_S27e P,L94V,R29Q DIVMTQSPDSLAVSLGERATINCKSSQSLLNPRTQKNYLAWYQQKPGQPPKLLIY 
WASTRESGVPDRFSGSGSGTDFTLTISSLQAEDVAVYYCKQSFIVRTFGQGTKVEI
K
SEQ ID NO: 269
>82VL8_S27e P,L94V,K30Q 
DIVMTQSPDSLAVSLGERATINCKSSQSLLNPRTRQNYLAWYQQKPGQPPKLLIY
WASTRESGVPDRFSGSGSGTDFTLTISSLQAEDVAVYYCKQSFIVRTFGQGTKVEI
K
SEQ ID NO: 270
>82VL8_S27e P,L94V,L27b V,T28S 
DIVMTQSPDSLAVSLGERATINCKSSQSVLNPRSRKNYLAWYQQKPGQPPKLLI
YWASTRESGVPDRFSGSGSGTDFTLTISSLQAEDVAVYYCKQSFIVRTFGQGTKV
EIK
SEQ ID NO: 271
>82VL8_S27e P,L94V,L27b V,R29Q 
DIVMTQSPDSLAVSLGERATINCKSSQSVLNPRTQKNYLAWYQQKPGQPPKLLI
YWASTRESGVPDRFSGSGSGTDFTLTISSLQAEDVAVYYCKQSFIVRTFGQGTKV
EIK
SEQ ID NO: 272
>82VL8_S27e P,L94V,T28S,R29Q 
DIVMTQSPDSLAVSLGERATINCKSSQSLLNPRSQKNYLAWYQQKPGQPPKLLIY
WASTRESGVPDRFSGSGSGTDFTLTISSLQAEDVAVYYCKQSFIVRTFGQGTKVEI
K
SEQ ID NO: 273
>82VH7_T28S 
QVQLVQSGAEVKKPGASVKVSCKASGYSFTSYYMHWVRQAPGQGLEWMGWI
NPGEGSIKYNEKFKGRVTLTRDTSTSTAYMELRSLTSEDTAVYYCARDGYSYYY
FDFWGQGTLVTVSS
SEQ ID NO: 274
>82VH7_N52Q 
QVQLVQSGAEVKKPGASVKVSCKASGYTFTSYYMHWVRQAPGQGLEWMGWI
QPGEGSIKYNEKFKGRVTLTRDTSTSTAYMELRSLTSEDTAVYYCARDGYSYYY
FDFWGQGTLVTVSS
SEQ ID NO: 275
>82VH11_N52Q 
EVQLVQSGAEVKKPGASVKVSCKASGYTFTSYYMHWVRQAPGQGLEWMGWI
QPGEGSIKYNEKFKGRVTLTRDTSTSTAYMELRSLTSEDTAVYYCARDGYSYYY
 
FDFWGQGTLVTVSS
SEQ ID NO: 358
>82VH7_N52S 
QVQLVQSGAEVKKPGASVKVSCKASGYTFTSYYMHWVRQAPGQGLEWMGWI
SPGEGSIKYNEKFKGRVTLTRDTSTSTAYMELRSLTSEDTAVYYCARDGYSYYYF
DFWGQGTLVTVSS
SEQ ID NO: 276
>82VH7_S56A 
QVQLVQSGAEVKKPGASVKVSCKASGYTFTSYYMHWVRQAPGQGLEWMGWI
NPGEGAIKYNEKFKGRVTLTRDTSTSTAYMELRSLTSEDTAVYYCARDGYSYYY
FDFWGQGTLVTVSS
SEQ ID NO: 277
>82VH7_I57T 
QVQLVQSGAEVKKPGASVKVSCKASGYTFTSYYMHWVRQAPGQGLEWMGWI
NPGEGSTKYNEKFKGRVTLTRDTSTSTAYMELRSLTSEDTAVYYCARDGYSYYY
FDFWGQGTLVTVSS
SEQ ID NO: 278
>82VH11_I57T 
EVQLVQSGAEVKKPGASVKVSCKASGYTFTSYYMHWVRQAPGQGLEWMGWI
NPGEGSTKYNEKFKGRVTLTRDTSTSTAYMELRSLTSEDTAVYYCARDGYSYYY
FDFWGQGTLVTVSS
SEQ ID NO: 357
>82VH7_K58S 
QVQLVQSGAEVKKPGASVKVSCKASGYTFTSYYMHWVRQAPGQGLEWMGWI
NPGEGSISYNEKFKGRVTLTRDTSTSTAYMELRSLTSEDTAVYYCARDGYSYYYF
DFWGQGTLVTVSS
SEQ ID NO: 279
>82VH11_K58S 
EVQLVQSGAEVKKPGASVKVSCKASGYTFTSYYMHWVRQAPGQGLEWMGWI
NPGEGSISYNEKFKGRVTLTRDTSTSTAYMELRSLTSEDTAVYYCARDGYSYYYF
DFWGQGTLVTVSS
SEQ ID NO: 359
>82VH7_F102Y 
QVQLVQSGAEVKKPGASVKVSCKASGYTFTSYYMHWVRQAPGQGLEWMGWI
NPGEGSIKYNEKFKGRVTLTRDTSTSTAYMELRSLTSEDTAVYYCARDGYSYYY
 
FDYWGQGTLVTVSS
SEQ ID NO: 280
>82VH7_N52Q,I57T 
QVQLVQSGAEVKKPGASVKVSCKASGYTFTSYYMHWVRQAPGQGLEWMGWI
QPGEGSTKYNEKFKGRVTLTRDTSTSTAYMELRSLTSEDTAVYYCARDGYSYYY
FDFWGQGTLVTVSS
SEQ ID NO: 281
>82VH11_N52Q,I57T 
EVQLVQSGAEVKKPGASVKVSCKASGYTFTSYYMHWVRQAPGQGLEWMGWI
QPGEGSTKYNEKFKGRVTLTRDTSTSTAYMELRSLTSEDTAVYYCARDGYSYYY
FDFWGQGTLVTVSS
SEQ ID NO: 360
>82VH7_N52Q,K58S 
QVQLVQSGAEVKKPGASVKVSCKASGYTFTSYYMHWVRQAPGQGLEWMGWI
QPGEGSISYNEKFKGRVTLTRDTSTSTAYMELRSLTSEDTAVYYCARDGYSYYYF
DFWGQGTLVTVSS
SEQ ID NO: 282
>82VH11_N52Q,K58S 
EVQLVQSGAEVKKPGASVKVSCKASGYTFTSYYMHWVRQAPGQGLEWMGWI
QPGEGSISYNEKFKGRVTLTRDTSTSTAYMELRSLTSEDTAVYYCARDGYSYYYF
DFWGQGTLVTVSS
SEQ ID NO: 361
>82VH7_I57T,K58S 
QVQLVQSGAEVKKPGASVKVSCKASGYTFTSYYMHWVRQAPGQGLEWMGWI
NPGEGSTSYNEKFKGRVTLTRDTSTSTAYMELRSLTSEDTAVYYCARDGYSYYY
FDFWGQGTLVTVSS
SEQ ID NO: 283
>82VH11_I57T,K58S

EVQLVQSGAEVKKPGASVKVSCKASGYTFTSYYMHWVRQAPGQGLEWMGWI
NPGEGSTSYNEKFKGRVTLTRDTSTSTAYMELRSLTSEDTAVYYCARDGYSYYY
FDFWGQGTLVTVSS
SEQ ID NO: 362

Note: In the above sequences, the regions are arranged in the following order: FR1-CDR1-FR2-CDR2-FR3-CDR3-FR4; the CDR sequences determined according to the Kabat numbering scheme are underlined, and the portions that are not underlined are the FR sequences.

The heavy chain variable region and the light chain variable region of the engineered anti-CD3 humanized antibody were recombined with the heavy chain constant region hIgG1: CH1-Fc-2 (SEQ ID NO: 120) and the light chain constant region kappa CL (SEQ ID NO: 91), respectively, to obtain the full-length humanized antibody.

Illustratively, the engineered anti-CD3 humanized antibodies are shown in the table below.

**Table 12. Engineering of 82# humanized antibody**

| Engineered antibody | Light chain | Heavy chain |
|---|---|---|
| 82L8_S91N H5 | 82VL8_S91N-CL (SEQ ID NO: 284) | 82VH5-CH1-Fc-2 (SEQ ID NO: 179) |
| 82L8_F92Y H5 | 82VL8_F92Y-CL (SEQ ID NO: 285) | 82VH5-CH1-Fc-2 (SEQ ID NO: 179) |
| 82L8_L94I H5 | 82VL8_L94I-CL (SEQ ID NO: 286) | 82VH5-CH1-Fc-2 (SEQ ID NO: 179) |
| 82L8_L94V H5 | 82VL8_L94V-CL (SEQ ID NO: 287) | 82VH5-CH1-Fc-2 (SEQ ID NO: 179) |
| 82L8_S27e D H5 | 82VL8_S27e D-CL (SEQ ID NO: 288) | 82VH5-CH1-Fc-2 (SEQ ID NO: 179) |
| 82L8_S27e E H5 | 82VL8_S27e E-CL (SEQ ID NO: 289) | 82VH5-CH1-Fc-2 (SEQ ID NO: 179) |
| 82L8_S27e K H5 | 82VL8_S27e K-CL (SEQ ID NO: 290) | 82VH5-CH1-Fc-2 (SEQ ID NO: 179) |
| 82L8_S27e P H5 | 82VL8_S27e P-CL (SEQ ID NO: 291) | 82VH5-CH1-Fc-2 (SEQ ID NO: 179) |
| 82L8_S27e Q H5 | 82VL8_S27e Q-CL (SEQ ID NO: 292) | 82VH5-CH1-Fc-2 (SEQ ID NO: 179) |
| 82L8_S27e R H5 | 82VL8_S27e R-CL (SEQ ID NO: 293) | 82VH5-CH1-Fc-2 (SEQ ID NO: 179) |
| 82L8_S27e W H5 | 82VL8_S27e W-CL (SEQ ID NO: 294) | 82VH5-CH1-Fc-2 (SEQ ID NO: 179) |
| 82L8H5_Y32E | 82VL8-CL (SEQ ID NO: 170) | 82VH5_Y32E-CH1-Fc-2 (SEQ ID NO: 295) |
| 82L8H5_Y32K | 82VL8-CL (SEQ ID NO: 170) | 82VH5_Y32K-CH1-Fc-2 (SEQ ID NO: 296) |
| 82L8H5_Y32Q | 82VL8-CL (SEQ ID NO: 170) | 82VH5_Y32Q-CH1-Fc-2 (SEQ ID NO: 297) |
| 82L8H5_I34E | 82VL8-CL (SEQ ID NO: 170) | 82VH5_I34E-CH1-Fc-2 (SEQ ID NO: 298) |
| 82L8H5_I34N | 82VL8-CL (SEQ ID NO: 170) | 82VH5_I34N-CH1-Fc-2 (SEQ ID NO: 299) |
| 82L8H5_I34Q | 82VL8-CL (SEQ ID NO: 170) | 82VH5_I34Q-CH1-Fc-2 (SEQ ID NO: 300) |
| 82L8H5_I34M | 82VL8-CL (SEQ ID NO: 170) | 82VH5_I34M-CH1-Fc-2 (SEQ ID NO: 301) |
| 82L8H5_A40G | 82VL8-CL (SEQ ID NO: 170) | 82VH5_A40G-CH1-Fc-2 (SEQ ID NO: 302) |
| 82L8_S27e D H5_I34M | 82VL8_S27e D-CL (SEQ ID NO: 288) | 82VH5_I34M-CH1-Fc-2 (SEQ ID NO: 301) |
| 82L8_S27e E H5_I34M | 82VL8_S27e E-CL (SEQ ID NO: 289) | 82VH5_I34M-CH1-Fc-2 (SEQ ID NO: 301) |
| 82L8_S27e K H5_I34M | 82VL8_S27e K-CL (SEQ ID NO: 290) | 82VH5_I34M-CH1-Fc-2 (SEQ ID NO: 301) |
| 82L8_S27e P H5_I34M | 82VL8_S27e P-CL (SEQ ID NO: 291) | 82VH5_I34M-CH1-Fc-2 (SEQ ID NO: 301) |
| 82L8_S27e Q H5_I34M | 82VL8_S27e Q-CL (SEQ ID NO: 292) | 82VH5_I34M-CH1-Fc-2 (SEQ ID NO: 301) |
| 82L8_S27e R H5_I34M | 82VL8_S27e R-CL (SEQ ID NO: 293) | 82VH5_I34M-CH1-Fc-2 (SEQ ID NO: 301) |
| 82L8_S27e W H5_I34M | 82VL8_S27e W-CL (SEQ ID NO: 294) | 82VH5_I34M-CH1-Fc-2 (SEQ ID NO: 301) |
| 82L8_S27e K H5_I34E | 82VL8_S27e K-CL (SEQ ID NO: 290) | 82VH5_I34E-CH1-Fc-2 (SEQ ID NO: 298) |
| 82L8_S27e Q H5_I34E | 82VL8_S27e Q-CL (SEQ ID NO: 292) | 82VH5_I34E-CH1-Fc-2 (SEQ ID NO: 298) |
| 82L8_S27e R H5_I34E | 82VL8_S27e R-CL (SEQ ID NO: 293) | 82VH5_I34E-CH1-Fc-2 (SEQ ID NO: 298) |
| 82L8_S27e W H5_I34E | 82VL8_S27e W-CL (SEQ ID NO: 294) | 82VH5_I34E-CH1-Fc-2 (SEQ ID NO: 298) |
| 82L8_S27e P,L94V H7 | 82VL8_S27e P,L94V-CL (SEQ ID NO: 303) | 82VH7-CH1-Fc-2 (SEQ ID NO: 181) |
| 82L8_S27e P,F92Y H5_I34M | 82VL8_S27e (SEQ ID NO: P,F92Y-CL 304) | 82VH5_I34M-CH1-Fc-2 (SEQ ID NO: 301) |
| 82L8_S27e P,L94V H5_I34M | 82VL8_S27e P,L94V-CL (SEQ ID NO: 303) | 82VH5_I34M-CH1-Fc-2 (SEQ ID NO: 301) |
| 82L8_S27e E,F92Y H5_I34M | 82VL8_S27e E,F92Y-CL (SEQ ID NO: 305) | 82VH5_I34M-CH1-Fc-2 (SEQ ID NO: 301) |
| 82L8_S27e P,L94I H5_I34M | 82VL8_S27e P,L94I-CL (SEQ ID NO: 306) | 82VH5_I34M-CH1-Fc-2 (SEQ ID NO: 301) |
| 82L8_S27e P,W50Y H5_I34M | 82VL8_S27e P,W50Y-CL (SEQ ID NO: 307) | 82VH5_I34M-CH1-Fc-2 (SEQ ID NO: 301) |
| 82L8_S27e E,L94V H5_I34M | 82VL8_S27e E,L94V-CL (SEQ ID NO: 308) | 82VH5_I34M-CH1-Fc-2 (SEQ ID NO: 301) |
| 82L8_S27e E,F92Y H4_I34M | 82VL8_S27e E,F92Y-CL (SEQ ID NO: 305) | 82VH4_I34M-CH1-Fc-2 (SEQ ID NO: 309) |
| 82L8_S27e P,W50Y H4_I34M | 82VL8_S27e P,W50Y-CL (SEQ ID NO: 307) | 82VH4_I34M-CH1-Fc-2 (SEQ ID NO: 309) |
| 82L8_S27e E,L94V H4_I34M | 82VL8_S27e E,L94V-CL (SEQ ID NO: 308) | 82VH4_I34M-CH1-Fc-2 (SEQ ID NO: 309) |
| 82L8_S27e E H7 | 82VL8_S27e E-CL (SEQ ID NO: 289) | 82VH7-CH1-Fc-2 (SEQ ID NO: 181) |
| 82L8_S27e E H6 | 82VL8_S27e E-CL (SEQ ID NO: 289) | 82VH6-CH1-Fc-2 (SEQ ID NO: 180) |
| 82L8_S27e P H7 | 82VL8_S27e P-CL (SEQ ID NO: 291) | 82VH7-CH1-Fc-2 (SEQ ID NO: 181) |
| 82L6H2_M37V | 82VL6-CL (SEQ ID NO: 168) | 82VH2_M37V-CH1-Fc-2 (SEQ ID NO: 176) |
| 82L8_S27e E H2_M37V | 82VL8_S27e E-CL (SEQ ID NO: 289) | 82VH2_M37V-CH1-Fc-2 (SEQ ID NO: 176) |
| 82L8_S27e P H2_M37V | 82VL8_S27e P-CL (SEQ ID NO: 291) | 82VH2_M37V-CH1-Fc-2 (SEQ ID NO: 176) |
| 82L8_S27e E H2 | 82VL8_S27e E-CL (SEQ ID NO: 289) | 82VH2-CH1-Fc-2 (SEQ ID NO: 175) |
| 82L8_S27e P H2 | 82VL8_S27e P-CL (SEQ ID NO: 291) | 82VH2-CH1-Fc-2 (SEQ ID NO: 175) |
| 82L8_S27e P,L94I,W50Y H2_M37V,I34N | 82VL8_S27e P,L94I,W50Y- CL (SEQ ID NO: 310) | 82VH2_M37V,I34N-CH1- Fc-2 (SEQ ID NO: 311) |
| 82L8_S27e P,L94I,W50Y H2_M37V,A40G | 82VL8_S27e P,L94I,W50Y- CL (SEQ ID NO: 310) | 82VH2_M37V,A40G-CH1- Fc-2 (SEQ ID NO: 312) |
| 82L8_S27e P,F92Y H2_M37V,I34N | 82VL8_S27e P,F92Y-CL (SEQ ID NO: 304) | 82VH2_M37V,I34N-CH1- Fc-2 (SEQ ID NO: 311) |
| 82L8_S27e P,L94V H2_M37V,I34N | 82VL8_S27e P,L94V-CL (SEQ ID NO: 303) | 82VH2_M37V,I34N-CH1- Fc-2 (SEQ ID NO: 311) |
| 82L8_S27e P,L94I H2_M37V,I34N | 82VL8_S27e P,L94I-CL (SEQ ID NO: 306) | 82VH2_M37V,I34N-CH1- Fc-2 (SEQ ID NO: 311) |
| 82L8_S27e E,F92Y H2_M37V,A40G | 82VL8_S27e E,F92Y-CL (SEQ ID NO: 305) | 82VH2_M37V,A40G-CH1- Fc-2 (SEQ ID NO: 312) |
| 82L6_S27e P,F92Y H2_M37V,Y32K | 82VL6_S27e P,F92Y-CL (SEQ ID NO: 313) | 82VH2_M37V,Y32K-CH1- Fc-2 (SEQ ID NO: 314) |
| 82L8_S27e E,L94V H2_M37V,A40G | 82VL8_S27e E,L94V-CL (SEQ ID NO: 308) | 82VH2_M37V,A40G-CH1- Fc-2 (SEQ ID NO: 312) |
| 82L6_S27e P,F92Y H2_M37V,A40G | 82VL6_S27e P,F92Y-CL (SEQ ID NO: 313) | 82VH2_M37V,A40G-CH1- Fc-2 (SEQ ID NO: 312) |
| 82L6_S27e P,F92Y H1_I34N | 82VL6_S27e P,F92Y-CL (SEQ ID NO: 313) | 82VH1_I34N-CH1-Fc-2 (SEQ ID NO: 315) |
| 82L6_S27e P,L94V H2_M37V,Y32K | 82VL6_S27e P,L94V-CL (SEQ ID NO: 316) | 82VH2_M37V,Y32K-CH1- Fc-2 (SEQ ID NO: 314) |
| 82L6_S27e P,L94V H2_M37V,A40G | 82VL6_S27e P,L94V-CL (SEQ ID NO: 316) | 82VH2_M37V,A40G-CH1- Fc-2 (SEQ ID NO: 312) |
| 82L6_S27e P,L94V H1_I34N | 82VL6_S27e P,L94V-CL (SEQ ID NO: 316) | 82VH1_I34N-CH1-Fc-2 (SEQ ID NO: 315) |
| 82L6_S27e P,F92Y H2_M37V,I34N | 82VL6_S27e P,F92Y-CL (SEQ ID NO: 313) | 82VH2_M37V,I34N-CH1- Fc-2 (SEQ ID NO: 311) |
| 82L6_S27e P,L94V H2_M37V,I34N | 82VL6_S27e P,L94V-CL (SEQ ID NO: 316) | 82VH2_M37V,I34N-CH1- Fc-2 (SEQ ID NO: 311) |
| 82L8_S27e P,L94V,K24T H7 | 82VL8_S27e P,L94V,K24T- CL (SEQ ID NO: 317) | 82VH7-CH1-Fc-2 (SEQ ID NO: 181) |
| 82L8_S27e P,L94V,L27b V H7 | 82VL8_S27e P,L94V,L27b V- CL (SEQ ID NO: 318) | 82VH7-CH1-Fc-2 (SEQ ID NO: 181) |
| 82L8_S27e P,L94V,T28S H7 | 82VL8_S27e P,L94V,T28S- CL (SEQ ID NO: 319) | 82VH7-CH1-Fc-2 (SEQ ID NO: 181) |
| 82L8_S27e P,L94V,R29Q H7 | 82VL8_S27e P,L94V,R29Q- CL (SEQ ID NO: 320) | 82VH7-CH1-Fc-2 (SEQ ID NO: 181) |
| 82L8_S27e P,L94V,K30Q H7 | 82VL8_S27e P,L94V,K30Q- CL (SEQ ID NO: 321) | 82VH7-CH1-Fc-2 (SEQ ID NO: 181) |
| 82L8_S27e P,L94V H7_T28S | 82VL8_S27e P,L94V-CL (SEQ ID NO: 303) | 82VH7_T28S-CH1-Fc-2 (SEQ ID NO: 322) |
| 82L8_S27e P,L94V H7_N52Q | 82VL8_S27e P,L94V-CL (SEQ ID NO: 303) | 82VH7_N52Q-CH1-Fc-2 (SEQ ID NO: 323) |
| 82L8_S27e P,L94V H7_N52S | 82VL8_S27e P,L94V-CL (SEQ ID NO: 303) | 82VH7_N52S-CH1-Fc-2 (SEQ ID NO: 324) |
| 82L8_S27e P,L94V H7_S56A | 82VL8_S27e P,L94V-CL (SEQ ID NO: 303) | 82VH7_S56A-CH1-Fc-2 (SEQ ID NO: 325) |
| 82L8_S27e P,L94V H7_I57T | 82VL8_S27e P,L94V-CL (SEQ ID NO: 303) | 82VH7_I57T-CH1-Fc-2 (SEQ ID NO: 326) |
| 82L8_S27e P,L94V H7_K58S | 82VL8_S27e P,L94V-CL (SEQ ID NO: 303) | 82VH7_K58S-CH1-Fc-2 (SEQ ID NO: 327) |
| 82L8_S27e P,L94V H7_F102Y | 82VL8_S27e P,L94V-CL (SEQ ID NO: 303) | 82VH7_F102Y-CH1-Fc-2 (SEQ ID NO: 328) |
| 82L8_S27e P,L94V,T28S H7_N52Q | 82VL8_S27e P,L94V,T28S- CL (SEQ ID NO: 319) | 82VH7_N52Q-CH1-Fc-2 (SEQ ID NO: 323) |
| 82L8_S27e P,L94V,T28S H7_I57T | 82VL8_S27e P,L94V,T28S- CL (SEQ ID NO: 319) | 82VH7_I57T-CH1-Fc-2 (SEQ ID NO: 326) |
| 82L8_S27e P,L94V,T28S H7_K58S | 82VL8_S27e P,L94V,T28S- CL (SEQ ID NO: 319) | 82VH7_K58S-CH1-Fc-2 (SEQ ID NO: 327) |
| 82L8_S27e P,L94V,R29Q H7_K58S | 82VL8_S27e P,L94V,R29Q- CL (SEQ ID NO: 320) | 82VH7_K58S-CH1-Fc-2 (SEQ ID NO: 327) |
| 82L8_S27e P,L94V,L27b V,T28S H7 | 82VL8_S27e P,L94V,L27b V,T28S-CL (SEQ ID NO: 329) | 82VH7-CH1-Fc-2 (SEQ ID NO: 181) |
| 82L8_S27e P,L94V,L27b V,R29Q H7 | 82VL8_S27e P,L94V,L27b V,R29Q-CL (SEQ ID NO: 330) | 82VH7-CH1-Fc-2 (SEQ ID NO: 181) |
| 82L8_S27e P,L94V H7_N52Q,I57T | 82VL8_S27e P,L94V-CL (SEQ ID NO: 303) | 82VH7_N52Q,I57T-CH1- Fc-2 (SEQ ID NO: 331) |
| 82L8_S27e P,L94V H7_N52Q,K58S | 82VL8_S27e P,L94V-CL (SEQ ID NO: 303) | 82VH7_N52Q,K58S-CH1- Fc-2 (SEQ ID NO: 332) |
| 82L8_S27e P,L94V H7_I57T,K58S | 82VL8_S27e P,L94V-CL (SEQ ID NO: 303) | 82VH7_I57T,K58S-CH1- Fc-2 (SEQ ID NO: 333) |
| 82L8_S27e P,L94V,R29Q H7_N52Q | 82VL8_S27e P,L94V,R29Q- CL (SEQ ID NO: 320) | 82VH7_N52Q-CH1-Fc-2 (SEQ ID NO: 323) |
| 82L8_S27e P,L94V,R29Q H7_I57T | 82VL8_S27e P,L94V,R29Q- CL (SEQ ID NO: 320) | 82VH7_I57T-CH1-Fc-2 (SEQ ID NO: 326) |
| 82L8_S27e P,L94V,L27b V H7_N52Q | 82VL8_S27e P,L94V,L27b V- CL (SEQ ID NO: 318) | 82VH7_N52Q-CH1-Fc-2 (SEQ ID NO: 323) |
| 82L8_S27e P,L94V,L27b V H7_I57T | 82VL8_S27e P,L94V,L27b V- CL (SEQ ID NO: 318) | 82VH7_I57T-CH1-Fc-2 (SEQ ID NO: 326) |
| 82L8_S27e P,L94V,L27b V H7_K58S | 82VL8_S27e P,L94V,L27b V- CL (SEQ ID NO: 318) | 82VH7_K58S-CH1-Fc-2 (SEQ ID NO: 327) |
| 82L8_S27e P,L94V,T28S,R29Q H7 | 82VL8_S27e P,L94V,T28S,R29Q-CL (SEQ ID NO: 334) | 82VH7-CH1-Fc-2 (SEQ ID NO: 181) |
| 82L8_S27e P,L94V,L27b V H7_N52Q,K58S | 82VL8_S27e P,L94V,L27b V- CL (SEQ ID NO: 318) | 82VH7_N52Q,K58S-CH1- Fc-2 (SEQ ID NO: 332) |
| 82L8_S27e P,L94V,T28S H7_N52Q,K58S | 82VL8_S27e P,L94V,T28S- CL (SEQ ID NO: 319) | 82VH7_N52Q,K58S-CH1- Fc-2 (SEQ ID NO: 332) |
| 82L8_S27e P,L94V,R29Q H7_N52Q,K58S | 82VL8_S27e P,L94V,R29Q- CL (SEQ ID NO: 320) | 82VH7_N52Q,K58S-CH1- Fc-2 (SEQ ID NO: 332) |
| 82L8_S27e P,L94V,L27b V,T28S H7_N52Q,K58S | 82VL8_S27e P,L94V,L27b V,T28S-CL (SEQ ID NO: 329) | 82VH7_N52Q,K58S-CH1- Fc-2 (SEQ ID NO: 332) |
| 82L8H2_M37V,T30A,G55D | 82VL8-CL (SEQ ID NO: 170) | 82VH2_M37V,T30A,G55D- CH1-Fc-2 (SEQ ID NO: 335) |
| 82L8H2_M37V,T30A,G55D, T107S | 82VL8-CL (SEQ ID NO: 170) | 82VH2_M37V,T30A,G55D, T107S-CH1-Fc-2 (SEQ ID NO: 336) |
| 82L8_S27e P,L94V H10_I34M | 82VL8_S27e P,L94V-CL (SEQ ID NO: 303) | 82VH10_I34M-CH1-Fc-2 (SEQ ID NO: 363) |
| 82L8_S27e P,F92Y H10_I34M | 82VL8_S27e P,F92Y-CL (SEQ ID NO: 304) | 82VH10_I34M-CH1-Fc-2 (SEQ ID NO: 363) |
| 82L8_S27e E,F92Y H10_I34M | 82VL8_S27e E,F92Y-CL (SEQ ID NO: 305) | 82VH10_I34M-CH1-Fc-2 (SEQ ID NO: 363) |
| 82L8_S27e P,L94V,T28S H11_I57T | 82VL8_S27e P,L94V,T28S- CL (SEQ ID NO: 319) | 82VH11_I57T-CH1-Fc-2 (SEQ ID NO: 365) |
| 82L8_S27e P,L94V,R29Q H11_N52Q | 82VL8_S27e P,L94V,R29Q- CL (SEQ ID NO: 320) | 82VH11_N52Q-CH1-Fc-2 (SEQ ID NO: 364) |
| 82L8_S27e P,L94V,R29Q H11_I57T | 82VL8_S27e P,L94V,R29Q- CL (SEQ ID NO: 320) | 82VH11_I57T-CH1-Fc-2 (SEQ ID NO: 365) |
| 82L8_S27e P,L94V,R29Q H11_K58S | 82VL8_S27e P,L94V,R29Q- CL (SEQ ID NO: 320) | 82VH11_K58S-CH1-Fc-2 (SEQ ID NO: 366) |
| 82L8_S27e P,L94V H11 | 82VL8_S27e P,L94V-CL (SEQ ID NO: 303) | 82VH11-CH1-Fc-2 (SEQ ID NO: 353) |
| 82L8_S27e P,L94V,T28S H11_N52Q | 82VL8_S27e P,L94V,T28S- CL (SEQ ID NO: 319) | 82VH11_N52Q-CH1-Fc-2 (SEQ ID NO: 364) |
| 82L8_S27e P,L94V,T28S H11_K58S | 82VL8_S27e P,L94V,T28S- CL (SEQ ID NO: 319) | 82VH11_K58S-CH1-Fc-2 (SEQ ID NO: 366) |
| 82L8_S27e P,L94V,L27b V,T28S H11 | 82VL8_S27e P,L94V,L27b V,T28S-CL (SEQ ID NO: 329) | 82VH11-CH1-Fc-2 (SEQ ID NO: 353) |
| 82L8_S27e P,L94V,L27b V,R29Q H11 | 82VL8_S27e P,L94V,L27b V,R29Q-CL (SEQ ID NO: 330) | 82VH11-CH1-Fc-2 (SEQ ID NO: 353) |
| 82L8_S27e P,L94V H11_N52Q,I57T | 82VL8_S27e P,L94V-CL (SEQ ID NO: 303) | 82VH11_N52Q,I57T-CH1- Fc-2 (SEQ ID NO: 367) |
| 82L8_S27e P,L94V H11_N52Q,K58S | 82VL8_S27e P,L94V-CL (SEQ ID NO: 303) | 82VH11_N52Q,K58S-CH1- Fc-2 (SEQ ID NO: 368) |
| 82L8_S27e P,L94V H11_I57T,K58S | 82VL8_S27e P,L94V-CL (SEQ ID NO: 303) | 82VH11_I57T,K58S-CH1- Fc-2 (SEQ ID NO: 369) |
| 82L8_S27e P,L94V,L27b V H11_N52Q,K58S | 82VL8_S27e P,L94V,L27b V- CL (SEQ ID NO: 318) | 82VH11_N52Q,K58S-CH1- Fc-2 (SEQ ID NO: 368) |
| 82L8_S27e P,L94V,T28S H11_N52Q,K58S | 82VL8_S27e P,L94V,T28S- CL (SEQ ID NO: 319) | 82VH11_N52Q,K58S-CH1- Fc-2 (SEQ ID NO: 368) |
| 82L8_S27e P,L94V,R29Q H11_N52Q,K58S | 82VL8_S27e P,L94V,R29Q- CL (SEQ ID NO: 320) | 82VH11_N52Q,K58S-CH1- Fc-2 (SEQ ID NO: 368) |

The specific full-length sequences are as follows:
>82VL8_S91N-CL 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQNFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 284
>82VL8_F92Y-CL 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSYILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
 SEQ ID NO: 285
>82VL6_F92Y-CL 
DIVMTQSPDSLAVSLGERATINCKSSQSLLNSRTRKNYLAWYQQKPGQSPKLLIY
YASTRESGVPDRFSGSGSGTDFTLTISSLQAEDVAVYYCKQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 198
>82VL8_L94I-CL 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSFIIRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 286
>82VL8_L94V-CL 
DIVMTQSPDSLAVSLGERATINCKSSQSLLNSRTRKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 287
>82VL6_L94V-CL 
DIVMTQSPDSLAVSLGERATINCKSSQSLLNSRTRKNYLAWYQQKPGQSPKLLIY
YASTRESGVPDRFSGSGSGTDFTLTISSLQAEDVAVYYCKQSFIVRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 342
>82VL8_S27e D-CL 
DIVMTQSPDSLAVSLGERATINCKSSQSLLNDRTRKNYLAWYQQKPGQPPKLLI
YWASTRESGVPDRFSGSGSGTDFTLTISSLQAEDVAVYYCKQSFILRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 288
>82VL8_S27e E-CL 
DIVMTQSPDSLAVSLGERATINCKSSQSLLNERTRKNYLAWYQQKPGQPPKLLIY
WASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
 SEQ ID NO: 289
>82VL8_S27e K-CL 
DIVMTQSPDSLAVSLGERATINCKSSQSLLNKRTRKNYLAWYQQKPGQPPKLLI
YWASTRESGVPDRFSGSGSGTDFTLTISSLQAEDVAVYYCKQSFILRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 290
>82VL8_S27e P-CL 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 291
>82VL8_S27e Q-CL 
DIVMTQSPDSLAVSLGERATINCKSSQSLLNQRTRKNYLAWYQQKPGQPPKLLI
YWASTRESGVPDRFSGSGSGTDFTLTISSLQAEDVAVYYCKQSFILRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 292
>82VL8_S27e R-CL 
DIVMTQSPDSLAVSLGERATINCKSSQSLLNRRTRKNYLAWYQQKPGQPPKLLI
YWASTRESGVPDRFSGSGSGTDFTLTISSLQAEDVAVYYCKQSFILRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 293
>82VL8_S27e W-CL 
DIVMTQSPDSLAVSLGERATINCKSSQSLLNWRTRKNYLAWYQQKPGQPPKLLI
YWASTRESGVPDRFSGSGSGTDFTLTISSLQAEDVAVYYCKQSFILRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 294
>82VH5_Y32E-CH1-Fc-2 
QVQLVQSGAEVKKPGASVKVSCKASGYTFTSEYIHWVRQAPGQGLEWMGWIN
PGEGSIKYNEKFKGRVTLTRDTSTSTAYMELRSLTSEDTAIYFCARDGYSYYYFD
FWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
 
KTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSRLTVDKSRWQQGNVFSCSVMHEALHNHYTQKSLSLSPGK
SEQ ID NO: 295
>82VH5_Y32K-CH1-Fc-2 
QVQLVQSGAEVKKPGASVKVSCKASGYTFTSKYIHWVRQAPGQGLEWMGWIN
PGEGSIKYNEKFKGRVTLTRDTSTSTAYMELRSLTSEDTAIYFCARDGYSYYYFD
FWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSRLTVDKSRWQQGNVFSCSVMHEALHNHYTQKSLSLSPGK
SEQ ID NO: 296
>82VH5_Y32Q-CH1-Fc-2 
QVQLVQSGAEVKKPGASVKVSCKASGYTFTSQYIHWVRQAPGQGLEWMGWIN
PGEGSIKYNEKFKGRVTLTRDTSTSTAYMELRSLTSEDTAIYFCARDGYSYYYFD
FWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSRLTVDKSRWQQGNVFSCSVMHEALHNHYTQKSLSLSPGK
SEQ ID NO: 297
>82VH5_I34E-CH1-Fc-2 
QVQLVQSGAEVKKPGASVKVSCKASGYTFTSYYEHWVRQAPGQGLEWMGWI
NPGEGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 298
>82VH5_I34N-CH1-Fc-2 
QVQLVQSGAEVKKPGASVKVSCKASGYTFTSYYNHWVRQAPGQGLEWMGWI
NPGEGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 299
>82VH5_I34Q-CH1-Fc-2 
QVQLVQSGAEVKKPGASVKVSCKASGYTFTSYYQHWVRQAPGQGLEWMGWI
NPGEGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 300
>82VH5_I34M-CH1-Fc-2 
QVQLVQSGAEVKKPGASVKVSCKASGYTFTSYYMHWVRQAPGQGLEWMGWI
NPGEGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 301
>82VH10_I34M-CH1-Fc-2 
EVQLVQSGAEVKKPGASVKVSCKASGYTFTSYYMHWVRQAPGQGLEWMGWI
NPGEGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
 
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 363
>82VH5_A40G-CH1-Fc-2 
QVQLVQSGAEVKKPGASVKVSCKASGYTFTSYYIHWVRQGPGQGLEWMGWIN
PGEGSIKYNEKFKGRVTLTRDTSTSTAYMELRSLTSEDTAIYFCARDGYSYYYFD
FWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSRLTVDKSRWQQGNVFSCSVMHEALHNHYTQKSLSLSPGK
SEQ ID NO: 302
>82VL8_S27e P,L94V-CL 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 303
>82VL8_S27e P,F92Y-CL 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSYILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 304
>82VL8_S27e E,F92Y-CL 
DIVMTQSPDSLAVSLGERATINCKSSQSLLNERTRKNYLAWYQQKPGQPPKLLIY
WASTRESGVPDRFSGSGSGTDFTLTISSLQAEDVAVYYCKQSYILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 305
>82VL8_S27e P,L94I-CL 
DIVMTQSPDSLAVSLGERATINCKSSQSLLNPRTRKNYLAWYQQKPGQPPKLLIY
WASTRESGVPDRFSGSGSGTDFTLTISSLQAEDVAVYYCKQSFIIRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
 SEQ ID NO: 306
>82VL8_S27e P,W50Y-CL 
DIVMTQSPDSLAVSLGERATINCKSSQSLLNPRTRKNYLAWYQQKPGQPPKLLIY
YASTRESGVPDRFSGSGSGTDFTLTISSLQAEDVAVYYCKQSFILRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 307
>82VL8_S27e E,L94V-CL 
DIVMTQSPDSLAVSLGERATINCKSSQSLLNERTRKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 308
>82VH4_I34M-CH1-Fc-2 
QVQLVQSGAEVKKPGASVKVSCKASGYTFTSYYMHWVRQAPGQGLEWMGWI
NPGDGSIKYNEKFKGRVTLTRDTSTSTAYMELRSLTSEDTAIYFCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 309
>82VH2_M37V-CH1-Fc-2 
EVQLVQSGAEVKKPGASVKVSCKASGYTFTSYYIHWVRQAPGQGLEWMGWIN
PGDGSIKYNEKFKGRVTLTADKSTSTAYMELRSLRSDDTAVYYCARDGYSYYYF
DFWGQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 176
>82VL8_S27e P,L94I,W50Y-CL 
DIVMTQSPDSLAVSLGERATINCKSSQSLLNPRTRKNYLAWYQQKPGQPPKLLIY
YASTRESGVPDRFSGSGSGTDFTLTISSLQAEDVAVYYCKQSFIIRTFGQGTKVEI
 
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 310
>82VH2_M37V,I34N-CH1-Fc-2 
EVQLVQSGAEVKKPGASVKVSCKASGYTFTSYYNHWVRQAPGQGLEWMGWI
NPGDGSIKYNEKFKGRVTLTADKSTSTAYMELRSLRSDDTAVYYCARDGYSYYY
FDFWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 311
>82VH2_M37V,A40G-CH1-Fc-2 
EVQLVQSGAEVKKPGASVKVSCKASGYTFTSYYIHWVRQGPGQGLEWMGWIN
PGDGSIKYNEKFKGRVTLTADKSTSTAYMELRSLRSDDTAVYYCARDGYSYYYF
DFWGQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 312
>82VL6_S27e P,F92Y-CL 
DIVMTQSPDSLAVSLGERATINCKSSQSLLNPRTRKNYLAWYQQKPGQSPKLLIY
YASTRESGVPDRFSGSGSGTDFTLTISSLQAEDVAVYYCKQSYILRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 313
>82VH2_M37V,Y32K-CH1-Fc-2 
EVQLVQSGAEVKKPGASVKVSCKASGYTFTSKYIHWVRQAPGQGLEWMGWIN
PGDGSIKYNEKFKGRVTLTADKSTSTAYMELRSLRSDDTAVYYCARDGYSYYYF
DFWGQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
 
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 314
>82VH1_I34N-CH1-Fc-2 
EVQLVQSGAEVKKPGASVKVSCKASGYTFTSYYNHWVRQAPGQGLEWMGWI
NPGDGSIKYNEKFKGRVTMTADKSTSTAYMELRSLRSDDTAVYYCARDGYSYY
YFDFWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKVEP
KSCDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFYPSDIAVEWESNGQPE
NNYKTTPPVLDSDGSFFLYSRLTVDKSRWQQGNVFSCSVMHEALHNHYTQKSLSLSP
GK
SEQ ID NO: 315
>82VL6_S27e P,L94V-CL 
DIVMTQSPDSLAVSLGERATINCKSSQSLLNPRTRKNYLAWYQQKPGQSPKLLIY
YASTRESGVPDRFSGSGSGTDFTLTISSLQAEDVAVYYCKQSFIVRTFGGGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 316
>82VL8_S27e P,L94V,K24T-CL 
DIVMTQSPDSLAVSLGERATINCTSSQSLLNPRTRKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 317
>82VL8_S27e P,L94V,L27b V-CL 
DIVMTQSPDSLAVSLGERATINCKSSQSVLNPRTRKNYLAWYQQKPGQPPKLLI
YWASTRESGVPDRFSGSGSGTDFTLTISSLQAEDVAVYYCKQSFIVRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 318
>82VL8_S27e P,L94V,T28S-CL 
DIVMTQSPDSLAVSLGERATINCKSSQSLLNPRSRKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
 
TEQDSKDSTYSLSSTLTLSKADYEKHKVYACEVTHQGLSSPVTKSFNRGEC
SEQ ID NO: 319
>82VL8_S27e P,L94V,R29Q-CL 
DIVMTQSPDSLAVSLGERATINCKSSQSLLNPRTQKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 320
>82VL8_S27e P,L94V,K30Q-CL 
DIVMTQSPDSLAVSLGERATINCKSSQSLLNPRTRQNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 321
>82VH7_T28S-CH1-Fc-2 
QVQLVQSGAEVKKPGASVKVSCKASGYSFTSYYMHWVRQAPGQGLEWMGWI
NPGEGSIKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 322
>82VH7_N52Q-CH1-Fc-2 
QVQLVQSGAEVKKPGASVKVSCKASGYTFTSYYMHWVRQAPGQGLEWMGWI
QPGEGSIKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 323
>82VH11_N52Q-CH1-Fc-2 EVQLVQSGAEVKKPGASVKVSCKASGYTFTSYYMHWVRQAPGQGLEWMGWI 
QPGEGSIKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 364
>82VH7_N52S-CH1-Fc-2 
QVQLVQSGAEVKKPGASVKVSCKASGYTFTSYYMHWVRQAPGQGLEWMGWI
SPGEGSIKYNEKFKGRVTLTRDTSTSTAYMELRSLTSEDTAVYYCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 324
>82VH7_S56A-CH1-Fc-2 
QVQLVQSGAEVKKPGASVKVSCKASGYTFTSYYMHWVRQAPGQGLEWMGWI
NPGEGAIKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 325
>82VH7_I57T-CH1-Fc-2 
QVQLVQSGAEVKKPGASVKVSCKASGYTFTSYYMHWVRQAPGQGLEWMGWI
NPGEGSTKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
 
KTTPPVLDSDGSFFLYSRLTVDKSRWQQGNVFSCSVMHEALHNHYTQKSLSLSPGK
SEQ ID NO: 326
>82VH11_I57T-CH1-Fc-2 
EVQLVQSGAEVKKPGASVKVSCKASGYTFTSYYMHWVRQAPGQGLEWMGWI
NPGEGSTKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 365
>82VH7_K58S-CH1-Fc-2 
QVQLVQSGAEVKKPGASVKVSCKASGYTFTSYYMHWVRQAPGQGLEWMGWI
NPGEGSISYNEKFKGRVTLTRDTSTSTAYMELRSLTSEDTAVYYCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 327
>82VH11_K58S-CH1-Fc-2 
EVQLVQSGAEVKKPGASVKVSCKASGYTFTSYYMHWVRQAPGQGLEWMGWI
NPGEGSISYNEKFKGRVTLTRDTSTSTAYMELRSLTSEDTAVYYCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 366
>82VH7_F102Y-CH1-Fc-2 
QVQLVQSGAEVKKPGASVKVSCKASGYTFTSYYMHWVRQAPGQGLEWMGWI
NPGEGSIKYNEKFKGRVTLTRDTSTSTAYMELRSLTSEDTAVYYCARDGYSYYY
FDYWGQGTLVTVSSASTKGPSVFPLAPSSKSTSGGTAALGCLVKDYFPEPVTVSWNS
 
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 328
>82VL8_S27e P,L94V,L27b V,T28S-CL 
DIVMTQSPDSLAVSLGERATINCKSSQSVLNPRSRKNYLAWYQQKPGQPPKLLI
YWASTRESGVPDRFSGSGSGTDFTLTISSLQAEDVAVYYCKQSFIVRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 329
>82VL8_S27e P,L94V,L27b V,R29Q-CL 
DIVMTQSPDSLAVSLGERATINCKSSQSVLNPRTQKNYLAWYQQKPGQPPKLLI
YWASTRESGVPDRFSGSGSGTDFTLTISSLQAEDVAVYYCKQSFIVRTFGQGTKV
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 330
>82VH7_N52Q,I57T-CH1-Fc-2 
QVQLVQSGAEVKKPGASVKVSCKASGYTFTSYYMHWVRQAPGQGLEWMGWI
QPGEGSTKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 331
>82VH11_N52Q,I57T-CH1-Fc-2 
EVQLVQSGAEVKKPGASVKVSCKASGYTFTSYYMHWVRQAPGQGLEWMGWI
QPGEGSTKYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
 
KTTPPVLDSDGSFFLYSRLTVDKSRWQQGNVFSCSVMHEALHNHYTQKSLSLSPGK
SEQ ID NO: 367
>82VH7_N52Q,K58S-CH1-Fc-2 
QVQLVQSGAEVKKPGASVKVSCKASGYTFTSYYMHWVRQAPGQGLEWMGWI
QPGEGSISYNEKFKGRVTLTRDTSTSTAYMELRSLTSEDTAVYYCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 332
>82VH11_N52Q,K58S-CH1-Fc-2 
EVQLVQSGAEVKKPGASVKVSCKASGYTFTSYYMHWVRQAPGQGLEWMGWI
QPGEGSISYNEKFKGRVTLTRDTSTSTAYMELRSLTSEDTAVYYCARDGYSYYYF
DFWGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 368
>82VH7_I57T,K58S-CH1-Fc-2 
QVQLVQSGAEVKKPGASVKVSCKASGYTFTSYYMHWVRQAPGQGLEWMGWI
NPGEGSTSYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 333
>82VH11_I57T,K58S-CH1-Fc-2 
EVQLVQSGAEVKKPGASVKVSCKASGYTFTSYYMHWVRQAPGQGLEWMGWI
NPGEGSTSYNEKFKGRVTLTRDTSTSTAYMELRSLTSEDTAVYYCARDGYSYYY
FDFWGQGTLVTVSSASTKGPSVFPLAPSSKSTSGGTAALGCLVKDYFPEPVTVSWNS
 
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 369
>82VL8_S27e P,L94V,T28S,R29Q-CL 
DIVMTQSPDSLAVSLGERATINCKSSQSLLNPRSQKNYLAWYQQKPGQPPKLLIY
WASTRESGVPDRFSGSGSGTDFTLTISSLQAEDVAVYYCKQSFIVRTFGQGTKVEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 334
>82VH2_M37V,T30A,G55D-CH1-Fc-2 
EVQLVQSGAEVKKPGASVKVSCKASGYTFASYYIHWVRQAPGQGLEWMGWIN
PGDDSIKYNEKFKGRVTLTADKSTSTAYMELRSLRSDDTAVYYCARDGYSYYYF
DFWGQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 335
>82VH2_M37V,T30A,G55D,T107S-CH1-Fc-2

EVQLVQSGAEVKKPGASVKVSCKASGYTFASYYIHWVRQAPGQGLEWMGWIN
PGDDSIKYNEKFKGRVTLTADKSTSTAYMELRSLRSDDTAVYYCARDGYSYYYF
DFWGQGSTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSRLTVDKSRWQQGNVFSCSVMHEALHNHYTQKSLSLSPGK
SEQ ID NO: 336

Note: In the sequences described above, the CDR sequences determined according to the Kabat numbering scheme are underlined, and the antibody constant regions are italicized.

### Example 5. In Vitro Assembly of CD20/CD3 Bispecific Antibodies

The anti-CD20 antibody was 7D8, the sequence of which was derived from the patent WO2019155008A1, wherein the VH/VL sequences were SEQ ID NO: 13 and SEQ ID NO: 14 in the patent, respectively, and the specific sequences are as follows:

**Table 13-1. CDR sequences of the anti-CD20 antibody**

| Antibody | Light chain | | Heavy chain | |
|---|---|---|---|---|
| | LCDR1 | RASQSVSSYLA (SEQ ID NO: 370) | HCDR1 | DYAMH (SEQ ID NO: 373) |
| CD20 | LCDR2 | DASNRAT (SEQ ID NO: 371) | HCDR2 | TISWNSGTIGYADSVKG (SEQ ID NO: 374) |
| | LCDR3 | QQRSNWPIT (SEQ ID NO: 372) | HCDR3 | DIQYGNYYYGMDV (SEQ ID NO: 375) |

| | | | | |
|---|---|---|---|---|
| Note: The CDRs are numbered according to the Kabat numbering scheme. | | | | |

>CD20 VL: 
EIVLTQSPATLSLSPGERATLSCRASQSVSSYLAWYQQKPGQAPRLLIYDASNRA
TGIPARFSGSGSGTDFTLTISSLEPEDFAVYYCQQRSNWPITFGQGTRLEIK
SEQ ID NO: 337
> CD20 VH:

EVQLVESGGGLVQPDRSLRLSCAASGFTFHDYAMHWVRQAPGKGLEWVSTISW
NSGTIGYADSVKGRFTISRDNAKNSLYLQMNSLRAEDTALYYCAKDIQYGNYY
YGMDVWGQGTTVTVSS
SEQ ID NO: 338

Note: The CDR sequences are underlined and numbered according to the Kabat numbering scheme.

The heavy chain variable region and the light chain variable region were recombined with the heavy chain constant region hIgG1:CH1-Fc-3 (SEQ ID NO: 339) and the light chain constant region CL (SEQ ID NO: 91), respectively, to obtain the full-length antibody.
> hIgG1:CH1-Fc-3 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFLLYSKLTVDKSRWQQGNVFSCSVMHEALHNHYTQKSL
SLSPGK
SEQ ID NO: 339
> 7D8-CH1-Fc-3 EVQLVESGGGLVQPDRSLRLSCAASGFTFHDYAMHWVRQAPGKGLEWVSTISW 
NSGTIGYADSVKGRFTISRDNAKNSLYLQMNSLRAEDTALYYCAKDIQYGNYY
YGMDVWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKKVE
PKSCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQP
ENNYKTTPPVLDSDGSFLLYSKLTVDKSRWQQGNVFSCSVMHEALHNHYTQKSLSLS
PGK
SEQ ID NO: 340
>7D8-CL

EIVLTQSPATLSLSPGERATLSCRASQSVSSYLAWYQQKPGQAPRLLIYDASNRAT
GIPARFSGSGSGTDFTLTISSLEPEDFAVYYCQQRSNWPITFGQGTRLEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC
SEQ ID NO: 341

The CD20/CD3 bispecific antibodies were obtained by *in vitro* assembly. For the specific assembly process, reference was made to the patent WO2013060867A2. The specific procedures are as follows:

The volume of the two monoclonal antibodies added was calculated according to the molar ratio of CD20 antibody:CD3 antibody = 1.05:1 and the protein concentration to prepare a reaction system; 0.1358 g of 2-MEA (2-mercaptoethylamine hydrochloride, Sangon, Cat. No. A506578-0005) was weighed out, and 796.9 µL of PBS (MeilunBio, Cat. No. PWL050) was added thereto to prepare 1.5 M 2-MEA, which was then added to the reaction system according to the volume, and 2-MEA was used as a reductant, with a final concentration of 75 mM; after the reaction system was prepared, the system was placed in a water bath at 31 °C and reacted for 5 h; the reaction system was transferred into a dialysis bag, and the dialysis bag was placed in PBS for complete dialysis; and then the reactants in the dialysis bag were taken out for concentration and quality verification, thus obtaining the *in vitro* assembled CD20/CD3 bispecific antibodies.

Illustratively, the full-length sequences of the *in vitro* assembled CD20/CD3 bispecific antibodies are shown in the table below:

**Table 13-2. CD20/CD3 bispecific antibodies**

| CD20/CD3 antibody | Light chain 1 | Heavy chain 1 | Light chain 2 | Heavy chain 2 |
|---|---|---|---|---|
| CD20-82L2H3 | 7D8-CL (SEQ ID NO: 341) | 7D8-CH1-Fc-3 (SEQ ID NO: 340) | 82VL2-CL (SEQ ID NO: 164) | 82VH3-CH1-Fc-2 (SEQ ID NO: 177) |
| CD20-82L3H3 | 7D8-CL (SEQ ID NO: 341) | 7D8-CH1-Fc-3 (SEQ ID NO: 340) | 82VL3-CL (SEQ ID NO: 165) | 82VH3-CH1-Fc-2 (SEQ ID NO: 177) |
| CD20-82L4H3 | 7D8-CL (SEQ ID NO: 341) | 7D8-CH1-Fc-3 (SEQ ID NO: 340) | 82VL4-CL (SEQ ID NO: 166) | 82VH3-CH1-Fc-2 (SEQ ID NO: 177) |
| CD20-82L5H1 | 7D8-CL (SEQ ID NO: 341) | 7D8-CH1-Fc-3 (SEQ ID NO: 340) | 82VL5-CL (SEQ ID NO: 167) | 82VH1-CH1-Fc-2 (SEQ ID NO: 174) |
| CD20-82L5H2 | 7D8-CL (SEQ ID NO: 341) | 7D8-CH1-Fc-3 (SEQ ID NO: 340) | 82VL5-CL (SEQ ID NO: 167) | 82VH2-CH1-Fc-2 (SEQ ID NO: 175) |
| CD20-82L5H3 | 7D8-CL (SEQ ID NO: 341) | 7D8-CH1-Fc-3 (SEQ ID NO: 340) | 82VL5-CL (SEQ ID NO: 167) | 82VH3-CH1-Fc-2 (SEQ ID NO: 177) |
| CD20-82L6H2 | 7D8-CL (SEQ ID NO: 341) | 7D8-CH1-Fc-3 (SEQ ID NO: 340) | 82VL6-CL (SEQ ID NO: 168) | 82VH2-CH1-Fc-2 (SEQ ID NO: 175) |
| CD20-82L6H3 | 7D8-CL (SEQ ID NO: 341) | 7D8-CH1-Fc-3 (SEQ ID NO: 340) | 82VL6-CL (SEQ ID NO: 168) | 82VH3-CH1-Fc-2 (SEQ ID NO: 177) |
| CD20-82L8H4 | 7D8-CL (SEQ ID NO: 341) | 7D8-CH1-Fc-3 (SEQ ID NO: 340) | 82VL8-CL (SEQ ID NO: 170) | 82VH4-CH1-Fc-2 (SEQ ID NO: 178) |
| CD20-82L8H5 | 7D8-CL (SEQ ID NO: 341) | 7D8-CH1-Fc-3 (SEQ ID NO: 340) | 82VL8-CL (SEQ ID NO: 170) | 82VH5-CH1-Fc-2 (SEQ ID NO: 179) |
| CD20-82L9H4 | 7D8-CL (SEQ ID NO: 341) | 7D8-CH1-Fc-3 (SEQ ID NO: 340) | 82VL9-CL (SEQ ID NO: 171) | 82VH4-CH1-Fc-2 (SEQ ID NO: 178) |
| CD20-82L10H4 | 7D8-CL (SEQ ID NO: 341) | 7D8-CH1-Fc-3 (SEQ ID NO: 340) | 82VL10-CL (SEQ ID NO: 172) | 82VH4-CH1-Fc-2 (SEQ ID NO: 178) |
| CD20-82L10H5 | 7D8-CL (SEQ ID NO: 341) | 7D8-CH1-Fc-3 (SEQ ID NO: 340) | 82VL10-CL (SEQ ID NO: 172) | 82VH5-CH1-Fc-2 (SEQ ID NO: 179) |

### Example 6. In Vitro Assembly of BCMA/CD3 Bispecific Antibodies

The variable region sequences of the anti-BCMA antibody were derived from the anti-BCMA antibody in teclistamab (the sequence of which was derived from WHO INN List PL120), and the specific sequences are as follows:

**Table 13-3. CDR sequences of the anti-BCMA antibody**

| Antibody | Light chain | | Heavy chain | |
|---|---|---|---|---|
| | LCDR1 | GGNNIGSKSVH (SEQ ID NO: 376) | HCDR1 | SGSYFWG (SEQ ID NO: 379) |
| BCMA | LCDR2 | DDSDRPS (SEQ ID NO: 377) | HCDR2 | SIYYSGITYYNPSLKS(S EQ ID NO: 380) |
| | LCDR3 | QVWDSSSDHVV (SEQ ID NO: 378) | HCDR3 | HDGAVAGLFDY (SEQ ID NO: 381) |

| | | | | |
|---|---|---|---|---|
| Note: The CDRs are numbered according to the Kabat numbering scheme. | | | | |

>BCMA VL: 
SYVLTQPPSVSVAPGQTARITCGGNNIGSKSVHWYQQPPGQAPVVVVYDDSDRP
SGIPERFSGSNSGNTATLTISRVEAGDEAVYYCQVWDSSSDHVVFGGGTKLTVL
SEQ ID NO: 382
>BCMA VH:

QLQLQESGPGLVKPSETLSLTCTVSGGSISSGSYFWGWIRQPPGKGLEWIGSIYYS
GITYYNPSLKSRVTISVDTSKNQFSLKLSSVTAADTAVYYCARHDGAVAGLFDY
WGQGTLVTVSS
SEQ ID NO: 383

Note: The CDR sequences are underlined and numbered according to the Kabat numbering scheme.

The heavy chain variable region and the light chain variable region were recombined with the heavy chain constant region hIgG1:CH1-Fc-3 (SEQ ID NO: 339) and the lambda light chain constant region CL (SEQ ID NO: 384), respectively, to obtain the full-length antibody.
> lambda CL 
GQPKAAPSVTLFPPSSEELQANKATLVCLISDFYPGAVTVAWKGDSSPVKAGVET
TTPSKQSNNKYAASSYLSLTPEQWKSHRSYSCQVTHEGSTVEKTVAPTECS
SEQ ID NO: 384
> BCMA-CH1-Fc-3 
QLQLQESGPGLVKPSETLSLTCTVSGGSISSGSYFWGWIRQPPGKGLEWIGSIYYS
GITYYNPSLKSRVTISVDTSKNQFSLKLSSVTAADTAVYYCARHDGAVAGLFDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDK
 
THTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFLLYSKLTVDKSRWQQGNVFSCSVMHEALHNHYTQKSLSLSPGK
SEQ ID NO: 385
>BCMA-CL

SYVLTQPPSVSVAPGQTARITCGGNNIGSKSVHWYQQPPGQAPVVVVYDDSDRP
SGIPERFSGSNSGNTATLTISRVEAGDEAVYYCQVWDSSSDHVVFGGGTKLTVLG
QPKAAPSVTLFPPSSEELQANKATLVCLISDFYPGAVTVAWKGDSSPVKAGVETTTPS
KQSNNKYAASSYLSLTPEQWKSHRSYSCQVTHEGSTVEKTVAPTECS
SEQ ID NO: 386

Note: The antibody constant regions are italicized.

The BCMA/CD3 bispecific antibodies were obtained by *in vitro* assembly. The specific assembly process was the same as in Example 5.

Illustratively, the full-length sequences of the *in vitro* assembled BCMA/CD3 bispecific antibodies are shown in the table below:

**Table 13-4. BCMA/CD3 bispecific antibodies**

| BCMA/CD3 antibody | Light chain 1 | Heavy chain 1 | Light chain 2 | Heavy chain 2 |
|---|---|---|---|---|
| BCMA-82L8_S27e P,L94V/H7 | BCMA-CL (SEQ ID NO: 386) | BCMA-CH1- Fc-3 (SEQ ID NO: 385) | 82L8_S27e P,L94V-CL (SEQ ID NO: 303) | 82VH7-CH1-Fc-2 (SEQ ID NO: 181) |
| BCMA-82L8_S27e P,F92Y/H5_I34M | BCMA-CL (SEQ ID NO: 386) | BCMA-CH1- Fc-3 (SEQ ID NO: 385) | 82VL8_S27e P,F92Y-CL (SEQ ID NO: 304) | 82VH5_I34M- CH1-Fc-2 (SEQ ID NO: 301) |
| BCMA-82L8_S27e E,F92Y/H5_I34M | BCMA-CL (SEQ ID NO: 386) | BCMA-CH1- Fc-3 (SEQ ID NO: 385) | 82VL8_S27e E,F92Y-CL (SEQ ID NO: 305) | 82VH5_I34M- CH1-Fc-2 (SEQ ID NO: 301) |
| BCMA-82L8_S27e P,L94V/H5_I34M | BCMA-CL (SEQ ID NO: 386) | BCMA-CH1- Fc-3 (SEQ ID NO: 385) | 82VL8_S27e P,L94V-CL (SEQ ID NO: 303) | 82VH5_I34M- CH1-Fc-2 (SEQ ID NO: 301) |
| BCMA-82L8_S27e P,L94V,T28S/H7_N5 2Q | BCMA-CL (SEQ ID NO: 386) | BCMA-CH1- Fc-3 (SEQ ID NO: 385) | 82VL8_S27e P,L94V,T28S-CL (SEQ ID NO: 319) | 82VH7_N52Q- CH1-Fc-2 (SEQ ID NO: 323) |
| BCMA-82L8_S27e P,L94V,T28S/H7_I57T | BCMA-CL (SEQ ID NO: 386) | BCMA-CH1- Fc-3 (SEQ ID NO: 385) | 82VL8_S27e P,L94V,T28S-CL (SEQ ID NO: 319) | 82VH7_I57T- CH1-Fc-2 (SEQ ID NO: 326) |
| BCMA-82L8_S27e P,L94V,R29Q/H7_N5 2Q | BCMA-CL (SEQ ID NO: 386) | BCMA-CH1- Fc-3 (SEQ ID NO: 385) | 82VL8_S27e P,L94V,R29Q-CL (SEQ ID NO: 320) | 82VH7_N52Q- CH1-Fc-2 (SEQ ID NO: 323) |
| BCMA-82L8_S27e P,L94V,R29Q/H7_I5 7T | BCMA-CL (SEQ ID NO: 386) | BCMA-CH1- Fc-3 (SEQ ID NO: 385) | 82VL8_S27e P,L94V,R29Q-CL (SEQ ID NO: 320) | 82VH7_I57T- CH1-Fc-2 (SEQ ID NO: 326) |
| BCMA-82L8_S27e P,L94V,R29Q/H7_K5 8S | BCMA-CL (SEQ ID NO: 386) | BCMA-CH1- Fc-3 (SEQ ID NO: 385) | 82VL8_S27e P,L94V,R29Q-CL (SEQ ID NO: 320) | 82VH7_K58S- CH1-Fc-2 (SEQ ID NO: 327) |
| BCMA-82L8_S27e P,L94V,L27b V,T28S/H7 | BCMA-CL (SEQ ID NO: 386) | BCMA-CH1- Fc-3 (SEQ ID NO: 385) | 82VL8_S27e P,L94V,L27b V,T28S-CL (SEQ ID NO: 329) | 82VH7-CH1-Fc-2 (SEQ ID NO: 181) |
| BCMA-82L8_S27e P,L94V,L27b V,R29Q H7 | BCMA-CL (SEQ ID NO: 386) | BCMA-CH1- Fc-3 (SEQ ID NO: 385) | 82VL8_S27e P,L94V,L27b V,R29Q-CL (SEQ ID NO: 330) | 82VH7-CH1-Fc-2 (SEQ ID NO: 181) |
| BCMA-82L8_S27e P,L94V/H7_N52Q,I5 7T | BCMA-CL (SEQ ID NO: 386) | BCMA-CH1- Fc-3 (SEQ ID NO: 385) | 82VL8_S27e P,L94V-CL (SEQ ID NO: 303) | 82VH7_N52Q,I57 T-CH1-Fc-2 (SEQ ID NO: 331) |
| BCMA-82L8_S27e P,L94V/H7_N52Q,K 58S | BCMA-CL (SEQ ID NO: 386) | BCMA-CH1- Fc-3 (SEQ ID NO: 385) | 82VL8_S27e P,L94V-CL (SEQ ID NO: 303) | 82VH7_N52Q,K5 8S-CH1-Fc-2 (SEQ ID NO: 332) |
| BCMA-82L8_S27e P,L94V/H7_I57T,K5 8S | BCMA-CL (SEQ ID NO: 386) | BCMA-CH1- Fc-3 (SEQ ID NO: 385) | 82VL8_S27e P,L94V-CL (SEQ ID NO: 303) | 82VH7_I57T,K58 S-CH1-Fc-2 (SEQ ID NO: 333) |
| BCMA-82L8_S27e P,L94V,R29Q/H7_N5 2Q,K58S | BCMA-CL (SEQ ID NO: 386) | BCMA-CH1- Fc-3 (SEQ ID NO: 385) | 82VL8_S27e P,L94V,R29Q-CL (SEQ ID NO: 320) | 82VH7_N52Q,K5 8S-CH1-Fc-2 (SEQ ID NO: 332) |

### Example 7. In Vitro Assembly of PSMA/CD3 Bispecific Antibodies

The variable region sequences of the anti-PSMA antibody were derived from the patent WO2023147784A1, wherein the VH/VL sequences were SEQ ID NO: 110 and SEQ ID NO: 114 in the patent, respectively, and the specific sequences are as follows:

**Table 13-5. CDR sequences of the anti-PSMA antibody**

| Antibody | Light chain | | Heavy chain | |
|---|---|---|---|---|
| | LCDR1 | KASQGVDTAVA (SEQ ID NO: 387) | HCDR1 | DHNMN (SEQ ID NO: 390) |
| PSMA | LCDR2 | WASTRHT (SEQ ID NO: 388) | HCDR2 | LINPNYGTADYNQKFKG(S EQ ID NO: 391) |
| | LCDR3 | QQYSRYPLT (SEQ ID NO: 389) | HCDR3 | GSSLFAY (SEQ ID NO: 392) |

| | | | | |
|---|---|---|---|---|
| Note: The CDRs are numbered according to the Kabat numbering scheme. | | | | |

>PSMA VL: 
DIQMTQSPSSLSASVGDRVTITCKASQGVDTAVAWYQQKPGKSPKLLIYWASTR
HTGVPDRFSGSGSGTDFTLTISSLQPEDVATYYCQQYSRYPLTFGQGTKLEIK
SEQ ID NO: 393
> PSMA VH:

EFQLVQSGAEVKKPGASVKVSCKASGYSFTDHNMNWVRQAPGQRLEWMGLIN
PNYGTADYNQKFKGRVTLTVDQSATTAYMELSSLRSEDTAVYYCARGSSLFAYW
GQGTLVTVSS
SEQ ID NO: 394

Note: The CDR sequences are underlined and numbered according to the Kabat numbering scheme.

The heavy chain variable region and the light chain variable region were recombined with the heavy chain constant region hIgG1:CH1-Fc-4 (SEQ ID NO: 399) and the kappa light chain constant region CL (SEQ ID NO: 91), respectively, to obtain the full-length antibody.
> hIgG1:CH1-Fc-4 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFLLYSKLTVDKSRWQQGNVFSCSVMHEALHNHYTQKSL
SLSPG
SEQ ID NO: 399
> PSMA-CH1-Fc-4 
EFQLVQSGAEVKKPGASVKVSCKASGYSFTDHNMNWVRQAPGQRLEWMGLIN
PNYGTADYNQKFKGRVTLTVDQSATTAYMELSSLRSEDTAVYYCARGSSLFAYW
GQGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTP
PVLDSDGSFLLYSKLTVDKSRWQQGNVFSCSVMHEALHNHYTQKSLSLSPG
SEQ ID NO: 396
>PSMA-CL

DIQMTQSPSSLSASVGDRVTITCKASQGVDTAVAWYQQKPGKSPKLLIYWASTR
HTGVPDRFSGSGSGTDFTLTISSLQPEDVATYYCQQYSRYPLTFGQGTKLEIKRTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

SEQ ID NO: 395

Note: The antibody constant regions are italicized.

The PSMA/CD3 bispecific antibodies were obtained by *in vitro* assembly. The specific assembly process was the same as in Example 5.

Illustratively, the full-length sequences of the *in vitro* assembled PSMA/CD3 bispecific antibodies are shown in the table below:

**Table 13-6. PSMA/CD3 bispecific antibodies**

| PSMA/CD3 antibody | Light chain 1 | Heavy chain 1 | Light chain 2 | Heavy chain 2 |
|---|---|---|---|---|
| PSMA-82L8_S27e P,L94V/H7 | PSMA-CL (SEQ ID NO: 395) | PSMA-CH1- Fc-4 (SEQ ID NO: 396) | 82L8_S27e P,L94V- CL (SEQ ID NO: 303) | 82VH7-CH1-Fc-2 (SEQ ID NO: 181) |
| PSMA-82L8_S27e P,F92Y/H5_I34M | PSMA-CL (SEQ ID NO: 395) | PSMA-CH1- Fc-4 (SEQ ID NO: 396) | 82VL8_S27e P,F92Y-CL (SEQ ID NO: 304) | 82VH5_I34M- CH1-Fc-2 (SEQ ID NO: 301) |
| PSMA-82L8_S27e E,F92Y/H5_I34M | PSMA-CL (SEQ ID NO: 395) | PSMA-CH1- Fc-4 (SEQ ID NO: 396) | 82VL8_S27e E,F92Y-CL (SEQ ID NO: 305) | 82VH5_I34M- CH1-Fc-2 (SEQ ID NO: 301) |
| PSMA-82L8_S27e P,L94V/H5_I34M | PSMA-CL (SEQ ID NO: 395) | PSMA-CH1- Fc-4 (SEQ ID NO: 396) | 82VL8_S27e P,L94V-CL (SEQ ID NO: 303) | 82VH5_I34M- CH1-Fc-2 (SEQ ID NO: 301) |
| PSMA-82L8_S27e P,L94V,T28S/H7_N 52Q | PSMA-CL (SEQ ID NO: 395) | PSMA-CH1- Fc-4 (SEQ ID NO: 396) | 82VL8_S27e P,L94V,T28S-CL (SEQ ID NO: 319) | 82VH7_N52Q- CH1-Fc-2 (SEQ ID NO: 323) |
| PSMA-82L8_S27e P,L94V,T28S/H7_I5 7T | PSMA-CL (SEQ ID NO: 395) | PSMA-CH1- Fc-4 (SEQ ID NO: 396) | 82VL8_S27e P,L94V,T28S-CL (SEQ ID NO: 319) | 82VH7_I57T-CH1- Fc-2 (SEQ ID NO: 326) |
| PSMA-82L8_S27e P,L94V,T28S/H7_K 58S | PSMA-CL (SEQ ID NO: 395) | PSMA-CH1- Fc-4 (SEQ ID NO: 396) | 82VL8_S27e P,L94V,T28S-CL (SEQ ID NO: 319) | 82VH7_K58S- CH1-Fc-2 (SEQ ID NO: 327) |
| PSMA-82L8_S27e P,L94V,R29Q/H7_ N52Q | PSMA-CL (SEQ ID NO: 395) | PSMA-CH1- Fc-4 (SEQ ID NO: 396) | 82VL8_S27e P,L94V,R29Q-CL (SEQ ID NO: 320) | 82VH7_N52Q- CH1-Fc-2 (SEQ ID NO: 323) |
| PSMA-82L8_S27e P,L94V,R29Q/H7_I 57T | PSMA-CL (SEQ ID NO: 395) | PSMA-CH1- Fc-4 (SEQ ID NO: 396) | 82VL8_S27e P,L94V,R29Q-CL (SEQ ID NO: 320) | 82VH7_I57T-CH1- Fc-2 (SEQ ID NO: 326) |
| PSMA-82L8_S27e P,L94V,R29Q/H7_ K58S | PSMA-CL (SEQ ID NO: 395) | PSMA-CH1- Fc-4 (SEQ ID NO: 396) | 82VL8_S27e P,L94V,R29Q-CL (SEQ ID NO: 320) | 82VH7_K58S- CH1-Fc-2 (SEQ ID NO: 327) |
| PSMA-82L8_S27e P,L94V,L27b V,T28S/H7 | PSMA-CL (SEQ ID NO: 395) | PSMA-CH1- Fc-4 (SEQ ID NO: 396) | 82VL8_S27e P,L94V,L27b V,T28S-CL (SEQ ID NO: 329) | 82VH7-CH1-Fc-2 (SEQ ID NO: 181) |
| PSMA-82L8_S27e P,L94V,L27b V,R29Q/H7 | PSMA-CL (SEQ ID NO: 395) | PSMA-CH1- Fc-4 (SEQ ID NO: 396) | 82VL8_S27e P,L94V,L27b V,R29Q-CL (SEQ ID NO: 330) | 82VH7-CH1-Fc-2 (SEQ ID NO: 181) |
| PSMA-82L8_S27e P,L94V/H7_N52Q,I 57T | PSMA-CL (SEQ ID NO: 395) | PSMA-CH1- Fc-4 (SEQ ID NO: 396) | 82VL8_S27e P,L94V-CL (SEQ ID NO: 303) | 82VH7_N52Q,I57T -CH1-Fc-2 (SEQ ID NO: 331) |
| PSMA-82L8_S27e P,L94V/H7_N52Q, K58S | PSMA-CL (SEQ ID NO: 395) | PSMA-CH1- Fc-4 (SEQ ID NO: 396) | 82VL8_S27e P,L94V-CL (SEQ ID NO: 303) | 82VH7_N52Q,K58 S-CH1-Fc-2 (SEQ ID NO: 332) |
| PSMA-82L8_S27e P,L94V/H7_I57T,K 58S | PSMA-CL (SEQ ID NO: 395) | PSMA-CH1- Fc-4 (SEQ ID NO: 396) | 82VL8_S27e P,L94V-CL (SEQ ID NO: 303) | 82VH7_I57T,K58S -CH1-Fc-2 (SEQ ID NO: 333) |
| PSMA-82L8_S27e P,L94V,R29Q/H7_ N52Q,K58S | PSMA-CL (SEQ ID NO: 395) | PSMA-CH1- Fc-4 (SEQ ID NO: 396) | 82VL8_S27e P,L94V,R29Q-CL (SEQ ID NO: 320) | 82VH7_N52Q,K58 S-CH1-Fc-2 (SEQ ID NO: 332) |

### Test Examples

### Test Example 1. Detection of Affinity of Anti-CD3 Antibodies for CD3 Antigens Using Biacore

The affinity of the antibodies for CD3 was detected in the form of antibody capturing. The antibody was affinity-captured by a CM5 biosensor chip (Cat. # BR-1005-30, GE) or a Protein A (Cat. # 29127556, GE) biosensor chip, each of which was coupled with an anti-human IgG antibody (Cat. # BR-1008-39, Lot. # 10260416, GE), and then an antigen molecule (the antigen for detection was purchased from Sino Biological or Acro) flowed through the surface of the chip. The reaction signals were detected in real time using a Biacore T200 instrument to obtain association and dissociation curves. After dissociation was completed for each assay cycle, the chip was washed clean and regenerated with a regeneration buffer, Glycine1.5 (Cat. # BR100354, GE) or 3 M MgCl₂ (from Human antibody capture kit, Cat. # BR100839, GE). After the assay was completed, the data were fitted with (1:1) Langmuir model using GE Biacore T200 Evaluation version 3.0 software to obtain affinity values.

**Table 14-1. Binding of different CD3 chimeric antibodies to human CD3E or human CD3D&CD3E antigen**

| Antibody | Human CD3E antigen | | | Human CD3D&CD3E antigen | | |
|---|---|---|---|---|---|---|
| | ka (1/Ms) | kd (1/s) | KD (M) | ka (1/Ms) | kd (1/s) | KD (M) |
| Chi8 | 3.95E+05 | 5.44E-04 | 1.38E-09 | 3.30E+05 | 1.83E-02 | 5.54E-08 |
| Chi12 | 2.93E+05 | 5.32E-04 | 1.82E-09 | 2.17E+05 | 9.82E-03 | 4.53E-08 |
| Chi21 | 4.01E+05 | 6.14E-04 | 1.53E-09 | 4.56E+05 | 3.08E-02 | 6.75E-08 |
| Chi29 | 4.32E+05 | 5.20E-04 | 1.21E-09 | 4.30E+05 | 1.71E-02 | 3.98E-08 |
| Chi31 | 2.99E+05 | 5.20E-04 | 1.74E-09 | 2.81E+05 | 1.62E-02 | 5.76E-08 |
| Chi57 | 1.18E+06 | 1.30E-03 | 1.10E-09 | 1.13E+06 | 3.37E-01 | 2.98E-07 |
| Chi61 | 2.14E+06 | 7.15E-04 | 3.35E-10 | 1.80E+06 | 1.32E-01 | 7.37E-08 |
| Chi82 | 3.41E+05 | 5.40E-04 | 1.58E-09 | 2.94E+05 | 1.02E-02 | 3.46E-08 |
| Chi89 | 3.54E+05 | 4.97E-04 | 1.41E-09 | 3.19E+05 | 7.77E-03 | 2.44E-08 |
| Chi92-2 | 3.30E+05 | 5.00E-04 | 1.52E-09 | 2.85E+05 | 1.08E-02 | 3.80E-08 |
| Chi96 | 2.87E+05 | 5.15E-04 | 1.80E-09 | 2.33E+05 | 1.07E-02 | 4.60E-08 |

**Table 14-2. Affinity of different CD3 humanized antibodies for human CD3D&CD3E**

| Antibody | Human CD3D&CD3E antigen | | |
|---|---|---|---|
| | ka (1/Ms) | kd (1/s) | KD (M) |
| 82L1H1 | 1.09E+06 | 1.39E-02 | 1.28E-08 |
| 82L8H5 | 1.00E+06 | 1.29E-02 | 1.29E-08 |

**Table 14-3. Affinity of different engineered CD3 humanized antibodies for human CD3D&CD3E**

| Antibody | Human CD3D&CD3E antigen | | |
|---|---|---|---|
| | ka (1/Ms) | kd (1/s) | KD (M) |
| 82L8_S27e D H5_I34M | 7.05E+05 | 1.19E-02 | 1.69E-08 |
| 82L8_S27e K H5_I34M | 7.58E+05 | 1.38E-02 | 1.82E-08 |
| 82L8_S27e P H5_I34M | 8.50E+05 | 8.34E-03 | 9.82E-09 |
| 82L8_S27e Q H5_I34M | 9.31E+05 | 1.33E-02 | 1.43E-08 |
| 82L8_S27e R H5_I34M | 8.41E+05 | 1.44E-02 | 1.71E-08 |
| 82L8_S27e W H5_I34M | 9.29E+05 | 9.25E-03 | 9.95E-09 |

**Table 14-4. Affinity of different CD3 antibodies for human CD3D&CD3E**

| Antibody | Human CD3D&CD3E antigen | | |
|---|---|---|---|
| | ka (1/Ms) | kd (1/s) | KD (M) |
| 82L6H2 | 7.22E+05 | 4.63E-02 | 6.42E-08 |
| 82L8_S27e E H5_I34M | 7.94E+05 | 1.20E-02 | 1.51E-08 |
| 82L8_S27e E H7 | 7.56E+05 | 1.12E-02 | 1.48E-08 |
| 82L8_S27e P,F92Y H5_I34M | 8.21E+05 | 1.72E-02 | 2.10E-08 |
| 82L8_S27e P,L94I H5_I34M | 9.90E+05 | 6.96E-03 | 7.03E-09 |
| 82L8_S27e P,L94V H5_I34M | 9.06E+05 | 9.46E-03 | 1.04E-08 |
| 82L8_S27e P,W50Y H5_I34M | 7.53E+05 | 4.83E-02 | 6.42E-08 |
| 82L8_S27e E,F92Y H5_I34M | 7.51E+05 | 2.01E-02 | 2.67E-08 |
| 82L8_S27e P,F92Y H2_M37V,I34N | 5.86E+05 | 6.91E-03 | 1.18E-08 |
| 82L8_S27e P,L94V H2_M37V,I34N | 7.88E+05 | 3.80E-03 | 4.83E-09 |
| 82L8_S27e E,F92Y H2_M37V,A40G | 6.81E+05 | 1.46E-02 | 2.14E-08 |
| 82L8_S27e E,L94V H2_M37V,A40G | 7.87E+05 | 1.05E-02 | 1.33E-08 |
| 82L8_S27e P,L94I,W50Y H2_M37V,I34N | 3.11E+05 | 1.85E-02 | 5.95E-08 |
| 82L8_S27e P,L94I,W50Y H2_M37V,A40G | 6.43E+05 | 2.36E-02 | 3.67E-08 |
| 82L8H2 | 1.15E+06 | 1.04E-02 | 9.04E-09 |
| 82L8_S27e E H2 | 1.20E+06 | 6.41E-03 | 5.33E-09 |
| 82L8_S27e P H2 | 1.01E+06 | 9.82E-03 | 9.74E-09 |

**Table 14-5. Affinity of different engineered CD3 humanized antibodies for human CD3D&CD3E**

| Antibody | Human CD3D&CD3E antigen | | |
|---|---|---|---|
| | ka (1/Ms) | kd (1/s) | KD (M) |
| 82L6H2_M37V | 4.89E+05 | 3.48E-02 | 7.12E-08 |
| 82L8_S27e E H2_M37V | 7.11E+05 | 8.10E-03 | 1.14E-08 |
| 82L8_S27e P H2_M37V | 8.15E+05 | 5.33E-03 | 6.55E-09 |

**Table 14-6. Affinity of different engineered CD3 humanized antibodies for human CD3D&CD3E**

| Antibody | Human CD3D&CD3E antigen | | |
|---|---|---|---|
| | ka (1/Ms) | kd (1/s) | KD (M) |
| 82L6_S27e P,F92Y H2_M37V,I34N | 1.72E+05 | 3.03E-02 | 1.76E-07 |
| 82L6_S27e P,F92Y H2_M37V,Y32K | 3.03E+05 | 3.16E-02 | 1.04E-07 |
| 82L6_S27e P,F92Y H2_M37V,A40G | 4.28E+05 | 4.87E-02 | 1.14E-07 |
| 82L6_S27e P,L94V H2_M37V,I34N | 2.52E+05 | 2.82E-02 | 1.12E-07 |
| 82L6_S27e P,L94V H2_M37V,Y32K | 3.82E+05 | 2.55E-02 | 6.67E-08 |
| 82L6_S27e P,L94V H2_M37V,A40G | 5.13E+05 | 3.74E-02 | 7.29E-08 |
| 82L8_S27e P,L94V,K24T H7 | 1.10E+06 | 9.24E-03 | 8.40E-09 |
| 82L8_S27e P,L94V,L27b V H7 | 9.83E+05 | 1.84E-02 | 1.88E-08 |
| 82L8_S27e P,L94V,T28S H7 | 9.65E+05 | 1.75E-02 | 1.81E-08 |
| 82L8_S27e P,L94V,R29Q H7 | 8.70E+05 | 1.12E-02 | 1.29E-08 |
| 82L8_S27e P,L94V,K30Q H7 | 4.21E+04 | 3.25E-03 | 7.73E-08 |
| 82L8_S27e P,L94V H7_T28S | 9.36E+05 | 8.33E-03 | 8.91E-09 |
| 82L8_S27e P,L94V H7_N52Q | 5.47E+05 | 1.09E-02 | 1.98E-08 |
| 82L8_S27e P,L94V H7_N52S | 6.85E+05 | 6.59E-03 | 9.62E-09 |
| 82L8_S27e P,L94V H7_S56A | 1.05E+06 | 9.52E-03 | 9.10E-09 |
| 82L8_S27e P,L94V H7_I57T | 1.02E+06 | 1.07E-02 | 1.06E-08 |
| 82L8_S27e P,L94V H7_K58S | 7.47E+05 | 1.60E-02 | 2.14E-08 |
| 82L8_S27e P,L94V H7_F102Y | 9.38E+05 | 8.28E-03 | 8.83E-09 |
| 82L6_S27e P,F92Y H1_I34N | 1.69E+05 | 4.10E-02 | 2.43E-07 |
| 82L6_S27e P,L94V H1_I34N | 2.28E+05 | 3.36E-02 | 1.47E-07 |
| 82L8_S27e P,L94V H7 | 8.61E+05 | 8.21E-03 | 9.54E-09 |

**Table 14-7. Affinity of different engineered CD3 humanized antibodies for human CD3D&CD3E**

| Antibody | Human CD3D&CD3E antigen | | |
|---|---|---|---|
| | ka (1/Ms) | kd (1/s) | KD (M) |
| 82L8_S27e P,L94V,L27b V H7_N52Q | 5.22E+05 | 2.39E-02 | 4.57E-08 |
| 82L8_S27e P,L94V,L27b V H7_I57T | 1.09E+06 | 2.21E-02 | 2.03E-08 |
| 82L8_S27e P,L94V,L27b V H7_K58S | 7.99E+05 | 3.73E-02 | 4.66E-08 |
| 82L8_S27e P,L94V,T28S H7_N52Q | 5.51E+05 | 2.36E-02 | 4.28E-08 |
| 82L8_S27e P,L94V,T28S H7_I57T | 1.09E+06 | 2.13E-02 | 1.95E-08 |
| 82L8_S27e P,L94V,T28S H7_K58S | 7.71E+05 | 3.70E-02 | 4.79E-08 |
| 82L8_S27e P,L94V,R29Q H7_N52Q | 5.69E+05 | 1.48E-02 | 2.60E-08 |
| 82L8_S27e P,L94V,R29Q H7_I57T | 1.28E+06 | 1.50E-02 | 1.17E-08 |
| 82L8_S27e P,L94V,R29Q H7_K58S | 6.98E+05 | 2.32E-02 | 3.33E-08 |
| 82L8_S27e P,L94V,L27b V,T28S H7 | 8.89E+05 | 4.22E-02 | 4.74E-08 |
| 82L8_S27e P,L94V,L27b V,R29Q H7 | 8.91E+05 | 2.28E-02 | 2.56E-08 |
| 82L8_S27e P,L94V,T28S,R29Q H7 | 8.94E+05 | 2.48E-02 | 2.78E-08 |
| 82L8_S27e P,L94V H7_N52Q,I57T | 6.24E+05 | 1.24E-02 | 1.98E-08 |
| 82L8_S27e P,L94V H7_N52Q,K58S | 4.19E+05 | 2.09E-02 | 5.00E-08 |
| 82L8_S27e P,L94V H7_I57T,K58S | 8.17E+05 | 2.12E-02 | 2.60E-08 |

**Table 14-8. Affinity of different engineered CD3 humanized antibodies for human CD3D&CD3E**

| Antibody | Human CD3D&CD3E antigen | | |
|---|---|---|---|
| | ka (1/Ms) | kd (1/s) | KD (M) |
| 82L8_S27e P,L94V H7 | 1.48E+06 | 1.11E-02 | 7.51E-09 |
| 82L8_S27e P,L94V,L27b V H7_N52Q,K58S | 6.43E+05 | 7.10E-02 | 1.11E-07 |
| 82L8_S27e P,L94V,T28S H7_N52Q,K58S | 5.82E+05 | 5.24E-02 | 9.01E-08 |
| 82L8_S27e P,L94V,R29Q H7_N52Q,K58S | 5.36E+05 | 3.73E-02 | 6.96E-08 |
| 82L8_S27e P,L94V,L27b V,T28S H7_N52Q,K58S | 3.64E+05 | 8.66E-02 | 2.38E-07 |

**Table 14-9. Affinity of different CD3 antibodies for human or monkey CD3E**

| Antibody | Human CD3E antigen | | | Cynomolgus monkey CD3E antigen | | |
|---|---|---|---|---|---|---|
| | ka (1/Ms) | kd (1/s) | KD (M) | ka (1/Ms) | kd (1/s) | KD (M) |
| 82L8_S27e E H7 | 1.36E+06 | 1.75E-04 | 1.29E-10 | 1.46E+06 | 2.13E-04 | 1.46E-10 |
| 82L8H2_M37V,T30A,G 55D | 1.03E+06 | 2.01E-04 | 1.96E-10 | 1.21E+06 | 2.17E-04 | 1.79E-10 |
| 82L8H2_M37V,T30A,G 55D,T107S | 1.08E+06 | 1.72E-04 | 1.59E-10 | 1.41E+06 | 2.52E-04 | 1.79E-10 |

**Table 14-10. Affinity of different CD3 antibodies for human CD3D&CD3E**

| Antibody | Human CD3D&CD3E antigen | | |
|---|---|---|---|
| | ka (1/Ms) | kd (1/s) | KD (M) |
| 82L8H2_M37V,T30A,G55D | 6.90E+05 | 6.01E-03 | 8.72E-09 |
| 82L8H2_M37V,T30A,G55D,T107S | 7.20E+05 | 5.76E-03 | 7.99E-09 |

**Table 14-11. Affinity of different CD20/CD3 bispecific antibodies for human CD3E**

| CD20/CD3 bispecific antibody | Human CD3E antigen | | |
|---|---|---|---|
| | ka (1/Ms) | kd (1/s) | KD (M) |
| CD20-82L2H3 | 5.75E+05 | 4.29E-04 | 7.46E-10 |
| CD20-82L3H3 | 5.19E+05 | 2.93E-04 | 5.65E-10 |
| CD20-82L4H3 | 4.87E+05 | 3.27E-04 | 6.72E-10 |
| CD20-82L5H1 | 2.62E+05 | 8.30E-04 | 3.17E-09 |
| CD20-82L5H2 | 4.07E+05 | 7.30E-04 | 1.79E-09 |
| CD20-82L5H3 | 3.92E+05 | 9.35E-04 | 2.39E-09 |
| CD20-82L6H2 | 3.31E+05 | 4.20E-04 | 1.27E-09 |
| CD20-82L6H3 | 3.07E+05 | 6.68E-04 | 2.17E-09 |
| CD20-82L8H4 | 4.91E+05 | 3.79E-04 | 7.72E-10 |
| CD20-82L8H5 | 4.48E+05 | 3.97E-04 | 8.88E-10 |
| CD20-82L9H4 | 3.37E+05 | 6.93E-04 | 2.06E-09 |
| CD20-82L10H4 | 3.36E+05 | 5.34E-04 | 1.59E-09 |
| CD20-82L10H5 | 2.88E+05 | 7.00E-04 | 2.43E-09 |

### Test Example 2. Determination of Antibody Binding Ability at Protein Level

To test the ability of the anti-CD3 antibodies of the present disclosure to bind to CD3 antigens, the binding activities of the anti-CD3 antibodies to human CD3E antigen (Sino Biological, Cat. No. 10977-H08H), cynomolgus monkey CD3E antigen (Sino Biological, Cat. No. 90047-C08H), and human CD3D&CD3E antigen (Sino Biological, Cat. No. CT038-H2508H) were detected by ELISA in this test example. Details are as follows: A 96-well plate was coated with a his-tagged antibody (GenScript, A00186) at 0.25 µg/well and placed at 4 °C overnight. After the supernatant was discarded, the plate was blocked with 5% skim milk for 2 h. The blocked 96-well plate was washed twice. A 2 µg/mL solution of a CD3 antigen was prepared using a buffer (BSA at a final concentration of 1% was added to PBS) and added to the 96-well plate at 50 µL/well. The plate was incubated at 37 °C for 1 h. The 96-well plate was washed four times, and the antibodies were diluted with a buffer (initial concentration: 200 nM, 4-fold dilution to obtain 8 concentrations). The antibodies were then added, and the plate was incubated at 37 °C for 1 h. After the plate was washed four times, an anti human IgG Fc-HRP secondary antibody (1:8000) was added, and the plate was incubated at 37 °C for 1 h. After color development with TMB, the reaction was stopped with 0.1 M sulfuric acid. The ELISA results are shown in the table below.

**Table 15-1. Binding of anti-CD3 antibodies to CD3 antigens**

| Antibody | Human CD3E antigen | | Cynomolgus monkey CD3E antigen | | Human CD3D&CD3E antigen | |
|---|---|---|---|---|---|---|
| | Emax | EC₅₀ (nM) | Emax | EC₅₀ (nM) | Emax | EC₅₀ (nM) |
| Chi8 | 0.4323 | 0.0303 | 0.4388 | 0.03544 | 1.075 | 0.02515 |
| Chi31 | 0.4583 | 0.07054 | 0.4378 | 0.07013 | 1.11 | 0.1046 |
| Chi21 | 0.4856 | 0.07959 | 0.4121 | 0.06223 | 0.9545 | 0.05627 |
| Chi29 | 0.5065 | 0.02612 | 0.498 | 0.04026 | 0.4437 | 0.02709 |
| Chi61 | 0.482 | 0.03912 | 0.4442 | 0.05082 | 0.3625 | 0.07249 |
| Chi57 | 0.4542 | 0.3265 | 0.4139 | 0.59 | 0.2842 | 0.4913 |
| Chi92-2 | 0.5327 | 0.1612 | ND | | 0.3974 | 0.06298 |

| | | | | | | |
|---|---|---|---|---|---|---|
| Note: ND means not detected, i.e., there was no binding. | | | | | | |

**Table 15-2. Binding of anti-CD3 antibodies to CD3 antigens**

| Antibody | Human CD3E antigen | | Cynomolgus monkey CD3E antigen | | Human CD3D&CD3E antigen | |
|---|---|---|---|---|---|---|
| | Emax | EC₅₀ (nM) | Emax | EC₅₀ (nM) | Emax | EC₅₀ (nM) |
| Chi12 | 2.487 | 0.1563 | 2.443 | 0.1511 | 1.746 | 1.065 |
| Chi82 | 2.597 | 0.1528 | 2.458 | 0.1664 | 1.964 | 0.309 |
| Chi89 | 2.609 | 0.17 | 2.508 | 0.1008 | 2.036 | 0.1333 |
| Chi96 | 2.508 | 0.22 | 2.461 | 0.2136 | 1.845 | 0.6839 |
| Chi103 | 2.79 | 15.33 | 2.73 | 18.03 | 2.598 | 0.3145 |

The results show that the anti-CD3 chimeric antibodies obtained by screening had relatively good specific binding ability to human CD3E or monkey CD3E, and also had a significant binding effect to the human CD3D&CD3E heterodimer.

### Test Example 3. Determination of Antibody Binding Ability at Cellular Level

The binding ability of the anti-CD3 antibodies to the cell surface antigen was determined by fluorescence-activated cell sorting (FACS). Jurkat cells (from ATCC, PTS-TIB-152) were used in the binding determination to the cell surface antigen CD3.

Cells resuspended in a FACS buffer (98% PBS, 2% FBS) were added to a 96-well U-shaped bottom plate (CORNING, 3795), and the antibody diluted in a gradient was added. The plate was incubated at 4 °C for 1 h and washed twice with a FACS buffer, and then Alexa Fluor 488 anti-human IgG H+L Antibody (Invitrogen, Cat. No. A11013, 1:500 dilution) was added to each well. The plate was incubated at 4 °C for 30 min and washed twice, and then the cells were resuspended in a FACS buffer. Finally, fluorescence signal values were read using FACS CantoII (BD).

**Table 16-1. Binding of anti-CD3 antibodies to Jurkat cells**

| Antibody | Emax | EC₅₀ (nM) |
|---|---|---|
| Chi8 | 11835 | 0.8309 |
| Chi31 | 11995 | 1.375 |
| Chi21 | 11790 | 0.9252 |
| Chi29 | 11221 | 1.354 |
| Chi92-2 | 12958 | 1.129 |
| Chi96 | 11205 | 1.149 |
| Chi103 | 11337 | 1.798 |

The results show that all the anti-CD3 chimeric antibodies obtained by screening had a significant binding effect on the CD3-positive Jurkat cell line.

**Table 16-2. Binding of anti-CD3 antibodies to Jurkat cells**

| Test I | | |
|---|---|---|
| Antibody | Emax | EC₅₀ (nM) |
| Chi12 | 4646 | 1.684 |
| 12L1H6 | 3495 | 200.8 |
| 12L2H2 | 7536 | 3.89 |
| 12L2H4 | 4863 | 1.363 |
| 12L2H5 | 5357 | 2.147 |

| Test II | | |
|---|---|---|
| Monoclonal antibody | Emax | EC50 (nM) |
| Chi12 | 13892 | 2.03 |
| 12L3H2 | 18946 | 1.577 |
| 12L3H3 | 8416 | 0.5701 |
| 12L3H4 | 12575 | 0.8337 |
| 12L3H5 | 12997 | 1.153 |
| 12L4H2 | 15622 | 0.7996 |
| 12L4H3 | 12219 | 0.8719 |

| Test III | | |
|---|---|---|
| Antibody | Emax | EC₅₀ (nM) |
| Chi12 | 16179 | 2.739 |
| 12L4H4 | 13542 | 1.182 |
| 12L4H5 | 15063 | 1.305 |
| 12L5H2 | 8078 | 26.83 |
| 12L5H3 | 6670 | 29.66 |
| 12L5H4 | 6466 | 16.09 |
| 12L5H5 | 6171 | 19.67 |

| Test IV | | |
|---|---|---|
| Antibody | Emax | EC₅₀ (nM) |
| Chi12 | 4766 | 2.625 |
| 12L6H8 | 4307 | 0.8406 |
| 12L6H9 | 3593 | 0.8065 |
| 12L7H8 | 2947 | 12.96 |
| 12L7H9 | 1925 | 5.868 |

The results show that most of the anti-CD3 humanized antibodies of 12# had a significant binding effect on the CD3-positive Jurkat cell line.

**Table 16-3. Binding of anti-CD3 antibodies to Jurkat cells**

| Antibody | Emax | EC₅₀ (nM) |
|---|---|---|
| 82L4H3 | 2494 | 0.008511 |
| 82L6H2 | 1820 | 0.2768 |
| 82L8H5 | 2209 | 0.3101 |
| 82L10H4 | 1567 | 0.3936 |
| 82L10H5 | 1287 | 0.4044 |

The results show that the anti-CD3 humanized antibodies of 82# all had a significant binding effect on the CD3-positive Jurkat cell line.

**Table 16-4. Binding of anti-CD3 antibodies to Jurkat cells**

| Antibody | Emax | EC₅₀ (nM) |
|---|---|---|
| 82L8_S27e D H5 | 13534 | 1.884 |
| 82L8_S27e E H5 | 13891 | 1.919 |
| 82L8_S27e K H5 | 14226 | 1.018 |
| 82L8_S27e P H5 | 13829 | 1.221 |
| 82L8_S27e Q H5 | 14081 | 1.305 |
| 82L8_S27e R H5 | 14296 | 0.8541 |
| 82L8_S27e W H5 | 13841 | 1.166 |
| 82L8H5 | 14140 | 0.8664 |

**Table 16-5. Binding of anti-CD3 antibodies to Jurkat cells**

| Antibody | Emax | EC₅₀ (nM) |
|---|---|---|
| 82L8_S27e D H5_I34M | 2255 | 0.4917 |
| 82L8_S27e K H5_I34M | 2559 | 0.3077 |
| 82L8_S27e P H5_I34M | 2211 | 0.2395 |
| 82L8_S27e Q H5_I34M | 2235 | 0.2807 |
| 82L8_S27e R H5_I34M | 2222 | 0.186 |
| 82L8_S27e W H5_I34M | 2084 | 0.1482 |

**Table 16-6. Binding of anti-CD3 antibodies to Jurkat cells**

| Antibody | Emax | EC₅₀ (nM) |
|---|---|---|
| 82L8H6 | 27646 | 2.198 |
| 82L8H7 | 28807 | 1.731 |

**Table 16-7. Binding of anti-CD3 antibodies to Jurkat cells**

| Antibody | Emax | EC₅₀ (nM) |
|---|---|---|
| 82L8_S27e E H5_I34M | 7363 | 0.1216 |
| 82L8_S27e P,F92Y H5_I34M | 7117 | 0.09055 |
| 82L8_S27e P,L94I H5_I34M | 7787 | 0.1108 |
| 82L8_S27e P,L94V H5_I34M | 7752 | 0.06511 |
| 82L8_S27e P,W50Y H5_I34M | 5033 | 0.06377 |
| 82L8_S27e P,W50Y H4_I34M | 5443 | 0.081 |

**Table 16-8. Binding of anti-CD3 antibodies to Jurkat cells**

| Antibody | Emax | EC₅₀ (nM) |
|---|---|---|
| 82L8_S27e E,F92Y H5_I34M | 7247 | 0.1572 |
| 82L8_S27e E,L94V H5_I34M | 7350 | 0.08692 |
| 82L8_S27e E,F92Y H4_I34M | 7025 | 0.1106 |
| 82L8_S27e E,L94V H4_I34M | 6670 | 0.08058 |
| 82L8_S27e E H7 | 7966 | 0.08298 |
| 82L8_S27e E H6 | 7688 | 0.07647 |

**Table 16-9. Binding of anti-CD3 antibodies to Jurkat cells**

| Antibody | Emax | EC₅₀ (nM) |
|---|---|---|
| 82L8H2 | 7885 | 0.07461 |
| 82L8_S27e P H2 | 7524 | 0.06238 |
| 82L8_S27e E H2 | 7476 | 0.1096 |
| 82L8_S27e P,F92Y H2_M37V,I34N | 7722 | 0.1241 |
| 82L8_S27e P,L94I H2_M37V,I34N | 8000 | 0.183 |
| 82L8_S27e P,L94V H2_M37V,I34N | 8177 | 0.1294 |
| 82L8_S27e E,F92Y H2_M37V,A40G | 7172 | 0.09092 |
| 82L8_S27e E,L94V H2_M37V,A40G | 7365 | 0.1264 |
| 82L8_S27e P,L94I,W50Y H2_M37V,I34N | 6589 | 0.1542 |
| 82L8_S27e P,L94I,W50Y H2_M37V,A40G | 6737 | 0.0592 |

The results show that all the anti-CD3 antibodies had a significant binding effect on the CD3-positive Jurkat cell line.

### Test Example 4. In Vitro T Cell Activation Effects of CD3 Monoclonal Antibodies

To test the activation effect of the anti-CD3 antibodies of the present disclosure, in this test example, Jurkat-NFAT luc (Promega, J133A) cells were used to detect the activation of Jurkat cells by CD3 monoclonal antibodies in solution. Method:
Jurkat-NFAT luc cells were subcultured in a complete culture medium. 5 µg/mL anti-human Fc antibody was prepared in PBS and added to a 96-well plate at 100 µL/well. The plate was coated at 37 °C for 3 h, and then the supernatant was discarded. The plate was washed three times with PBST, 300 µL each time. The antibody was diluted in a gradient with an RPMI1640 culture medium containing 10% FBS, and added to a 96-well microplate at 50 µL/well. Jurkat-NFAT luc cells were collected by centrifugation, resuspended in an RPMI1640 culture medium containing 10% FBS, and counted, and the cell density was adjusted to 1 × 10⁶ cells/mL. 50 µL of the cells were added to each well of the 96-well microplate containing the antibody (5 × 10⁴ cells/well). After the plate was left to stand at 37 °C for 6 h, a firefly-glo luciferase reporter gene detection reagent (MeilunBio, Cat. # MA0519-2) was added at 50 µL/well. The plate was left to stand at room temperature for 8 min, and then the solution was transferred to a white plate. The plate was read using a multi-mode microplate reader (BMG, Cat. # PHERAstar). The results are shown in the table below.

**Table 17-1. Activation of T cells by anti-CD3 antibodies**

| Antibody | Emax | EC₅₀ (nM) |
|---|---|---|
| Chi12 | 6329 | 0.7726 |
| Chi21 | 5543 | 0.292 |
| Chi8 | 5195 | 0.2944 |
| Chi29 | 5253 | 0.3712 |
| Chi31 | 6413 | 0.3723 |
| Chi57 | 3398 | 1.577 |
| Chi61 | 2521 | 1.146 |
| Chi82 | 6305 | 0.1933 |
| Chi89 | 6440 | 0.1276 |
| Chi92-2 | 6118 | 0.1704 |
| Chi96 | 7112 | 0.4061 |
| Chi103 | 5917 | 0.1397 |

The results show that the anti-CD3 antibodies had a significant activation effect on Jurkat-NFAT cells, and the anti-CD3 antibodies obtained by screening showed different degrees of T cell activation effects.

**Table 17-2. Activation of T cells by 12# anti-CD3 antibodies**

| Test I | | |
|---|---|---|
| Antibody | Emax | EC₅₀ (nM) |
| Chi12 | 83684 | 0.05043 |
| 12L1H6 | 20120 | 0.07146 |
| 12L2H2 | 87707 | 0.09127 |
| 12L2H3 | 86812 | 0.2095 |
| 12L2H4 | 93920 | 0.08541 |
| 12L2H5 | 85590 | 0.1589 |

| Test II | | |
|---|---|---|
| Antibody | Emax | EC₅₀ (nM) |
| Chi12 | 92095 | 0.0341 |
| 12L3H2 | 87347 | 0.08024 |
| 12L3H3 | 86333 | 0.09433 |
| 12L3H4 | 95560 | 0.07646 |
| 12L3H5 | 84200 | 0.09338 |
| 12L4H2 | 98427 | 0.077 |
| 12L4H3 | 97000 | 0.07251 |

| Test III | | |
|---|---|---|
| Antibody | Emax | EC₅₀ (nM) |
| 12L4H4 | 86747 | 0.07755 |
| 12L4H5 | 76760 | 0.07456 |
| 12L5H2 | 25480 | 1.929 |
| 12L5H4 | 33525 | 1.102 |
| 12L5H5 | 8600 | 1.792 |

| Test IV | | |
|---|---|---|
| Antibody | Emax | EC₅₀ (nM) |
| Chi12 | 75307 | 0.08 |
| 12L6H8 | 72893 | 0.07947 |
| 12L6H9 | 80840 | 0.07872 |
| 12L7H8 | 28560 | 2.005 |
| 12L7H9 | 29360 | 1.459 |

The results show that 12# antibodies had a significant activation effect on Jurkat-NFAT cells, and exhibited different degrees of T cell activation effects.

**Table 17-3. Activation of T cells by 82# anti-CD3 antibodies**

| Test I | | |
|---|---|---|
| Antibody | Emax | EC₅₀ (nM) |
| Chi82 | 94567 | 0.2102 |
| 82L1H1 | 84969 | 0.1967 |
| 82L1H2 | 87561 | 0.08525 |
| 82L2H1 | 92294 | 0.1635 |

| Test II | | |
|---|---|---|
| Antibody | Emax | EC₅₀ (nM) |
| Chi82 | 89506 | 0.1829 |
| 82L2H2 | 85835 | 0.1797 |
| 82L2H3 | 85650 | 0.1373 |
| 82L3H1 | 87877 | 0.1679 |
| 82L3H2 | 92434 | 0.1711 |

| Test III | | |
|---|---|---|
| Antibody | Emax | EC₅₀ (nM) |
| Chi82 | 85382 | 0.1786 |
| 82L3H3 | 78719 | 0.1528 |
| 82L4H1 | 87922 | 0.2213 |
| 82L4H2 | 81078 | 0.1138 |
| 82L4H3 | 92659 | 0.2984 |

| Test IV | | |
|---|---|---|
| Antibody | Emax | EC₅₀ (nM) |
| Chi82 | 82729 | 0.1113 |
| 82L5H1 | 47886 | 2.007 |
| 82L5H2 | 57118 | 2.169 |
| 82L5H3 | 59534 | 1.258 |
| 82L6H1 | 74206 | 1.27 |

| Test V | | |
|---|---|---|
| Antibody | Emax | EC₅₀ (nM) |
| Chi82 | 77150 | 0.1503 |
| 82L6H2 | 54119 | 0.5209 |
| 82L6H3 | 55180 | 0.5733 |
| 82L7H2 | 13532 | 2.864 |

| Test VI | | |
|---|---|---|
| Antibody | Emax | EC₅₀ (nM) |
| Chi82 | 79913 | 0.177 |
| 82L7H3 | 4770 | 2.204 |
| 82L8H4 | 66932 | 0.1834 |
| 82L8H5 | 70516 | 0.1633 |
| 82L9H4 | 56631 | 1.341 |
| 82L9H5 | 52034 | 2.131 |
| 82L10H4 | 58726 | 1.23 |
| 82L10H5 | 58626 | 1.552 |

The results show that 82# antibodies had a significant activation effect on Jurkat-NFAT cells, and exhibited different degrees of T cell activation effects.

### Test Example 5. In Vitro T Cell Activation Effects of CD20/CD3 Bispecific Antibodies

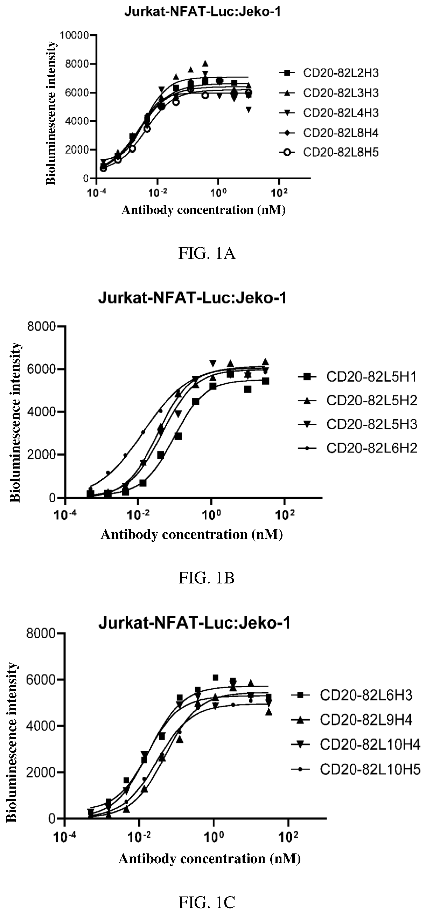

To test the conditional activation effect of the CD20/CD3 antibodies in the examples, in this test example, Jurkat-NFAT luc (Promega, J133A) cells were used to detect the activation of Jurkat cells by CD20/CD3 bispecific antibodies in the presence of CD20-positive cells Jeko-1 (ATCC, CRL3006). Method:
Jurkat-NFAT luc cells were subcultured in a complete culture medium. The antibody was prepared in an RPMI1640 culture medium containing 10% FBS and subjected to 3-fold dilution from an initial concentration of 200 nM (the final concentration was 100 nM) for a total of 10 concentration points, or to 3-fold dilution from an initial concentration of 30 nM (the final concentration was 10 nM) for a total of 10 concentration points. After the preparation was completed, the antibody was added to a 96-well plate at 40 µL/well. CD20-positive cells Jeko-1 were prepared and resuspended to 8 × 10⁵ cells/mL in an RPMI1640 culture medium containing 10% FBS. Jurkat-NFAT luc cells were collected by centrifugation and resuspended to 4 × 10⁶ cells/mL in a culture medium. Jeko-1 and Jurkat-NFAT luc were mixed in a volume ratio of 1:1, and then the mixture was added to the 96-well plate at 80 µL/well, with a final E:T ratio of 5:1. After the plate was left to stand at 37 °C for 5 h, a firefly-glo luciferase reporter gene detection reagent (MeilunBio, Cat. # MA0519-2) was added at 50 µL/well. The plate was left to stand at room temperature for 8 min, and then the solution was transferred to a white plate. The plate was read using a multi-mode microplate reader (BMG, Cat. # PHERAstar). The results are shown in FIGs. 1A-1C.

The results show that the CD20/CD3 bispecific antibodies exhibited different degrees of T cell activation effects in the presence of Jeko-1 cells.

### Test Example 6. In Vitro Killing Effects of CD20/CD3 Bispecific Antibodies

This test example was intended to test the killing of tumor cells by CD20/CD3 bispecific antibody-mediated human peripheral blood mononuclear cells (PBMCs). In this assay, luciferase-containing CD20-positive Daudi cells (Daudi-luc) were constructed, and the killing effects of CD20/CD3 bispecific antibody-mediated PBMCs on CD20-positive tumor cells were analyzed by detecting luciferase signals of Daudi-Luc cells in a co-incubation system containing PBMCs, bispecific antibodies, and tumor cells. Method: The 2× antibody was prepared in a culture medium (RPMI1640 + 15% FBS) and subjected to 5-fold dilution from an initial concentration of 20 nM for a total of 9 concentration points, or to 3-fold dilution from an initial concentration of 60 nM for a total of 9 concentration points. After the preparation was completed, the antibody was added to a 96-well plate at 50 µL/well. Daudi-Luc cells were prepared, collected, and resuspended to 3×10⁵ cells/mL in a culture medium (RPMI1640 + 15% FBS). PBMCs were taken out from a liquid nitrogen tank and rapidly thawed at 37 °C. The cells were slowly added to 20 mL of pre-heated RPMI1640 + 15% FBS culture medium. The cells were collected and resuspended to 1.5 × 10⁶ cells/mL with the culture medium. Daudi-luc and PBMCs were mixed in a volume ratio of 1:1, and the mixture was added to the 96-well plate at 50 µL/well, with a final E:T ratio of 5:1. The 96-well plate was placed in an incubator for 48 h.

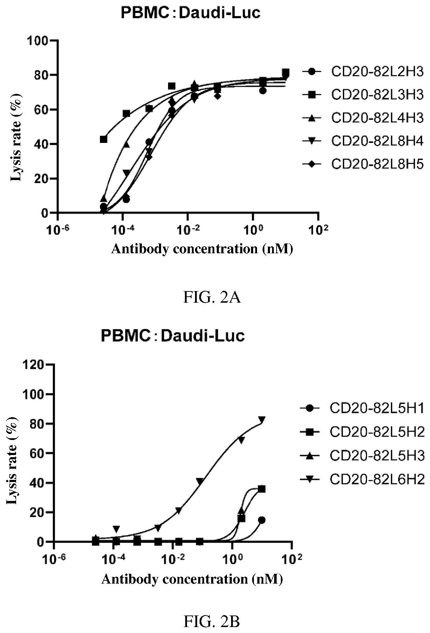

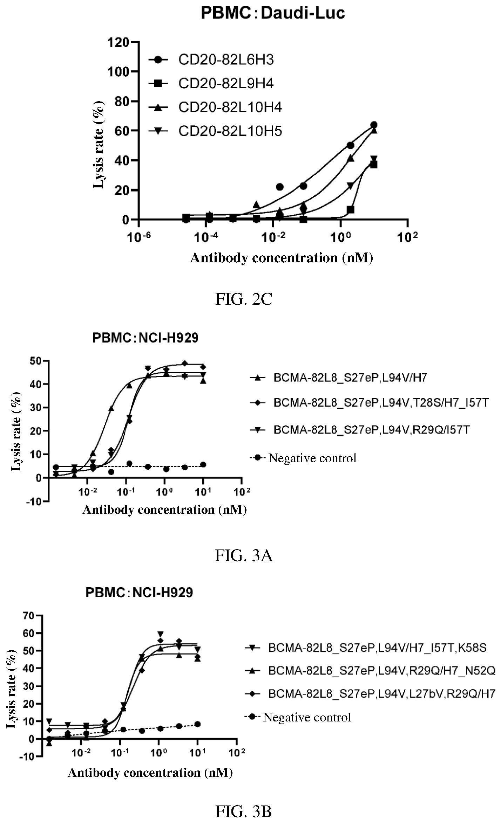

One-Glo detection method: A reagent was directly added at 50 µL/well, and the plate was shaken and read for chemiluminescence. The results are shown in FIGs. 2A-2C.

The results show that all the CD20/CD3 bispecific antibodies constructed with different humanized anti-CD3 antibodies were able to show a certain killing effect on tumor cells.

### Test Example 7. In Vitro Killing Effects of BCMA/CD3 Bispecific Antibodies

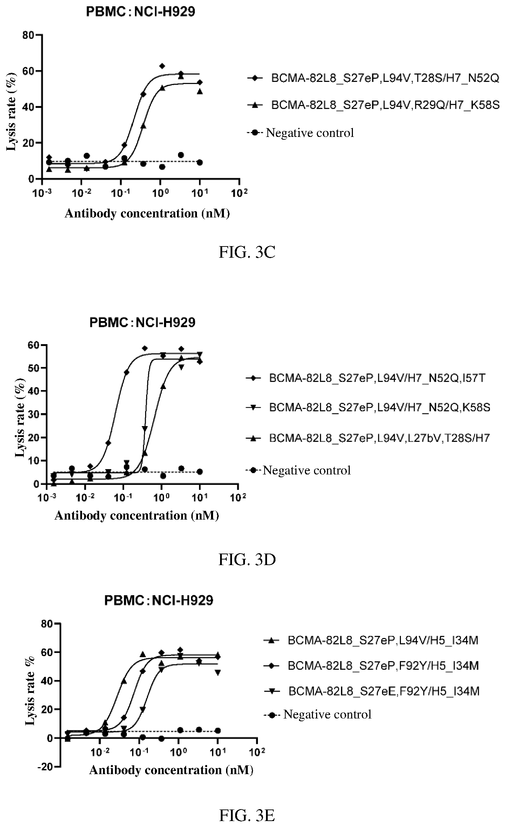

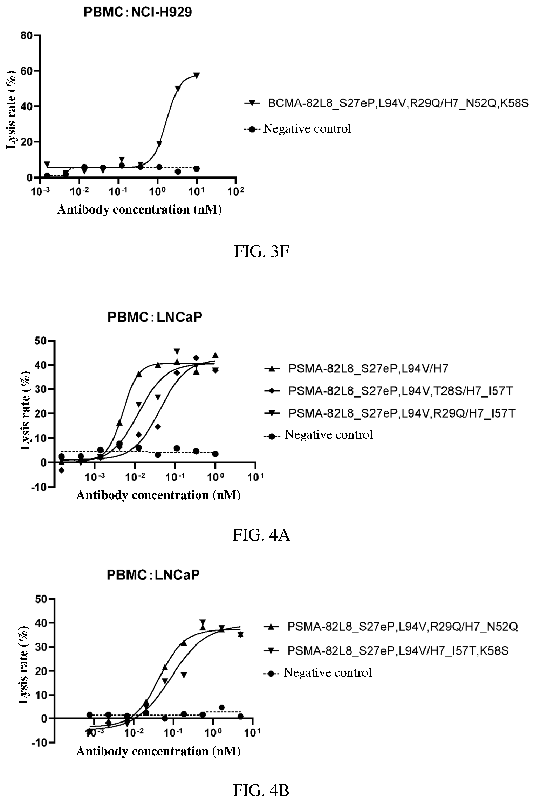

To test the killing effects of the BCMA/CD3 bispecific antibody-mediated human peripheral blood mononuclear cells (PBMCs) on tumor cells, the BCMA/CD3 bispecific antibodies were diluted in a gradient with a complete culture medium (an RPMI1640 culture medium containing 15% FBS) and added to a 96-well plate at 40 µL/well. BCMA-expressing NCI-H929 cells (CRL-3580, ATCC) were centrifuged at 1000 rpm for 5 min, resuspended in a complete culture medium, and counted. The cell density was adjusted to 5 × 10⁵ cells/mL. Cryopreserved PBMCs (Shanghai Xuanfeng Biotechnology Co., Ltd.) were rapidly thawed at 37 °C. The cells were slowly added to 20 mL of pre-heated complete culture medium and centrifuged at 1500 rpm for 10 min. The cells were resuspended in the complete culture medium and counted, and the cell density was adjusted to 2.5 × 10⁶ cells/mL. The NCI-H929 cells and PBMCs described above were mixed in equal volumes (with a final effector-to-target ratio (E:T) of 5:1). After mixing, the mixture was added to the 96-well plate at 160 µL/well. The 96-well plate was placed in a 5% CO₂ incubator at 37 °C for 48 h, and then the culture plate was taken out. The supernatant was pipetted. The content of LDH released by dead cells was detected according to the instructions of the LDH assay kit (Cat. # G1780, Promega), and the percentage of tumor cell death induced by the BCMA/CD3 bispecific antibodies was determined. The results are shown in FIGs. 3A-3F.

The results show that all the BCMA/CD3 bispecific antibodies constructed with different humanized anti-CD3 antibodies were able to show a certain killing effect on tumor cells.

### Test Example 8. In Vitro Killing of Prostate Cancer Cells by PSMA/CD3 Bispecific Antibody-Induced PBMCs

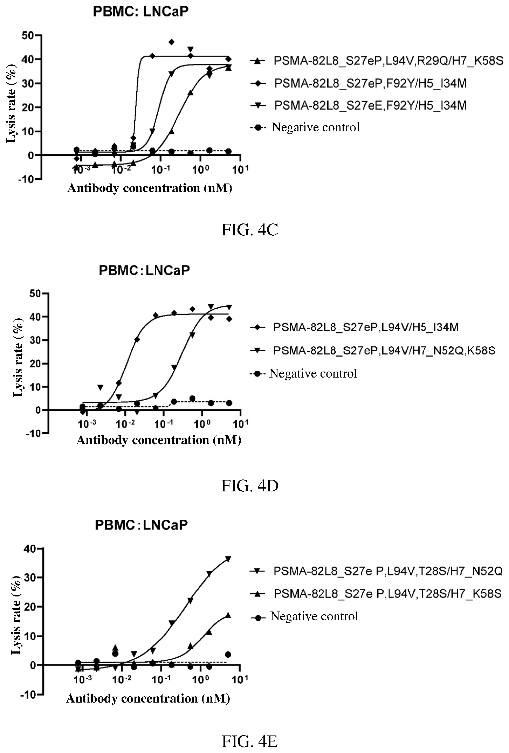

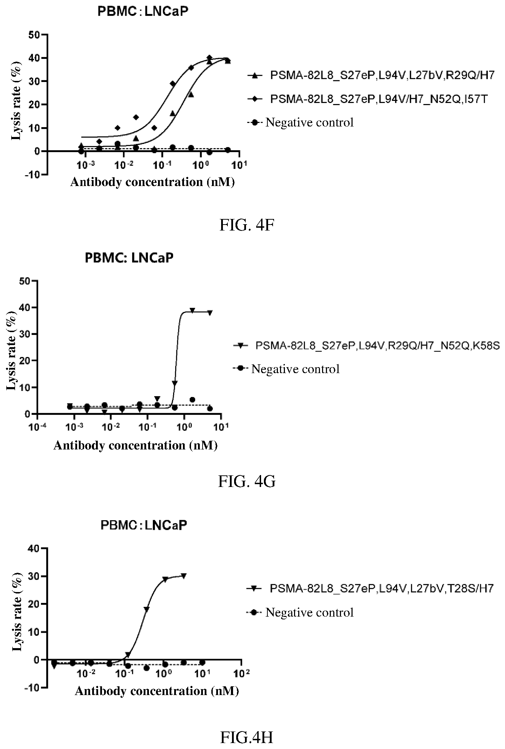

The PSMA/CD3 bispecific antibody was diluted in a gradient with a complete culture medium (an RPMI1640 culture medium containing 15% FBS), and added to a 96-well plate at 40 µL/well. PSMA-expressing LnCAP cells (CRL-1740, ATCC) were centrifuged at 1000 rpm for 5 min, resuspended in a complete culture medium, and counted. The cell density was adjusted to 5 × 10⁵ cells/mL. Cryopreserved PBMCs (Shanghai Xuanfeng Biotechnology Co., Ltd.) were rapidly thawed at 37 °C. The cells were slowly added to 20 mL of pre-heated complete culture medium and centrifuged at 1500 rpm for 10 min. The cells were resuspended in the complete culture medium and counted, and the cell density was adjusted to 2.5 × 10⁶ cells/mL. The LnCAP cells and PBMCs described above were mixed in equal volumes (with a final effector-to-target ratio of 5:1). After mixing, the mixture was added to the 96-well plate at 160 µL/well. The 96-well plate was placed in a 5% CO₂ incubator at 37 °C for 48 h, and then the culture plate was taken out. The supernatant was pipetted. The content of LDH released by dead cells was detected according to the instructions of the LDH assay kit (Cat. # G1780, Promega), and the percentage of tumor cell death induced by the bispecific antibodies in the examples was determined. The results are shown in FIGs. 4A-4H.

The results show that all the PSMA/CD3 bispecific antibodies constructed with different humanized anti-CD3 antibodies were able to show a certain killing effect on tumor cells. Although the above invention has been described in detail using drawings and examples for a clear understanding, the description and examples should not be construed as limiting the scope of the present disclosure. The disclosures of all the patents and scientific documents cited herein are clearly incorporated by reference in their entirety.

## Claims

1. An anti-CD3 antibody, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. in the heavy chain variable region, the HCDR1 comprises the amino acid sequence of SEQ ID NO: 343, the HCDR2 comprises the amino acid sequence of SEQ ID NO: 344, and the HCDR3 comprises the amino acid sequence of SEQ ID NO: 345, and in the light chain variable region, the LCDR1 comprises the amino acid sequence of SEQ ID NO: 346, the LCDR2 comprises the amino acid sequence of SEQ ID NO: 347, and the LCDR3 comprises the amino acid sequence of SEQ ID NO: 348; or
b. in the heavy chain variable region, the HCDR1 comprises the amino acid sequence of SEQ ID NO: 8, the HCDR2 comprises the amino acid sequence of SEQ ID NO: 400, and the HCDR3 comprises the amino acid sequence of SEQ ID NO: 401, and in the light chain variable region, the LCDR1 comprises the amino acid sequence of SEQ ID NO: 402, the LCDR2 comprises the amino acid sequence of SEQ ID NO: 2, and the LCDR3 comprises the amino acid sequence of SEQ ID NO: 7;
preferably,
a. in the heavy chain variable region, the HCDR1 comprises the amino acid sequence of SEQ ID NO: 137, the HCDR2 comprises the amino acid sequence of SEQ ID NO: 403, and the HCDR3 comprises the amino acid sequence of SEQ ID NO: 36, and in the light chain variable region, the LCDR1 comprises the amino acid sequence of SEQ ID NO: 404, the LCDR2 comprises the amino acid sequence of SEQ ID NO: 2, and the LCDR3 comprises the amino acid sequence of SEQ ID NO: 405; or
b. in the heavy chain variable region, the HCDR1 comprises the amino acid sequence of SEQ ID NO: 137, 34, 193, 194, 195, 196, 197, or 199, the HCDR2 comprises the amino acid sequence of SEQ ID NO: 138, 217, 214, 218, 219, 220, 221, 35, 38, 215, or 216, and the HCDR3 comprises the amino acid sequence of SEQ ID NO: 36 or 43, and in the light chain variable region, the LCDR1 comprises the amino acid sequence of SEQ ID NO: 189, 187, 207, 208, 210, 211, 206, 1, 96, 186, 188, 190, 191, 192, 205, 209, or 212, the LCDR2 comprises the amino acid sequence of SEQ ID NO: 2 or 136, and the LCDR3 comprises the amino acid sequence of SEQ ID NO: 185, 183, 17, 182, or 184; or
b. in the heavy chain variable region, the HCDR1 comprises the amino acid sequence of SEQ ID NO: 8, the HCDR2 comprises the amino acid sequence of SEQ ID NO: 9, 97, 98, or 100, and the HCDR3 comprises the amino acid sequence of SEQ ID NO: 10 or 99, and in the light chain variable region, the LCDR1 comprises the amino acid sequence of SEQ ID NO: 1 or 96, the LCDR2 comprises the amino acid sequence of SEQ ID NO: 2, and the LCDR3 comprises the amino acid sequence of SEQ ID NO: 7;
more preferably, in the heavy chain variable region, the HCDR1 comprises the amino acid sequence of SEQ ID NO: 137, the HCDR2 comprises the amino acid sequence of SEQ ID NO: 138, 214, 217, 218, 219, 220, or 221, and the HCDR3 comprises the amino acid sequence of SEQ ID NO: 36, and in the light chain variable region, the LCDR1 comprises the amino acid sequence of SEQ ID NO: 189, 187, 206, 207, 208, 210, or 211, the LCDR2 comprises the amino acid sequence of SEQ ID NO: 2, and the LCDR3 comprises the amino acid sequence of SEQ ID NO: 185 or 183;
most preferably,
1) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
2) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 183; or
3) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 187, the LCDR2 comprises the amino acid sequence of SEQ ID NO: 2, and the LCDR3 comprises the amino acid sequence of SEQ ID NO: 183; or
4) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
5) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
6) in the heavy chain variable region, the HCDR1 comprises the amino acid sequence of SEQ ID NO: 137, the HCDR2 comprises the amino acid sequence of SEQ ID NO: 217, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
7) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185; or
8) in the heavy chain variable region, the HCDR1 comprises the amino acid sequence of SEQ ID NO: 137, the HCDR2 comprises the amino acid sequence of SEQ ID NO: 214, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
9) in the heavy chain variable region, the HCDR1 comprises the amino acid sequence of SEQ ID NO: 137, the HCDR2 comprises the amino acid sequence of SEQ ID NO: 218, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
10) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 210, the LCDR2 comprises the amino acid sequence of SEQ ID NO: 2, and the LCDR3 comprises the amino acid sequence of SEQ ID NO: 185; or
11) in the heavy chain variable region, the HCDR1 comprises the amino acid sequence of SEQ ID NO: 137, the HCDR2 comprises the amino acid sequence of SEQ ID NO: 138, and the HCDR3 comprises the amino acid sequence of SEQ ID NO: 36, and in the light chain variable region, the LCDR1 comprises the amino acid sequence of SEQ ID NO: 211, the LCDR2 comprises the amino acid sequence of SEQ ID NO: 2, and the LCDR3 comprises the amino acid sequence of SEQ ID NO: 185; or
12) in the heavy chain variable region, the HCDR1 comprises the amino acid sequence of SEQ ID NO: 137, the HCDR2 comprises the amino acid sequence of SEQ ID NO: 219, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
13) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
14) in the heavy chain variable region, the HCDR1 comprises the amino acid sequence of SEQ ID NO: 137, the HCDR2 comprises the amino acid sequence of SEQ ID NO: 221, and the HCDR3 comprises the amino acid sequence of SEQ ID NO: 36, and in the light chain variable region, the LCDR1 comprises the amino acid sequence of SEQ ID NO: 189, the LCDR2 comprises the amino acid sequence of SEQ ID NO: 2, and the LCDR3 comprises the amino acid sequence of SEQ ID NO: 185; or
15) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 206, the LCDR2 comprises the amino acid sequence of SEQ ID NO: 2, and the LCDR3 comprises the amino acid sequence of SEQ ID NO: 185; or
16) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 207, the LCDR2 comprises the amino acid sequence of SEQ ID NO: 2, and the LCDR3 comprises the amino acid sequence of SEQ ID NO: 185; or
17) in the heavy chain variable region, the HCDR1 comprises the amino acid sequence of SEQ ID NO: 137, the HCDR2 comprises the amino acid sequence of SEQ ID NO: 220, and the HCDR3 comprises the amino acid sequence of SEQ ID NO: 36, and in the light chain variable region, the LCDR1 comprises the amino acid sequence of SEQ ID NO: 208, the LCDR2 comprises the amino acid sequence of SEQ ID NO: 2, and the LCDR3 comprises the amino acid sequence of SEQ ID NO: 185.

2. The anti-CD3 antibody according to claim 1, being a murine-derived antibody, a chimeric antibody, a humanized antibody, or a fully human-derived antibody, preferably a humanized antibody.

3. The anti-CD3 antibody according to claim 1 or 2, wherein:
a. the heavy chain variable region comprises an amino acid sequence having at least 80% sequence identity to SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, and the light chain variable region comprises an amino acid sequence having at least 80% sequence identity to SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273; or
the heavy chain variable region comprises an amino acid sequence having at least 80% sequence identity to SEQ ID NO: 75, and the light chain variable region comprises an amino acid sequence having at least 80% sequence identity to SEQ ID NO: 74; or
b. the heavy chain variable region comprises an amino acid sequence having at least 80% sequence identity to SEQ ID NO: 108, 109, 110, 111, 112, 113, 114, 115, 116, 117, 118, or 119, and the light chain variable region comprises an amino acid sequence having at least 80% sequence identity to SEQ ID NO: 101, 102, 103, 104, 105, 106, or 107; or the heavy chain variable region comprises an amino acid sequence having at least 80% sequence identity to SEQ ID NO: 60, and the light chain variable region comprises an amino acid sequence having at least 80% sequence identity to SEQ ID NO: 59;
preferably,
a. the heavy chain variable region comprises the amino acid sequence of SEQ ID NO: 255, 278, 279, 275, 157, 281, 282, 283, 150, 151, 152, 153, 154, 155, 156, 158, 159, 160, 161, 162, 245, 246, 247, 248, 249, 250, 251, 252, 253, 254, 256, 257, 258, 259, 260, 261, 262, 263, 264, 265, 274, 276, 277, 280, 349, 356, 357, 358, 359, 360, 361, or 362, and the light chain variable region comprises the amino acid sequence of SEQ ID NO: 236, 237, 238, 268, 269, 271, 272, 267, 139, 140, 141, 142, 143, 144, 145, 146, 147, 148, 149, 222, 223, 224, 225, 226, 227, 228, 229, 230, 231, 232, 233, 234, 235, 239, 240, 241, 242, 243, 244, 266, 270, or 273; or
the heavy chain variable region comprises the amino acid sequence of SEQ ID NO: 75, and the light chain variable region comprises the amino acid sequence of SEQ ID NO: 74; or
b. the heavy chain variable region comprises the amino acid sequence of SEQ ID NO: 108, 109, 110, 111, 112, 113, 114, 115, 116, 117, 118, or 119, and the light chain variable region comprises the amino acid sequence of SEQ ID NO: 101, 102, 103, 104, 105, 106, or 107; or
the heavy chain variable region comprises the amino acid sequence of SEQ ID NO: 60, and the light chain variable region comprises the amino acid sequence of SEQ ID NO: 59;
more preferably,
1) the heavy chain variable region comprises the amino acid sequence of SEQ ID NO: 255, 157, 156, 158, 161, 162, 349, 356, or 274, and the light chain variable region comprises the amino acid sequence of SEQ ID NO: 236; or
2) the heavy chain variable region comprises the amino acid sequence of SEQ ID NO: 255, 157, 156, 158, 161, 162, 349, 356, or 274, and the light chain variable region comprises the amino acid sequence of SEQ ID NO: 237; or
3) the heavy chain variable region comprises the amino acid sequence of SEQ ID NO: 255, 157, 156, 158, 161, 162, 349, 356, or 274, and the light chain variable region comprises the amino acid sequence of SEQ ID NO: 238; or
4) the heavy chain variable region comprises the amino acid sequence of SEQ ID NO: 278 or 357, and the light chain variable region comprises the amino acid sequence of SEQ ID NO: 268; or
5) the heavy chain variable region comprises the amino acid sequence of SEQ ID NO: 275 or 358, and the light chain variable region comprises the amino acid sequence of SEQ ID NO: 269; or
6) the heavy chain variable region comprises the amino acid sequence of SEQ ID NO: 278 or 357, and the light chain variable region comprises the amino acid sequence of SEQ ID NO: 269; or
7) the heavy chain variable region comprises the amino acid sequence of SEQ ID NO: 279 or 359, and the light chain variable region comprises the amino acid sequence of SEQ ID NO: 269; or
8) the heavy chain variable region comprises the amino acid sequence of SEQ ID NO: 275 or 358, and the light chain variable region comprises the amino acid sequence of SEQ ID NO: 268; or
9) the heavy chain variable region comprises the amino acid sequence of SEQ ID NO: 279 or 359, and the light chain variable region comprises the amino acid sequence of SEQ ID NO: 268; or
10) the heavy chain variable region comprises the amino acid sequence of SEQ ID NO: 157, 255, 156, 158, 161, 162, 349, 356, or 274, and the light chain variable region comprises the amino acid sequence of SEQ ID NO: 271; or
11) the heavy chain variable region comprises the amino acid sequence of SEQ ID NO: 157, 255, 156, 158, 161, 162, 349, 356, or 274, and the light chain variable region comprises the amino acid sequence of SEQ ID NO: 272; or
12) the heavy chain variable region comprises the amino acid sequence of SEQ ID NO: 281 or 360, and the light chain variable region comprises the amino acid sequence of SEQ ID NO: 236; or
13) the heavy chain variable region comprises the amino acid sequence of SEQ ID NO: 282 or 361, and the light chain variable region comprises the amino acid sequence of SEQ ID NO: 236; or
14) the heavy chain variable region comprises the amino acid sequence of SEQ ID NO: 283, and the light chain variable region comprises the amino acid sequence of SEQ ID NO: 236; or
15) the heavy chain variable region comprises the amino acid sequence of SEQ ID NO: 282 or 361, and the light chain variable region comprises the amino acid sequence of SEQ ID NO: 267; or
16) the heavy chain variable region comprises the amino acid sequence of SEQ ID NO: 282 or 361, and the light chain variable region comprises the amino acid sequence of SEQ ID NO: 268; or
17) the heavy chain variable region comprises the amino acid sequence of SEQ ID NO: 282 or 361, and the light chain variable region comprises the amino acid sequence of SEQ ID NO: 269;
most preferably,
1) the heavy chain variable region comprises the amino acid sequence of SEQ ID NO: 255, and the light chain variable region comprises the amino acid sequence of SEQ ID NO: 236; or
the heavy chain variable region comprises the amino acid sequence of SEQ ID NO: 157, and the light chain variable region comprises the amino acid sequence of SEQ ID NO: 236; or
2) the heavy chain variable region comprises the amino acid sequence of SEQ ID NO: 255, and the light chain variable region comprises the amino acid sequence of SEQ ID NO: 237; or
3) the heavy chain variable region comprises the amino acid sequence of SEQ ID NO: 255, and the light chain variable region comprises the amino acid sequence of SEQ ID NO: 238; or
4) the heavy chain variable region comprises the amino acid sequence of SEQ ID NO: 278, and the light chain variable region comprises the amino acid sequence of SEQ ID NO: 268; or
5) the heavy chain variable region comprises the amino acid sequence of SEQ ID NO: 275, and the light chain variable region comprises the amino acid sequence of SEQ ID NO: 269; or
6) the heavy chain variable region comprises the amino acid sequence of SEQ ID NO: 278, and the light chain variable region comprises the amino acid sequence of SEQ ID NO: 269; or
7) the heavy chain variable region comprises the amino acid sequence of SEQ ID NO: 279, and the light chain variable region comprises the amino acid sequence of SEQ ID NO: 269; or
8) the heavy chain variable region comprises the amino acid sequence of SEQ ID NO: 275, and the light chain variable region comprises the amino acid sequence of SEQ ID NO: 268; or
9) the heavy chain variable region comprises the amino acid sequence of SEQ ID NO: 279, and the light chain variable region comprises the amino acid sequence of SEQ ID NO: 268; or
10) the heavy chain variable region comprises the amino acid sequence of SEQ ID NO: 157, and the light chain variable region comprises the amino acid sequence of SEQ ID NO: 271; or
11) the heavy chain variable region comprises the amino acid sequence of SEQ ID NO: 157, and the light chain variable region comprises the amino acid sequence of SEQ ID NO: 272; or
12) the heavy chain variable region comprises the amino acid sequence of SEQ ID NO: 281, and the light chain variable region comprises the amino acid sequence of SEQ ID NO: 236; or
13) the heavy chain variable region comprises the amino acid sequence of SEQ ID NO: 282, and the light chain variable region comprises the amino acid sequence of SEQ ID NO: 236; or
14) the heavy chain variable region comprises the amino acid sequence of SEQ ID NO: 283, and the light chain variable region comprises the amino acid sequence of SEQ ID NO: 236; or
15) the heavy chain variable region comprises the amino acid sequence of SEQ ID NO: 282, and the light chain variable region comprises the amino acid sequence of SEQ ID NO: 267; or
16) the heavy chain variable region comprises the amino acid sequence of SEQ ID NO: 282, and the light chain variable region comprises the amino acid sequence of SEQ ID NO: 268; or
17) the heavy chain variable region comprises the amino acid sequence of SEQ ID NO: 282, and the light chain variable region comprises the amino acid sequence of SEQ ID NO: 269.

4. The anti-CD3 antibody according to any one of claims 1 to 3, wherein the anti-CD3 antibody is an antigen-binding fragment; preferably, the antigen-binding fragment is a Fab, Fab', F(ab')2, Fd, Fv, scFv, dsFv, or dAb.

5. The anti-CD3 antibody according to any one of claims 1 to 3, wherein the anti-CD3 antibody comprises a heavy chain constant region and a light chain constant region; preferably, the heavy chain constant region is a heavy chain constant region of human IgG1, IgG2, IgG3, or IgG4, or a variant thereof, and the light chain constant region is a light chain constant region of human kappa or lambda or a variant thereof;
further preferably, the heavy chain constant region is a human IgG1 heavy chain constant region or a variant thereof, and the light chain constant region is a human kappa light chain constant region or a variant thereof;
more preferably, the heavy chain constant region comprises the amino acid sequence of SEQ ID NO: 120, 90, 339, or 399, and the light chain constant region comprises the amino acid sequence of SEQ ID NO: 91 or 384;
most preferably, the heavy chain constant region comprises the amino acid sequence of SEQ ID NO: 120, and the light chain constant region comprises the amino acid sequence of SEQ ID NO: 91.

6. The anti-CD3 antibody according to claim 5, wherein the anti-CD3 antibody comprises a heavy chain and a light chain, wherein:
a. the heavy chain comprises an amino acid sequence having at least 80% sequence identity to SEQ ID NO: 301, 326, 323, 327, 181, 331, 332, 333, 174, 175, 176, 177, 178, 179, 180, 295, 296, 297, 298, 299, 300, 302, 309, 311, 312, 314, 315, 322, 324, 325, 328, 335, 336, 350, 351, 352, 353, 354, 355, 363, 364, 365, 366, 367, 368, or 369, and the light chain comprises an amino acid sequence having at least 80% sequence identity to SEQ ID NO: 303, 304, 305, 319, 320, 329, 330, 318, 163, 164, 165, 166, 167, 168, 169, 170, 171, 172, 173, 198, 284, 285, 286, 287, 288, 289, 290, 291, 292, 293, 294, 306, 307, 308, 310, 313, 316, 317, 321, 334, or 342; or
the heavy chain comprises an amino acid sequence having at least 80% sequence identity to SEQ ID NO: 95, and the light chain comprises an amino acid sequence having at least 80% sequence identity to SEQ ID NO: 94; or
b. the heavy chain comprises an amino acid sequence having at least 80% sequence identity to SEQ ID NO: 128, 129, 130, 131, 132, 133, 134, or 135, and the light chain comprises an amino acid sequence having at least 80% sequence identity to SEQ ID NO: 121, 122, 123, 124, 125, 126, or 127; or
the heavy chain comprises an amino acid sequence having at least 80% sequence identity to SEQ ID NO: 93, and the light chain comprises an amino acid sequence having at least 80% sequence identity to SEQ ID NO: 92;
preferably,
a. the heavy chain comprises the amino acid sequence of SEQ ID NO: 301, 326, 323, 327, 181, 331, 332, 333, 174, 175, 176, 177, 178, 179, 180, 295, 296, 297, 298, 299, 300, 302, 309, 311, 312, 314, 315, 322, 324, 325, 328, 335, 336, 350, 351, 352, 353, 354, 355, 363, 364, 365, 366, 367, 368, or 369, and the light chain comprises the amino acid sequence of SEQ ID NO: 303, 304, 305, 319, 320, 329, 330, 318, 163, 164, 165, 166, 167, 168, 169, 170, 171, 172, 173, 198, 284, 285, 286, 287, 288, 289, 290, 291, 292, 293, 294, 306, 307, 308, 310, 313, 316, 317, 321, 334, or 342; or
the heavy chain comprises the amino acid sequence of SEQ ID NO: 95, and the light chain comprises the amino acid sequence of SEQ ID NO: 94; or
b. the heavy chain comprises the amino acid sequence of SEQ ID NO: 128, 129, 130, 131, 132, 133, 134, or 135, and the light chain comprises the amino acid sequence of SEQ ID NO: 121, 122, 123, 124, 125, 126, or 127; or
the heavy chain comprises the amino acid sequence of SEQ ID NO: 93, and the light chain comprises the amino acid sequence of SEQ ID NO: 92;
more preferably,
1) the heavy chain comprises the amino acid sequence of SEQ ID NO: 301, 181, 180, 350, 353, 354, 355, 363, or 322, and the light chain comprises the amino acid sequence of SEQ ID NO: 303; or
2) the heavy chain comprises the amino acid sequence of SEQ ID NO: 301, 181, 180, 350, 353, 354, 355, 363, or 322, and the light chain comprises the amino acid sequence of SEQ ID NO: 304; or
3) the heavy chain comprises the amino acid sequence of SEQ ID NO: 301, 181, 180, 350, 353, 354, 355, 363, or 322, and the light chain comprises the amino acid sequence of SEQ ID NO: 305; or
4) the heavy chain comprises the amino acid sequence of SEQ ID NO: 326 or 365, and the light chain comprises the amino acid sequence of SEQ ID NO: 319; or
5) the heavy chain comprises the amino acid sequence of SEQ ID NO: 323 or 364, and the light chain comprises the amino acid sequence of SEQ ID NO: 320; or
6) the heavy chain comprises the amino acid sequence of SEQ ID NO: 326 or 365, and the light chain comprises the amino acid sequence of SEQ ID NO: 320; or
7) the heavy chain comprises the amino acid sequence of SEQ ID NO: 327 or 366, and the light chain comprises the amino acid sequence of SEQ ID NO: 320; or
8) the heavy chain comprises the amino acid sequence of SEQ ID NO: 323 or 364, and the light chain comprises the amino acid sequence of SEQ ID NO: 319; or
9) the heavy chain comprises the amino acid sequence of SEQ ID NO: 327 or 366, and the light chain comprises the amino acid sequence of SEQ ID NO: 319; or
10) the heavy chain comprises the amino acid sequence of SEQ ID NO: 181, 301, 180, 350, 353, 354, 355, 363, or 322, and the light chain comprises the amino acid sequence of SEQ ID NO: 329; or
11) the heavy chain comprises the amino acid sequence of SEQ ID NO: 181, 301, 180, 350, 353, 354, 355, 363, or 322, and the light chain comprises the amino acid sequence of SEQ ID NO: 330; or
12) the heavy chain comprises the amino acid sequence of SEQ ID NO: 331 or 367, and the light chain comprises the amino acid sequence of SEQ ID NO: 303; or
13) the heavy chain comprises the amino acid sequence of SEQ ID NO: 332 or 368, and the light chain comprises the amino acid sequence of SEQ ID NO: 303; or
14) the heavy chain comprises the amino acid sequence of SEQ ID NO: 333 or 369, and the light chain comprises the amino acid sequence of SEQ ID NO: 303; or
15) the heavy chain comprises the amino acid sequence of SEQ ID NO: 332 or 368, and the light chain comprises the amino acid sequence of SEQ ID NO: 318; or
16) the heavy chain comprises the amino acid sequence of SEQ ID NO: 332 or 368, and the light chain comprises the amino acid sequence of SEQ ID NO: 319; or
17) the heavy chain comprises the amino acid sequence of SEQ ID NO: 332 or 368, and the light chain comprises the amino acid sequence of SEQ ID NO: 320;
most preferably,
1) the heavy chain comprises the amino acid sequence of SEQ ID NO: 301, and the light chain comprises the amino acid sequence of SEQ ID NO: 303; or
the heavy chain comprises the amino acid sequence of SEQ ID NO: 181, and the light chain comprises the amino acid sequence of SEQ ID NO: 303; or
2) the heavy chain comprises the amino acid sequence of SEQ ID NO: 301, and the light chain comprises the amino acid sequence of SEQ ID NO: 304; or
3) the heavy chain comprises the amino acid sequence of SEQ ID NO: 301, and the light chain comprises the amino acid sequence of SEQ ID NO: 305; or
4) the heavy chain comprises the amino acid sequence of SEQ ID NO: 326, and the light chain comprises the amino acid sequence of SEQ ID NO: 319; or
5) the heavy chain comprises the amino acid sequence of SEQ ID NO: 323, and the light chain comprises the amino acid sequence of SEQ ID NO: 320; or
6) the heavy chain comprises the amino acid sequence of SEQ ID NO: 326, and the light chain comprises the amino acid sequence of SEQ ID NO: 320; or
7) the heavy chain comprises the amino acid sequence of SEQ ID NO: 327, and the light chain comprises the amino acid sequence of SEQ ID NO: 320; or
8) the heavy chain comprises the amino acid sequence of SEQ ID NO: 323, and the light chain comprises the amino acid sequence of SEQ ID NO: 319; or
9) the heavy chain comprises the amino acid sequence of SEQ ID NO: 327, and the light chain comprises the amino acid sequence of SEQ ID NO: 319; or
10) the heavy chain comprises the amino acid sequence of SEQ ID NO: 181, and the light chain comprises the amino acid sequence of SEQ ID NO: 329; or
11) the heavy chain comprises the amino acid sequence of SEQ ID NO: 181, and the light chain comprises the amino acid sequence of SEQ ID NO: 330; or
12) the heavy chain comprises the amino acid sequence of SEQ ID NO: 331, and the light chain comprises the amino acid sequence of SEQ ID NO: 303; or
13) the heavy chain comprises the amino acid sequence of SEQ ID NO: 332, and the light chain comprises the amino acid sequence of SEQ ID NO: 303; or
14) the heavy chain comprises the amino acid sequence of SEQ ID NO: 333, and the light chain comprises the amino acid sequence of SEQ ID NO: 303; or
15) the heavy chain comprises the amino acid sequence of SEQ ID NO: 332, and the light chain comprises the amino acid sequence of SEQ ID NO: 318; or
16) the heavy chain comprises the amino acid sequence of SEQ ID NO: 332, and the light chain comprises the amino acid sequence of SEQ ID NO: 319; or
17) the heavy chain comprises the amino acid sequence of SEQ ID NO: 332, and the light chain comprises the amino acid sequence of SEQ ID NO: 320.

7. A multispecific antibody, comprising the anti-CD3 antibody according to any one of claims 1 to 6, wherein preferably, the multispecific antibody specifically binds to human CD3 and further binds to a tumor-associated antigen or a cell surface antigen, receptor, or receptor ligand; more preferably, the multispecific antibody binds to human CD3 and further binds to one or more antigens selected from the group consisting of CD20, BCMA, and PSMA.

8. The multispecific antibody according to claim 7, wherein the multispecific antibody is a bispecific antibody.

9. A pharmaceutical composition, comprising the anti-CD3 antibody according to any one of claims 1 to 6, or the multispecific antibody according to claim 7 or 8, and one or more pharmaceutically acceptable carriers, diluents, or excipients.

10. An isolated nucleic acid molecule encoding the anti-CD3 antibody according to any one of claims 1 to 6 or the multispecific antibody according to claim 7 or 8.

11. A host cell, comprising the isolated nucleic acid molecule according to claim 10.

12. A method for producing the anti-CD3 antibody according to any one of claims 1 to 6 or the multispecific antibody according to claim 7 or 8, comprising a step of culturing the host cell according to claim 11 in a culture medium to form and accumulate the anti-CD3 antibody according to any one of claims 1 to 6 or the multispecific antibody according to claim 7 or 8, and recovering the antibody or the multispecific antibody from the culture.

13. A method for treating, preventing, or ameliorating a disease or disorder, comprising administering to a subject a therapeutically, prophylactically, or amelioratively effective amount of the anti-CD3 antibody according to any one of claims 1 to 6, or the multispecific antibody according to claim 7 or 8, or the pharmaceutical composition according to claim 9, or the nucleic acid molecule according to claim 10, or the host cell according to claim 11,
wherein preferably, the disease or disorder is selected from the group consisting of a tumor, an autoimmune disease, and an inflammatory disease.

14. Use of the anti-CD3 antibody according to any one of claims 1 to 6, the multispecific antibody according to claim 7 or 8, the pharmaceutical composition according to claim 9, the nucleic acid molecule according to claim 10, or the host cell according to claim 11 in the manufacture of a medicament for treating, preventing, or ameliorating a disease or disorder,
wherein preferably, the disease or disorder is selected from the group consisting of a tumor, an autoimmune disease, and an inflammatory disease.

15. A method for activating T cells, comprising administering to a subject the anti-CD3 antibody according to any one of claims 1 to 6, or the multispecific antibody according to claim 7 or 8, or the pharmaceutical composition according to claim 9.
